# EUROPEAN PATENT APPLICATION

(11) **EP 2 644 588 A2**
(43) Date of publication of application: **02.10.2013**
(21) Application number: 13173408.9
(22) Date of filing: 24.05.2007
(51) Int. Cl.: C07C 59/62, C07C 235/08, C07C 59/105, C07D 311/72, C07F 9/09, C07F 9/10, G01N 33/483, G01N 33/487, G01N 33/50

(54) **Biomarkers for diagnosing multiple sclerosis, and methods thereof**

(30) Priority: 26.05.2006 US 803267 P
(62) Divisional of application: 07719854.7
(71) Applicant: Phenomenome Discoveries Inc., Saskatoon, Saskatchewan S7N 4L8 (CA)
(72) Inventor: Cook, Lisa, Lethbridge, Alberta T1J 0N1 (CA); Goodenowe, Dayan, Saskatoon, Saskatchewan S7N 4L8 (CA)
(74) Representative: McConchie, Connor Fraser

(57) **Abstract**

The present invention describes methods for the diagnosis and differential diagnosis of the different forms of multiple sclerosis. The methods measure the intensities of specific small molecules called metabolites in samples from patients with clinically diagnosed relapsing-remitting or primary-progressive forms of multiple sclerosis and compare these intensities to the intensities observed in a population of healthy individuals, thus identifying markers of multiple sclerosis. A method is also provided for the differential diagnosis of subjects afflicted with relapsing-remitting multiple sclerosis from secondary-progressive multiple sclerosis.

## Description

### FIELD OF INVENTION

The present invention relates to small molecules or metabolites that are found to have significantly different abundances or intensities between clinically diagnosed MULTIPLE SCLEROSIS or other neurological disorders, and normal patients. The present invention also relates to methods for diagnosing MULTIPLE SCLEROSIS and other neurological disorders, or individuals at risk of getting MULTIPLE SCLEROSIS or other neurological disorders.

### BACKGROUND OF THE INVENTION

MULTIPLE SCLEROSIS is the most common neurological disorder effecting people under the age of 30, and is second only to epilepsy as the most common disease of the central nervous system (CNS) [1]. It is generally accepted that MULTIPLE SCLEROSIS is an autoimmune disorder that results in focal and discrete areas of inflammation and demyelination throughout the white matter of the CNS.

The prevalence rate of MULTIPLE SCLEROSIS throughout North America ranges from 1 per 500 to 1 per 1000, affecting an estimated 50,000 Canadians and 400,000 Americans; there are approximately 2 million people affected world-wide. Epidemiological studies have revealed females are twice as likely to develop the disease, the age of onset is relatively early (peak age of 30), and there is a greater susceptibility in people of northern European descent [2]. Although differing theories have implicated the involvement of various environmental factors [3-6], immune dysfunction [3,4], and genetic anomalies [3,4] in the development of this disorder, the etiology is still unknown. It is reasonable to assume that any factor that results in an autoimmune reaction against myelin proteins results in MULTIPLE SCLEROSIS. The most accepted theory involving its etiology takes into account several factors and suggests genetically susceptible individuals are exposed to a foreign entity, such as a virus or a toxin, and through some type of molecular mimicry, an autoimmune reaction against myelin proteins is initiated. Approximately five to fifteen years later, the first clinical symptoms become apparent/evident [7].

The pathological hallmark of MULTIPLE SCLEROSIS is discrete and focal areas of myelin loss, known as plaques or lesions. These plaques can consist of varying amounts of demyelination, gliosis, inflammation, edema and axonal degradation [8]. Although the exact locations of the plaques vary among patients, a general anatomical pattern is evident. Plaques within the human brain are located periventricular, within the temporal lobe, corpus callosum, optic nerves, brain stem, and/or cerebellum and tend to surround one or more blood vessels [7,9]. More than half of MULTIPLE SCLEROSIS patients have plaques within the cervical portion of the spinal cord [10]. The physiological consequence of the plaques is the slowing or blocked transmission of nerve impulses which manifests itself as sensory and/or motor impairment. In 2000, Lucchinetti et al [11] described four distinct patterns of MULTIPLE SCLEROSIS plaques in terms of their histological features. Two of these patterns suggest that demyelination results from the destruction of the myelin-producing cells within the CNS, oligodendrocytes, whereas the other two patterns indicate that myelin destruction results from T-cell or T-cell plus antibody targeting of the myelin sheath. The two patterns where oligodendrocytes are destroyed differ from one another by the selective destruction of specific myelin proteins in one pattern. The demyelinated lesions that contain T cells differ due to immunoglobulin-containing deposition and activated complement characteristic of one pattern. The discovery of the four patterns of MULTIPLE SCLEROSIS plaques was important since it indicates that the process of demyelination within this disorder can be achieved in several ways, and, hence, supports the notion that any process which triggers the formation of these plaques results in the clinical manifestation of MULTIPLE SCLEROSIS.

However, the pathological examination of MULTIPLE SCLEROSIS plaques is problematic in that it is derived primarily from post-mortem tissue, which represents only a snapshot of the disease at a given time. The majority of this tissue is acquired from individuals who had MULTIPLE SCLEROSIS for several years, and therefore represent tissue from the chronic stage of the disease. While post-mortem tissue may provide some information about the pathology of the disease, but it cannot elucidate how the disease progresses or where the lesions began. Magnetic resonance imaging (MRI) is commonly used to visualize MULTIPLE SCLEROSIS lesions *in vivo.* The use of MRI to study MULTIPLE SCLEROSIS lesions is limited, however, because it cannot provide information about the pathological composition of the lesions.

The initial diagnosis of MULTIPLE SCLEROSIS is typically either relapsing-remitting (RR-MULTIPLE SCLEROSIS) or primary-progressive (PP-MULTIPLE SCLEROSIS). PP-MULTIPLE SCLEROSIS is the initial diagnosis in 10-15% of patients and is defined as a gradual worsening of symptoms throughout the course of the disease without any clinical remissions [4,12]. RR-MULTIPLE SCLEROSIS is the most common form as it is the initial diagnosis in 80% of patients, and is defined by clinical attacks (relapses) that last at least 24 hours followed by partial or complete recovery (remission). Within 20 years of initial diagnosis, 90% of RR-MULTIPLE SCLEROSIS patients will proceed to the secondary-progressive form of MULTIPLE SCLEROSIS (SP-MULTIPLE SCLEROSIS), where the symptoms worsen and remission periods eventually disappear. Some RR-MULTIPLE SCLEROSIS patients within a 15-year time period experience few relapses with no worsening of symptoms and long remission periods; these patients would have developed benign-MULTIPLE SCLEROSIS (BN-MULTIPLE SCLEROSIS). Currently, there is no evidence that indicates why a patient would initially manifest either PP-MULTIPLE SCLEROSIS or RR-MULTIPLE SCLEROSIS.

In 2001, the McDonald Criteria [13] was published to standardize the diagnosis of MULTIPLE SCLEROSIS. The fundamental feature of the criteria involves the objective evidence of lesions disseminated in both time and space. Clinical evidence alone can be adequate to secure a diagnosis if: 1) the individual has experienced two attacks/relapses and 2) there is clinical evidence of two or more lesions separated by time and space. If the individual does not reach this clinical criterion, additional paraclinical tests from MRI, cerebrospinal fluid (CSF) analysis and/or visual evoked potentials (VEP) are performed. MRI is the most sensitive and specific paraclinical test as it can provide objective evidence for dissemination of lesions in both time and space. CSF analysis can provide evidence of immune or inflammatory reactions of lesions and can aid in diagnosis when the clinical presentation and MRI criteria are not met, but it cannot provide information about dissemination of lesions or events in time or space. VEP in MULTIPLE SCLEROSIS are delayed, but exhibit a well-preserved waveform and can be used to provide evidence of a second lesion if the first lesion does not affect the visual pathway. The supplemental evidence provided by the paraclinical tests might result in a diagnosis of either: a) having MULTIPLE SCLEROSIS, b) not having MULTIPLE SCLEROSIS, or c) having possible MULTIPLE SCLEROSIS. The majority of individuals diagnosed with having MULTIPLE SCLEROSIS exhibit the RR-MULTIPLE SCLEROSIS form, so the dissemination of lesions in time and space is often evident. However, since there are no remission periods in PP-MULTIPLE SCLEROSIS, paraclinical tests are particularly important to secure a diagnosis. CSF analysis and either MRI or VEP must be obtained to provide objective evidence about space, whereas the use of MRI and continued progression of clinical symptoms for one year could provide evidence about dissemination over time.

Prior to the utilization of these paraclinical tests, it took an average of seven years before a physician could secure a diagnosis. Today, the use of these tests can secure a diagnosis of RR-MULTIPLE SCLEROSIS within months. The McDonald Criteria decreased the time required for diagnosis substantially, but for those individuals who are diagnosed with possible MULTIPLE SCLEROSIS, or will eventually receive a diagnosis of PP-MULTIPLE SCLEROSIS, it has fallen short.

While the paraclinical tests may aid in the diagnosis of multiple sclerosis and provide information regarding the dissemination of lesions, no specific information regarding the pathological composition of the lesions is obtained. In addition, the interpretation of paraclinical test results is subjective and requires the expertise of trained personnel. Furthermore, tools such as the pathological examination of multiple sclerosis plaques and the paraclinical test do not provide any information on susceptibility to the disease, but rather are used once symptoms become apparent.

### SUMMARY OF THE INVENTION

The present invention relates to small molecules or metabolites that are found to have significantly different abundances or intensities between persons with MULTIPLE SCLEROSIS or other neurological disorders, and normal patients. The present invention also relates to small molecules or metabolites that have significantly different abundances or intensities between persons with neuropathology associated with MULTIPLE SCLEROSIS and persons absent of such pathology such that these small molecules or metabolites may be indicative of a pre-clinical pathological state. The present invention also relates to methods for diagnosing MULTIPLE SCLEROSIS and other neurological disorders.

The present invention provides novel methods for discovering, validating, and implementing a diagnostic method for one or more diseases or particular health-states. In particular, the present invention provides a method for the diagnosis and differential diagnosis of MULTIPLE SCLEROSIS in humans by measuring the levels of specific small molecules present in a sample and comparing them to "normal" reference levels.

The type of neurological disorder diagnosed by the above method may be MULTIPLE SCLEROSIS, or other type of demyelinating disorder. The sample obtained from the human may be a blood sample.

A method is provided for the diagnosis of subjects afflicted with MULTIPLE SCLEROSIS (relapsing-remitting or primary-progressive) and/or for the differential diagnosis of subjects transitioning from relapsing-remitting to secondary progressive MULTIPLE SCLEROSIS.

The methods of the present invention, including high throughput screening (HTS) assays, can be used for the following, wherein the specific "health-state" in this application may refer to, but is not limited to, MULTIPLE SCLEROSIS:

1. identifying small-molecule metabolite biomarkers that can discriminate between multiple health-states using any biological sample taken from an individual;

2. specifically diagnosing a health-state using metabolites identified in serum, plasma, whole blood, CSF, and/or other tissue biopsy as described in this application;

3. selecting the minimal number of metabolite features required for optimal diagnostic assay performance statistics using supervised statistical methods such as those mentioned in this application;

4. identifying structural characteristics of biomarker metabolites selected from non-targeted metabolomic analysis using LC-MS/MS, MSⁿ and NMR;

5. developing a high-throughput LC-MS/MS method for assaying selected metabolite levels in serum, plasma, whole blood, CSF, saliva, urine, hair, and/or other tissue biopsy; and

6. diagnosing a given health-state, or risk for development of a health-state by determining the levels of any combination of metabolite features disclosed from the Fourier Transform Mass Spectrometry (FTMS) analysis patient serum or other biological fluid or tissue, using any method including, but not limited to, mass spectrometry, NMR, UV detection, ELISA (enzyme-linked immunosorbant assay), chemical reaction, image analysis, or other.

The present invention provides for the longitudinal monitoring or screening of the general population for one or more health-states using any single or combination of features disclosed in the method, described above.

The present invention also provides several hundred metabolite masses that have statistically significant differential abundances between clinically diagnosed RR-MULTIPLE SCLEROSIS, clinically diagnosed PP-MULTIPLE SCLEROSIS, clinically diagnosed SP-MULTIPLE SCLEROSIS, and normal samples, also referred to herein as a reference sample. Of the metabolite masses identified, an optimal panel of between four and 45 metabolite masses can be used, or any number there between; for example, an optimal panel of 4, 5, 6, 7, 8, 9, 10, 11,12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, or 45 metabolite masses can be used to differentiate between clinically diagnosed RR-MULTIPLE SCLEROSIS, clinically diagnosed PP-MULTIPLE SCLEROSIS, clinically diagnosed SP-MULTIPLE SCLEROSIS and normal states. In a specific, non-limiting example, an optimal panel of 36 metabolite masses can be used.

The present invention also provides a panel of about 257 metabolite masses that can be used as a diagnostic indicator of RR-MULTIPLE SCLEROSIS disease course in serum samples compared to normal samples (see Table 1); in a further example, the panel may contain about 240 metabolite masses. In a more specific example, an optimal panel of nine metabolite masses can be extracted and used as a diagnostic indicator of RR-MULTIPLE SCLEROSIS disease course in serum samples compared to normal samples; for example, the panel of nine metabolites can include those with masses (measured in Daltons) 452.3868, 496.4157, 524.4448, 540.4387, 578.4923, 580.5089, 594.4848, 596.5012, 597.5062 where a +/- 5 ppm difference would indicate the same metabolite.

Also, the invention provides a panel of about 100 metabolite masses that can be used as a diagnostic indicator of PP-MULTIPLE SCLEROSIS disease course in serum samples compared to normal samples (see Table 2); in a further example, the panel may contain about 60 metabolite masses. In a more specific example, an optimal panel of five metabolite masses can be extracted and used as a diagnostic indicator of PP-MULTIPLE SCLEROSIS disease course in serum samples compared to normal samples; for example, the optimal panel of five metabolites can include those with masses (measured in Daltons) 202.0453, 216.04, 243.0719, 244.0559, 857.7516, where a +/- 5 ppm difference would indicate the same metabolite.

In addition, the invention provides a panel of about 226 metabolite masses that can be used as a diagnostic indicator of SP-MULTIPLE SCLEROSIS disease course in serum samples compared to normal samples (see Table 3); in a further example, the panel may contain about 129 metabolite masses. In a more specific example, an optimal panel of eighteen metabolite masses can be extracted and used as a diagnostic indicator of SP-MULTIPLE SCLEROSIS disease course in serum samples compared to normal samples; for example, the optimal panel of eighteen metabolites can include those with masses (measured in Daltons) 194.0803, 428.3653, 493.385, 541.3415, 565.3391, 576.4757, 578.4923, 590.4964, 594.4848, 495.4883, 596.5012, 596.5053, 597.5062, 597.5068, 805.5609, 806.5643, 827.5446, 886.5582, where a+/- 5 ppm difference would indicate the same metabolite.

Furthermore, the invention provides a panel of about 142 metabolite masses that can be used as a diagnostic indicator of RR-MULTIPLE SCLEROSIS disease course in serum samples compared to SP - MULTIPLE SCLEROSIS samples (see Table 4); in a further example, the panel may contain about 135 metabolite masses. In a more specific example, an optimal panel of six metabolite masses that can be extracted and used as an indicator of RR-MULTIPLE SCLEROSIS disease course in serum samples compared to SP- MULTIPLE SCLEROSIS samples, also referred to herein as a reference sample; for example, the optimal panel of six metabolites can include those with masses (measured in Daltons) 540.4387, 576.4757, 594.4848, 595.4883, 596.5012, 597.5062, where a +/- 5 ppm difference would indicate the same metabolite.

The present invention further provides a panel of about 148 metabolite masses that can be used as a diagnostic indicator of the transition from RR-MULTIPLE SCLEROSIS patients to SP-MULTIPLE SCLEROSIS compared to RR-MULTIPLE SCLEROSIS, also referred to herein as a reference sample (see Table 5); in a more specific example, an optimal panel of 5 metabolites masses that can be extracted and used as an indicator of early neuropathology changes within the transition from RR-MULTIPLE SCLEROSIS patients to SP-MULTIPLE SCLEROSIS compared to RR-MULTIPLE SCLEROSIS; for example, the optimal panel of five metabolites can include those with masses (measured in Daltons) 576.4757, 578.4923, 594.4848, 596.5012, 597.5062, where a +/- 5 ppm difference would indicate the same metabolite.

Moreover, the invention provides a panel of about 309 metabolite masses that can be used as a diagnostic indicator of the transition from RR-MULTIPLE SCLEROSIS to SP-MULTIPLE SCLEROSIS compared to SP- MULTIPLE SCLEROSIS (see Table 6), also referred to herein as a reference sample; in a further example, the panel may contain about 42 metabolite masses. In a more specific example, an optimal panel of eight metabolite masses that can be extracted and used as an indicator of early neuropathology changes within the transition from RR-MULTIPLE SCLEROSIS to SP-MULTIPLE SCLEROSIS compared to SP- MULTIPLE SCLEROSIS; for example, the optimal panel of eight metabolites can include those with masses (measured in Daltons) 617.0921, 746.5118, 760.5231, 770.5108, 772.5265, 784.5238, 786.5408, and 787.5452, where a +/- 5 ppm difference would indicate the same metabolite.

The present invention further provides a method for diagnosing RR-MULTIPLE SCLEROSIS, PP-MULTIPLE SCLEROSIS, and SP-MULTIPLE SCLEROSIS, comprising the steps of: introducing one or more samples from one or more patients with clinically diagnosed RR-MULTIPLE SCLEROSIS, clinically diagnosed PP-MULTIPLE SCLEROSIS or clinically diagnosed SP-MULTIPLE SCLEROSIS, introducing said sample containing a plurality of metabolites into a high resolution mass spectrometer, for example, a Fourier Transform Ion Cyclotron Resonance Mass Spectrometer (FTICR-MS); obtaining, identifying and quantifying data for the metabolites; creating a database of said identifying and quantifying data; comparing, identifying and quantifying data from the sample with corresponding data from a sample from normal subject (one who does not have MULTIPLE SCLEROSIS); identifying one or more metabolites that differ; and selecting the minimal number of metabolite markers needed for optimal diagnosis.

In a further embodiment of the present invention there is provided a method for identifying specific biomarkers for RR-MULTIPLE SCLEROSIS, PP-MULTIPLE SCLEROSIS, and SP-MULTIPLE SCLEROSIS, comprising the steps of: introducing one or more samples from one or more patients with clinically diagnosed RR-MULTIPLE SCLEROSIS, clinically diagnosed PP-MULTIPLE SCLEROSIS, or clinically diagnosed SP-MULTIPLE SCLEROSIS, said sample containing a plurality of metabolites into an FTICT-MS; obtaining, identifying, and quantifying data for the metabolites; creating a database of said identifying and quantifying data; comparing the identifying and quantifying data from the sample with corresponding data from a sample from a normal subject (one who does not have MULTIPLE SCLEROSIS) identifying one or more metabolites that differ; and selecting the minimal number of metabolite markers needed for optimal diagnosis. The metabolite markers needed for optimal diagnosis of RR-MULTIPLE SCLEROSIS in a serum sample may be selected from the group consisting of metabolites with accurate masses (measured in Daltons) 452.3868, 496.4157, 524.4448, 540.4387, 578.4923, 580.5089, 594.4848, 596.5012, 597.5062, where a +/-5 ppm difference would indicate the same metabolite. The metabolite markers needed for optimal diagnosis of PP-MULTIPLE SCLEROSIS in a serum sample may be selected from the group consisting of metabolites with accurate masses (measured in Daltons) 202.0453, 216.04, 243.0719, 244.0559, 857.7516, where a +/- 5 ppm difference would indicate the same metabolite. The metabolite markers needed for optimal diagnosis of SP-MULTIPLE SCLEROSIS in a serum sample may be selected from the group consisting of metabolites with accurate masses (measured in Daltons) 194.0803, 428.3653, 493.385, 541.3415, 565.3391, 576.4757, 578.4923, 590.4964, 594.4848, 495.4883, 596.5012, 596.5053, 597.5062, 597.5068, 805.5609, 806.5643, 827.5446, 886.5582, where a +/- 5 ppm difference would indicate the same metabolite. The metabolite markers needed for optimal differentiation of RR-MULTIPLE SCLEROSIS patients from SP-MULTIPLE SCLEROSIS in a serum sample may be selected from the group consisting of metabolites with accurate masses (measured in Daltons) 540.4387, 576.4757, 594.4848, 595.4883, 596.5012, 597.5062, where a +/- 5 ppm difference would indicate the same metabolite. The metabolite markers needed for optimal differentiation of RR-MULTIPLE SCLEROSIS transitioning to SP-MULTIPLE SCLEROSIS (RR-SP) as compared to SP-MULTIPLE SCLEROSIS in a serum sample may be selected from the group consisting of metabolites with accurate masses (measured in Daltons) 617.0921, 746.5118, 760.5231, 770.5108, 772.5265, 784.5238, 786.5408, and 787.5452, where a +/- 5 ppm difference would indicate the same metabolite. The metabolite markers needed for optimal differentiation of RR-MULTIPLE SCLEROSIS transitioning to SP-MULTIPLE SCLEROSIS (RR-SP) as compared to RR-MULTIPLE SCLEROSIS in a serum sample may be selected from the group consisting of metabolites with accurate masses (measured in Daltons) 576.4757, 578.4923, 594.4848, 596.5012, 597.5062, where a +/- 5 ppm difference would indicate the same metabolite.

In a further embodiment of the present invention there is provided a method for diagnosing a patient for RR-MULTIPLE SCLEROSIS, PP-MULTIPLE SCLEROSIS, and SP-MULTIPLE SCLEROSIS, comprising the steps of: screening a sample from said patient for quantification of one or more metabolic markers and comparing the amounts of metabolite markers to corresponding data from a sample from a normal subject (one who does not have MULTIPLE SCLEROSIS). The metabolite markers for diagnosis of RR-MULTIPLE SCLEROSIS in a serum sample may be selected from the group consisting of metabolites with accurate masses (measured in Daltons) 452.3868, 496.4157, 524.4448, 540.4387, 578.4923, 580.5089, 594.4848, 596.5012, 597.5062, where a +/- 5 ppm difference would indicate the same metabolite. The metabolite markers for diagnosis of PP-MULTIPLE SCLEROSIS in a serum sample may be selected from the group consisting of metabolites with accurate masses (measured in Daltons) 202.0453, 216.04, 243.0719, 244.0559, 857.7516, where a +/- 5 ppm difference would indicate the same metabolite. The metabolite markers for diagnosis of SP-MULTIPLE SCLEROSIS from healthy controls in a serum sample may be selected from the group consisting of metabolites with accurate masses (measured in Daltons) 194.0803, 428.3653, 493.385, 541.3415, 565.3391, 576.4757, 578.4923, 590.4964, 594.4848, 495.4883, 596.5012, 596.5053, 597.5062, 597.5068, 805.5609, 806.5643, 827.5446, 886.5582,, where a +/- 5 ppm difference would indicate the same metabolite. The metabolite markers for diagnosis of RR-MULTIPLE SCLEROSIS from SP- MULTIPLE SCLEROSIS in a serum sample may be selected from the group consisting of metabolites with accurate masses (measured in Daltons) 540.4387, 576.4757, 594.4848, 595.4883, 596.5012, 597.5062, where a +/- 5 ppm difference would indicate the same metabolite. The metabolite markers needed for optimal differentiation of RR-MULTIPLE SCLEROSIS transitioning to SP-MULTIPLE SCLEROSIS (RR-SP) as compared to SP-MULTIPLE SCLEROSIS in a serum sample may be selected from the group consisting of metabolites with accurate masses (measured in Daltons) 617.0921, 746.5118, 760.5231, 770.5108, 772.5265, 784.5238, 786.5408, and 787.5452, where a +/- 5 ppm difference would indicate the same metabolite. The metabolite markers needed for optimal differentiation of RR-MULTIPLE SCLEROSIS transitioning to SP-MULTIPLE SCLEROSIS (RR-SP) as compared to RR-MULTIPLE SCLEROSIS in a serum sample may be selected from the group consisting of metabolites with accurate masses (measured in Daltons) 576.4757, 578.4923, 594.4848, 596.5012, 597.5062, where a +/- 5 ppm difference would indicate the same metabolite.

The molecular formulae and proposed structure for some of the MULTIPLE SCLEROSIS biomarkers referred to above were determined in one embodiment of the present invention. These are summarized below. According to results the biomarkers are thoughts to be derivatives of sugars, phospholipids and tocopherols.

RR-MULTIPLE SCLEROSIS as compared to a Normal patient

| Mass | Formula | Structure |
|---|---|---|
| 496.4157 | C₃₀H₅₆O₅ | 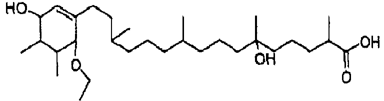 |
| 524.4448 | C₃₂H₆₀O₅ | 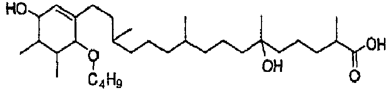 |
| 540.4387 | C₃₂H₆₀O₆ | 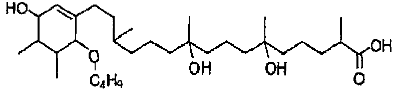 |
| 580.5089 | C₃₆H₆₈O₅ | 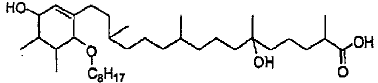 |
| 594.4848 | C₃₆H₆₆O₆ | 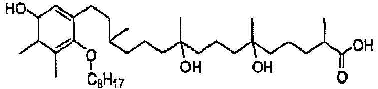 |
| 596.5012 | C₃₆H₆₈O₆ | 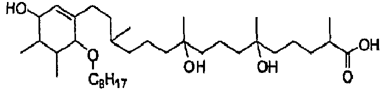 |
| 578.4923 | C₃₆H₆₆O₅ | 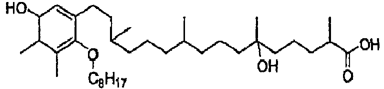 |

PP-MULTIPLE SCLEROSIS as compared to a Normal patient

| Mass | Formulae | Structure |
|---|---|---|
| 216.04 | C₅H₁₃O₇P | 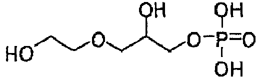 |
| 202.0453 | C₆H₁₁O₆Na | 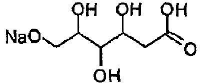 |
| 244.0559 | C₈H₁₃O₇Na | 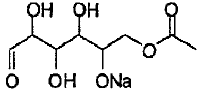 |
| 857.7516 | C₅₄H₉₉NO₆ | 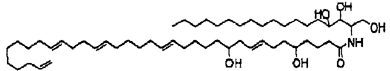 |

SP-MULTIPLE SCLEROSIS as compared to a Normal patient

| Mass | Formulae | Structure |
|---|---|---|
| 541.3415 | C₂₅H₅₂NO₉P | 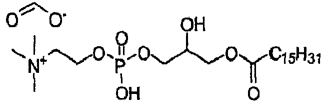 |
| 565.3391 | C₂₇H₅₂NO₉P | 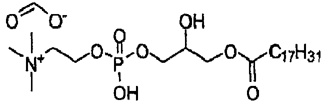 |
| 428.3653 | C₂₉H₄₈O₂ | 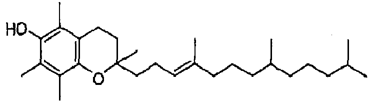 |
| 805.5609 | C₄₈H₈₀NO₈P | 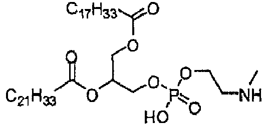 |
| 194.0803 | C₇H₁₄O₆ | 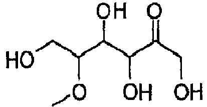 |
| 578.4923 | C₃₆H₆₆O₅ | 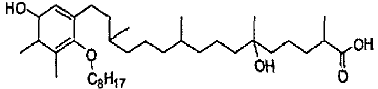 |

RR-MULTIPLE SCLEROSIS as compared to a SP -MULTIPLE SCLEROSIS patient

| Mass | Formulae | Structure |
|---|---|---|
| 540.4387 | C₃₂H₆₀O₆ | 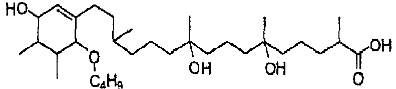 |
| 576.4757 | C₃₆H₆₄O₅ | 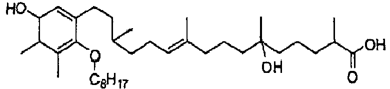 |

RR-MULTIPLE SCLEROSIS transitioning to SP -MULTIPLE SCLEROSIS as compared to a SP -MULTIPLE SCLEROSIS patient

| Mass | Formulae | Structure |
|---|---|---|
| 786.5408 | C₄₃H₇₉O₁₀P | 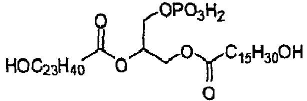 |

RR-MULTIPLE SCLEROSIS transitioning to SP -MULTIPLE SCLEROSIS as compared to a RR -MULTIPLE SCLEROSIS patient

| Mass | Formulae | Structure |
|---|---|---|
| 576.4757 | C₃₆H₆₄O₅ | 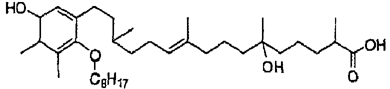 |
| 578.4923 | C₃₆H₆₆O₅ | 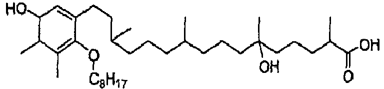 |

The identification of MULTIPLE SCLEROSIS-specific biomarkers in human serum is extremely useful since it is minimally invasive, and can be used to detect the presence of MULTIPLE SCLEROSIS pathology prior to the manifestation of clinical symptoms. A serum test is minimally invasive and would be accepted by the general population. The metabolite masses presently identified were found to have statistically significantly differential abundances between RR-MULTIPLE SCLEROSIS, PP-MULTIPLE SCLEROSIS, SP-MULTIPLE SCLEROSIS and normal serum, of which an optimal panels can be extracted and used as a diagnostic indicator of disease presence. A diagnostic assay based on small molecules or metabolites in serum can be developed into a relatively simple and cost-effective assay that is capable of detecting specific metabolites. Translation of the method into a clinical assay, compatible with current clinical chemistry laboratory hardware, is commercially acceptable and effective, and could result in a rapid deployment worldwide. Furthermore, the requirement for highly trained personnel to perform and interpret the test would be eliminated.

Since the present invention relates to panels of molecules that are increased in individuals with RR-MULTIPLE SCLEROSIS and PP-MULTIPLE SCLEROSIS as compared to healthy individuals, there the test can be used as an indicator of susceptibility to the specific type of MULTIPLE SCLEROSIS or, alternatively, an indicator of very early disease onset. The possibility of a highly accurate MULTIPLE SCLEROSIS predisposition assay in serum would be the first of its kind.

The impact of the present invention on the diagnosis of MULTIPLE SCLEROSIS would be tremendous, as literally everyone could be screened longitudinally throughout their lifetime to assess risk. Given that the performance characteristics of the test of the present invention are representative for the general population, this test alone may be superior to any other currently available screening method, as it may have the potential to detect disease progression prior to the emergence of clinical symptoms.

This summary of the invention does not necessarily describe all features of the invention.

### BRIEF DESCRIPTION OF THE DRAWINGS

These and other features of the invention will become more apparent from the following description in which reference is made to the appended drawings, wherein:

FIGURE 1A shows a Prediction Analysis of Microarray (PAM) training error plot and FIGURE 1B shows a cross validated misclassification error plot, in accordance with an embodiment of the present invention.

FIGURE 2 shows cross-validated diagnostic probabilities for clinically diagnosed RR-MULTIPLE SCLEROSIS patients and controls, in accordance with an embodiment of the present invention.

FIGURE 3 shows a receiver-operator characteristic (ROC) curve based on cross-validated probabilities, in accordance with a further embodiment of the present invention.

FIGURE 4 shows diagnostic predictions for blinded test set, in accordance with a further embodiment of the present invention.

FIGURE 5 shows a ROC curve based on predicted test set of clinically diagnosed RR-MULTIPLE SCLEROSIS patients and controls, in accordance with a further embodiment of the present invention.

FIGURE 6 shows a ROC curve based on clinically diagnosed PP-MULTIPLE SCLEROSIS and controls, in accordance with a further embodiment of the present invention.

FIGURE 7 shows a ROC curve based on clinically diagnosed SP-MULTIPLE SCLEROSIS and controls, in accordance with a further embodiment of the present invention.

FIGURE 8 shows a ROC curve based on clinically diagnosed RR-MULTIPLE SCLEROSIS and SP-MULTIPLE SCLEROSIS in accordance with a further embodiment of the present invention.

FIGURE 9 shows a ROC curve based on clinically diagnosed RR-MULTIPLE SCLEROSIS patients and RR-MULTIPLE SCLEROSIS transitioning to SP-MULTIPLE SCLEROSIS, in accordance with a further embodiment of the present invention.

FIGURE 10 shows a ROC curve based on clinically diagnosed SP-MULTIPLE SCLEROSIS and RR-MULTIPLE SCLEROSIS patients transitioning to SP-MULTIPLE SCLEROSIS, in accordance with a further embodiment of the present invention.

FIGURE 11 shows a mean signal-to-noise +/-SEM of the RR-MULTIPLE SCLEROSIS 9 serum biomarker panel relative to controls, in accordance with a further embodiment of the present invention.

FIGURE 12 shows a mean signal-to-noise +/-SEM of the PP-MULTIPLE SCLEROSIS 5 serum biomarker panel relative to controls, in accordance with a further embodiment of the present invention.

FIGURE 13 shows a mean signal-to-noise +/-SEM of the SP-MULTIPLE SCLEROSIS 18 serum biomarker panel relative to controls, in accordance with a further embodiment of the present invention.

FIGURE 14 shows a mean signal-to-noise +/-SEM of the RR-MULTIPLE SCLEROSIS 6 serum biomarker panel relative to SP-MULTIPLE SCLEROSIS, in accordance with a further embodiment of the present invention.

FIGURE 15 shows a mean signal-to-noise +/-SEM of the RR-MULTIPLE SCLEROSIS 5 serum biomarker panel relative to RR-MULTIPLE SCLEROSIS patients transitioning to SP-MULTIPLE SCLEROSIS, in accordance with a further embodiment of the present invention.

FIGURE 16 shows a mean signal-to-noise +/-SEM of the RR-MULTIPLE SCLEROSIS patients transitioning to SP-MULTIPLE SCLEROSIS 8 serum biomarker panel relative to SP-MULTIPLE SCLEROSIS, in accordance with a further embodiment of the present invention.

### DETAILED DESCRIPTION

The present invention relates to small molecules or metabolites that are found to have significantly different abundances or intensities between clinically diagnosed MULTIPLE SCLEROSIS or other neurological disorders, and normal patients. The present invention also relates to methods for diagnosing MULTIPLE SCLEROSIS and other neurological disorders.

The present invention provides novel methods for discovering, validating, and implementing a diagnosis method for one or more diseases or particular health-states. In particular, the present invention provides a method for the diagnosis and differential diagnosis of MULTIPLE SCLEROSIS in humans by measuring the levels of specific small molecules present in a sample and comparing them to "normal" reference levels. A reference sample can be a normal sample or a sample from a patient with other forms of MULTIPLE SCLEROSIS. The sample may be any biological sample, including, but not exclusive to blood, urine, saliva, hair, cerebrospinal fluid (CSF), biopsy or autopsy samples. The methods measure the intensities of specific small molecules, also referred to as metabolites, in the sample from patients with MULTIPLE SCLEROSIS and compare these intensities to the intensities observed in a population of healthy (non-MULTIPLE SCLEROSIS) individuals.

The small molecules measured in a sample may also be referred to herein as "markers", "biomarkers", or "metabolites". The metabolites may be characterized in any manner known in the art, for example but not limited to, by mass (also referred to as "metabolite mass" or "accurate mass"), molecular formula, polarity, acid/base properties, NMR spectra, MS/MS or MSⁿ spectra, molecular structure, or any combination thereof The term "metabolite feature" refers to a metabolite, a fragment thereof, an analogue thereof, or a chemical equivalent thereof.

The diagnosis or the exclusion of any type(s) of neurological disorders is contemplated by the present invention, using all or a subset of the metabolites disclosed herein. The types of neurological disorders include, but are not limited to: Alzheimer's disease (AD), dementia with Lewy bodies (DLB), frontotemporal lobe dementia (FTD), vascular induced dementia (e.g. multi-infarct dementia), anoxic event induced dementia (e.g. cardiac arrest), trauma to the brain induced dementia (e.g. dementia pugilistica [boxer's dementia]), dementia resulting from exposure to an infectious (e.g. Creutzfeldt-Jakob Disease) or toxic agent (e.g. alcohol-induced dementia), Acute Disseminated Encephalomyelitis, Guillain-Barré Syndrome, Adrenoleukodystrophy, Adrenomyeloneuropathy, Leber's Hereditary Optic Neuropathy, HTLV-associated Myelopathy, Krabbe's Disease, phenylketonuria, Canavan Disease, Pelizaeus-Merzbacher Disease, Alexander's Disease, Neuromyelitis Optica, Central Pontine Myelinolysis, Metachromatic Leukodystrophy, Schilder's Disease, Autism, Multiple Sclerosis, Parkinson's Disease, Bipolar Disorder, Ischemia, Huntington's Chorea, Major Depressive Disorder, Closed Head Injury, Hydrocephalus, Amnesia, Anxiety Disorder, Traumatic Brain Injury, Obsessive Compulsive Disorder, Schizophrenia, Mental Retardation, Epilepsy and/or any other condition that is associated with an immune response, demyelination, myelitis or encephalomyelitis.

The present invention provides a method of diagnosing MULTIPLE SCLEROSIS and its subtypes by measuring the levels of specific small molecules present in a sample obtained from a human and comparing them to "normal" reference levels.

In order to determine whether there are biochemical markers of a given health-state in particular population, a group of patients representative of the health state (i.e. a particular disease) and a group of "normal" counterparts are required. Biological samples taken from the patients in a particular health-state category are then compared to the same samples taken from the normal population as well as to patients in similar health-state categories to identify biochemical differences between the two groups, by analyzing the biochemicals present in the samples using FTMS and/or LC-MS. The biological samples could originate from anywhere within the body, including, but not limited to, blood (serum/plasma), cerebrospinal fluid (CSF), urine, stool, saliva, or biopsy of any solid tissue including tumor, adjacent normal, smooth and skeletal muscle, adipose tissue, liver, skin, hair, brain, kidney, pancreas, lung, colon, stomach, or other. Of particular interest are samples that are serum. While the term "serum" is used herein, those skilled in the art will recognize that plasma, whole blood, or a subfraction of whole blood may be used.

The method of the present invention, based on small molecules or metabolites in a sample, makes an ideal screening test as the development of assays capable of detecting specific metabolites is relatively simple and cost effective. The test is minimally invasive and is indicative of MULTIPLE SCLEROSIS pathology, and may be useful to differentiate MULTIPLE SCLEROSIS subtypes from each other. Translation of the method into a clinical assay compatible with current clinical chemistry laboratory hardware is commercially acceptable and effective. Furthermore, the method of the present invention does not require highly trained personnel to perform and/or interpret the test.

The present invention also provides several hundred metabolite masses that were found to have statistically significantly differential abundances between clinically diagnosed RR-MULTIPLE SCLEROSIS, clinically diagnosed PP-MULTIPLE SCLEROSIS, clinically diagnosed SP-MULTIPLE SCLEROSIS and normal serum.

*Non-Targeted Metabolomic Strategies.* Multiple non-targeted metabolomics strategies have been described in the scientific literature including NMR [14], GC-MS [15-17], LC-MS, and FTMS strategies [14,18-20]. The metabolic profiling strategy employed for the discovery of differentially expressed metabolites in this application was the non-targeted FTMS strategy developed by Phenomenome Discoveries [17,20-23; see also US Published Application No. 2004-0029120 A1, Canadian Application No. 2,298,181, and WO 01/57518]. Non-targeted analysis involves the measurement of as many molecules in a sample as possible, without any prior knowledge or selection of components prior to the analysis. Therefore, the potential for non-targeted analysis to discover novel metabolite biomarkers is high versus targeted methods, which detect a predefined list of molecules. The present invention uses a non-targeted method to identify metabolite components in serum samples that differ between:

1. Clinically diagnosed RR-MULTIPLE SCLEROSIS patients and healthy controls;

2. Clinically diagnosed PP-MULTIPLE SCLEROSIS patients and healthy controls;

3. Clinically diagnosed SP-MULTIPLE SCLEROSIS patients and healthy controls;

4. Clinically diagnosed RR-MULTIPLE SCLEROSIS patients and clinically diagnosed SP-MULTIPLE SCLEROSIS patients;

5. Clinically diagnosed RR-MULTIPLE SCLEROSIS transitioning to SP-MULTIPLE SCLEROSIS patients and clinically diagnosed SP-MULTIPLE SCLEROSIS patients; and

5. Clinically diagnosed RR-MULTIPLE SCLEROSIS transitioning to SP-MULTIPLE SCLEROSIS patients and clinically diagnosed RR-MULTIPLE SCLEROSIS patients.

*Sample Processing.* When a blood sample is drawn from a patient there are several ways in which the sample can be processed. The range of processing can be as little as none (i.e. frozen whole blood) or as complex as the isolation of a particular cell type. The most common and routine procedures involve the preparation of either serum or plasma from whole blood. All blood sample processing methods, including spotting of blood samples onto solid-phase supports, such as filter paper or other immobile materials, are also contemplated by the present invention.

*Sample Extraction.* The processed blood sample described above is then further processed to make it compatible with the methodical analysis technique to be employed in the detection and measurement of the biochemicals contained within the processed serum sample. The types of processing can range from as little as no further processing to as complex as differential extraction and chemical derivatization. Extraction methods may include sonication, soxhlet extraction, microwave assisted extraction (MAE), supercritical fluid extraction (SFE), accelerated solvent extraction (ASE), pressurized liquid extraction (PLE), pressurized hot water extraction (PHWE), and/or surfactant assisted extraction (PHWE) in common solvents such as methanol, ethanol, mixtures of alcohols and water, or organic solvents such as ethyl acetate or hexane. The preferred method of extracting metabolites for FTMS non-targeted analysis is to perform a liquid/liquid extraction whereby non-polar metabolites dissolve in an organic solvent and polar metabolites dissolve in an aqueous solvent.

*Mass spectrometry analysis of extracts.* Extracts of biological samples are amenable to analysis on essentially any mass spectrometry platform, either by direct injection or following chromatographic separation. Typical mass spectrometers are comprised of a source, which ionizes molecules within the sample, and a detector for detecting the ionized molecules or fragments of molecules. Examples of common sources include electron impact, electrospray ionization (ESI), atmospheric pressure chemical ionization, atmospheric pressure photo ionization (APPI), matrix assisted laser desorption ionization (MALDI), surface enhanced laser desorption ionization (SELDI), and derivations thereof. Common mass separation and detection systems can include quadrupole, quadrupole ion trap, linear ion trap, time-of-flight (TOF), magnetic sector, ion cyclotron (FTMS), Orbitrap, and derivations and combinations thereof. The advantage of FTMS over other MS-based platforms is its high resolving capability that allows for the separation of metabolites differing by only hundredths of a Dalton, many of which would be missed by lower resolution instruments.

*Training classifier.* Cross-validated training classifier was created using the Prediction Analysis of Microarrays (PAM) (http://www-stat.stanford.edu/∼tibs/PAM/) algorithm [24]. The method involves training a classifier algorithm using samples with known diagnosis that can then be applied to blinded diagnosed samples (i.e. a test set). Several supervised methods exist, of which any could have been used to identify the best feature set, including artificial neural networks (ANNs), support vector machines (SVMs), partial least squares discriminative analysis (PLSDA), sublinear association methods, Bayesian inference methods, supervised principle component analysis, shrunken centroids, or others (see [25] for review).

With reference to Examples 1 to 4, and based on the similarity of molecular formula, MS/MS fragmentation patterns, and NMR data, the metabolites identified in serum, or subsets thereof, comprising the diagnostic feature set maybe chemically related. In addition, there are many other related compounds present in the FTMS dataset that also show increased abundance in the MULTIPLE SCLEROSIS population, and which share similar molecular formulas to the subset identified. Therefore, the results suggest that an entire family of metabolites sharing common structural properties is abnormal in MULTIPLE SCLEROSIS patients. Without wishing to be bound by theory, the biochemical pathway responsible for regulating the levels of these metabolites may be perturbed in MULTIPLE SCLEROSIS patients, and consequently may be a putative interventional target for treatment. Possible types of intervention include the development of agonists or antagonists for proteins involved in the implicated pathways and/or the development of nutritional supplements that would decrease the concentration of the implicated metabolites or the development of pro-drugs or pro-nutrients to decrease the concentration of these metabolites.

The present invention also provides the structural characteristics of the metabolites used for the differential diagnosis of RR-MULTIPLE SCLEROSIS, PP-MULTIPLE SCLEROSIS, and SP-MULTIPLE SCLEROSIS, which may include accurate mass and molecular formula determination, polarity, acid/base properties, NMR spectra, and MS/MS or MSⁿ spectra. Techniques used to determine these characteristics include, but are not limited to, reverse phase LC-MS using a C18 column followed by analysis by MS, MS/MS fragmentation using collision induced dissociation (CID), nuclear magnetic resonance (NMR), and extraction. The characteristics of the metabolites obtained by various methods are then used to determine the structure of the metabolites.

The present invention also provides high throughput methods for differential diagnosis of MULTIPLE SCLEROSIS and normal states. The method involves fragmentation of the parent molecule; in a non-limiting example, this may be accomplished by a Q-Trap^{™} system. Detection of the metabolites may be performed using one of various assay platforms, including colorimetric chemical assays (UV, or other wavelength), antibody-based enzyme-linked immunosorbant assays (ELISAs), chip- and PCR-based assays for nucleic acid detection, bead-based nucleic-acid detection methods, dipstick chemical assays or other chemical reaction, image analysis such as magnetic resonance imaging (MRI), positron emission tomography (PET) scan, computerized tomography (CT) scan, nuclear magnetic resonance (NMR), and various mass spectrometry-based systems.

The metabolites and the methods of the present invention may also be combined with the current diagnostic tools for MULTIPLE SCLEROSIS, which include clinical history, neuroimaging analysis, evoked potentials, and cerebrospinal fluid analysis of proteinaceous and inflammatory components within the cerebrospinal fluid. Imaging techniques include, but are not limited to, structural magnetic resonance imaging (MRI), contrast-enhanced MRI, positron emission tomography (PET), computerized tomography (CT), functional magnetic resonance imaging (fMRI), electroencephalography (EEG), single positron emission tomography (SPECT), event related potentials, magnetoencephalography, and/or multi-modal imaging. The clinical assessment may include, but is not limited to, the Kurtzke's extended disability status scale (EDSS), multiple sclerosis impact scale (MSIS), Scripps neurologic rating scale (NRS), ambulation index (AI), MS-related symptoms scale, 15-item activities of daily living self-care scale for MS Persons, Incapacity status scale, functional independent measure, and/or internuclear ophthalmoplegia. A person skilled in the art would recognize that the combination of metabolites and methods as described herein with current techniques has the potential to diagnosis or differentiate any form of multiple sclerosis and/or its pathology.

The present invention will be further illustrated in the following examples.

### Example 1: Identification of Differentially Expressed Metabolites

Differentially expressed metabolites are identified in individuals with clinically diagnosed RR-MULTIPLE SCLEROSIS, clinically diagnosed PP-MULTIPLE SCLEROSIS, clinically diagnosed SP-MULTIPLE SCLEROSIS, as well as healthy controls.

***Clinical Samples.*** For the MULTIPLE SCLEROSIS serum diagnostic assay described, samples were obtained from representative populations of healthy individuals and those with clinically diagnosed RR-MULTIPLE SCLEROSIS, clinically diagnosed PP-MULTIPLE SCLEROSIS, and clinically diagnosed SP-MULTIPLE SCLEROSIS patients. The biochemical markers of RR-MULTIPLE SCLEROSIS described in the invention were derived from the analysis of 93 serum samples from patients clinically diagnosed with RR-MULTIPLE SCLEROSIS, serum samples from 18 patients with clinically diagnosed PP-MULTIPLE SCLEROSIS, serum samples from 22 patients with clinically diagnosed SP-MULTIPLE SCLEROSIS, and 51 serum samples from controls. The 93 patients with RR-MULTIPLE SCLEROSIS were further divided into one of two groups: those still exhibiting a relapsing-remitting disease course (mean disease duration 5.9 years, n = 46) and those transitioning into the chronic secondary-progressive disease course (mean disease duration 11.4 years, n = 47). Samples in the four groups were from a diverse population of individuals, ranging in age, demographic, weight, occupation, and displaying varying non-MULTIPLE SCLEROSIS-related health-states. All samples were single time-point collections

The metabolites contained within the 184 serum samples used in this example were separated into polar and non-polar extracts through sonication and vigorous mixing (vortex mixing).

Analysis of serum extracts collected from 184 individuals (93 clinically diagnosed RR-MULTIPLE SCLEROSIS, 18 clinically diagnosed PP-MULTIPLE SCLEROSIS, 22 clinically diagnosed SP-MULTIPLE SCLEROSIS, and 51 healthy controls) was performed by direct injection into a FTMS and ionization by either ESI or atmospheric pressure chemical ionization (APCI) in both positive and negative modes. Sample extracts were diluted either three or six-fold in methanol:0.1%(v/v) ammonium hydroxide (50:50, v/v) for negative ionization modes, or in methanol:0.1% (v/v) formic acid (50:50, v/v) for positive ionization modes. For APCI, sample extracts were directly injected without diluting. All analyses were performed on a Bruker Daltonics APEX III Fourier transform ion cyclotron resonance mass spectrometer equipped with a 7.0 T actively shielded superconducting magnet (Bruker Daltonics, Billerica, MA). Samples were directly injected using electrospray ionization (ESI) and APCI at a flow rate of 1200 µL per hour. Ion transfer/detection parameters were optimized using a standard mix of serine, tetra-alanine, reserpine, Hewlett-Packard tuning mix and the adrenocorticotrophic hormone fragment 4-10. In addition, the instrument conditions were tuned to optimize ion intensity and broadband accumulation over the mass range of 100-1000 amu according to the instrument manufacturer's recommendations. A mixture of the abovementioned standards was used to internally calibrate each sample spectrum for mass accuracy over the acquisition range of 100-1000 amu.

In total six separate analyses comprising combinations of extracts and ionization modes were obtained for each sample:
Aqueous Extract
   1. Positive ESI (analysis mode 1101)
   2. Negative ESI (analysis mode 1102)
Organic Extract
   3. Positive ESI (analysis mode 1201)
   4. Negative ESI (analysis mode 1202)
   5. Positive APCI (analysis mode 1203)
   6. Negative APCI (analysis mode 1204)

***Mass Spectrometry Data Processing.*** Using a linear least-squares regression line, mass axis values were calibrated such that each internal standard mass peak had a mass error of <1 ppm compared with its theoretical mass. Using XMASS software from Bruker Daltonics Inc., data file sizes of 1 megaword were acquired and zero-filled to 2 megawords. A sinm data transformation was performed prior to Fourier transform and magnitude calculations. The mass spectra from each analysis were integrated, creating a peak list that contained the accurate mass and absolute intensity of each peak. Compounds in the range of 100-2000 m/z were analyzed. In order to compare and summarize data across different ionization modes and polarities, all detected mass peaks were converted to their corresponding neutral masses assuming hydrogen adduct formation. A self-generated two-dimensional (mass vs. sample intensity) array was then created using DISCOVAmetrics™ software (Phenomenome Discoveries Inc., Saskatoon, SK, Canada). The data from multiple files were integrated, and this combined file was then processed to determine all of the unique masses. The average of each unique mass was determined, representing the y axis. This value represents the average of all of the detected accurate masses that were statistically determined to be equivalent. Considering that the mass accuracy of the instrument for the calibration standards is approximately 1 ppm, a person skilled in the art will recognize that these average masses may include individual masses that fall within +/- 5 ppm of this average mass. A column was created for each file that was originally selected to be analyzed, representing the x axis. The intensity for each mass found in each of the files selected was then filled into its representative x,y coordinate. Coordinates that did not contain an intensity value were left blank. Once in the array, the data were further processed, visualized and interpreted, and putative chemical identities were assigned. Each of the spectra were then peak picked to obtain the mass and intensity of all metabolites detected. These data from all of the modes were then merged to create one data file per sample. The data from all 184 samples were then merged and aligned to create a two-dimensional metabolite array in which each sample is represented by a column and each unique metabolite is represented by a single row. In the cell corresponding to a given metabolite sample combination, the intensity of the metabolite in that sample is displayed. When the data is represented in this format, metabolites showing differences between groups of samples can be determined.

***Advanced Data Interpretation - Serum Biomarkers.*** A student's T-test was used to select for metabolites which differed significantly between the following different clinical groups in serum:
1. clinically diagnosed RR-MULTIPLE SCLEROSIS patients (n=46) and controls (n=51), [240 metabolites, see Table 1];
2. clinically diagnosed PP-MULTIPLE SCLEROSIS patients (n=18) and controls (n=51), [60 metabolites, see Table 2];
3. clinically diagnosed SP-MULTIPLE SCLEROSIS patients (n=22) and controls (n=51), [129 metabolites, see Table 3];
4. clinically diagnosed RR-MULTIPLE SCLEROSIS patients (n=46) and clinically diagnosed SP-MULTIPLE SCLEROSIS (n=22), [135 metabolites, see Table 4];
5. clinically diagnosed RR-MULTIPLE SCLEROSIS patients (n=46) and RR-MULTIPLE SCLEROSIS patients transitioning to SP-MULTIPLE SCLEROSIS [RR-SP] (n=47), [148 metabolites, see Table 5];
6. RR-MULTIPLE SCLEROSIS patients transitioning to SP-MULTIPLE SCLEROSIS [RR-SP] (n=47) and SP-MULTIPLE SCLEROSIS patients (n=22), [42 metabolites, see Table 6].

Metabolites that were less than p<0.05 were considered significant.

Tables 1-6 show metabolite features whose concentrations or amounts in serum are significantly different (p<0.05) between the tested populations and therefore have potential diagnostic utility for identifying each of the aforesaid populations. The features are described by their accurate mass and analysis mode, which together are sufficient to provide the putative molecular formulas and chemical characteristics (such as polarity and putative functional groups) of each metabolite.

For each clinical pairing, a cross-validated training classifier was created using the PAM algorithm, previously described. The classifier algorithm was trained using samples with known diagnosis and then applied to blinded sample (i.e. a test set).

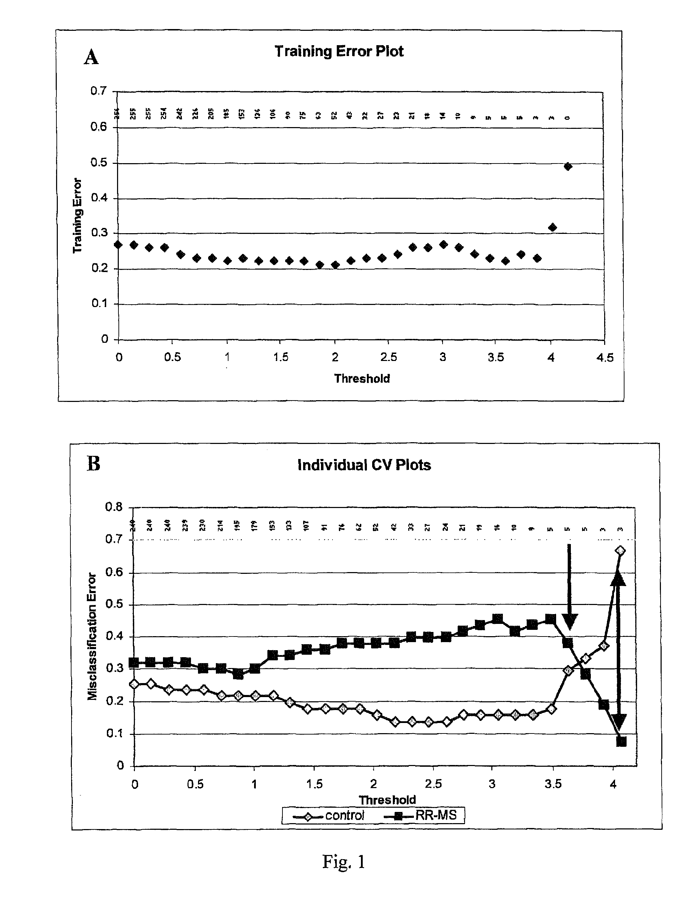

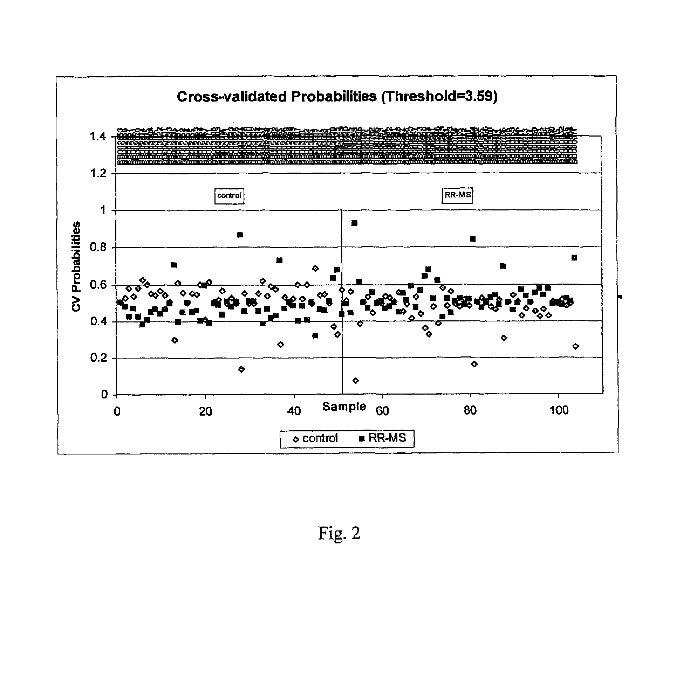

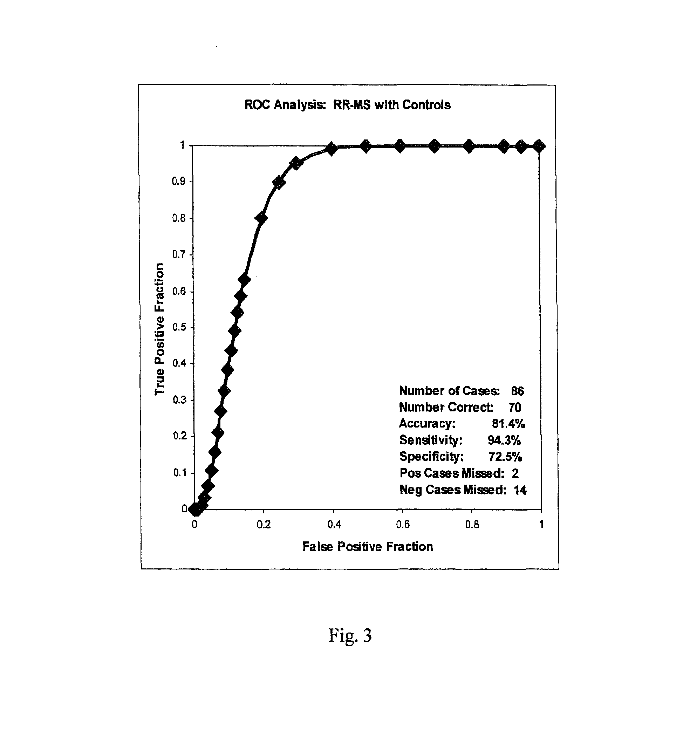

The lowest training classifier obtained with the fewest number of metabolites was selected for each clinical pairing. The graph in Figure 1A shows the number of metabolites required to achieve a given training error at various threshold values (a user-definable PAM parameter). The plot shows that a training classifier with less than 22% error rate (0.22 training error) is possible with five metabolite features (threshold value of approximately 3.59, see arrow). The graph in Figure 1B is conceptually similar to that in 1A, however, the graph in 1B shows the misclassification error of the trained classifier for clinically diagnosed RR-MULTIPLE SCLEROSIS patients and control patients following the cross-validation procedure integral to the PAM program. The line connected by the diamonds mirrors the previous result, showing that minimal cross-validated misclassification error for controls were achieved using as few as five metabolites. It also shows that clinically diagnosed RR-MULTIPLE SCLEROSIS patients, depicted by the squares, were 93% accurately diagnosed as having RR-MULTIPLE SCLEROSIS using only three metabolite feature, but at this threshold, the misclassification for the controls was 66% (see arrows). The individual cross-validated diagnostic probabilities for each of the RR-MULTIPLE SCLEROSIS patients and controls are shown in Figure 2. All of clinically diagnosed RR-MULTIPLE SCLEROSIS patients are listed on right side of the graph, and the controls are on the left. Each sample contains two points on the graph, one showing the probability of having RR-MULTIPLE SCLEROSIS (squares), and one showing the probability of not having RR-MULTIPLE SCLEROSIS (i.e. normal, diamonds). From the graph, six RR- MULTIPLE SCLEROSIS samples were classified as non- MULTIPLE SCLEROSIS and five control samples were classified as RR-MULTIPLE SCLEROSIS. The five metabolites are listed in Table 7. The predicted probabilities were then used to create the receiver-operating characteristic (ROC) curve in Figure 3 using JROCFIT (http://www.rad.jhmi.edu/jeng/javarad/roc/JROCFITi.html), which shows the true positive fraction (those with RR-MULTIPLE SCLEROSIS being predicted to have RR-MULTIPLE SCLEROSIS) versus the false positive fraction (control individuals predicted as having RR-MULTIPLE SCLEROSIS). The area under the curve is 81.4%, with a sensitivity of 94.3%, and a specificity of 72.5%. Overall, the diagnostic accuracy is 81.4% based on the cross-validated design.

The above first principle component analysis allowed the initial identification of the optimal metabolites for each clinical pairing. In order to confirm these findings, a second PAM analysis was performed. For each clinical pairing, the second analysis (discussed below) generally provided a larger number of metabolites than the first principle component analysis. From this expanded set of metabolites, the best candidates for differentiation between clinical health states, which generally correspond to the initially identified metabolites, were identified.

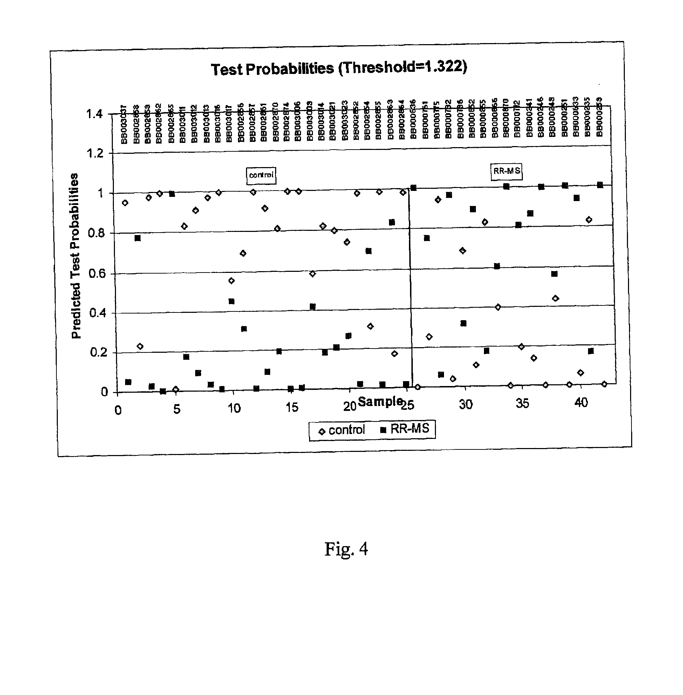

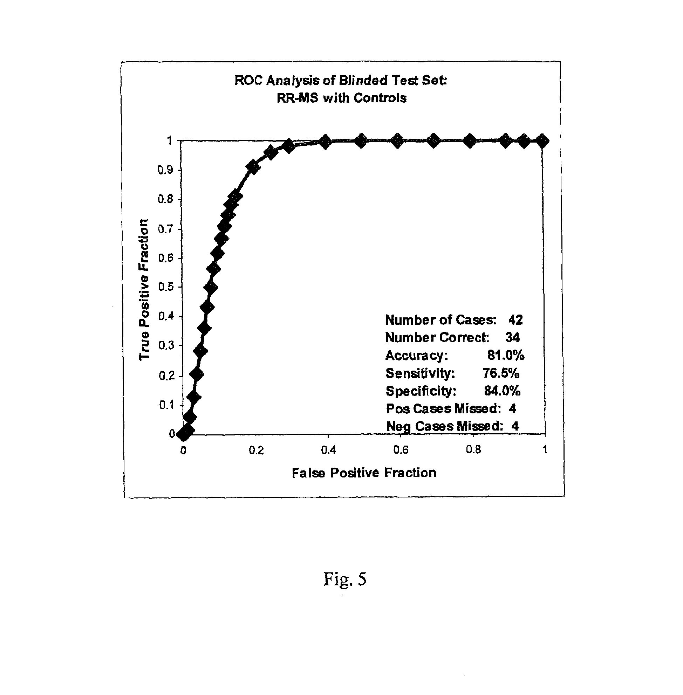

In the second PAM method, the samples for each clinical pairing were randomly split in half, using one half to generate a classifier and other half as a blinded "test set" for diagnosis. Since the first method creates the classifier using more samples, its predictive accuracy would be expected to be higher than the second approach, and consequently requires a fewer number of metabolites for high diagnostic accuracy. Following the previous example of all clinically diagnosed RR-MULTIPLE SCLEROSIS patients and controls, the training set was comprised of 30 clinically diagnosed RR-MULTIPLE SCLEROSIS patients and 26 controls. The predicted probabilities of the blinded test samples as either being RR-MULTIPLE SCLEROSIS-specific or controls are plotted in Figure 4. The results show four of the clinically-diagnosed RR-MULTIPLE SCLEROSIS samples were given a higher probability of being controls and four of the controls were given a higher probability of being RR-MULTIPLE SCLEROSIS. The optimal number of metabolites required for the lowest misclassification error using these samples was 16, listed in Table 8. The classifier was next used to predict the diagnosis of the remaining samples (blinded; 17 clinically diagnosed RR-MULTIPLE SCLEROSIS patients and 25 controls). Table 9 contains the patients that were used in the test set and their actual and predicted diagnosis. The probabilities from Figure 4 were then translated into a ROC curve (Figure 5). The performance characteristics based on classification of the blinded test set were sensitivity of 76.5%, specificity of 84.0%, and overall diagnostic accuracy of 81.0%.

The PAM analysis was repeated for each of the clinical pairings. The sample numbers used in each training set as well as the optimal number of metabolites required for the lowest misclassification error are listed in Table 10. The classifiers for the training sets were next used to predict the diagnosis of the remaining samples for each clinical pairing.

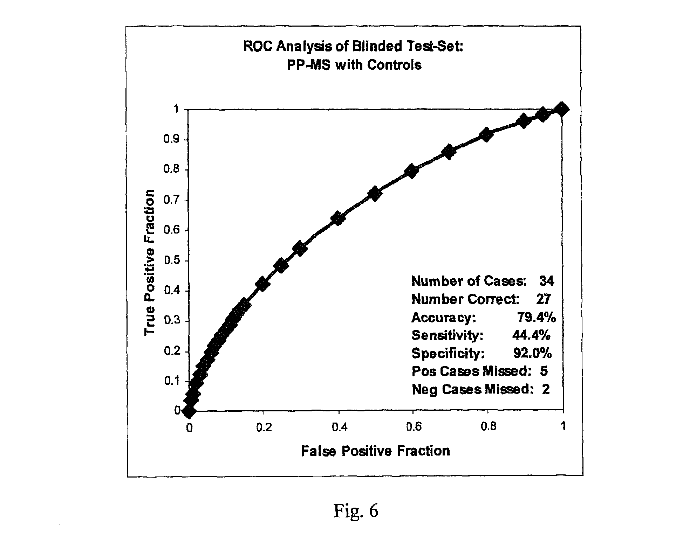

***i) Clinically diagnosed PP-MULTIPLE SCLEROSIS patients and controls.*** Table 11 contains the expanded set of metabolites and the actual and predicted diagnosis of the patients that were used in the test set. The probabilities from Table 11 were translated into a ROC curve (Figure 6). The performance characteristics based on the classification of the blinded test set were: sensitivity of 44.4%, specificity of 92%, and overall diagnostic accuracy of 79.4%.

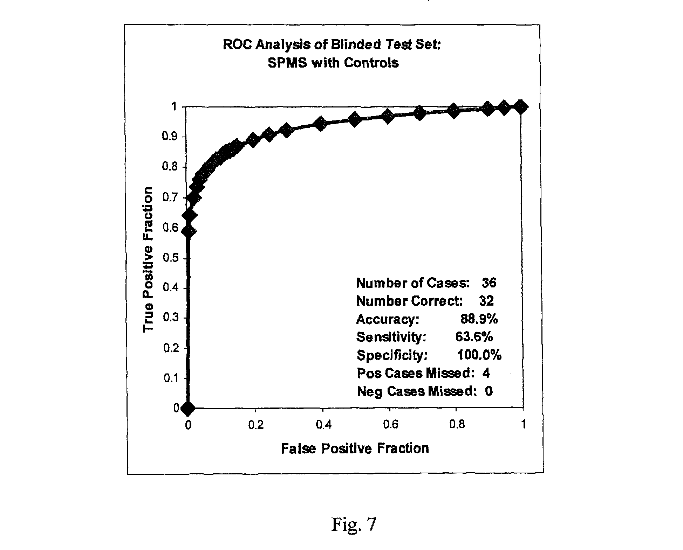

***ii) Clinically diagnosed SP-MULTIPLE SCLEROSIS patients and controls.*** Table 12 contains the expanded set of metabolites and the actual and predicted diagnosis of the patients that were used in the test set. The probabilities from Table 12 were translated into a ROC curve (Figure 7). The performance characteristics based on the classification of the blinded test set were: sensitivity of 63.6%, specificity of 100%, and overall diagnostic accuracy of 88.9%.

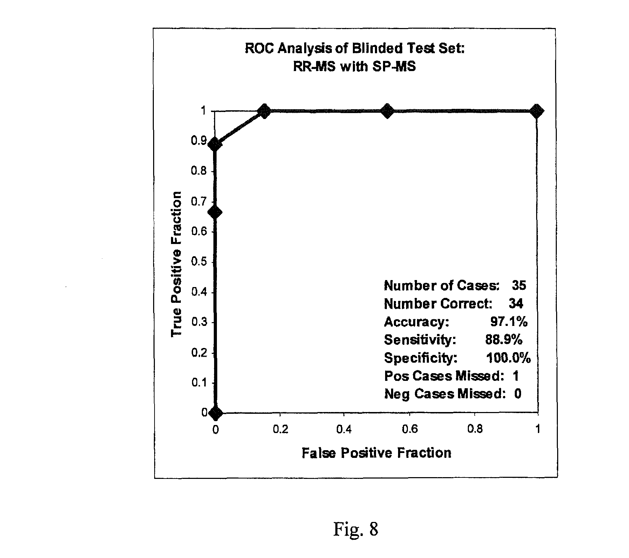

***iii) Clinically diagnosed RR-MULTIPLE SCLEROSIS patients and SP-MULTIPLE SCLEROSIS patients.*** Table 13 contains the expanded set of metabolites and the actual and predicted diagnosis of the patients that were used in the test set. The probabilities from Table 13 were translated into a ROC curve (Figure 8). The performance characteristics based on the classification of the blinded test set were: sensitivity of 88.9%, specificity of 100%, and overall diagnostic accuracy of 97.1 %.

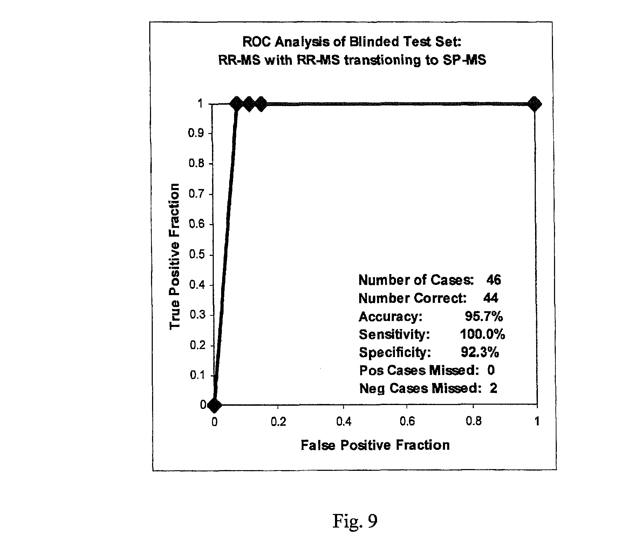

***iv) Clinically diagnosed RR-MULTIPLE SCLEROSIS patients transitioning to SP-MULTIPLE SCLEROSIS [RR-SP] and RR-MULTIPLE SCLEROSIS patients.*** Table 14 contains the expanded set of metabolites and the actual and predicted diagnosis of the patients that were used in the test set. The probabilities from Table 14 were translated into a ROC curve (Figure 9). The performance characteristics based on the classification of the blinded test set were: sensitivity of 100%, specificity of 92.3%, and overall diagnostic accuracy of 95.7%.

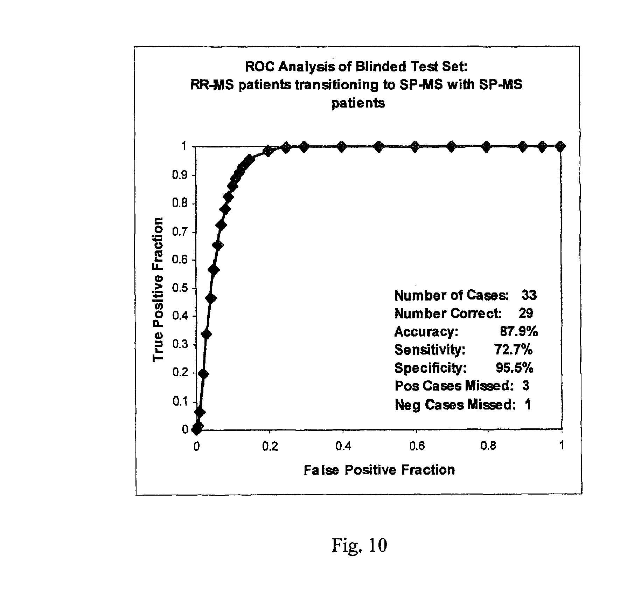

***v) Clinically diagnosed RR-MULTIPLE SCLEROSIS patients transitioning to SP-MULTIPLE SCLEROSIS [RR-SP] and SP-MULTIPLE SCLEROSIS patients.*** Table 15 contains the expanded set of metabolites and the actual and predicted diagnosis of the patients that were used in the test set. The probabilities from Table 15 were translated into a ROC curve (Figure 10). The performance characteristics based on the classification of the blinded test set were: sensitivity of 72.7%, specificity of 95.5%, and overall diagnostic accuracy of 87.9%.

Using an initial panel of about 240 metabolites, and an expanded set of about 16 metabolites, it was determined that a combination of nine metabolites fulfills the criteria for a serum diagnostic test of RR-MULTIPLE SCLEROSIS compared to normal samples. The best combination of nine metabolites includes the metabolites with masses (measured in Daltons) 452.3868, 496.4157, 524.4448, 540.4387, 578.4923, 580.5089, 594.4848, 596.5012, 597.5062. Although these are the actual masses, a person skilled in the art of this technology would recognize that +/- 5 ppm difference would indicate the same metabolite.

Using an initial panel of about 60 metabolites, and an expanded set of about 7 metabolites, it was determined that a combination of five metabolites fulfills the criteria for a serum diagnostic test of PP-MULTIPLE SCLEROSIS compared to normal samples. The best combination of five metabolites includes the metabolites with masses (measured in Daltons) 202.0453, 216.04, 243.0719, 244.0559, 857.7516, where a +/- 5 ppm difference would indicate the same metabolite.

Using an initial panel of about 129 metabolites, and an expanded set of about 16 metabolites, it was determined that a combination of eighteen metabolites fulfills the criteria for a serum diagnostic test of SP-MULTIPLE SCLEROSIS compared to normal samples. The best combination of eighteen metabolites includes the metabolites with masses (measured in Daltons) 194.0803, 428.3653, 493.385, 541.3415, 565.3391, 576.4757, 578.4923, 590.4964, 594.4848, 495.4883, 596.5012, 596.5053, 597.5062, 597.5068, 805.5609, 806.5643, 827.5446, 886.5582, where a +/-5 ppm difference would indicate the same metabolite.

Using an initial panel of about 135 metabolites, and an expanded set of about 16 metabolites, it was determined that a combination of six metabolites fulfills the criteria for a serum indicator of RR-MULTIPLE SCLEROSIS compared to SP-MULTIPLE SCLEROSIS. The best combination of six metabolites includes the metabolites with masses (measured in Daltons) 540.4387, 576.4757, 594.4848, 595.4883, 596.5012, 597.5062, where a +/- 5 ppm difference would indicate the same metabolite.

Using an initial panel of about 148 metabolites, and an expanded set of about 9 metabolites, it was determined that a combination of 5 metabolites fulfills the criteria for a serum indicator of RR-MULTIPLE SCLEROSIS patients transitioning to SP-MULTIPLE SCLEROSIS [RR-SP] compared to RR-MULTIPLE SCLEROSIS patients. The best combination of five metabolites includes the metabolites with masses (measured in Daltons) 576.4757, 578.4923, 594.4848, 596.5012, 597.5062, where a +/- 5 ppm difference would indicate the same metabolite.

Using an initial panel of about 42 metabolites, and an expanded set of about 17 metabolites, it was determined that a combination of 8 metabolites fulfills the criteria for a serum indicator of RR-MULTIPLE SCLEROSIS patients transitioning to SP-MULTIPLE SCLEROSIS [RR-SP] compared to SP-MULTIPLE SCLEROSIS patients. The best combination of eight metabolites includes the metabolites with masses (measured in Daltons) 617.0921, 746.5118, 760.5231, 770.5108, 772.5265, 784.5238, 786.5408, 787.5452, where a +/- 5 ppm difference would indicate the same metabolite.

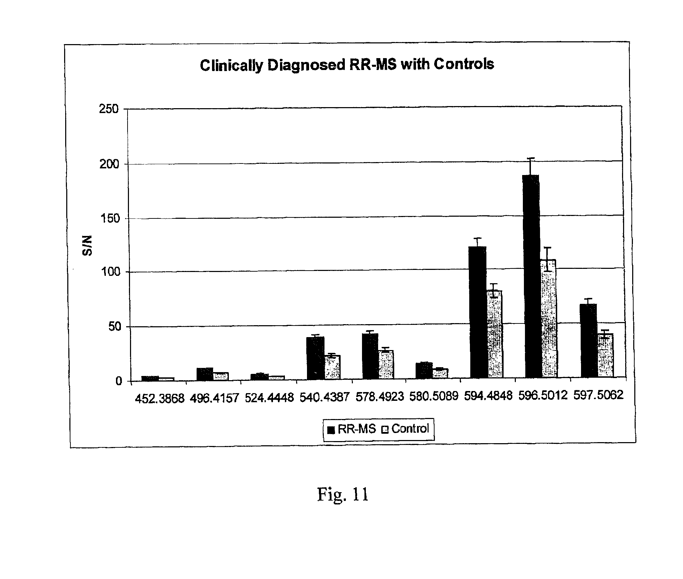

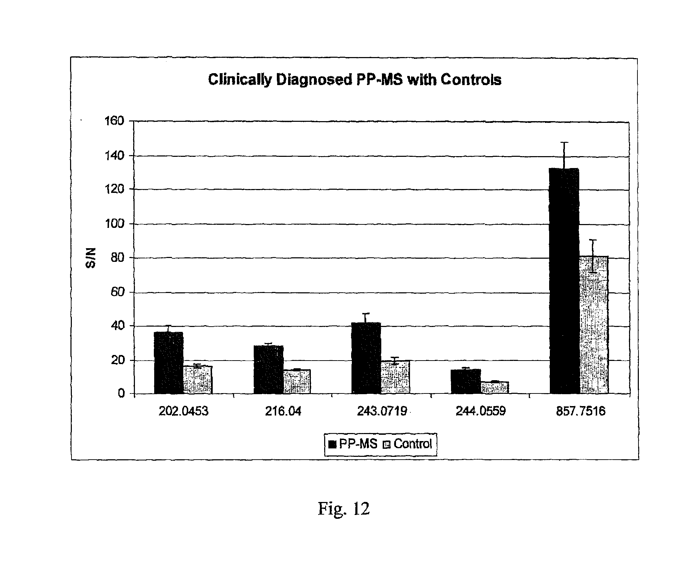

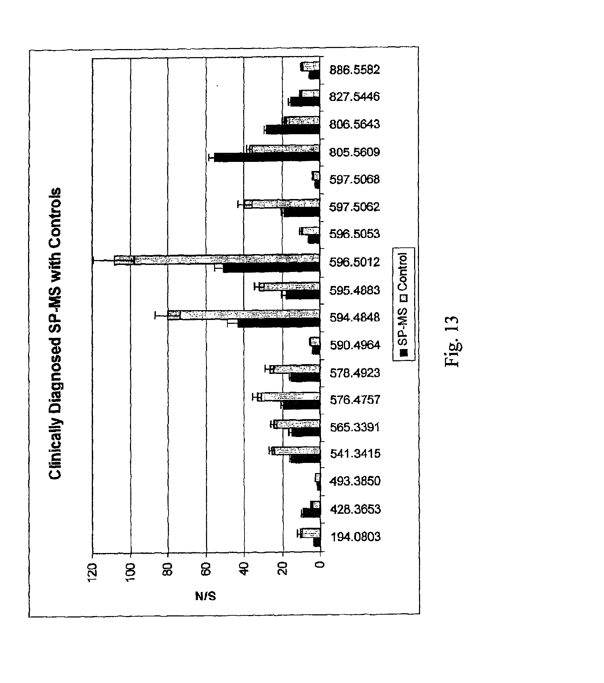

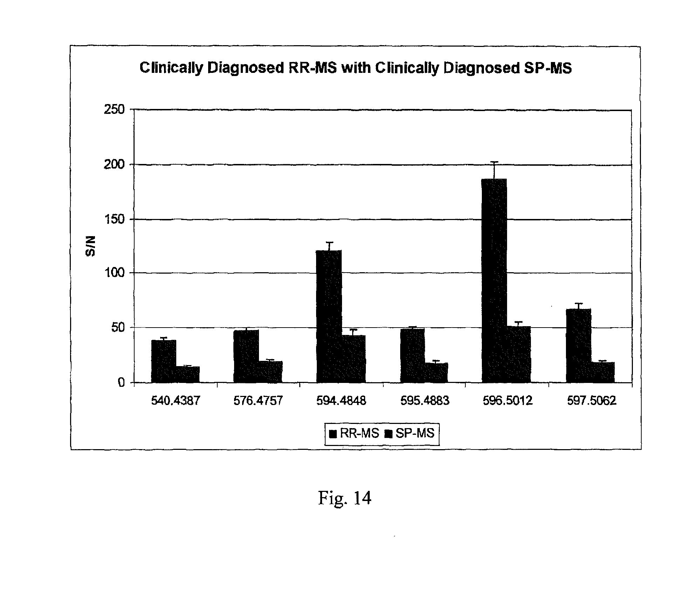

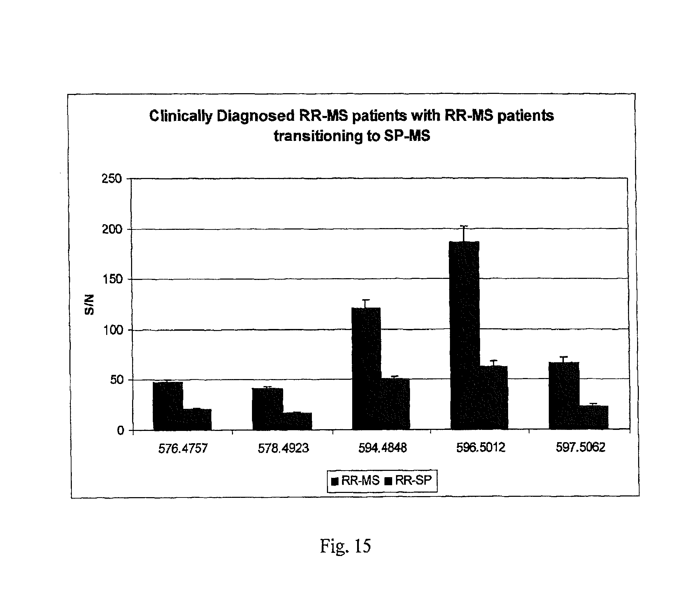

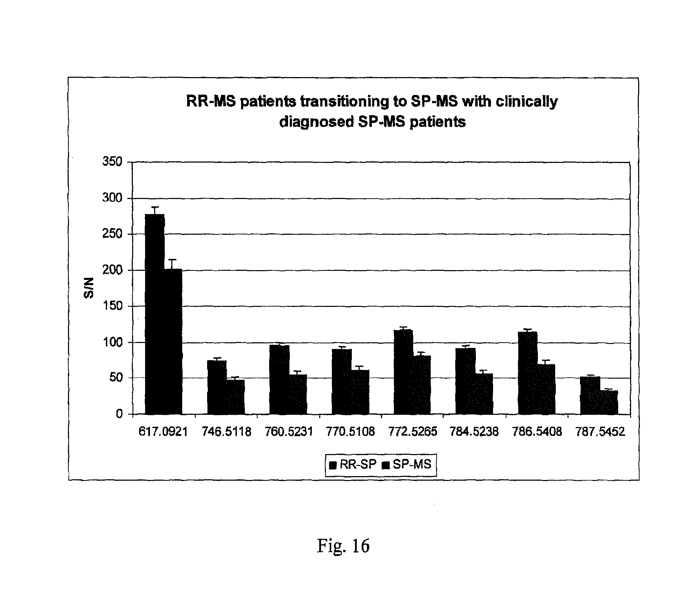

Bar graphs representing the mean +/- SEM of the biomarkers for the different clinical groups are shown in Figures 11-16. Relative to control individuals, the three non-control states can be described as follows:

1. RR-MULTIPLE SCLEROSIS vs. control:
a. Biomarker 452.3868 - increased
b. Biomarker 496.4157 - increased
c. Biomarker 524.4448 - increased
d. Biomarker 540.4387 - increased
e. Biomarker 578.4923 - increased
f. Biomarker 580.5089 - increased
g. Biomarker 594.4848 - increased i. Biomarker 596.5012 - increased
h. Biomarker 597.5062 - increased

2. PP-MULTIPLE SCLEROSIS vs. control:
a. Biomarker 202.0453 - increased
b. Biomarker 216.0400 - increased
c. Biomarker 243.0719 - increased
d. Biomarker 244.0559 - increased
e. Biomarker 857.7516 - increased

3. SP-MULTIPLE SCLEROSIS vs. control:
a. Biomarker 194.0803 - decreased
b. Biomarker 428.3653 - increased
c. Biomarker 493.3850 - decreased
d. Biomarker 541.3415 - decreased
e. Biomarker 565.3391 - decreased
f. Biomarker 576.4757 - decreased
g. Biomarker 578.4923 - decreased
h. Biomarker 590.4964 - decreased
i. Biomarker 594.4848 - decreased
j. Biomarker 595.4883 -decreased
k. Biomarker 596.5012 - decreased
l. Biomarker 596.5053 - decreased
m. Biomarker 597.5062 - decreased
n. Biomarker 597.5068 - decreased
o. Biomarker 805.5609 - increased
p. Biomarker 806.5643 - increased
q. Biomarker 827.5446 - increased
r. Biomarker 886.5582 - decreased

Relative to RR-MULTIPLE SCLEROSIS patients, the two chronic clinical groups can be described as follows:

1. SP-MULTIPLE SCLEROSIS vs. RR-MULTIPLE SCLEROSIS:
a. Biomarker 540.4387 - decreased
b. Biomarker 576.4757 - decreased
c. Biomarker 594.4848 - decreased
d. Biomarker 595.4883 - decreased
e. Biomarker 596.5012 - decreased
f. Biomarker 597.5062 - decreased

2. RR-MULTIPLE SCLEROSIS transitioning to SP-MULTIPLE SCLEROSIS [RR-SP] vs. RR-MULTIPLE SCLEROSIS:
a. Biomarker 576.4757 - decreased
b. Biomarker 578.4923 - decreased
c. Biomarker 594.4848 - decreased
d. Biomarker 596.5012 - decreased
e. Biomarker 597.5062 - decreased

Relative to SP-MULTIPLE SCLEROSIS patients, the PR-MULTIPLE SCLEROSIS patients transitioning to SP-MULTIPLE SCLEROSIS [RR-SP] can be described as follows:

1. RR-MULTIPLE SCLEROSIS transitioning to SP-MULTIPLE SCLEROSIS [RR-SP] vs. SP-MULTIPLE SCLEROSIS:
a. Biomarker 617.0921 - increased
b. Biomarker 746.5118 - increased
c. Biomarker 760.5231 - increased
d. Biomarker 770.5108 - increased
e. Biomarker 772.5265 - increased
f. Biomarker 784.5238 - increased
g. Biomarker 786.5408 - increased
e. Biomarker 787.5452 - increased

The biomarker panels were then applied to the various clinical groups and the ten patients for each clinical group that showed the best separation were selected. A student's T-test was performed on all the serum metabolites using only ten patients per clinical group.
1. Clinically diagnosed RR-MULTLPLE SCLEROSIS patients (n=10) and controls
   (n=10), [257 metabolites, see Table 16];
2. Clinically diagnosed PP-MULTIPLE SCLEROSIS patients (n=10) and controls
   (n=10), [100 metabolites, see Table 17];
3. Clinically diagnosed SP-MULTIPLE SCLEROSIS patients (n=10) and controls
   (n=10), [226 metabolites, see Table 18];
4. Clinically diagnosed RR-MULTIPLE SCLEROSIS patients (n=10) and clinically
   diagnosed SP-MULTIPLE SCLEROSIS (n=10), [142 metabolites, see Table 19];
5. RR- MULTIPLE SCLEROSIS patients transitioning to SP-MULTIPLE SCLEROSIS
   [RR-SP] (n=10) and clinically diagnosed RR-MULTIPLE SCLEROSIS patients (n=10), [148 metabolites, see Table 20];
6. RR-MULTIPLE SCLEROSIS patients transitioning to SP-MULTIPLE SCLEROSIS
   [RR-SP] (n=10) and clinically diagnosed SP-MULTIPLE SCLEROSIS patients (n=10), [309 metabolites, see Table 19].

The sample set (184 individuals) used for this example was comprised of individuals of various geographical backgrounds, and of varying age and health status. Therefore, it is expected that the findings are representative of the general MULTIPLE SCLEROSIS population.

### Example 2: Independent Method Confirmation of Discovered Metabolites

The metabolites and their associations with the clinical variables described in this invention are further confirmed using an independent mass spectrometry system. Representative sample extracts from each variable group are re-analyzed by LC-MS using an HP 1050 high-performance liquid chromatography (HPLC), or equivalent, interfaced to an ABI Q-Star, or equivalent, mass spectrometer to obtain mass and intensity information for the purpose of identifying metabolites that differ in intensity between the clinical variables under investigation.

By determining the levels of the identified metabolites in a person's blood and comparing these levels to levels in a normal "reference" population, a prediction is made whether the person has RR-MULTIPLE SCLEROSIS, PP-MULTIPLE SCLEROSIS, or early stages of SP-MULTIPLE SCLEROSIS. This is carried out in one of several ways: 1) Using a prediction algorithm to classify the test sample, as previously described, which outputs a percentage probability for having a form of MULTIPLE SCLEROSIS. A predictive approach would work independently of the assay method, as long as the intensities of the metabolites are measured. 2) Applying a method based on setting a threshold intensity level from the mass spectrometer, and determining whether a person's profile is above or below the threshold, which indicates their disease status. 3) Using a quantitative assay to determine the molar concentration of the 36 serum metabolites in the normal and disease population. An absolute threshold concentration is then determined for MULTIPLE SCLEROSIS-positivity versus non-MULTIPLE SCLEROSIS-positivity. In a clinical setting, this means that if the measured levels of the metabolites, or combinations of the metabolites, are above a certain concentration, there would be an associated probability that the individual is positive for a type of MULTIPLE SCLEROSIS.

### Example 3: Structure Elucidation of the Primary Metabolite Biomarkers

Characteristics that can be used for structure elucidation of metabolites include accurate mass and molecular formula, polarity, acid/base properties, NMR spectra, and MS/MS or MSⁿ spectra. These data can be used as fingerprints of a particular metabolite and are unique identifiers of a particular metabolite regardless of whether the complete structure has been determined. The data include:

1. *LC retention time.* The extracts containing the metabolites of interest are subjected to reverse phase LC-MS using a C18 column and analysis by MS to determine their retention time under standardized conditions.

2. *MS*/*MS spectra.* Metabolites of interest are further characterized by performing MS/MS fragmentation using collision induced dissociation (CID). This MS/MS analysis is performed in real time (i.e. during the chromatographic elution process) or off-line on fractions collected from the chromatographic separation process. The structure of a given molecule dictates a specific fragmentation pattern under defined conditions and is specific for that molecule (equivalent to a person's fingerprint). Even slight changes to the molecule's structure can result in a different fragmentation pattern. In addition to providing a fingerprint of the molecule's identity, the fragments generated by CID are used to gain insights about the structure of a molecule, and for generating a very specific high-throughput quantitative detection method (see [26-29] for examples).

3. *NMR spectra.* The MS/MS fragmentation provides highly specific descriptive information about a metabolite. However, NMR can solve and confirm the structures of the molecules. As NMR analysis techniques are typically less sensitive than mass spectrometry techniques, multiple injections are performed on the HPLC and the retention time window corresponding to the metabolites of interest collected and combined. The combined extract is then evaporated to dryness and reconstituted in the appropriate solvent for NMR analysis.

Multiple NMR techniques and instruments are available, for example, NMR spectral data are recorded on Bruker Avance 600 MHz spectrometer with cryogenic probe after the chromatographic separation and purification of the metabolites of interest. 1H NMR, 13C NMR, no-difference spec, as well as 2-D NMR techniques like heteronuclear multiple quantum correlation (HMQC), and heteronuclear multiple bond correlation (HMBC) are used for structure elucidation work on the biomarkers.

4. *Extraction conditions.* The conditions of extraction also provide insights about the chemical properties of the biomarkers. All nine metabolites in the serum (from Example 1) were ionized in negative mode (APCI), which is indicative of a molecule containing an acidic moiety such as a carboxylic acid or phosphate. Any moiety capable of losing a hydrogen atom can be detected in negative ionization mode. The metabolite markers were extracted into an organic ethyl acetate fraction, indicating that these metabolites are non-polar under acidic condition.

All chemicals and media were purchased from Sigma-Aldrich Canada Ltd., Oakville, ON., Canada. All solvents were HPLC grade. HPLC analysis were carried out with a high performance liquid chromatograph equipped with quaternary pump, automatic injector, degasser, and a Hypersil ODS column (5 µm particle size silica, 4.6 i.d x 200 mm) with an inline filter. Mobile phase: linear gradient H₂O-MeOH to 100% MeOH in a 52 min period at a flow rate of 1.0 ml/min. High resolution (HR) mass spectra (MS) were recorded on Bruker apex 7T Fourier transform ion cyclotron resonance (FT-ICR) spectrometer and MS/MS data collected using QStar XL TOF mass spectrometer with atmospheric pressure chemical ionization (APCI) and electro spray ionization (ESI) sources in both positive and negative modes.

Metabolite Characterization Data

Biomarker **1**

HRAPCI-MS *m*/*z*: [M - H]⁻, C₂₈H₅₁O₄⁻ measured; 451.3795, calcd. 451.3793. MS/MS *m*/*z* (relative intensity): 451 ([M - H]⁻, 20%), 433 (100%), 407(30%), 389 (90%), 281 (10%), 279 (25%), 183 (20%), 169 (10%), 153 (10%), 125 (20%), 111(25%), 97 (25%).

Biomarker **2**

HRAPCI-MS *m*/*z*: [M - H]⁻, C₃₀H₅₅O₅⁻ measured; 495.4054. calcd. 495.4055 MS/MS *m*/*z* (relative intensity): 495 ([M - H]⁻, 5%), 451 (5%), 477 (15%), (433 (15%), 415(5%), 307 (5%), 297 (45%), 279 (100%), 235 (5%), 223 (20%), 215 (70%), 197 (90%), 179(50%),181 (10%), 169 (100%),157 (25%),155 (10%), 153 (5%), 141 (10%), 139 (5%),127 (10%), 125 (10%), 113 (5%).

Biomarker **3**

HRAPCI-MS *m*/*z*: [M - H]⁻, C₃₂H₅₉O₅⁻ measured; 523.4375, calcd; 523.4368. MS/MS *m*/*z* (relative intensity): 523 ([M - H]⁻, 30%), 505 (100%), 487 (25%), 479 (40%), 463 (40%), 461 (45%), 443 (40%), 365 (30%), 337 (20%), 299 (25%), 297 (25%), 281 (25%), 279 (40%), 271 (65%), 269 (20%), 253 (35%), 251 (55%), 243 (30%), 225 (65%), 197 (55%), 171 (20%), 169(25%), 157 (20%), 155 (10%), 143 (10%), 141 (20%), 139 (20%).

Biomarker **4**

HRAPCI-MS *m*/*z*: [M - H]⁻, C₃₂H₅₉O₆⁻ measured; 539.4312, calcd; 539.4317. MS/MS *m*/*z* (relative intensity): 539 ([M - H]⁻, 20%), 521 (100%), 503 (50%), 495 (40%), 477 (40%), 461 (30%), 459 (40%), 419 (30%), 335 (70%), 315 (40%), 313 (40%), 297 (60%), 279 (90%), 259 (40%), 255 (40%), 253 (20%), 243 (20%), 241 (30%), 225 (20%), 223 (30%), 213 (30%), 179 (20%), 171 (40%), 155 (30%), 141 (50%), 127 (40%).

Biomarker **5**

HRAPCI-MS *m*/*z*: [M - H]⁻, C₃₆H₆₃O₅⁻ measured; 575.4678, calcd; 575.4681. MS/MS *m*/*z* (relative intensity): 575 ([M - H]⁻, 45%), 557 (75%), 539 (70%), 531 (30%), 513 (60%), 495 (100%), 417 (50%), 403 (60%), 371 (25%), 297 (15%), 279 (40%).

Biomarker **6**

HRAPCI-MS *m*/*z*: [M - H]⁻, C₃₆H₆₅O₅⁻ measured; 577.4850, calcd; 577.4837. MS/MS *m*/*z* (relative intensity): 577 ([M - H]⁻, 45%), 559 (75%), 541 (70%), 533 (30%), 515 (60%), 497 (100%), 419 (50%), 405 (60%), 387 (25%), 373 (25%), 297 (15%), 281 (25%), 279 (40%).

Biomarker **7**

HRAPCI-MS *m*/*z*: [M - H]⁻, C₃₆H₆₇O₅⁻ measured; 579.5016, calcd; 579.4994. MS/MS *m*/*z* (relative intensity): 579 ([M - H]⁻, 45%), 561 (90%), 543 (40%), 535 (25%), 517 (60%), 499 (100%), 421 (20%), 407 (20%), 389 (20%), 375 (20%), 299 (25%), 281 (30%), 279 (40%), 263 (10%), 253 (15%), 185 (10%), 171 (25%).

Biomarker **8**

HRAPCI-MS *m*/*z*: [M - H]⁻, C₃₆H₆₅O₆⁻ measured; 593.4775, calcd; 593.4787. MS/MS *m*/*z* (relative intensity): 593 ([M - H]⁻, 50%), 575 (55%), 557 (30%), 549 (15%), 531 (20%), 513 (25%), 495 (10%), 421 (15%), 371 (30%), 315 (50%), 297 (100%), 279 (90%). 201 (30%), 171 (60%), 141 (25%), 127 (25%).

Biomarker **9**

HRAPCI-MS *m*/*z*: [M - H]⁻, C₃₆H₆₇O₆⁻ measured; 595.4939, calcd; 595.4943. MS/MS *m*/*z* (relative intensity): 595 ([M - H]⁻, 20%), 577 (20%), 559 (15%), 551 (5%), 515 (15%), 497 (5%), 423 (5%), 373 (15%), 315 (75%), 297 (70%), 281 (40%), 279 (100%), 269 (5%), 251 (5%), 171 (25%), 155 (15%), 153 (10%), 141 (15%), 139 (10%), 127(15%).

Biomarker **10**

HRAPCI-MS *m*/*z*: [M - H]⁻, C₄₃H₇₈O₁₀P⁻ measured; 785.5329, calcd; 785.5338. MS/MS *m*/*z* (relative intensity): 758 ([M - H]⁻, 100%), 529 (10%), 425 (20%), 273 (73%), 169 (5%), 125 (100%), 97 (5%).

Biomarker **11**

HPAPCI-MS *m*/*z*: [M - H]⁻, C₅H₁₂O₇P⁻ measured; 215.0322, calcd; 215.0326. MS/MS *m*/*z* (relative intensity): 215 ([M - H]⁻, 100%), 197 (30%), 171 (40%), 153 (90%), 135 (20%).

Biomarker **12**

HRAPCI-MS *m*/*z*: [M - H]⁻, C₂₅H₅₁NO₉P⁻ measured; 540.3337, calcd; 540.3301. MS/MS *m*/*z* (relative intensity): 540 ([M - H]⁻, >1%), 480 (17%), 255 (100%), 242 (>1%), 224 (5%), 168 (>1%), 153 (>1%), 78 (>1%).

Biomarker **13**

HRAPCI-MS *m*/*z*: [M - H]⁻, C₂₇H₅₁NO₉P⁻ measured; 564.3313, calcd; 564.3307. MS/MS *m*/*z* (relative intensity): 564 ([M - H]⁻, >1%), 504 (10%), 279 (100%), 242 (>1%), 224 (5%), 168 (>1%), 153 (>1%), 78 (>1%).

Biomarker **14**

HRAPCI-MS *m*/*z*: [M + H]⁺, C₆H₁₂O₆Na⁺ measured; 203.0531, calcd; 205.0526. MS/MS *m*/*z* (relative intensity): 203 ([M + H]⁺, 100%), 159 (15%), 115 (23%), 89 (38%), 97 (5%).

Biomarker **15**

HRAPCI-MS *m*/*z*: [M + H]⁺, C₈H₁₃O₇Na⁺ measured; 245.0637, calcd; 245.0631. MS/MS *m*/*z* (relative intensity): 245 ([M + H]⁺, 100%), 227 (5%), 209 (5%), 155 (10%), 125 (15%), 83 (5%).

Biomarker **16**

HRAPCI-MS *m*/*z*: [M + H]⁺, C₂₉H₄₉O₂⁺ measured; 429.3732, calcd; 429.3727. MS/MS *m*/*z* (relative intensity): 429 ([M + H]⁺, 1%), 205 (5%), 165 (100%).

Biomarker **17**

HRAPCI-MS *m*/*z*: [M + H]⁺, C₄₆H₈₁NO₈P⁺ measured; 806.5687, calcd; 806.5694. MS/MS *m*/*z* (relative intensity): 806 ([M +H]⁺, 21%), 478 (>1%), 237 (>1%), 184 (100%).

Biomarker **18**

HRAPCI-MS *m*/*z*: [M + H]⁺, C₇H₁₅O₆⁺ measured; 195.0881, calcd; 195.0863. MS/MS *m*/*z* (relative intensity): 195 ([M + H]⁺, 2%), 177 (>1%), 165 (>1%), 163 (>1%), 138 (100%), 123 (6%).

Biomarker **19**

HRAPCI-MS *m*/*z*: [M + H]⁺, C₅₄H₁₀₀NO₆⁺ measured; 858.7594, calcd; 858.7545. MS/MS *m*/*z* (relative intensity): 858 ([M + H]⁺, 100%), 576 (10%), 314 (12%), 165 (7%), 151 (10%), 95 (2%).

The accurate masses of the biomarkers were used to deduce the molecular formulae. Tandem mass spectrometry on the biomarkers were used to propose the structures that are summarized in Table 22. The biomarkers were thought to be derivatives of sugars, phospholipids and tocopherols.

The MS/MS spectral data obtained for each of the multiple sclerosis biomarkers was used to deduce their structures. Upon comparing the MS/MS fragmentation patterns of MS biomarkers **1- 9** against that of the CRC panel (see applicant's co-pending application PCT/CA 2006/001502; published as WO/CA2007/030928 on March 22, 2007) a number of similarities were observed. In addition to the common ionization modes for both CRC and these MS biomarkers, their MS/MS spectra also showed signals due to fragment ions corresponding to phytyl chain type fatty acid entities, C18:1 or C18:2 (m/z 281, 279) for all of the detected biomarkers as well as fragment losses due to [M-H-CO₂], [M-H-H₂O] and [M-H-CO₂-H₂O]⁻. Another similarity is that, the MS/MS spectra of MS biomarkers **1** - **9** showed fragment ions deduced as loss of chroman type ring system after cleavage of phytyl side chain [(153 (**1**), 197 (**2**), 225 (**3, 4**), 279 (**5, 6, 7**) and 281 **(8, 9**), Tables 23 - 31)]. These observations led to the assignment of tocopherol type structures for biomarkers **1 - 9**. The loss(es) of water and carbon dioxide suggest the presence of free hydroxyl and carboxylic acid groups. The main differences between MS biomarkers and the CRC's as observed in the MS/MS spectra are the open chroman ring system and chain elongation proposed at position 1.

The molecular formula of **1** was determined as C₂₈H₅₂O₄ by HRAPCI-MS, with three degrees of unsaturation. As indicated above, MS/MS spectra of **1** showed fragment ions due to loss of water *(m*/*z* 433), carbon dioxide (*m*/*z* 407) and presence of phytyl side chain (*m*/*z* 279). Fragment ion observed at *m*/*z* 153 was assigned as a cyclohexenyl ring system generated after the loss of the phytyl side chain. Based on these deductions the structure of metabolite **1** was assigned as shown in Table 22.

As indicated above, metabolites **2-9** have all the structural similarities to **1** and additional hydroxylations and chain elongations via ether linkages with the oxygen atom at position 1. The cyclohexenyl ring unit left after the cleavage of the phytyl side chains of these biomarkers gave unique fragment ions having some variation in the degrees of unsaturation and the number of hydroxylations. These ions observed at *m*/*z* 197 for **2**, *m*/*z* 225 for **3** and **4**, *m*/*z* 279 for **5, 6** and **8** and *m*/*z* 281 for **7** and **9** (See Tables 23 - 31) were used to assign the different alkyl chain elongations; ethyl, butyl and octyl respectively with the appropriate hydroxylations. In some detail, these fragmentation patterns clearly show the differences between each cyclohexenyl ring system. For **1** where there is no chain elongation at position 1, the cyclohexenyl ring fragment resulted when cleaved at C2-C3, generating the formula C₁₀H₁₇O (*m*/*z* 153). In **2** where the ethylation is thought to occur at position 1, and with an additional hydroxy group on the ring, the formula of the cyclohexenyl ring fragment showed an increase by C₂H₄O entity compared to **1**, thus the fragment having C₁₂H₂₁O₂ (m/z 197) as formula. These predictions complied with the observation in the MS/MS spectra of **2** thus validating the structural assignments. In **3** and **4,** the chain elongation was thought to occur with a butyl unit (C₄H₉), thus an increase by C₂H₄ entity with formula C₁₄H₂₅O₂ (*m*/*z* 225) observed when compared to **2.** For biomarkers **5 - 9** the alkoxy chain elongation at position 1 was by C₈H₁₇ entity. Upon comparison of their formulae and MS/MS spectra, **7** and **9** (C₃₆H₆₈O₅ and C₃₆H₆₈O₆) showed similar features except for an additional oxygen atom in **9**. This was consequently assigned on the phytyl chain. Therefore for **7** and **9** the cyclohexenyl ring component fragment ion was observed at *m*/*z* 281 (C₁₈H₃₃O₂)., In the same vane, biomarkers **6** (C₃₆H₆₆O₅) and **8** (C₃₆H₆₆O₆) showed similarity like **7** and **9,** the only difference being an added unsaturation, thus their cyclohexenyl fragment was at m/z 279 (C₁₈H₃₁O₂). An additional degree of unsaturation in **5**(C₃₆H₆₄O₅) compared to **6** and **8** but with ring fragment m/z 279 (C₁₈H₃₁O₂) suggested the additional unsaturation was on the phytyl chain. Based on these deductions, the structures of metabolites 2 to 9 were assigned as shown in Table 22.

Biomarker 10 which was detected in the same mode as 1-9 suggested a different class of metabolite based on the molecular formula FT-ICRMS data. The obtained formula, C₄₃H₇₉O₁₀P suggests a hydroxylated diacylglycerol-phospholipid type structure. The proposed structure and the MS/MS fragments are given in Table 22 and 32 respectively.

MS/MS data obtained on aqueous extracts of serum in the negative mode with electro spray ionization for biomarkers 11-13 were individually analyzed to deduce their structures. The biomarkers identified in this panel were with the formulae of C₅H₁₃O₇P, C₂₅H₅₂NO₉P, and C₂₇H₅₂NO₉P. MS/MS data of 11 (C₅H₁₃O₇P) shows the fragments due to loss of two water molecules as well as a HPO₃ group (Table 33), which can be assigned using the proposed structure. Biomarkers 12 and 13, *(m*/*z* 541.3415, C₂₅H₅₂NO₉P and *m*/*z* 565.3391, C₂₇H₅₂NO₉P) were found to be the same as two Prostrate cancer biomarkers (see applicant's co-pending application PCT/CA 2007/000469, filed on March 23, 2007). In the negative mode with electro spray ionization, (ESI), the most commonly observed ions are the acidic phospholipids such as glycerophosphoinisitol, glycerophosphoserine, glycerophosphatidic acid and glycerophosphoethanolamine. But under certain circumstances it is possible that the phosphocholines can be detected as an adduct of [M + Cl]⁻ or [M + acetate/formate]⁻ as ion species in the negative ESI mode. Since the laboratory procedure of ESI aqueous extractions involves the use of formic acid there is a good probability that these ions could be the formate adduct of phosphocholines. As a result of the addition of the formate group forms a neutral cluster of glycerophosphocholine which forms the corresponding molecular ion ([M-H⁺]⁻) upon subjected to negative ESI now that the ionization site is the phosphatidic group. This suggests the de-protonation of the phosphate group leaving the negatively charged phosphate ion as the parent ion. The fragmentation analysis of biomarkers 12 and 13 are given in Tables 34 and 35.

MS/MS data was obtained on organic and aqueous extracts of serum in positive mode with ESI and APCI for biomarkers 14-19. Biomarkers 14 and 15 (aqueous extract) were identified as sodium adducts of small monosaccharide related metabolites using their MS/MS fragment fingerprint (Tables 36 and 37). Biomarker 16 (Table 38), (m/z 428.3653, C₂₉H₄₈O₂) from organic extracts was assigned as a derivative of α tocopherol since its MS/MS spectra was quite similar to that of α tocopherol standard except for an additional degree of unsaturation. Biomarker 17 (Table 39), (m/z 805.5609, C₄₆H₈₀NO₈P) also from organic extracts of serum was proposed as Oleyl, eicosapentenoic(EPA), N-methyl phosphoethanolamine since the MS/MS data showed fragment ions for the presence of EPA and oleyl groups as well as the N-methyl substituted phosphoethanolamine back bone. The MS/MS spectral data of metabolites 18 (Table 40) and 19 (Table 41) using APCI source, were putatively assigned as monosaccharide and sphingolipid derived biomarkers respectively.

### Example 4: High Throughput Commercial Method Development

For routine analysis of a subset of the metabolites described, a high throughput analysis method is developed. There are multiple types of cost-effective assay platform options currently available depending on the molecules being detected. These include colorimetric chemical assays (UV, or other wavelength), antibody-based enzyme-linked immunosorbant assays (ELISAs), chip- and PCR-based assays for nucleic acid detection, bead-based nucleic-acid detection methods, dipstick chemical assays, image analysis such as magnetic resonance imaging (MRI), positron emission tomography (PET) scan, computerized tomography (CT) scan, and various mass spectrometry-based systems.

The method involves the development of a high-throughput MS/MS method that is compatible with current laboratory instrumentation and triple-quadrupole mass spectrometers which are readily in place in many labs around the world. A Q-Trap™ system is used to isolate the parent molecule, fragment it; and then the fragments are measured.

All citations are hereby incorporated by reference.

The present invention has been described with regard to one or more embodiments. However, it will be apparent to persons skilled in the art that a number of variations and modifications can be made without departing from the scope of the invention as defined in the claims.

**Table 1: Accurate mass features differing between clinically diagnosed RR-MULTIPLE SCLEROSIS patients and controls (p<0.05).**

| | | | | | | | |
|---|---|---|---|---|---|---|---|
| 581.5126 | 1204 | 5.573 | 0.279 | 3.307 | 0.250 | 1.685 | 6.02E-09 |
| 452.3868 | 1204 | 3.933 | 0.137 | 2.758 | 0.161 | 1.426 | 9.63E-09 |
| 496.4157 | 1204 | 10.848 | 0.581 | 6.751 | 0.455 | 1.607 | 3.72E-08 |
| 524.4448 | 1204 | 5.474 | 0.257 | 3.608 | 0.241 | 1.517 | 7.83E-08 |
| 469.3863 | 1204 | 5.090 | 0.204 | 3.536 | 0.206 | 1.439 | 1.25E-07 |
| 580.5089 | 1204 | 13.697 | 0.678 | 8.725 | 0.641 | 1.570 | 1.26E-07 |
| 534.4645 | 1204 | 3.935 | 0.175 | 2.771 | 0.144 | 1.420 | 1.81E-07 |
| 510.3937 | 1204 | 6.354 | 0.265 | 4.445 | 0.265 | 1.429 | 1.99E-07 |
| 552.4784 | 1204 | 8.370 | 0.465 | 5.140 | 0.379 | 1.628 | 2.64E-07 |
| 468.384 | 1204 | 18.514 | 0.778 | 12.977 | 0.754 | 1.427 | 2.69E-07 |
| 506.2853 | 1201 | 4.224 | 0.345 | 3.261 | 0.273 | 1.295 | 2.95E-07 |
| 541.4422 | 1204 | 12.488 | 0.710 | 7.651 | 0.568 | 1.632 | 4.16E-07 |
| 484.3788 | 1204 | 8.292 | 0.379 | 5.832 | 0.359 | 1.422 | 5.37E-07 |
| 450.3729 | 1204 | 9.249 | 0.323 | 6.881 | 0.364 | 1.344 | 5.51E-07 |
| 494.3968 | 1204 | 14.347 | 0.613 | 10.018 | 0.645 | 1.432 | 5.77E-07 |
| 540.4387 | 1204 | 36.603 | 2.086 | 22.346 | 1.749 | 1.638 | 5.92E-07 |
| 522.4313 | 1204 | 15.891 | 0.597 | 11.438 | 0.681 | 1.389 | 9.00E-07 |
| 508.3782 | 1204 | 5.343 | 0.248 | 3.819 | 0.212 | 1.399 | 1.27E-06 |
| 578.4923 | 1204 | 41.017 | 2.169 | 26.563 | 2.068 | 1.544 | 1.37E-06 |
| 466.3656 | 1204 | 13.731 | 0.553 | 10.077 | 0.552 | 1.363 | 1.46E-06 |
| 610.482 | 1204 | 9.001 | 0.477 | 6.136 | 0.359 | 1.467 | 1.46E-06 |
| 536.41 | 1204 | 11.063 | 0.461 | 7.896 | 0.469 | 1.401 | 1.75E-06 |
| 566.454 | 1204 | 8.805 | 0.369 | 5.967 | 0.419 | 1.475 | 1.81E-06 |
| 440.3526 | 1204 | 4.439 | 0.187 | 3.273 | 0.180 | 1.356 | 1.84E-06 |
| 579.4958 | 1204 | 16.171 | 0.852 | 10.661 | 0.805 | 1.517 | 2.19E-06 |
| 480.3473 | 1204 | 3.955 | 0.153 | 3.030 | 0.142 | 1.305 | 2.27E-06 |
| 562.4989 | 1204 | 8.091 | 0.419 | 7.162 | 0.349 | 1.130 | 2.47E-06 |
| 482.3604 | 1204 | 5.195 | 0.241 | 3.772 | 0.196 | 1.377 | 2.55E-06 |
| 512.4079 | 1204 | 16.119 | 0.815 | 10.772 | 0.864 | 1.496 | 2.64E-06 |
| 568.4723 | 1204 | 13.768 | 0.729 | 8.786 | 0.743 | 1.567 | 3.70E-06 |
| 448.3562 | 1204 | 10.559 | 0.375 | 8.119 | 0.373 | 1.301 | 4.90E-06 |
| 569.4769 | 1204 | 5.509 | 0.287 | 3.508 | 0.309 | 1.570 | 6.73E-06 |
| 523.4337 | 1204 | 5.325 | 0.209 | 3.934 | 0.237 | 1.354 | 8.23E-06 |
| 495.4018 | 1204 | 4.656 | 0.199 | 3.441 | 0.203 | 1.353 | 8.62E-06 |
| 550.4602 | 1204 | 10.983 | 0.490 | 7.791 | 0.513 | 1.410 | 8.79E-06 |
| 538.4257 | 1204 | 30.014 | 1.397 | 21.271 | 1.294 | 1.411 | 9.34E-06 |
| 327.0307 | 1204 | 7.570 | 0.224 | 6.421 | 0.164 | 1.179 | 1.08E-05 |
| 513.4116 | 1204 | 5.468 | 0.275 | 3.821 | 0.296 | 1.431 | 1.18E-05 |
| 521.4188 | 1204 | 5.877 | 0.205 | 4.437 | 0.265 | 1.324 | 1.49E-05 |
| 564.513 | 1204 | 4.550 | 0.230 | 3.320 | 0.217 | 1.371 | 1.52E-05 |
| 493.385 | 1204 | 3.460 | 0.129 | 2.549 | 0.165 | 1.357 | 1.94E-05 |
| 467.3711 | 1204 | 4.522 | 0.194 | 3.431 | 0.188 | 1.318 | 2.01E-05 |
| 598.5107 | 1204 | 14.432 | 1.001 | 8.823 | 0.842 | 1.636 | 2.07E-05 |
| 520.4131 | 1204 | 16.144 | 0.600 | 12.295 | 0.696 | 1.313 | 2.10E-05 |
| 590.4585 | 1204 | 11.382 | 0.604 | 8.041 | 0.547 | 1.415 | 2.17E-05 |
| 548.4438 | 1204 | 7.502 | 0.289 | 5.581 | 0.347 | 1.344 | 2.51E-05 |
| 537.4142 | 1204 | 4.383 | 0.178 | 3.299 | 0.207 | 1.329 | 2.53E-05 |
| 596.5053 | 1202 | 15.513 | 0.919 | 9.864 | 0.894 | 1.573 | 2.78E-05 |
| 438.3354 | 1204 | 3.474 | 0.150 | 2.674 | 0.147 | 1.299 | 3.05E-05 |
| 597.5062 | 1204 | 64.543 | 4.659 | 39.473 | 3.816 | 1.635 | 3.06E-05 |
| 564.4396 | 1204 | 3.613 | 0.169 | 2.625 | 0.183 | 1.377 | 3.32E-05 |
| 596.5012 | 1204 | 181.033 | 13.876 | 108.540 | 10.921 | 1.668 | 3.44E-05 |
| 378.9906 | 1204 | 4.143 | 0.107 | 3.556 | 0.099 | 1.165 | 4.07E-05 |
| 492.3832 | 1204 | 9.413 | 0.388 | 7.222 | 0.415 | 1.303 | 4.33E-05 |
| 618.4834 | 1201 | 6.333 | 0.434 | 3.880 | 0.406 | 1.632 | 4.41E-05 |
| 570.4903 | 1204 | 4.537 | 0.344 | 2.752 | 0.258 | 1.649 | 4.42E-05 |
| 597.5068 | 1202 | 5.874 | 0.358 | 3.833 | 0.321 | 1.532 | 4.66E-05 |
| 188.0143 | 1102 | 4.309 | 0.492 | 2.424 | 0.354 | 1.778 | 0.0001 |
| 253.8165 | 1101 | 13.068 | 0.529 | 11.088 | 0.490 | 1.179 | 0.0001 |
| 462.3346 | 1204 | 3.673 | 0.137 | 2.982 | 0.148 | 1.232 | 0.0001 |
| 464.3524 | 1204 | 9.234 | 0.360 | 7.293 | 0.403 | 1.266 | 0.0001 |
| 478.4044 | 1204 | 3.745 | 0.161 | 2.814 | 0.173 | 1.331 | 0.0001 |
| 539.4274 | 1204 | 9.897 | 0.627 | 6.743 | 0.574 | 1.468 | 0.0001 |
| 551.4646 | 1204 | 4.024 | 0.184 | 2.946 | 0.193 | 1.366 | 0.0001 |
| 563.5013 | 1204 | 4.904 | 0.196 | 3.762 | 0.208 | 1.303 | 0.0001 |
| 576.4757 | 1204 | 45.791 | 2.161 | 33.088 | 2.440 | 1.384 | 0.0001 |
| 577.4795 | 1204 | 16.958 | 0.784 | 12.368 | 0.894 | 1.371 | 0.0001 |
| 594.4848 | 1204 | 116.663 | 6.054 | 80.027 | 6.363 | 1.458 | 0.0001 |
| 595.4883 | 1204 | 46.584 | 2.416 | 32.020 | 2.556 | 1.455 | 0.0001 |
| 462.3716 | 1204 | 3.016 | 0.089 | 2.549 | 0.120 | 1.183 | 0.0002 |
| 534.3912 | 1204 | 4.631 | 0.213 | 3.585 | 0.213 | 1.292 | 0.0002 |
| 546.3413 | 1204 | 3.789 | 0.217 | 2.844 | 0.150 | 1.332 | 0.0002 |
| 576.4765 | 1202 | 4.474 | 0.255 | 3.174 | 0.267 | 1.410 | 0.0002 |
| 594.4875 | 1202 | 10.060 | 0.505 | 7.204 | 0.579 | 1.396 | 0.0002 |
| 612.4994 | 1204 | 6.141 | 0.393 | 4.493 | 0.288 | 1.367 | 0.0002 |
| 616.4675 | 1201 | 4.908 | 0.294 | 3.410 | 0.291 | 1.439 | 0.0003 |
| 255.8135 | 1101 | 16.977 | 0.699 | 14.569 | 0.623 | 1.165 | 0.0005 |
| 384.3399 | 1203 | 69.859 | 1.997 | 62.624 | 1.789 | 1.116 | 0,0005 |
| 595.4928 | 1202 | 4.276 | 0.204 | 3.142 | 0.244 | 1.361 | 0.0006 |
| 366.3284 | 1203 | 26.455 | 0.868 | 23.895 | 0.747 | 1.107 | 0.0007 |
| 519.3998 | 1204 | 4.316 | 0.197 | 3.389 | 0.235 | 1.273 | 0.0007 |
| 572.4455 | 1204 | 4.831 | 0.251 | 3.693 | 0.223 | 1.308 | 0.0007 |
| 592.4717 | 1204 | 38.481 | 2.350 | 27.621 | 2.399 | 1.393 | 0.0007 |
| 769.5638 | 1204 | 124.642 | 6.236 | 106.685 | 4.871 | 1.168 | 0.0007 |
| 518.3969 | 1204 | 12.360 | 0.553 | 9.780 | 0.625 | 1.264 | 0.0008 |
| 593.4736 | 1204 | 16.057 | 1.005 | 11.506 | 1.011 | 1.396 | 0.0008 |
| 763.5153 | 1204 | 21.461 | 2.719 | 12.389 | 1.529 | 1.732 | 0.0008 |
| 770.569 | 1204 | 56.365 | 2.587 | 49.027 | 2.067 | 1.150 | 0.0008 |
| 591.4614 | 1204 | 3.999 | 0.218 | 3.096 | 0.219 | 1.292 | 0.001 |
| 476.3869 | 1204 | 4.746 | 0.185 | 3.852 | 0.239 | 1.232 | 0.0011 |
| 502.4054 | 1204 | 6.672 | 0.292 | 5.301 | 0.330 | 1.258 | 0.0011 |
| 716.4323 | 1204 | 14.005 | 0.923 | 10.954 | 0.494 | 1.279 | 0.0011 |
| 381.311 | 1203 | 68.023 | 2.436 | 59.906 | 2.515 | 1.136 | 0.0014 |
| 385.3428 | 1203 | 22.370 | 0.617 | 20.338 | 0.543 | 1.100 | 0.0017 |
| 446.341 | 1204 | 13.375 | 0.593 | 10.949 | 0.621 | 1.222 | 0.0017 |
| 271.8051 | 1102 | 6.853 | 0.380 | 5.799 | 0.449 | 1.182 | 0.0018 |
| 1018.9399 | 1203 | 14.299 | 1.077 | 10.464 | 0.789 | 1.367 | 0.0018 |
| 211.8495 | 1102 | 5.945 | 0.287 | 5.125 | 0.303 | 1.160 | 0.0019 |
| 367.3325 | 1203 | 11.717 | 0.255 | 10.717 | 0.271 | 1.093 | 0.0019 |
| 1254.1311 | 1203 | 6.597 | 0.466 | 4.773 | 0.405 | 1.382 | 0.002 |
| 713.5097 | 1204 | 12.498 | 0.743 | 10.692 | 0.559 | 1.169 | 0.0021 |
| 765.5316 | 1204 | 32.360 | 2.588 | 24.079 | 1.770 | 1.344 | 0.0022 |
| 1016.9279 | 1203 | 23.892 | 2.182 | 17.413 | 1.667 | 1.372 | 0.0023 |
| 257.8106 | 1101 | 8.432 | 0.356 | 7.358 | 0.334 | 1.146 | 0.0026 |
| 532.4503 | 1204 | 4.833 | 0.212 | 3.972 | 0.232 | 1.217 | 0.0027 |
| 546.4298 | 1204 | 4.437 | 0.233 | 3.508 | 0.247 | 1.265 | 0.0027 |
| 1253.1236 | 1203 | 8.820 | 0.595 | 6,551 | 0.561 | 1.346 | 0.0028 |
| 345.8738 | 1101 | 5.102 | 0.275 | 5.977 | 0.235 | 0.854 | 0.0033 |
| 855.6798 | 1204 | 5.567 | 0.503 | 3.533 | 0.454 | 1.576 | 0.0033 |
| 474.3731 | 1204 | 4.936 | 0.232 | 4.069 | 0.229 | 1.213 | 0.0034 |
| 886.5582 | 1102 | 7.913 | 0.483 | 9.581 | 0.608 | 0.826 | 0.0035 |
| 646.5702 | 1203 | 7.748 | 0.465 | 6.225 | 0.383 | 1.245 | 0.0038 |
| 488.2996 | 1204 | 5.809 | 0.309 | 4.794 | 0.190 | 1.212 | 0.0041 |
| 793.5663 | 1204 | 55.191 | 2.714 | 46.988 | 2.378 | 1.175 | 0.0043 |
| 380.3079 | 1203 | 231.313 | 8.275 | 207.315 | 8.508 | 1.116 | 0.0046 |
| 792.555 | 1204 | 29.746 | 2.033 | 24.379 | 1.428 | 1.220 | 0.0047 |
| 202.0453 | 1101 | 27.711 | 2.242 | 21.457 | 0.892 | 1.291 | 0.0048 |
| 448.3194 | 1204 | 3.729 | 0.121 | 3.241 | 0.133 | 1.151 | 0.0048 |
| 791.5488 | 1204 | 57.558 | 4.280 | 46.668 | 2.924 | 1.233 | 0.0048 |
| 490.3676 | 1204 | 6.262 | 0.288 | 5.209 | 0.308 | 1.202 | 0.0049 |
| 382.1084 | 1101 | 3.227 | 0.518 | 1.965 | 0.158 | 1.642 | 0.0051 |
| 468.3577 | 1201 | 3.557 | 0.279 | 2.792 | 0.208 | 1.274 | 0.0053 |
| 504.4188 | 1204 | 6.954 | 0.301 | 5.648 | 0.349 | 1.231 | 0.0053 |
| 376.2759 | 1203 | 18.398 | 0.666 | 17.053 | 0.629 | 1.079 | 0.0057 |
| 378.2921 | 1203 | 40.341 | 1.816 | 37.340 | 1.732 | 1.080 | 0.0057 |
| 634.3951 | 1204 | 8.858 | 0.797 | 6.233 | 0.437 | 1.421 | 0.0057 |
| 712.5074 | 1204 | 28.299 | 1.742 | 24.760 | 1.335 | 1.143 | 0.0061 |
| 702.4175 | 1204 | 10.640 | 0.718 | 8.121 | 0.375 | 1.310 | 0.0063 |
| 781.6001 | 1204 | 9.460 | 0.461 | 8.087 | 0.423 | 1.170 | 0.0063 |
| 745.5643 | 1204 | 120.519 | 6.555 | 105.213 | 5.381 | 1.145 | 0.0068 |
| 741.5302 | 1204 | 30.672 | 2.256 | 25.435 | 1.820 | 1.206 | 0.007 |
| 832.6022 | 1102 | 18.856 | 1.613 | 22.020 | 1.676 | 0.856 | 0.0071 |
| 306.2568 | 1204 | 9.978 | 0.463 | 8.702 | 0.360 | 1.147 | 0.0072 |
| 736.5031 | 1204 | 14.415 | 0.870 | 12.305 | 0.672 | 1.171 | 0.0075 |
| 556.4497 | 1204 | 5.617 | 0.316 | 5.615 | 0.346 | 1.001 | 0.0076 |
| 460.2681 | 1204 | 10.495 | 0.519 | 8.750 | 0.386 | 1.200 | 0.0078 |
| 610.3691 | 1201 | 12.407 | 0.915 | 9.705 | 0.850 | 1.278 | 0.0081 |
| 530.4379 | 1204 | 5.260 | 0.269 | 4.259 | 0.279 | 1.235 | 0.0085 |
| 559.4688 | 1204 | 4.738 | 0.324 | 4.029 | 0.291 | 1.176 | 0.0085 |
| 575.4628 | 1204 | 13.125 | 0.926 | 9.966 | 0.854 | 1.317 | 0.0085 |
| 766.5372 | 1204 | 15.044 | 1.259 | 12.065 | 0.926 | 1.247 | 0.009 |
| 746.5701 | 1204 | 52.385 | 2.663 | 46.291 | 2.263 | 1.132 | 0.0092 |
| 364.3123 | 1203 | 5.078 | 0.151 | 4.629 | 0.136 | 1.097 | 0.0094 |
| 447.3433 | 1204 | 4.155 | 0.228 | 3.361 | 0.217 | 1.236 | 0.0096 |
| 574.4594 | 1204 | 33.564 | 2.435 | 25.417 | 2.231 | 1.321 | 0.0096 |
| 432.3252 | 1204 | 6.136 | 0.165 | 5.573 | 0.150 | 1.101 | 0.0098 |
| 831.5992 | 1102 | 43.218 | 3.967 | 50.976 | 4.257 | 0.848 | 0.0098 |
| 708.4632 | 1201 | 2.976 | 0.180 | 2.474 | 0.195 | 1.203 | 0,0101 |
| 739.5146 | 1204 | 22.411 | 2.380 | 17.043 | 1.472 | 1.315 | 0.0103 |
| 311.7754 | 1101 | 5.487 | 0.325 | 4.484 | 0.299 | 1.224 | 0.0107 |
| 611.3724 | 1201 | 4.409 | 0.327 | 3.525 | 0.273 | 1.251 | 0.0108 |
| 312.231 | 1204 | 5.225 | 0.185 | 4.654 | 0.155 | 1.123 | 0.0112 |
| 794.5718 | 1204 | 28.572 | 1,318 | 25.149 | 1.154 | 1.136 | 0.0114 |
| 446.2525 | 1204 | 5.762 | 0.262 | 4.871 | 0.192 | 1.183 | 0.0122 |
| 737.5045 | 1204 | 6.771 | 0.410 | 5.827 | 0.333 | 1.162 | 0.0122 |
| 558.4649 | 1204 | 6.289 | 0.344 | 5.208 | 0.244 | 1.208 | 0.0123 |
| 243.0719 | 1101 | 33.095 | 2.902 | 26.746 | 1.469 | 1.237 | 0.0125 |
| 296.2357 | 1204 | 10.315 | 0.333 | 9.220 | 0.275 | 1.119 | 0.0128 |
| 218.0192 | 1101 | 8.365 | 0.788 | 6.159 | 0.509 | 1.358 | 0.0134 |
| 574.4635 | 1202 | 3.433 | 0.250 | 2.672 | 0.242 | 1.285 | 0.0134 |
| 743.5461 | 1204 | 365.902 | 20.760 | 321.870 | 20.695 | 1.137 | 0.0135 |
| 379.2957 | 1203 | 10.549 | 0.454 | 9.864 | 0.447 | 1.069 | 0.0143 |
| 273.8743 | 1101 | 9.119 | 0.350 | 8.331 | 0.298 | 1.095 | 0.0146 |
| 747.5761 | 1204 | 16.251 | 0.779 | 14.555 | 0.671 | 1.117 | 0.0149 |
| 263.8453 | 1101 | 8.079 | 0.306 | 7.317 | 0.276 | 1.104 | 0.0154 |
| 474.2846 | 1204 | 9.713 | 0.515 | 7.933 | 0.332 | 1.224 | 0.0158 |
| 290.1737 | 1204 | 3.105 | 0.157 | 2.605 | 0.118 | 1.192 | 0.0159 |
| 377.2801 | 1203 | 6.240 | 0.226 | 5.860 | 0.228 | 1.065 | 0.0162 |
| 495.3322 | 1201 | 3.653 | 0.262 | 4.284 | 0.287 | 0.853 | 0.0163 |
| 730.4535 | 1204 | 27.705 | 1.725 | 22.138 | 0.909 | 1.251 | 0.0165 |
| 244.0559 | 1101 | 9.936 | 0.826 | 8.100 | 0.241 | 1.227 | 0.0169 |
| 267.811 | 1102 | 6.583 | 0.399 | 5.420 | 0.385 | 1.215 | 0.0169 |
| 775.5514 | 1204 | 26.705 | 1.913 | 22.066 | 1.818 | 1.210 | 0.0169 |
| 833.7541 | 1203 | 5.373 | 0.673 | 8.021 | 1.021 | 0.670 | 0.0169 |
| 557.4527 | 1204 | 10.976 | 0.676 | 8.715 | 0.561 | 1.259 | 0.0175 |
| 744.5516 | 1204 | 151.209 | 7.754 | 135.492 | 7.998 | 1.116 | 0.0176 |
| 734.488 | 1204 | 15.280 | 1.078 | 12.822 | 0.881 | 1.192 | 0.018 |
| 551.4976 | 1203 | 38.060 | 4.291 | 54.579 | 6.668 | 0.697 | 0.0182 |
| 689.5083 | 1204 | 11.213 | 0.726 | 9.921 | 0.544 | 1.130 | 0.0183 |
| 314.2461 | 1204 | 5.729 | 0.255 | 5.145 | 0.220 | 1.114 | 0.0189 |
| 743.5475 | 1203 | 17.087 | 1.399 | 13.178 | 0.986 | 1.297 | 0.019 |
| 205.8867 | 1101 | 8.505 | 0.247 | 7.734 | 0.228 | 1.100 | 0.0192 |
| 260.004 | 1101 | 5.043 | 0.421 | 3.917 | 0.255 | 1.288 | 0.0197 |
| 209.8525 | 1102 | 4.762 | 0.278 | 3.920 | 0.272 | 1.215 | 0.0198 |
| 428.3653 | 1201 | 5.297 | 0.338 | 4.617 | 0.315 | 1.147 | 0.0203 |
| 544.3636 | 1204 | 4.414 | 0.247 | 3.576 | 0.180 | 1.234 | 0.0207 |
| 1017.9316 | 1203 | 21.209 | 2.096 | 15.726 | 1.093 | 1.349 | 0.0209 |
| 855.6009 | 1102 | 21.610 | 1.823 | 26.526 | 2.217 | 0.815 | 0.0215 |
| 552.5008 | 1203 | 7.495 | 0.783 | 10.398 | 1.173 | 0.721 | 0.022 |
| 282.2572 | 1204 | 168.937 | 10.620 | 141.140 | 7.379 | 1.197 | 0.0227 |
| 333.9539 | 1102 | 4.018 | 0.340 | 3.076 | 0.264 | 1.306 | 0.0229 |
| 744.55 | 1203 | 7.721 | 0.649 | 5.952 | 0.454 | 1.297 | 0.0242 |
| 502.3165 | 1204 | 33.700 | 1.944 | 27.434 | 1.147 | 1.228 | 0.0243 |
| 693.631 | 1204 | 16.868 | 1.766 | 12.232 | 1.297 | 1.379 | 0.0248 |
| 550.4954 | 1203 | 105.271 | 12.069 | 147.523 | 18.043 | 0.714 | 0.0249 |
| 503.3194 | 1204 | 9.409 | 0.559 | 7.602 | 0.307 | 1.238 | 0.025 |
| 318.1421 | 1201 | 8.664 | 0.503 | 7.285 | 0.537 | 1.189 | 0.0252 |
| 758.4785 | 1204 | 68.915 | 3.462 | 58.805 | 2.587 | 1.172 | 0.0256 |
| 524.296 | 1201 | 4.652 | 0.264 | 3.922 | 0.281 | 1.186 | 0.0258 |
| 269.8081 | 1102 | 10.759 | 0.685 | 8.876 | 0.662 | 1.212 | 0.026 |
| 268.1287 | 1201 | 46.799 | 2.560 | 40.474 | 2.525 | 1.156 | 0.0265 |
| 277.8861 | 1101 | 11.485 | 0.501 | 12.370 | 0.526 | 0.928 | 0.0268 |
| 688.5048 | 1204 | 26.803 | 1.813 | 23.685 | 1.393 | 1.132 | 0.0269 |
| 304.2398 | 1202 | 9.267 | 0.531 | 8.084 | 0.527 | 1.146 | 0.0272 |
| 694.6323 | 1204 | 10.428 | 0.979 | 7.889 | 0.727 | 1.322 | 0.0272 |
| 632.5038 | 1204 | 3.332 | 0.305 | 2.653 | 0.213 | 1.256 | 0.0277 |
| 283.2602 | 1204 | 32.186 | 1.992 | 27.097 | 1.398 | 1.188 | 0.0278 |
| 648.5861 | 1203 | 27.564 | 1.377 | 24.101 | 1.204 | 1.144 | 0.0279 |
| 374.2613 | 1203 | 7.381 | 0.254 | 6.920 | 0.250 | 1.067 | 0.028 |
| 781.5619 | 1204 | 10.474 | 0.737 | 8.753 | 0.595 | 1.197 | 0.0281 |
| 558.3761 | 1204 | 4.507 | 0.282 | 3.634 | 0.242 | 1.240 | 0.0295 |
| 274.1778 | 1202 | 44.020 | 1.999 | 37.866 | 1.523 | 1.163 | 0.0305 |
| 275.1811 | 1202 | 6.901 | 0.302 | 5.925 | 0.241 | 1.165 | 0.0306 |
| 687.4916 | 1204 | 30.334 | 1.910 | 27.231 | 1.812 | 1.114 | 0.0307 |
| 558.4663 | 1202 | 40.924 | 2.515 | 32.452 | 2.372 | 1.261 | 0.0308 |
| 766.5051 | 1201 | 3.411 | 0.211 | 2.896 | 0.207 | 1.178 | 0.0309 |
| 207.8836 | 1101 | 6.751 | 0.210 | 6.223 | 0.230 | 1.085 | 0.0316 |
| 789.5658 | 1204 | 10.254 | 0.434 | 9.339 | 0.370 | 1.098 | 0.0321 |
| 686.4879 | 1204 | 72.574 | 4.856 | 64.772 | 4.573 | 1.120 | 0.0329 |
| 649.5895 | 1203 | 13.385 | 0.671 | 11.763 | 0.593 | 1.138 | 0.0331 |
| 856.6045 | 1102 | 10.911 | 0.876 | 13.159 | 1.067 | 0.829 | 0.0341 |
| 542.3447 | 1102 | 6.632 | 0.354 | 7.305 | 0.314 | 0.908 | 0.0347 |
| 280.2413 | 1204 | 143.389 | 6.365 | 127.689 | 5.733 | 1.123 | 0.0351 |
| 715.5228 | 1204 | 22.831 | 1.767 | 19.876 | 1.283 | 1.149 | 0.0353 |
| 767.5473 | 1204 | 204.597 | 14.465 | 176.048 | 11.501 | 1.162 | 0.0355 |
| 722.479 | 1201 | 3.450 | 0.199 | 3.002 | 0.200 | 1.149 | 0.0356 |
| 265.8424 | 1101 | 7.574 | 0.284 | 6.876 | 0.249 | 1.102 | 0.0365 |
| 296.1601 | 1201 | 95.136 | 5.065 | 83.281 | 5.046 | 1.142 | 0.0383 |
| 328.2393 | 1202 | 4.172 | 0.377 | 3.300 | 0.355 | 1.264 | 0,0394 |
| 249.9677 | 1102 | 6.420 | 0.393 | 5.792 | 0.353 | 1.109 | 0.0397 |
| 768.5525 | 1204 | 93.141 | 5.972 | 81.499 | 4.915 | 1.143 | 0.0407 |
| 281.2447 | 1204 | 28.015 | 1.237 | 25.049 | 1.101 | 1.118 | 0.0408 |
| 560.478 | 1203 | 15.556 | 0.914 | 12.279 | 0.877 | 1.267 | 0.0412 |
| 1251.1042 | 1203 | 7.618 | 0.586 | 6.046 | 0.642 | 1.260 | 0.0418 |
| 333.8302 | 1101 | 7.201 | 0.306 | 6.569 | 0.258 | 1.096 | 0.0441 |
| 742.5366 | 1204 | 15.072 | 1.088 | 13.383 | 0.905 | 1.126 | 0.0443 |
| 256.24 | 1202 | 4.238 | 0.364 | 3.529 | 0.339 | 1.201 | 0.0449 |
| 246.1467 | 1202 | 16.897 | 0.885 | 14.462 | 0.560 | 1.168 | 0.045 |
| 392.294 | 1204 | 5.413 | 0.407 | 4.474 | 0.399 | 1.210 | 0.0485 |
| 552.3273 | 1201 | 6.177 | 0.323 | 5.319 | 0.366 | 1.161 | 0.0488 |

**Table 2: Accurate mass features differing between clinically diagnosed PP- MULTIPLE SCLEROSIS patients and controls (p<0.05).**

| | | | | | | | |
|---|---|---|---|---|---|---|---|
| 188.0143 | 1102 | 5.494 | 0.988 | 2.424 | 0.354 | 2.267 | 7.38E-08 |
| 244.0559 | 1101 | 11.378 | 0.934 | 8.100 | 0.241 | 1.405 | 1.73E-06 |
| 202.0453 | 1101 | 29.842 | 2.721 | 21.457 | 0.892 | 1.391 | 2.59E-05 |
| 218.0371 | 1102 | 7.872 | 0.566 | 6.067 | 0.256 | 1.297 | 3.72E-05 |
| 216.04 | 1102 | 23.392 | 1.656 | 18.040 | 0.754 | 1.297 | 4.97E-05 |
| 243.0719 | 1101 | 33.520 | 3.834 | 26.746 | 1.469 | 1.253 | 0.0003 |
| 273.9985 | 1102 | 4.594 | 0.455 | 3.181 | 0.267 | 1.444 | 0.0003 |
| 218.0192 | 1101 | 9.506 | 1.366 | 6.159 | 0.509 | 1.543 | 0.0004 |
| 226.0688 | 1102 | 12.009 | 1.155 | 10.686 | 0.540 | 1.124 | 0.0004 |
| 290.1737 | 1204 | 3.446 | 0.253 | 2.605 | 0.118 | 1.323 | 0.0006 |
| 278.1494 | 1201 | 11.298 | 1.626 | 6.249 | 0.625 | 1.808 | 0.0008 |
| 260.004 | 1101 | 5.873 | 0.706 | 3.917 | 0.255 | 1.499 | 0.0014 |
| 326.1708 | 1201 | 6.913 | 0.832 | 4.477 | 0.371 | 1.544 | 0.0017 |
| 613.3404 | 1202 | 6.307 | 0.559 | 5.304 | 0.302 | 1.189 | 0.0045 |
| 827.5445 | 1101 | 5.066 | 0.511 | 4.010 | 0.235 | 1.263 | 0.005 |
| 546.3413 | 1204 | 3.704 | 0.305 | 2.844 | 0.150 | 1.303 | 0.0052 |
| 246.1467 | 1202 | 17.624 | 1.405 | 14.462 | 0.560 | 1.219 | 0.0054 |
| 269.132 | 1201 | 7.977 | 0.617 | 5.987 | 0.374 | 1.332 | 0.006 |
| 634.3951 | 1204 | 8.465 | 0.758 | 6.233 | 0.437 | 1.358 | 0.007 |
| 506.4338 | 1204 | 3.033 | 0.356 | 2.406 | 0.161 | 1.260 | 0.0082 |
| 268.1287 | 1201 | 53.182 | 4.401 | 40.474 | 2.525 | 1.314 | 0.0085 |
| 273.8743 | 1101 | 8.819 | 0.350 | 8.331 | 0.298 | 1.059 | 0.01 |
| 1228.1101 | 1203 | 12.407 | 1.879 | 9.994 | 0.940 | 1.241 | 0.0104 |
| 257.8106 | 1101 | 8.396 | 0.392 | 7.358 | 0.334 | 1.141 | 0.0119 |
| 474.2846 | 1204 | 9.786 | 0.712 | 7.933 | 0.332 | 1.234 | 0.0133 |
| 432.2365 | 1204 | 3.775 | 0.254 | 3.251 | 0.139 | 1.161 | 0.0136 |
| 623.5003 | 1203 | 8.814 | 0.720 | 6.953 | 0.309 | 1.268 | 0.0139 |
| 333.9539 | 1102 | 4.124 | 0.515 | 3.076 | 0.264 | 1.341 | 0.0148 |
| 611.3724 | 1201 | 5.093 | 0.575 | 3.525 | 0.273 | 1.445 | 0.0149 |
| 828.5479 | 1101 | 2.853 | 0.300 | 2.322 | 0.123 | 1.229 | 0.015 |
| 282.1444 | 1201 | 10.169 | 0.739 | 7.891 | 0.482 | 1.289 | 0.0162 |
| 622.4973 | 1203 | 18.911 | 1.515 | 14.995 | 0.688 | 1.261 | 0.0174 |
| 296.1601 | 1201 | 105.884 | 8.348 | 83.281 | 5.046 | 1.271 | 0.0175 |
| 488.2996 | 1204 | 5.767 | 0.402 | 4.794 | 0.190 | 1.203 | 0.021 |
| 203.1157 | 1101 | 5.032 | 0.697 | 4.122 | 0.337 | 1.221 | 0.0222 |
| 263.8453 | 1101 | 7.812 | 0.304 | 7.317 | 0.276 | 1.068 | 0.0228 |
| 246.1472 | 1204 | 12.531 | 0.803 | 10.559 | 0.477 | 1.187 | 0.0248 |
| 253.8165 | 1101 | 12.096 | 0.565 | 11.088 | 0.490 | 1.091 | 0.0257 |
| 792.555 | 1204 | 27.305 | 2.818 | 24.379 | 1.428 | 1.120 | 0.0282 |
| 161.1051 | 1101 | 4.361 | 0.462 | 3.749 | 0.267 | 1.163 | 0.0289 |
| 793.4936 | 1204 | 38.528 | 3.782 | 33.573 | 1.651 | 1.148 | 0.0292 |
| 791.5488 | 1204 | 52.376 | 6.009 | 46.668 | 2.924 | 1.122 | 0.03 |
| 517.3141 | 1201 | 2.684 | 0.302 | 2.994 | 0.162 | 0.897 | 0.0315 |
| 610.3691 | 1201 | 14.143 | 1.697 | 9.705 | 0.850 | 1.457 | 0.0322 |
| 310.1758 | 1201 | 7.318 | 0.503 | 5.609 | 0.387 | 1.305 | 0.0323 |
| 217.9124 | 1101 | 11.644 | 0.463 | 11.018 | 0.336 | 1.057 | 0.0347 |
| 446.2525 | 1204 | 5.623 | 0.324 | 4.871 | 0.192 | 1.154 | 0.0356 |
| 328.2393 | 1202 | 5.586 | 1.013 | 3.300 | 0.355 | 1.693 | 0.0365 |
| 318.1421 | 1201 | 9.677 | 0.763 | 7.285 | 0.537 | 1.328 | 0.0383 |
| 274.1778 | 1202 | 44.628 | 3.137 | 37.866 | 1.523 | 1.179 | 0.0389 |
| 297.1634 | 1201 | 16.418 | 1.267 | 13.359 | 0.793 | 1.229 | 0.0391 |
| 831.5992 | 1102 | 33.924 | 6.553 | 50.976 | 4.257 | 0.666 | 0.0391 |
| 275.8713 | 1101 | 5.707 | 0.219 | 5.459 | 0.187 | 1.046 | 0.0393 |
| 819.5831 | 1204 | 15.703 | 1.548 | 14.148 | 0.734 | 1.110 | 0.0423 |
| 460.2681 | 1204 | 10.050 | 0.633 | 8.750 | 0.386 | 1.149 | 0.0429 |
| 506.2853 | 1201 | 5.202 | 0.690 | 3.261 | 0.273 | 1.595 | 0.0431 |
| 832.6022 | 1102 | 15.107 | 2.641 | 22.020 | 1.676 | 0.686 | 0.0439 |
| 899.5871 | 1102 | 7.493 | 1.294 | 10.990 | 0.852 | 0.682 | 0.0462 |
| 328.2415 | 1204 | 4.683 | 0.491 | 3.818 | 0.259 | 1.227 | 0.0465 |
| 503.3194 | 1204 | 8.986 | 0.646 | 7.602 | 0.307 | 1.182 | 0.0475 |

**Table 3: Accurate mass features differing between clinically diagnosed SP- MULTIPLE SCLEROSIS patients and controls (p<0.05).**

| | | | | | | | |
|---|---|---|---|---|---|---|---|
| 428.3653 | 1201 | 9.177 | 0.839 | 4.617 | 0.315 | 1.988 | 2.84E-05 |
| 590.4964 | 1204 | 3.690 | 0.441 | 5.275 | 0.443 | 0.700 | 0.0003 |
| 597.5068 | 1202 | 2.135 | 0.223 | 3.833 | 0.321 | 0.557 | 0.0003 |
| 596.5053 | 1202 | 5.345 | 0.487 | 9.864 | 0.894 | 0.542 | 0.0005 |
| 493.385 | 1204 | 1.599 | 0.143 | 2.549 | 0.165 | 0.627 | 0.001 |
| 594.4875 | 1202 | 4.484 | 0.610 | 7.204 | 0.579 | 0.622 | 0.0014 |
| 763.5153 | 1204 | 19.590 | 3.550 | 12.389 | 1.529 | 1.581 | 0.0016 |
| 764.5196 | 1204 | 8.495 | 1.885 | 4.784 | 0.817 | 1.776 | 0.0017 |
| 194.0803 | 1203 | 3.200 | 0.530 | 10.851 | 1.415 | 0.295 | 0.0019 |
| 872.6715 | 1204 | 4.860 | 0.498 | 2.578 | 0.305 | 1.885 | 0.0019 |
| 597.5062 | 1204 | 18.785 | 1.588 | 39.473 | 3.816 | 0.476 | 0.0022 |
| 616.4675 | 1201 | 2.340 | 0.315 | 3.410 | 0.291 | 0.686 | 0.0023 |
| 495.4018 | 1204 | 2.381 | 0.174 | 3.441 | 0.203 | 0.692 | 0.0025 |
| 595.4928 | 1202 | 2.005 | 0.277 | 3.142 | 0.244 | 0.638 | 0.0025 |
| 523.4337 | 1204 | 2.645 | 0.225 | 3.934 | 0.237 | 0.672 | 0.0026 |
| 598.5107 | 1204 | 4.542 | 0.376 | 8.823 | 0.842 | 0.515 | 0.0028 |
| 596.5012 | 1204 | 50.842 | 4.367 | 108.540 | 10.921 | 0.468 | 0.003 |
| 618.4834 | 1201 | 2.583 | 0.254 | 3.880 | 0.406 | 0.666 | 0.0032 |
| 610.5204 | 1204 | 7.767 | 0.828 | 12.405 | 1.447 | 0.626 | 0.0033 |
| 539.4274 | 1204 | 4.835 | 0.723 | 6.743 | 0.574 | 0.717 | 0.0037 |
| 791.5488 | 1204 | 52.523 | 5.677 | 46.668 | 2.924 | 1.125 | 0.0038 |
| 577.4795 | 1204 | 7.187 | 0.718 | 12.368 | 0.894 | 0.581 | 0.0041 |
| 578.4923 | 1204 | 15.340 | 1.107 | 26.563 | 2.068 | 0.578 | 0.0042 |
| 821.5288 | 1204 | 17.973 | 1.220 | 15.743 | 0.732 | 1.142 | 0.0043 |
| 792.555 | 1204 | 27.350 | 2.825 | 24.379 | 1.428 | 1.122 | 0.0046 |
| 576.4757 | 1204 | 19.011 | 1.969 | 33.088 | 2.440 | 0.575 | 0.0047 |
| 490.3676 | 1204 | 3.541 | 0.400 | 5.209 | 0.308 | 0.680 | 0.0048 |
| 594.4848 | 1204 | 43.087 | 5.319 | 80.027 | 6.363 | 0.538 | 0.0048 |
| 579.4958 | 1204 | 6.320 | 0.458 | 10.661 | 0.805 | 0.593 | 0.0049 |
| 793.4936 | 1204 | 37.612 | 3.233 | 33.573 | 1.651 | 1.120 | 0.0049 |
| 595.4883 | 1204 | 17.967 | 2.221 | 32.020 | 2.556 | 0.561 | 0.0051 |
| 492.3832 | 1204 | 4.994 | 0.446 | 7.222 | 0.415 | 0.692 | 0.0054 |
| 851.5686 | 1102 | 6.306 | 0.461 | 9.813 | 0.818 | 0.643 | 0.0056 |
| 541.4422 | 1204 | 4.920 | 0.418 | 7.651 | 0.568 | 0.643 | 0.0068 |
| 466.3656 | 1204 | 7.059 | 0.607 | 10.077 | 0.552 | 0.700 | 0.0069 |
| 550.4602 | 1204 | 5.085 | 0.560 | 7.791 | 0.513 | 0.653 | 0.007 |
| 606.4872 | 1204 | 4.658 | 0.508 | 6.560 | 0.561 | 0.710 | 0.0072 |
| 806.5643 | 1201 | 27.948 | 1.637 | 18.717 | 0.860 | 1.493 | 0.0075 |
| 522.4313 | 1204 | 7.993 | 0.637 | 11.438 | 0.681 | 0.699 | 0.0076 |
| 551.4646 | 1204 | 1.920 | 0.203 | 2.946 | 0.193 | 0.652 | 0.0078 |
| 495.3321 | 1101 | 10.510 | 0.594 | 11.115 | 0.423 | 0.946 | 0.0081 |
| 440.3526 | 1204 | 2.306 | 0.202 | 3.273 | 0.180 | 0.705 | 0.0083 |
| 558.4663 | 1202 | 3.020 | 0.486 | 4.029 | 0.291 | 0.749 | 0.0084 |
| 467.3711 | 1204 | 2.462 | 0.223 | 3.431 | 0.188 | 0.718 | 0.009 |
| 519.3322 | 1101 | 5.123 | 0.498 | 6.008 | 0.350 | 0.853 | 0.009 |
| 520.4131 | 1204 | 8.374 | 0.748 | 12.295 | 0.696 | 0.681 | 0.009 |
| 548.4438 | 1204 | 3.745 | 0.372 | 5.581 | 0.347 | 0.671 | 0.0091 |
| 805.5609 | 1201 | 55.027 | 3.212 | 36.921 | 1.704 | 1.490 | 0.0093 |
| 468.3577 | 1201 | 5.153 | 0.520 | 2.792 | 0.208 | 1.846 | 0.0094 |
| 538.4257 | 1204 | 14.296 | 1.804 | 21.271 | 1.294 | 0.672 | 0.0094 |
| 464.3524 | 1204 | 5.162 | 0.501 | 7.293 | 0.403 | 0.708 | 0.0097 |
| 542.3447 | 1102 | 4.282 | 0.293 | 7.305 | 0.314 | 0.586 | 0.0098 |
| 446.341 | 1204 | 7.886 | 0.859 | 10.949 | 0.621 | 0.720 | 0.01 |
| 513.4116 | 1204 | 2.379 | 0.211 | 3.821 | 0.296 | 0.623 | 0.0107 |
| 540.4387 | 1204 | 14.521 | 1.256 | 22.346 | 1.749 | 0.650 | 0.0108 |
| 202.0453 | 1101 | 24.111 | 1.347 | 21.457 | 0.892 | 1.124 | 0.0109 |
| 328.2415 | 1204 | 4.988 | 0.862 | 3.818 | 0.259 | 1.306 | 0.0109 |
| 819.5831 | 1204 | 15.250 | 1.244 | 14.148 | 0.734 | 1.078 | 0.0112 |
| 569.3687 | 1102 | 4.492 | 0.482 | 7.792 | 0.345 | 0.576 | 0.0117 |
| 568.4723 | 1204 | 5.475 | 0.537 | 8.786 | 0.743 | 0.623 | 0.0123 |
| 518.3969 | 1204 | 6.830 | 0.808 | 9.780 | 0.625 | 0.698 | 0.0125 |
| 828.5477 | 1201 | 7.801 | 0.619 | 5.137 | 0.244 | 1.519 | 0.0126 |
| 494.3968 | 1204 | 6.860 | 0.516 | 10.018 | 0.645 | 0.685 | 0.0129 |
| 576.4765 | 1202 | 2.269 | 0.250 | 3.174 | 0.267 | 0.715 | 0.0129 |
| 249.9677 | 1102 | 5.069 | 0.455 | 5.792 | 0.353 | 0.875 | 0.0143 |
| 468.384 | 1204 | 9.354 | 0.797 | 12.977 | 0.754 | 0.721 | 0.0147 |
| 382.1084 | 1101 | 2.573 | 0.251 | 1.965 | 0.158 | 1.309 | 0.015 |
| 566.454 | 1204 | 3.969 | 0.446 | 5.967 | 0.419 | 0.665 | 0.0154 |
| 484.3788 | 1204 | 4.070 | 0.305 | 5.832 | 0.359 | 0.698 | 0.0157 |
| 512.4079 | 1204 | 6.864 | 0.551 | 10.772 | 0.864 | 0.637 | 0.0157 |
| 610.482 | 1204 | 4.242 | 0.241 | 6.136 | 0.359 | 0.691 | 0.0159 |
| 537.4142 | 1204 | 2.263 | 0.275 | 3.299 | 0.207 | 0.686 | 0.0167 |
| 720.4696 | 1204 | 6.131 | 0.514 | 4.968 | 0.257 | 1.234 | 0.0167 |
| 580.5089 | 1204 | 5.730 | 0.402 | 8.725 | 0.641 | 0.657 | 0.017 |
| 855.6798 | 1204 | 5.942 | 0.996 | 3.533 | 0.454 | 1.682 | 0.017 |
| 448.3194 | 1204 | 4.225 | 0.221 | 3.241 | 0.133 | 1.304 | 0.0177 |
| 508.3782 | 1204 | 2.805 | 0.263 | 3.819 | 0.212 | 0.735 | 0.0178 |
| 438.3354 | 1204 | 2.071 | 0.176 | 2.674 | 0.147 | 0.774 | 0.0181 |
| 574.4594 | 1204 | 15.435 | 2.101 | 25.417 | 2.231 | 0.607 | 0.0187 |
| 613.3404 | 1202 | 5.155 | 0.432 | 5.304 | 0.302 | 0.972 | 0.0189 |
| 482.3604 | 1204 | 2.926 | 0.227 | 3.772 | 0.196 | 0.776 | 0.019 |
| 827.5446 | 1201 | 15.396 | 1.216 | 9.932 | 0.505 | 1.550 | 0.0191 |
| 564.4396 | 1204 | 1.877 | 0.175 | 2.625 | 0.183 | 0.715 | 0.0192 |
| 448.3562 | 1204 | 6.275 | 0.562 | 8.119 | 0.373 | 0.773 | 0.0194 |
| 541.3415 | 1102 | 15.111 | 1.031 | 25.470 | 1.129 | 0.593 | 0.0203 |
| 622.4973 | 1203 | 20.247 | 1.598 | 14.995 | 0.688 | 1.350 | 0.0219 |
| 311.7754 | 1101 | 4.688 | 0.288 | 4.484 | 0.299 | 1.045 | 0.022 |
| 385.3428 | 1203 | 21.477 | 0.879 | 20.338 | 0.543 | 1.056 | 0.022 |
| 574.4635 | 1202 | 1.995 | 0.279 | 2.672 | 0.242 | 0.747 | 0.0231 |
| 566.3431 | 1102 | 4.439 | 0.519 | 7.334 | 0.380 | 0.605 | 0.024 |
| 521.3478 | 1101 | 3.649 | 0.287 | 4.014 | 0.207 | 0.909 | 0.0244 |
| 328.2393 | 1202 | 6.800 | 1.193 | 3.300 | 0.355 | 2.061 | 0.0248 |
| 480.3473 | 1204 | 2.492 | 0.216 | 3.030 | 0.142 | 0.823 | 0.0249 |
| 253.8165 | 1101 | 10.790 | 0.555 | 11.088 | 0.490 | 0.973 | 0.025 |
| 510.3937 | 1204 | 3.222 | 0.237 | 4.445 | 0.265 | 0.725 | 0.0251 |
| 1228.1101 | 1203 | 8.624 | 1.735 | 9.994 | 0.940 | 0.863 | 0.0253 |
| 565.3391 | 1102 | 14.619 | 1.846 | 24.344 | 1.318 | 0.601 | 0.0256 |
| 593.4736 | 1204 | 7.260 | 0.952 | 11.506 | 1.011 | 0.631 | 0.0256 |
| 519.3998 | 1204 | 2.331 | 0.340 | 3.389 | 0.235 | 0.688 | 0.0265 |
| 886.5582 | 1102 | 5.104 | 0.302 | 9.581 | 0.608 | 0.533 | 0.0267 |
| 694.6323 | 1204 | 12.155 | 1.212 | 7.889 | 0.727 | 1.541 | 0.0283 |
| 820.589 | 1204 | 9.217 | 0.740 | 8.675 | 0.401 | 1.062 | 0.0291 |
| 384.3399 | 1203 | 65.198 | 3.300 | 62.624 | 1.789 | 1.041 | 0.0303 |
| 546.4298 | 1204 | 2.524 | 0.327 | 3.508 | 0.247 | 0.719 | 0.0305 |
| 766.5372 | 1204 | 13.388 | 1.664 | 12.065 | 0.926 | 1.110 | 0.0308 |
| 469.3863 | 1204 | 2.523 | 0.248 | 3.536 | 0.206 | 0.714 | 0.0312 |
| 312.231 | 1204 | 5.656 | 0.280 | 4.654 | 0.155 | 1.215 | 0.0313 |
| 592.4717 | 1204 | 17.304 | 2.411 | 27.621 | 2.399 | 0.626 | 0.0313 |
| 541.3141 | 1201 | 2.734 | 0.234 | 2.835 | 0.227 | 0.964 | 0.0315 |
| 474.3731 | 1204 | 3.158 | 0.377 | 4.069 | 0.229 | 0.776 | 0.0326 |
| 575.4628 | 1204 | 6.499 | 0.860 | 9.966 | 0.854 | 0.652 | 0.0329 |
| 723.6395 | 1204 | 9.645 | 0.666 | 6.930 | 0.437 | 1.392 | 0.0333 |
| 244.0559 | 1101 | 9.067 | 0.491 | 8.100 | 0.241 | 1.119 | 0.0335 |
| 246.1468 | 1201 | 6.273 | 0.755 | 4.636 | 0.356 | 1.353 | 0.0339 |
| 765.5316 | 1204 | 26.162 | 3.135 | 24.079 | 1.770 | 1.087 | 0.0342 |
| 521.4188 | 1204 | 3.362 | 0.267 | 4.437 | 0.265 | 0.758 | 0.0343 |
| 534.3912 | 1204 | 2.755 | 0.256 | 3.585 | 0.213 | 0.769 | 0.0346 |
| 569.4769 | 1204 | 2.292 | 0.255 | 3.508 | 0.309 | 0.653 | 0.0349 |
| 523.3637 | 1101 | 2.894 | 0.313 | 3.422 | 0.124 | 0.846 | 0.0357 |
| 243.0719 | 1101 | 24.190 | 1.697 | 26.746 | 1.469 | 0.904 | 0.0366 |
| 255.8135 | 1101 | 14.393 | 0.624 | 14.569 | 0.623 | 0.988 | 0.0374 |
| 536.41 | 1204 | 6.261 | 0.609 | 7.896 | 0.469 | 0.793 | 0.0387 |
| 541.3141 | 1101 | 5.759 | 0.624 | 6.840 | 0.414 | 0.842 | 0.0414 |
| 768.5468 | 1102 | 1.679 | 0.306 | 3.219 | 0.321 | 0.522 | 0.0415 |
| 590.4585 | 1204 | 5.278 | 0.564 | 2.834 | 0.368 | 1.862 | 0.0421 |
| 684.6037 | 1203 | 4.261 | 0.453 | 3.097 | 0.392 | 1.376 | 0.0431 |
| 852.5724 | 1102 | 3.428 | 0.268 | 5.434 | 0.416 | 0.631 | 0.0436 |
| 552.4784 | 1204 | 16.345 | 1.998 | 22.583 | 1.626 | 0.724 | 0.0454 |
| 560.4821 | 1204 | 5.721 | 0.595 | 8.041 | 0.547 | 0.712 | 0.049 |

**Table 4: Accurate mass features differing between clinically diagnosed SP- MULTIPLE SCLEROSIS patients and RR- MULTIPLE SCLEROSIS patients (p<0.05).**

| | | | | | | | |
|---|---|---|---|---|---|---|---|
| 452.3868 | 1204 | 2.163 | 0.154 | 3.906 | 0.137 | 0.554 | 4.29E-11 |
| 580.5089 | 1204 | 5.730 | 0.402 | 13.528 | 0.711 | 0.424 | 1.98E-10 |
| 578.4923 | 1204 | 15.340 | 1.107 | 40.496 | 2.263 | 0.379 | 2.21E-10 |
| 493.385 | 1204 | 1.599 | 0.143 | 3.412 | 0.131 | 0.469 | 2.85E-10 |
| 523.4337 | 1204 | 2.645 | 0.225 | 5.249 | 0.219 | 0.504 | 3.26E-10 |
| 522.4313 | 1204 | 7.993 | 0.637 | 15.695 | 0.623 | 0.509 | 3.42E-10 |
| 512.4079 | 1204 | 6.864 | 0.551 | 15.906 | 0.838 | 0.432 | 4.59E-10 |
| 579.4958 | 1204 | 6.320 | 0.458 | 15.978 | 0.888 | 0.396 | 5.47E-10 |
| 494.3968 | 1204 | 6.860 | 0.516 | 14.131 | 0.626 | 0.485 | 6.51E-10 |
| 495.4018 | 1204 | 2.381 | 0.174 | 4.575 | 0.202 | 0.521 | 8.69E-10 |
| 484.3788 | 1204 | 4.070 | 0.305 | 8.180 | 0.389 | 0.497 | 1.00E-09 |
| 513.4116 | 1204 | 2.379 | 0.211 | 5.401 | 0.281 | 0.440 | 1.14E-09 |
| 596.5053 | 1202 | 5.345 | 0.487 | 15.297 | 0.951 | 0.349 | 1.54E-09 |
| 581.5126 | 1204 | 2.422 | 0.183 | 5.487 | 0.296 | 0.441 | 1.69E-09 |
| 466.3656 | 1204 | 7.059 | 0.607 | 13.494 | 0.566 | 0.523 | 1.72E-09 |
| 550.4602 | 1204 | 5.085 | 0.560 | 10.852 | 0.521 | 0.469 | 1.85E-09 |
| 510.3937 | 1204 | 3.222 | 0.237 | 6.276 | 0.278 | 0.513 | 2.63E-09 |
| 468.384 | 1204 | 9.354 | 0.797 | 18.294 | 0.792 | 0.511 | 2.96E-09 |
| 469.3863 | 1204 | 2.523 | 0.248 | 5.002 | 0.209 | 0.504 | 4.28E-09 |
| 597.5068 | 1202 | 2.135 | 0.223 | 5.788 | 0.369 | 0.369 | 4.59E-09 |
| 440.3526 | 1204 | 2.306 | 0.202 | 4.382 | 0.192 | 0.526 | 5.93E-09 |
| 568.4723 | 1204 | 5.475 | 0.537 | 13.607 | 0.761 | 0.402 | 6.09E-09 |
| 618.4834 | 1201 | 2.583 | 0.254 | 6.236 | 0.449 | 0.414 | 9.48E-09 |
| 577.4795 | 1204 | 7.187 | 0.718 | 16.759 | 0.829 | 0.429 | 1.26E-08 |
| 524.4448 | 1204 | 2.878 | 0.241 | 5.382 | 0.265 | 0.535 | 1.34E-08 |
| 450.3729 | 1204 | 5.537 | 0.435 | 9.090 | 0.331 | 0.609 | 1.38E-08 |
| 594.4875 | 1202 | 4.484 | 0.610 | 9.946 | 0.526 | 0.451 | 1.40E-08 |
| 551.4646 | 1204 | 1.920 | 0.203 | 3.993 | 0.192 | 0.481 | 1.71E-08 |
| 566.454 | 1204 | 3.969 | 0.446 | 8.691 | 0.393 | 0.457 | 1.85E-08 |
| 552.4784 | 1204 | 3.804 | 0.378 | 8.282 | 0.478 | 0.459 | 1.89E-08 |
| 598.5107 | 1204 | 4.542 | 0.376 | 14.230 | 1.030 | 0.319 | 1.97E-08 |
| 576.4757 | 1204 | 19.011 | 1.969 | 45.354 | 2.261 | 0.419 | 2.02E-08 |
| 548.4438 | 1204 | 3.745 | 0.372 | 7.407 | 0.309 | 0.506 | 2.90E-08 |
| 448.3562 | 1204 | 6.275 | 0.562 | 10.433 | 0.390 | 0.601 | 3.15E-08 |
| 569.4769 | 1204 | 2.292 | 0.255 | 5.444 | 0.298 | 0.421 | 3.44E-08 |
| 520.4131 | 1204 | 8.374 | 0.748 | 15.943 | 0.634 | 0.525 | 3.59E-08 |
| 467.3711 | 1204 | 2.462 | 0.223 | 4.437 | 0.193 | 0.555 | 3.62E-0B |
| 597.5062 | 1204 | 18.785 | 1.588 | 63.633 | 4.798 | 0.295 | 4.01E-08 |
| 508.3782 | 1204 | 2.805 | 0.263 | 5.241 | 0.265 | 0.535 | 4.46E-08 |
| 564.4396 | 1204 | 1.877 | 0.175 | 3.584 | 0.173 | 0.524 | 4.64E-08 |
| 521.4188 | 1204 | 3.362 | 0.267 | 5.796 | 0.222 | 0.580 | 4.75E-08 |
| 541.4422 | 1204 | 4.920 | 0.418 | 12.302 | 0.734 | 0.400 | 5.43E-08 |
| 496.4157 | 1204 | 5.182 | 0.354 | 10.685 | 0.591 | 0.485 | 5.52E-08 |
| 492.3832 | 1204 | 4.994 | 0.446 | 9.311 | 0.399 | 0.536 | 5.78E-08 |
| 594.4848 | 1204 | 43.087 | 5.319 | 115.274 | 6.359 | 0.374 | 5.81E-08 |
| 537.4142 | 1204 | 2.263 | 0.275 | 4.317 | 0.185 | 0.524 | 5.85E-08 |
| 536.41 | 1204 | 6.261 | 0.609 | 10.884 | 0.483 | 0.575 | 6.25E-08 |
| 540.4387 | 1204 | 14.521 | 1.256 | 36.081 | 2.150 | 0.402 | 6.32E-08 |
| 595.4883 | 1204 | 17.967 | 2.221 | 46.028 | 2.536 | 0.390 | 6.43E-08 |
| 595.4928 | 1202 | 2.005 | 0.277 | 4.218 | 0.215 | 0.475 | 6.44E-08 |
| 616.4675 | 1201 | 2.340 | 0.315 | 4.851 | 0.305 | 0.482 | 7.05E-08 |
| 482.3604 | 1204 | 2.926 | 0.227 | 5.143 | 0.245 | 0.569 | 7.54E-08 |
| 596.5012 | 1204 | 50.842 | 4.367 | 178.485 | 14.249 | 0.285 | 7.94E-08 |
| 610.482 | 1204 | 4.242 | 0.241 | 8.903 | 0.494 | 0.476 | 1.17E-07 |
| 464.3524 | 1204 | 5.162 | 0.501 | 9.142 | 0.365 | 0.565 | 1.72E-07 |
| 480.3473 | 1204 | 2.492 | 0.216 | 3.911 | 0.157 | 0.637 | 1.78E-07 |
| 438.3354 | 1204 | 2.071 | 0.176 | 3.426 | 0.150 | 0.604 | 2.41E-07 |
| 539.4274 | 1204 | 4.835 | 0.723 | 9.733 | 0.636 | 0.497 | 2.43E-07 |
| 576.4765 | 1202 | 2.269 | 0.250 | 4.415 | 0.265 | 0.514 | 3.05E-07 |
| 538.4257 | 1204 | 14.296 | 1.804 | 29.559 | 1.464 | 0.484 | 3.34E-07 |
| 562.4989 | 1204 | 7.595 | 0.589 | 12.437 | 0.512 | 0.611 | 3.83E-07 |
| 590.4585 | 1204 | 5.721 | 0.595 | 11.249 | 0.631 | 0.509 | 4.39E-07 |
| 563.5013 | 1204 | 2.892 | 0.258 | 4.830 | 0.203 | 0.599 | 5.17E-07 |
| 478.4044 | 1204 | 2.256 | 0.210 | 3.682 | 0.161 | 0.613 | 5.30E-07 |
| 518.3969 | 1204 | 6.830 | 0.808 | 12.262 | 0.569 | 0.557 | 1.07E-06 |
| 462.3716 | 1204 | 1.914 | 0.187 | 3.007 | 0.090 | 0.636 | 1.33E-06 |
| 446.341 | 1204 | 7.886 | 0.859 | 13.291 | 0.599 | 0.593 | 2.02E-06 |
| 476.3869 | 1204 | 3.097 | 0.261 | 4.685 | 0.186 | 0.661 | 2.08E-06 |
| 519.3998 | 1204 | 2.331 | 0.340 | 4.275 | 0.202 | 0.545 | 2.45E-06 |
| 593.4736 | 1204 | 7.260 | 0.952 | 15.915 | 1.032 | 0.456 | 2.57E-06 |
| 592.4717 | 1204 | 17.304 | 2.411 | 38.114 | 2.424 | 0.454 | 2.82E-06 |
| 570.4903 | 1204 | 1.853 | 0.317 | 4.480 | 0.352 | 0.414 | 3.57E-06 |
| 534.3912 | 1204 | 2.755 | 0.256 | 4.595 | 0.215 | 0.600 | 3.63E-06 |
| 534.4645 | 1204 | 2.436 | 0.202 | 3.904 | 0.180 | 0.624 | 4.64E-06 |
| 532.4503 | 1204 | 3.116 | 0.284 | 4.752 | 0.228 | 0.656 | 6.03E-06 |
| 490.3676 | 1204 | 3.541 | 0.400 | 6.198 | 0.296 | 0.571 | 7.12E-06 |
| 462.3346 | 1204 | 2.328 | 0.276 | 3.632 | 0.138 | 0.641 | 1.43E-05 |
| 502.4054 | 1204 | 4.125 | 0.456 | 6.631 | 0.298 | 0.622 | 1.47E-05 |
| 591.4614 | 1204 | 2.320 | 0.217 | 3.990 | 0.220 | 0.582 | 1.63E-05 |
| 574.4594 | 1204 | 15.435 | 2.101 | 32.679 | 2.206 | 0.472 | 2.00E-05 |
| 546.4298 | 1204 | 2.524 | 0.327 | 4.374 | 0.245 | 0.577 | 2.51E-05 |
| 504.4188 | 1204 | 4.556 | 0.423 | 6.780 | 0.309 | 0.672 | 3.45E-05 |
| 575.4628 | 1204 | 6.499 | 0.860 | 12.808 | 0.831 | 0.507 | 3.95E-05 |
| 572.4455 | 1204 | 2.656 | 0.324 | 4.775 | 0.263 | 0.556 | 4.64E-05 |
| 574.4635 | 1202 | 1.995 | 0.279 | 3.453 | 0.242 | 0.578 | 4.77E-05 |
| 327.0307 | 1204 | 6.113 | 0.237 | 7.511 | 0.224 | 0.814 | 0.0001 |
| 447.3433 | 1204 | 2.679 | 0.300 | 4.068 | 0.211 | 0.658 | 0.0001 |
| 474.3731 | 1204 | 3.158 | 0.377 | 4.916 | 0.233 | 0.642 | 0.0001 |
| 530.4379 | 1204 | 3.465 | 0.344 | 5.085 | 0.240 | 0.681 | 0.0001 |
| 558.4649 | 1204 | 22.470 | 2.918 | 39.813 | 2.242 | 0.564 | 0.0001 |
| 558.4663 | 1202 | 3.020 | 0.486 | 4.836 | 0.312 | 0.624 | 0.0001 |
| 559.4688 | 1204 | 8.680 | 1.100 | 15.071 | 0.830 | 0.576 | 0.0001 |
| 561.4863 | 1204 | 6.581 | 0.759 | 9.904 | 0.461 | 0.664 | 0.0001 |
| 560.4821 | 1204 | 16.345 | 1.998 | 24.793 | 1.209 | 0.659 | 0.0002 |
| 564.513 | 1204 | 2.848 | 0.261 | 4.456 | 0.242 | 0.639 | 0.0002 |
| 612.4994 | 1204 | 3.494 | 0.237 | 6.110 | 0.396 | 0.572 | 0.0002 |
| 532.1851 | 1204 | 1.780 | 0.243 | 1.088 | 0.050 | 1.636 | 0.0003 |
| 506.4338 | 1204 | 2.504 | 0.192 | 3.502 | 0.168 | 0.715 | 0.0004 |
| 610.5204 | 1204 | 7.767 | 0.828 | 13.914 | 1.696 | 0.558 | 0.0005 |
| 556.4497 | 1204 | 6.304 | 0.714 | 10.754 | 0.704 | 0.586 | 0.0013 |
| 590.4964 | 1204 | 4.097 | 0.371 | 3.690 | 0.441 | 1.110 | 0.0023 |
| 821.5288 | 1204 | 17.973 | 1.220 | 15.703 | 0.731 | 1.145 | 0.0026 |
| 557.4527 | 1204 | 2.788 | 0.293 | 4.409 | 0.298 | 0.632 | 0.004 |
| 606.4872 | 1204 | 4.658 | 0.508 | 6.362 | 0.467 | 0.732 | 0.0041 |
| 340.2407 | 1204 | 6.645 | 0.655 | 5.152 | 0.169 | 1.290 | 0.0042 |
| 851.5686 | 1102 | 6.306 | 0.461 | 9.698 | 1.102 | 0.650 | 0.0046 |
| 886.7896 | 1203 | 5.463 | 1.422 | 4.340 | 0.882 | 1.259 | 0.0075 |
| 378.9906 | 1204 | 3.602 | 0.183 | 4.124 | 0.106 | 0.873 | 0.0121 |
| 852.5724 | 1102 | 3.428 | 0.268 | 5.055 | 0.553 | 0.678 | 0.0135 |
| 194.0803 | 1203 | 3.200 | 0.530 | 9.406 | 1.860 | 0.340 | 0.0144 |
| 834.5963 | 1201 | 7.969 | 0.614 | 5.250 | 0.377 | 1.518 | 0.0154 |
| 264.9759 | 1204 | 6.124 | 0.234 | 6.909 | 0.130 | 0.886 | 0.0158 |
| 872.6715 | 1204 | 4.860 | 0.498 | 2.907 | 0.376 | 1.671 | 0.0173 |
| 477.3218 | 1201 | 6.130 | 0.392 | 3.865 | 0.260 | 1.586 | 0.0183 |
| 551.4991 | 1201 | 2.961 | 0.530 | 1.953 | 0.210 | 1.516 | 0.0199 |
| 833.5931 | 1201 | 14.601 | 1.100 | 9.580 | 0.695 | 1.524 | 0.0221 |
| 539.4286 | 1204 | 1.364 | 0.195 | 1.714 | 0.365 | 0.795 | 0.0222 |
| 428.3653 | 1201 | 9.177 | 0.839 | 5.455 | 0.349 | 1.682 | 0.024 |
| 634.3951 | 1204 | 5.491 | 0.664 | 8.401 | 0.739 | 0.654 | 0.0305 |
| 662.4267 | 1204 | 4.861 | 0.451 | 6.334 | 0.360 | 0.768 | 0.0305 |
| 274.1777 | 1203 | 1.377 | 0.177 | 2.279 | 0.299 | 0.604 | 0.0311 |
| 835.6094 | 1201 | 5.680 | 0.531 | 3.714 | 0.244 | 1.529 | 0.0314 |
| 780.5303 | 1204 | 7.194 | 0.535 | 9.693 | 0.351 | 0.742 | 0.033 |
| 793.4936 | 1204 | 37.612 | 3.233 | 35.440 | 1.943 | 1.061 | 0.0353 |
| 368.1656 | 1102 | 1.679 | 0.306 | 3.982 | 0.611 | 0.422 | 0.0354 |
| 646.5702 | 1203 | 6.529 | 0.770 | 7.797 | 0.471 | 0.837 | 0.0375 |
| 632.5038 | 1204 | 2.055 | 0.470 | 3.021 | 0.265 | 0.680 | 0.0379 |
| 729.5727 | 1204 | 6.518 | 0.512 | 9.419 | 0.497 | 0.692 | 0.0397 |
| 806.5643 | 1201 | 27.948 | 1.637 | 19.725 | 1.161 | 1.417 | 0.0444 |
| 786.51 | 1204 | 31.030 | 2.459 | 41.726 | 1.808 | 0.744 | 0.0451 |
| 805.5609 | 1201 | 55.027 | 3.212 | 38.808 | 2.258 | 1.418 | 0.0463 |
| 541.3141 | 1201 | 2.734 | 0.234 | 2.773 | 0.153 | 0.986 | 0.0464 |
| 856.6045 | 1102 | 7.004 | 0.557 | 11.014 | 0.891 | 0.636 | 0.0464 |
| 366.3284 | 1203 | 21.097 | 1.078 | 26.496 | 0.868 | 0.796 | 0.0474 |
| 501.3217 | 1201 | 5.313 | 0.419 | 3.495 | 0.214 | 1.520 | 0.0498 |

**Table 5: Accurate mass features differing between clinically diagnosed RR- MULTIPLE SCLEROSIS patients transitioning to SP- MULTIPLE SCLEROSIS and RR-MULTIPLE SCLEROSIS patients (p<0.05).**

| | | | | | | | |
|---|---|---|---|---|---|---|---|
| 580.5089 | 1204 | 7.612 | 0.633 | 13.528 | 0.711 | 0.563 | 2.52E-15 |
| 452.3868 | 1204 | 2.747 | 0.171 | 3.906 | 0.137 | 0.703 | 6.69E-15 |
| 522.4313 | 1204 | 10.338 | 0.808 | 15.695 | 0.623 | 0.659 | 8.43E-15 |
| 578.4923 | 1204 | 22.148 | 2.145 | 40.496 | 2.263 | 0.547 | 9.92E-15 |
| 450.3729 | 1204 | 6.629 | 0.358 | 9.090 | 0.331 | 0.729 | 1.01E-14 |
| 579.4958 | 1204 | 8.772 | 0.838 | 15.978 | 0.888 | 0.549 | 1.08E-14 |
| 581.5126 | 1204 | 3.136 | 0.254 | 5.487 | 0.296 | 0.571 | 1.80E-14 |
| 484.3788 | 1204 | 5.246 | 0.379 | 8.180 | 0.389 | 0.641 | 1.89E-14 |
| 466.3656 | 1204 | 9.116 | 0.702 | 13.494 | 0.566 | 0.676 | 2.23E-14 |
| 494.3968 | 1204 | 9.307 | 0.747 | 14.131 | 0.626 | 0.659 | 3.02E-14 |
| 550.4602 | 1204 | 6.431 | 0.504 | 10.852 | 0.521 | 0.593 | 3.96E-14 |
| 523.4337 | 1204 | 3.432 | 0.263 | 5.249 | 0.219 | 0.654 | 4.34E-14 |
| 510.3937 | 1204 | 4.078 | 0.340 | 6.276 | 0.278 | 0.650 | 5.04E-14 |
| 495.4018 | 1204 | 2.979 | 0.244 | 4.575 | 0.202 | 0.651 | 8.95E-14 |
| 512.4079 | 1204 | 9.451 | 0.830 | 15.906 | 0.838 | 0.594 | 2.38E-13 |
| 448.3562 | 1204 | 7.324 | 0.439 | 10.433 | 0.390 | 0.702 | 3.03E-13 |
| 552.4784 | 1204 | 4.480 | 0.334 | 8.282 | 0.478 | 0.541 | 4.73E-13 |
| 524.4448 | 1204 | 3.500 | 0.239 | 5.382 | 0.265 | 0.650 | 5.83E-13 |
| 536.41 | 1204 | 7.097 | 0.572 | 10.884 | 0.483 | 0.652 | 6.16E-13 |
| 568.4723 | 1204 | 7.427 | 0.835 | 13.607 | 0.761 | 0.546 | 9.45E-13 |
| 577.4795 | 1204 | 10.116 | 1.038 | 16.759 | 0.829 | 0.604 | 9.63E-13 |
| 576.4757 | 1204 | 26.727 | 2.897 | 45.354 | 2.261 | 0.589 | 1.30E-12 |
| 467.3711 | 1204 | 3.093 | 0.231 | 4.437 | 0.193 | 0.697 | 1.48E-12 |
| 468.384 | 1204 | 12.450 | 0.935 | 18.294 | 0.792 | 0.681 | 1.56E-12 |
| 493.385 | 1204 | 2.195 | 0.216 | 3.412 | 0.131 | 0.643 | 1.71E-12 |
| 513.4116 | 1204 | 3.325 | 0.286 | 5.401 | 0.281 | 0.616 | 1.91E-12 |
| 521.4188 | 1204 | 3.983 | 0.338 | 5.796 | 0.222 | 0.687 | 2.15E-12 |
| 596.5053 | 1202 | 7.401 | 1.039 | 15.297 | 0.951 | 0.484 | 2.15E-12 |
| 594.4875 | 1202 | 5.256 | 0.642 | 9.946 | 0.526 | 0.528 | 3.39E-12 |
| 537.4142 | 1204 | 2.863 | 0.247 | 4.317 | 0.185 | 0.663 | 4.81E-12 |
| 548.4438 | 1204 | 4.947 | 0.496 | 7.407 | 0.309 | 0.668 | 5.93E-12 |
| 469.3863 | 1204 | 3.607 | 0.253 | 5.002 | 0.209 | 0.721 | 7.00E-12 |
| 440.3526 | 1204 | 3.040 | 0.235 | 4.382 | 0.192 | 0.694 | 7.60E-12 |
| 551.4646 | 1204 | 2.450 | 0.191 | 3.993 | 0.192 | 0.614 | 8.25E-12 |
| 597.5068 | 1202 | 2.933 | 0.380 | 5.788 | 0.369 | 0.507 | 9.90E-12 |
| 569.4769 | 1204 | 2.998 | 0.330 | 5.444 | 0.298 | 0.551 | 1.02E-11 |
| 520.4131 | 1204 | 11.310 | 0.945 | 15.943 | 0.634 | 0.709 | 1.03E-11 |
| 566.454 | 1204 | 5.597 | 0.588 | 8.691 | 0.393 | 0.644 | 1.22E-11 |
| 492.3832 | 1204 | 6.447 | 0.555 | 9.311 | 0.399 | 0.692 | 1.26E-11 |
| 598.5107 | 1204 | 7.070 | 0.979 | 14.230 | 1.030 | 0.497 | 1.80E-11 |
| 595.4883 | 1204 | 25.969 | 3.279 | 46.028 | 2.536 | 0.564 | 1.93E-11 |
| 595.4928 | 1202 | 2.356 | 0.274 | 4.218 | 0.215 | 0.558 | 2.23E-11 |
| 594.4848 | 1204 | 65.843 | 8.436 | 115.274 | 6.359 | 0.571 | 2.35E-11 |
| 591.4614 | 1204 | 2.460 | 0.213 | 3.990 | 0.220 | 0.617 | 2.90E-11 |
| 482.3604 | 1204 | 3.414 | 0.245 | 5.143 | 0.245 | 0.664 | 3.32E-11 |
| 576.4765 | 1202 | 2.471 | 0.241 | 4.415 | 0.265 | 0.560 | 3.37E-11 |
| 476.3869 | 1204 | 3.426 | 0.183 | 4.685 | 0.186 | 0.731 | 3.85E-11 |
| 496.4157 | 1204 | 6.968 | 0.586 | 10.685 | 0.591 | 0.652 | 4.23E-11 |
| 508.3782 | 1204 | 3.587 | 0.251 | 5.241 | 0.265 | 0.684 | 4.43E-11 |
| 464.3524 | 1204 | 6.563 | 0.496 | 9.142 | 0.365 | 0.718 | 5.30E-11 |
| 618.4834 | 1201 | 2.975 | 0.365 | 6.236 | 0.449 | 0.477 | 7.22E-11 |
| 590.4585 | 1204 | 6.938 | 0.556 | 11.249 | 0.631 | 0.617 | 7.43E-11 |
| 597.5062 | 1204 | 32.711 | 4.840 | 63.633 | 4.798 | 0.514 | 8.79E-11 |
| 438.3354 | 1204 | 2.443 | 0.185 | 3.426 | 0.150 | 0.713 | 1.03E-10 |
| 541.4422 | 1204 | 7.441 | 0.963 | 12.302 | 0.734 | 0.605 | 1.26E-10 |
| 596.5012 | 1204 | 89.261 | 13.368 | 178.485 | 14.249 | 0.500 | 1.62E-10 |
| 540.4387 | 1204 | 21.969 | 2.791 | 36.081 | 2.150 | 0.609 | 1.82E-10 |
| 564.4396 | 1204 | 2.322 | 0.202 | 3.584 | 0.173 | 0.648 | 2.11E-10 |
| 538.4257 | 1204 | 19.272 | 2.025 | 29.559 | 1.464 | 0.652 | 2.93E-10 |
| 592.4717 | 1204 | 21.832 | 2.186 | 38.114 | 2.424 | 0.573 | 3.20E-10 |
| 593.4736 | 1204 | 8.928 | 0.908 | 15.915 | 1.032 | 0.561 | 3.21E-10 |
| 539.4274 | 1204 | 5.471 | 0.803 | 9.733 | 0.636 | 0.562 | 4.48E-10 |
| 534.3912 | 1204 | 3.038 | 0.259 | 4.595 | 0.215 | 0.661 | 5.76E-10 |
| 518.3969 | 1204 | 8.400 | 0.712 | 12.262 | 0.569 | 0.685 | 6.53E-10 |
| 532.4503 | 1204 | 3.489 | 0.190 | 4.752 | 0.228 | 0.734 | 7.60E-10 |
| 610.482 | 1204 | 5.842 | 0.532 | 8.903 | 0.494 | 0.656 | 8.00E-10 |
| 616.4675 | 1201 | 2.688 | 0.263 | 4.851 | 0.305 | 0.554 | 8.68E-10 |
| 462.3346 | 1204 | 2.692 | 0.199 | 3.632 | 0.138 | 0.741 | 9.35E-10 |
| 480.3473 | 1204 | 2.852 | 0.191 | 3.911 | 0.157 | 0.729 | 1.26E-09 |
| 446.341 | 1204 | 9.664 | 0.647 | 13.291 | 0.599 | 0.727 | 2.74E-09 |
| 504.4188 | 1204 | 4.955 | 0.270 | 6.780 | 0.309 | 0.731 | 3.65E-09 |
| 478.4044 | 1204 | 2.730 | 0.154 | 3.682 | 0.161 | 0.742 | 5.27E-09 |
| 570.4903 | 1204 | 2.348 | 0.203 | 4.480 | 0.352 | 0.524 | 6.68E-09 |
| 560.4821 | 1204 | 17.670 | 1.056 | 24.793 | 1.209 | 0.713 | 1.21E-08 |
| 502.4054 | 1204 | 4.726 | 0.317 | 6.631 | 0.298 | 0.713 | 1.51E-08 |
| 561.4863 | 1204 | 7.248 | 0.403 | 9.904 | 0.461 | 0.732 | 1.82E-08 |
| 490.3676 | 1204 | 4.358 | 0.414 | 6.198 | 0.296 | 0.703 | 2.12E-08 |
| 574.4594 | 1204 | 19.303 | 2.070 | 32.679 | 2.206 | 0.591 | 2.38E-08 |
| 575.4628 | 1204 | 7.733 | 0.793 | 12.808 | 0.831 | 0.604 | 3.58E-08 |
| 546.4298 | 1204 | 3.032 | 0.284 | 4.374 | 0.245 | 0.693 | 4.07E-08 |
| 574.4635 | 1202 | 1.927 | 0.171 | 3.453 | 0.242 | 0.558 | 4.32E-08 |
| 519.3998 | 1204 | 3.134 | 0.264 | 4.275 | 0.202 | 0.733 | 6.07E-08 |
| 572.4455 | 1204 | 3.261 | 0.227 | 4.775 | 0.263 | 0.683 | 8.21E-08 |
| 506.4338 | 1204 | 2.578 | 0.156 | 3.502 | 0.168 | 0.736 | 1.86E-07 |
| 474.3731 | 1204 | 3.602 | 0.287 | 4.916 | 0.233 | 0.733 | 6.45E-07 |
| 558.4663 | 1202 | 2.842 | 0.237 | 4.836 | 0.312 | 0.588 | 8.38E-07 |
| 559.4688 | 1204 | 10.587 | 0.932 | 15.071 | 0.830 | 0.703 | 9.35E-07 |
| 447.3433 | 1204 | 2.967 | 0.213 | 4.068 | 0.211 | 0.729 | 1.19E-06 |
| 562.4989 | 1204 | 9.973 | 0.554 | 12.437 | 0.512 | 0.802 | 1.25E-06 |
| 558.4649 | 1204 | 27.632 | 2.484 | 39.813 | 2.242 | 0.694 | 1.66E-06 |
| 534.4645 | 1204 | 3.046 | 0.155 | 3.904 | 0.180 | 0.780 | 1.89E-06 |
| 556.4497 | 1204 | 7.612 | 0.401 | 10.976 | 0.668 | 0.694 | 2.99E-06 |
| 557.4527 | 1204 | 3.388 | 0.213 | 4.409 | 0.298 | 0.769 | 6.92E-06 |
| 563.5013 | 1204 | 3.903 | 0.201 | 4.830 | 0.203 | 0.808 | 1.74E-05 |
| 530.4379 | 1204 | 4.240 | 0.273 | 5.085 | 0.240 | 0.834 | 0.0001 |
| 590.4964 | 1204 | 4.097 | 0.371 | 4.965 | 0.456 | 0.825 | 0.0003 |
| 784.6228 | 1204 | 19.037 | 1.831 | 10.803 | 1.101 | 1.762 | 0.0004 |
| 612.4994 | 1204 | 4.706 | 0.316 | 6.110 | 0.396 | 0.770 | 0.0005 |
| 327.0307 | 1204 | 7.030 | 0.236 | 7.511 | 0.224 | 0.936 | 0.0012 |
| 462.3716 | 1204 | 2.793 | 0.143 | 3.007 | 0.090 | 0.929 | 0.0012 |
| 783.6174 | 1204 | 30.598 | 3.318 | 16.139 | 1.805 | 1.896 | 0.0014 |
| 816.5159 | 1204 | 8.856 | 0.270 | 8.243 | 0.279 | 1.074 | 0.0027 |
| 560.478 | 1203 | 6.582 | 0.404 | 8.188 | 0.375 | 0.804 | 0.0031 |
| 244.2189 | 1203 | 6.901 | 0.266 | 7.322 | 0.161 | 0.943 | 0.0034 |
| 333.9539 | 1102 | 3.572 | 0.276 | 3.748 | 0.334 | 0.953 | 0.0047 |
| 744.55 | 1203 | 6.217 | 0.552 | 7.484 | 0.600 | 0.831 | 0.0079 |
| 747.5121 | 1204 | 54.149 | 2.246 | 45.567 | 2.452 | 1.188 | 0.0096 |
| 564.513 | 1204 | 4.140 | 0.278 | 4.456 | 0.242 | 0.929 | 0.0099 |
| 779.5828 | 1204 | 20.561 | 1.093 | 23.925 | 1.363 | 0.859 | 0.01 |
| 832.5211 | 1204 | 6.584 | 0.407 | 5.016 | 0.421 | 1.313 | 0.0101 |
| 743.5475 | 1203 | 14.077 | 1.093 | 16.578 | 1.335 | 0.849 | 0.0102 |
| 260.2137 | 1203 | 5.339 | 0.154 | 5.622 | 0.119 | 0.950 | 0.0106 |
| 828.5477 | 1201 | 5.936 | 0.361 | 5.534 | 0.275 | 1.073 | 0.0132 |
| 246.2345 | 1203 | 7.833 | 0.456 | 8.467 | 0.280 | 0.925 | 0.0143 |
| 584.2641 | 1202 | 4.241 | 0.433 | 5.003 | 0.562 | 0.848 | 0.015 |
| 821.5288 | 1204 | 18.114 | 1.077 | 15.703 | 0.731 | 1.154 | 0.0151 |
| 216.1877 | 1203 | 7.189 | 0.385 | 7.496 | 0.290 | 0.959 | 0.0166 |
| 831.5758 | 1201 | 16.234 | 0.935 | 16.394 | 0.762 | 0.990 | 0.017 |
| 239.939 | 1102 | 4.791 | 0.275 | 4.879 | 0.341 | 0.982 | 0.0174 |
| 830.5634 | 1201 | 5.708 | 0.313 | 5.574 | 0.235 | 1.024 | 0.0198 |
| 726.5438 | 1204 | 5.481 | 0.552 | 7.236 | 0.574 | 0.757 | 0.0201 |
| 214.1721 | 1203 | 9.114 | 0.523 | 9.867 | 0.358 | 0.924 | 0.0206 |
| 823.5427 | 1204 | 9.742 | 0.414 | 8.824 | 0.338 | 1.104 | 0.0242 |
| 200.1566 | 1203 | 7.319 | 0.271 | 7.980 | 0.158 | 0.917 | 0.0251 |
| 610.5204 | 1204 | 12.409 | 1.532 | 13.914 | 1.696 | 0.892 | 0.0258 |
| 839.6019 | 1202 | 7.634 | 0.269 | 8.499 | 0.363 | 0.898 | 0.0264 |
| 277.8861 | 1101 | 13.450 | 0.560 | 11.133 | 0.491 | 1.208 | 0.0276 |
| 303.108 | 1202 | 33.776 | 3.719 | 37.528 | 3.650 | 0.900 | 0.0286 |
| 731.5464 | 1201 | 4.127 | 0.502 | 3.918 | 0.335 | 1.053 | 0.0292 |
| 181.9806 | 1102 | 5.275 | 0.323 | 5.215 | 0.335 | 1.011 | 0.0298 |
| 188.1567 | 1203 | 9.264 | 0.414 | 9.796 | 0.299 | 0.946 | 0.0302 |
| 834.5372 | 1204 | 16.761 | 0.942 | 13.473 | 0.712 | 1.244 | 0.0311 |
| 781.6001 | 1204 | 8.456 | 0.370 | 9.429 | 0.471 | 0.897 | 0.0316 |
| 835.5417 | 1204 | 9.435 | 0.518 | 7.667 | 0.387 | 1.231 | 0.0327 |
| 202.1721 | 1203 | 9.681 | 0.507 | 10.182 | 0.375 | 0.951 | 0.0335 |
| 345.8738 | 1101 | 6.088 | 0.293 | 5.198 | 0.260 | 1.171 | 0.0344 |
| 331.957 | 1102 | 3.787 | 0.310 | 3.746 | 0.376 | 1.011 | 0.0373 |
| 546.3413 | 1204 | 3.436 | 0.266 | 3.807 | 0.217 | 0.903 | 0.0374 |
| 813.5871 | 1202 | 5.325 | 0.192 | 6.026 | 0.297 | 0.884 | 0.038 |
| 378.9906 | 1204 | 3.925 | 0.109 | 4.124 | 0.106 | 0.952 | 0.0398 |
| 718.4736 | 1204 | 6.848 | 0.489 | 5.319 | 0.469 | 1.287 | 0.0401 |
| 384.3399 | 1203 | 68.518 | 2.885 | 70.221 | 1.973 | 0.976 | 0.0403 |
| 804.5476 | 1201 | 16.738 | 1.052 | 16.471 | 0.725 | 1.016 | 0.043 |
| 174.1411 | 1203 | 6.924 | 0.220 | 7.701 | 0.168 | 0.899 | 0.0441 |
| 780.5872 | 1204 | 10.743 | 0.554 | 12.203 | 0.690 | 0.880 | 0.0443 |
| 793.4936 | 1204 | 40.783 | 2.537 | 35.440 | 1.943 | 1.151 | 0.0443 |
| 834.5963 | 1201 | 5.412 | 0.351 | 4.956 | 0.368 | 1.092 | 0.0476 |
| 541.3141 | 1201 | 2.684 | 0.174 | 2.773 | 0.153 | 0.968 | 0.048 |

**Table 6: Accurate mass features differing between clinically diagnosed RR- MULTIPLE SCLEROSIS patients transitioning to SP- MULTIPLE SCLEROSIS and SP-MULTIPLE SCLEROSIS patients (p<0.05).**

| | | | | | | | |
|---|---|---|---|---|---|---|---|
| 541.3141 | 1201 | 2.876 | 0.170 | 2.734 | 0.234 | 1.052 | 0.0007 |
| 567.3547 | 1102 | 12.556 | 0.589 | 7.173 | 0.794 | 1.750 | 0.0022 |
| 239.939 | 1102 | 4.477 | 0.259 | 4.397 | 0.495 | 1.018 | 0.0034 |
| 872.6715 | 1204 | 2.361 | 0.259 | 4.860 | 0.498 | 0.486 | 0.0052 |
| 555.3102 | 1102 | 6.389 | 0.294 | 3.908 | 0.545 | 1.635 | 0.0061 |
| 760.5231 | 1204 | 95.686 | 4.233 | 54.548 | 5.832 | 1.754 | 0.0067 |
| 761.529 | 1204 | 40.261 | 1.759 | 24.002 | 2.250 | 1.677 | 0.0071 |
| 788.5549 | 1204 | 20.553 | 0.816 | 13.393 | 0.984 | 1.535 | 0.0075 |
| 566.3431 | 1102 | 7.397 | 0.292 | 4.439 | 0.519 | 1.666 | 0.0081 |
| 786.5408 | 1204 | 114.078 | 4.957 | 69.256 | 6.251 | 1.647 | 0.0081 |
| 565.3391 | 1102 | 24.577 | 1.046 | 14.619 | 1.846 | 1.681 | 0.0084 |
| 784.5238 | 1204 | 91.976 | 3.932 | 55.254 | 6.193 | 1.665 | 0.0099 |
| 783.6174 | 1204 | 32.380 | 3.488 | 8.590 | 2.388 | 3.769 | 0.0106 |
| 746.5118 | 1204 | 74.992 | 3.148 | 46.823 | 4.708 | 1.602 | 0.0107 |
| 303.1081 | 1102 | 4.422 | 0.334 | 2.767 | 0.507 | 1.598 | 0.0108 |
| 249.9677 | 1102 | 6.051 | 0.305 | 5.069 | 0.455 | 1.194 | 0.0115 |
| 787.5452 | 1204 | 52.453 | 2.226 | 32.632 | 2.794 | 1.607 | 0.0124 |
| 305.8792 | 1102 | 6.257 | 0.388 | 3.671 | 0.627 | 1.704 | 0.0125 |
| 784.6228 | 1204 | 20.109 | 1.910 | 6.221 | 1.486 | 3.232 | 0.0125 |
| 684.6037 | 1204 | 2.308 | 0.286 | 5.278 | 0.564 | 0.437 | 0.0145 |
| 785.5287 | 1204 | 46.467 | 1.990 | 27.795 | 3.186 | 1.672 | 0.0157 |
| 718.4736 | 1204 | 6.929 | 0.499 | 4.007 | 0.540 | 1.729 | 0.0175 |
| 770.5108 | 1204 | 90.825 | 3.423 | 61.142 | 5.177 | 1.485 | 0.0185 |
| 331.957 | 1102 | 3.358 | 0.274 | 3.566 | 0.398 | 0.942 | 0.0212 |
| 808.5225 | 1204 | 43.754 | 2.334 | 26.220 | 2.895 | 1.669 | 0.0215 |
| 633.3232 | 1102 | 4.455 | 0.197 | 2.881 | 0.303 | 1.546 | 0.025 |
| 809.5264 | 1204 | 22.485 | 1.215 | 13.576 | 1.484 | 1.656 | 0.0258 |
| 333.9539 | 1102 | 3.182 | 0.238 | 3.120 | 0.333 | 1.020 | 0.0276 |
| 772.5265 | 1204 | 117.162 | 4.503 | 81.159 | 5.960 | 1.444 | 0.028 |
| 733.501 | 1204 | 31.675 | 1.880 | 16.611 | 2.461 | 1.907 | 0.0297 |
| 747.5121 | 1204 | 54.603 | 2.277 | 41.134 | 3.281 | 1.327 | 0.0317 |
| 246.1468 | 1201 | 4.033 | 0.358 | 6.273 | 0.755 | 0.643 | 0.0334 |
| 828.7213 | 1201 | 2.551 | 0.267 | 3.744 | 0.802 | 0.681 | 0.0346 |
| 856.7527 | 1201 | 9.061 | 1.308 | 14.037 | 3.456 | 0.645 | 0.0369 |
| 617.0921 | 1204 | 277.341 | 10.025 | 201.035 | 13.034 | 1.380 | 0.0378 |
| 574.4958 | 1201 | 6.712 | 0.792 | 12.295 | 2.617 | 0.546 | 0.0382 |
| 742.4745 | 1204 | 10.479 | 0.392 | 7.859 | 0.576 | 1.333 | 0.0398 |
| 716.4987 | 1204 | 25.168 | 1.344 | 20.579 | 1.653 | 1.223 | 0.0403 |
| 757.5008 | 1204 | 47.638 | 2.832 | 25.711 | 4.126 | 1.853 | 0.0403 |
| 854.737 | 1201 | 6.333 | 0.933 | 10.183 | 2.684 | 0.622 | 0.0403 |
| 379.2536 | 1204 | 3.069 | 0.171 | 1.706 | 0.224 | 1.799 | 0.0454 |
| 734.508 | 1204 | 14.722 | 1.041 | 6.522 | 1.436 | 2.257 | 0.0475 |

**Table 7: Metabolites identified in first principle component analysis for RR-multiple sclerosis.**

| | | | | | | | |
|---|---|---|---|---|---|---|---|
| 540.4387 | 1204 | 36.603 | 2.086 | 22.346 | 1.749 | 1.638 | 5.92E-07 |
| 578.4923 | 1204 | 41.017 | 2.169 | 26.563 | 2.068 | 1.544 | 1.37E-06 |
| 596.5012 | 1204 | 181.033 | 13.876 | 108.540 | 10.921 | 1.668 | 3.44E-05 |
| 597.5062 | 1204 | 64.543 | 4.659 | 39.473 | 3.816 | 1.635 | 3.06E-05 |
| 594.4848 | 1204 | 116.663 | 6.054 | 80.027 | 6.363 | 1.458 | 0.0001 |

**Table 8: Expanded set of metabolites identified in second PAM analysis for RR-multiple sclerosis.**

| | | | | | | | |
|---|---|---|---|---|---|---|---|
| 540.4387 | 1204 | 36.603 | 2.086 | 22.346 | 1.749 | 1.638 | 5.92E-07 |
| 538.4257 | 1204 | 30.014 | 1.397 | 21.271 | 1.294 | 1.411 | 9.34E-06 |
| 594.4848 | 1204 | 116.663 | 6.054 | 80.027 | 6.363 | 1.458 | 0.0001 |
| 578.4923 | 1204 | 41.017 | 2.169 | 26.563 | 2.068 | 1.544 | 1.37E-06 |
| 596.5012 | 1204 | 181.033 | 13.876 | 108.540 | 10.921 | 1.668 | 3.44E-05 |
| 468.384 | 1204 | 18.514 | 0.778 | 12.977 | 0.754 | 1.427 | 2.69E-07 |
| 595.4883 | 1204 | 46.584 | 2.416 | 32.020 | 2.556 | 1.455 | 0.0001 |
| 597.5062 | 1204 | 64.543 | 4.659 | 39.473 | 3.816 | 1.635 | 3.06E-05 |
| 384.3399 | 1203 | 69.859 | 1.997 | 62.624 | 1.789 | 1.116 | 0.0005 |
| 576.4757 | 1204 | 45.791 | 2.161 | 33.088 | 2.440 | 1.384 | 0.0001 |
| 763.5153 | 1204 | 21.461 | 2.719 | 12.389 | 1.529 | 1.732 | 0.0008 |
| 541.4422 | 1204 | 12.488 | 0.710 | 7.651 | 0.568 | 1.632 | 4.16E-07 |
| 522.4313 | 1204 | 15.891 | 0.597 | 11.438 | 0.681 | 1.389 | 9.00E-07 |
| 496.4157 | 1204 | 10.848 | 0.581 | 6.751 | 0.455 | 1.607 | 3.72E-08 |
| 765.5316 | 1204 | 32.360 | 2.588 | 24.079 | 1.770 | 1.344 | 0.0022 |
| 745.5643 | 1204 | 120.519 | 6.555 | 105.213 | 5.381 | 1.145 | 0.0068 |

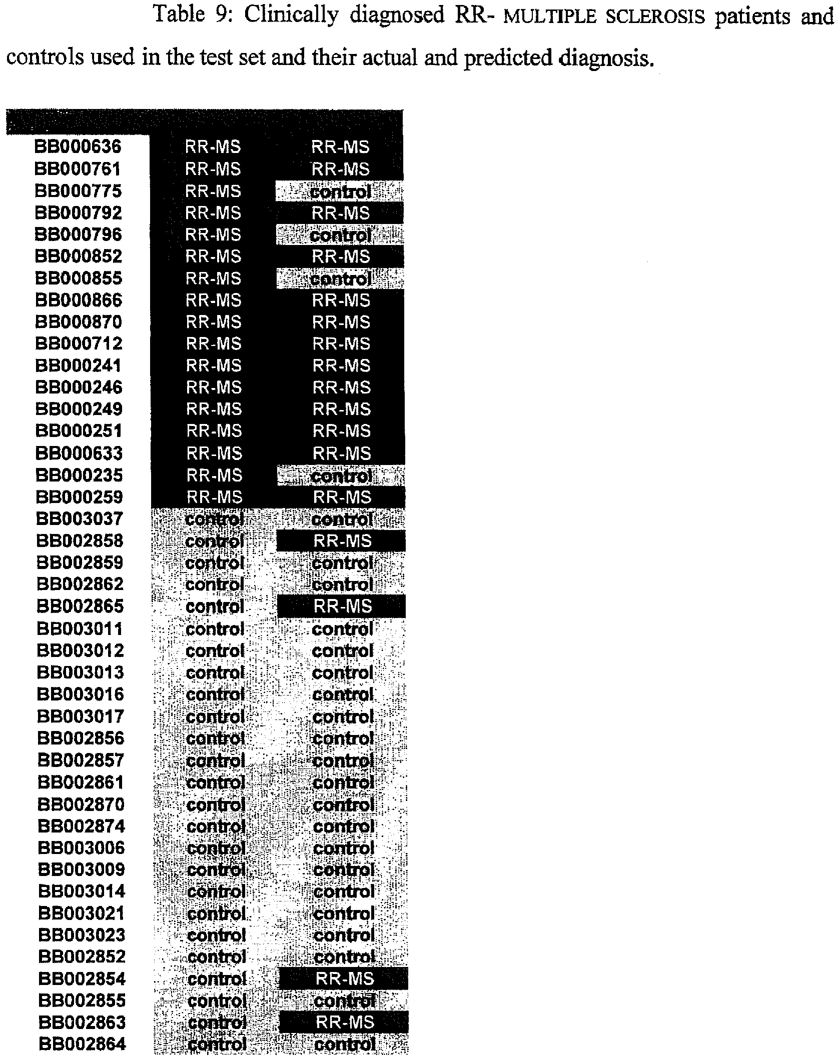

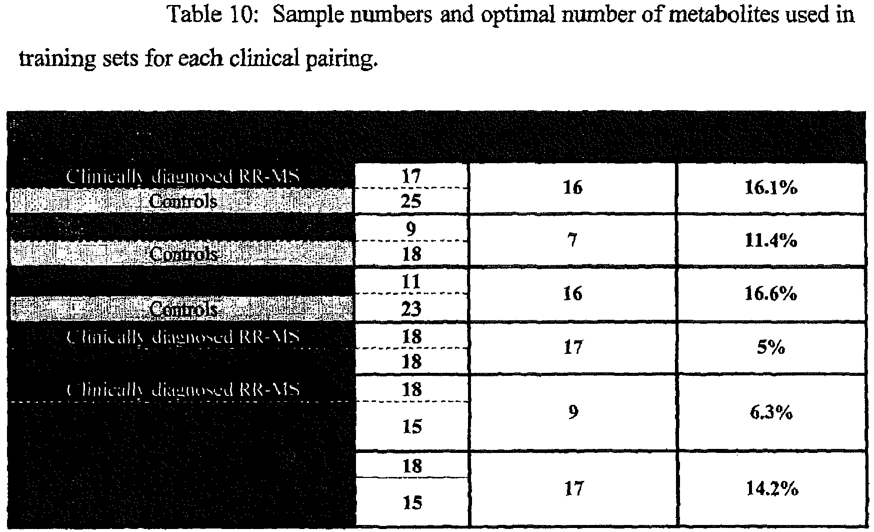

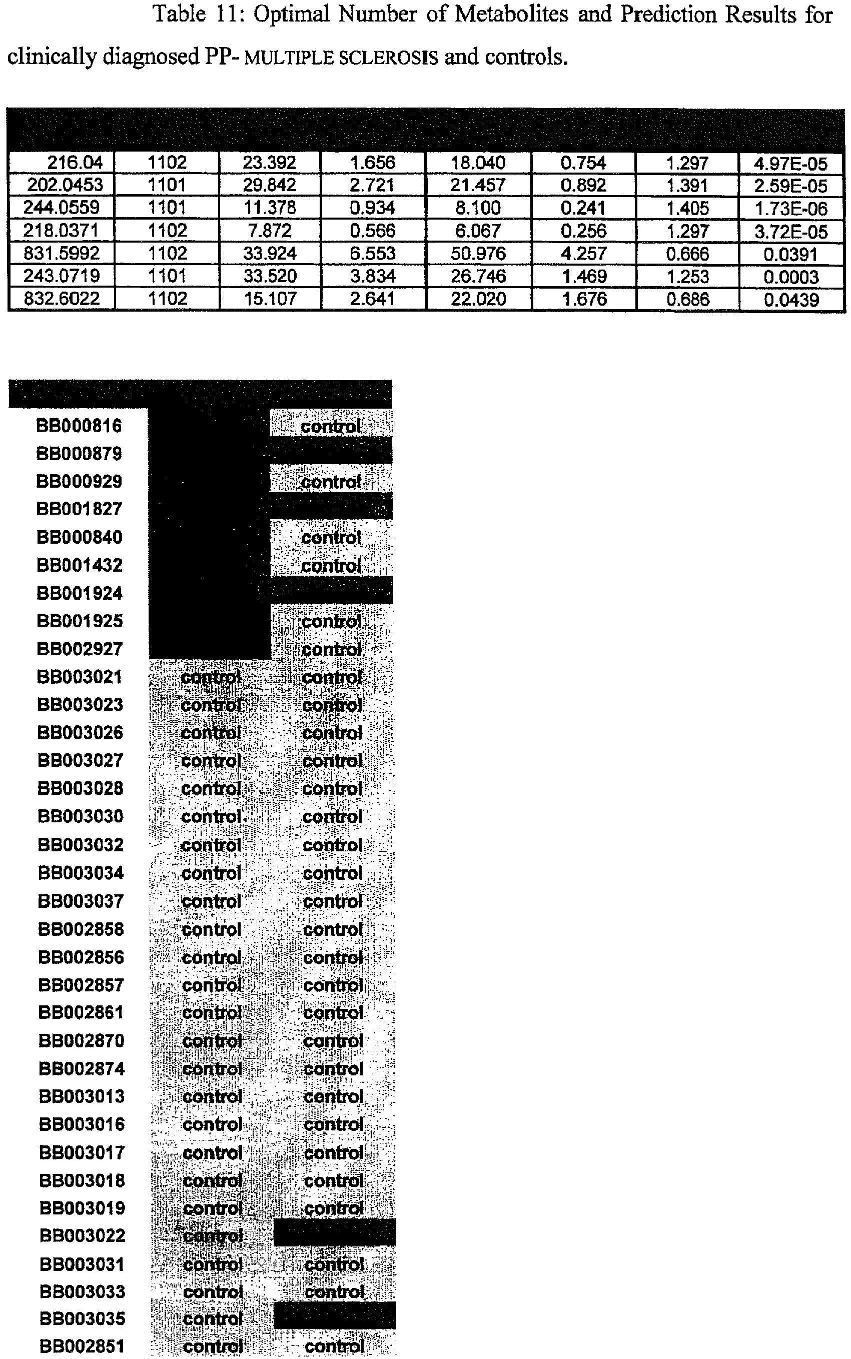

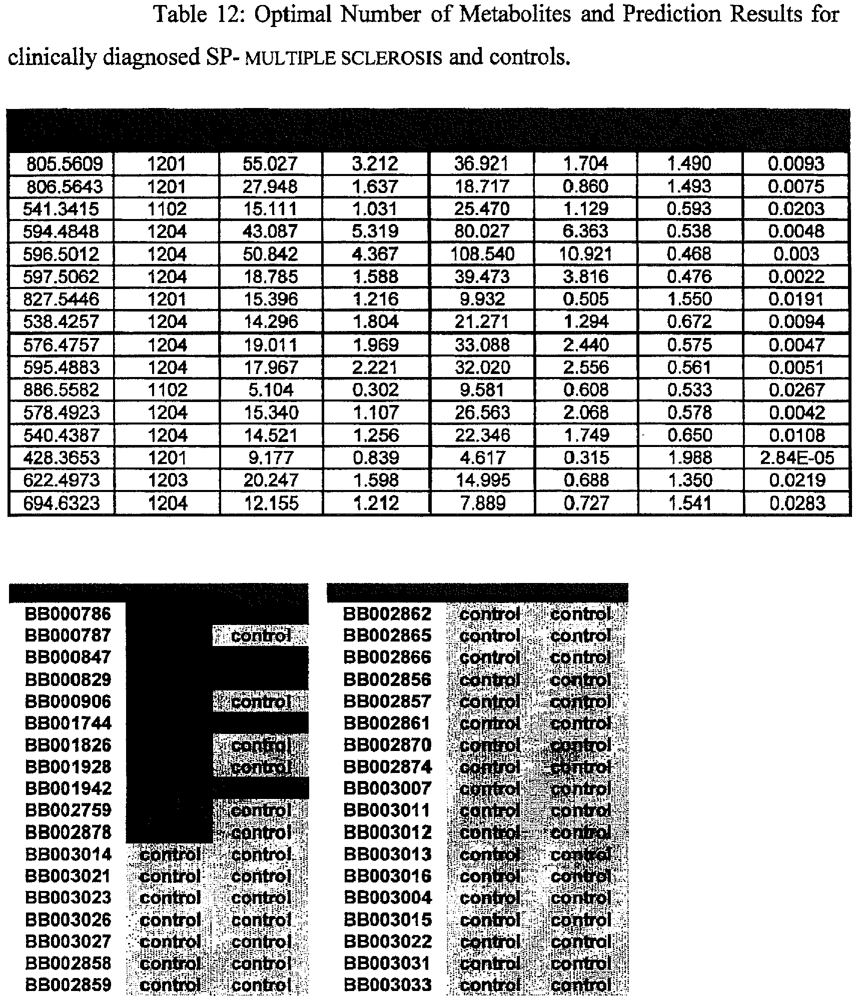

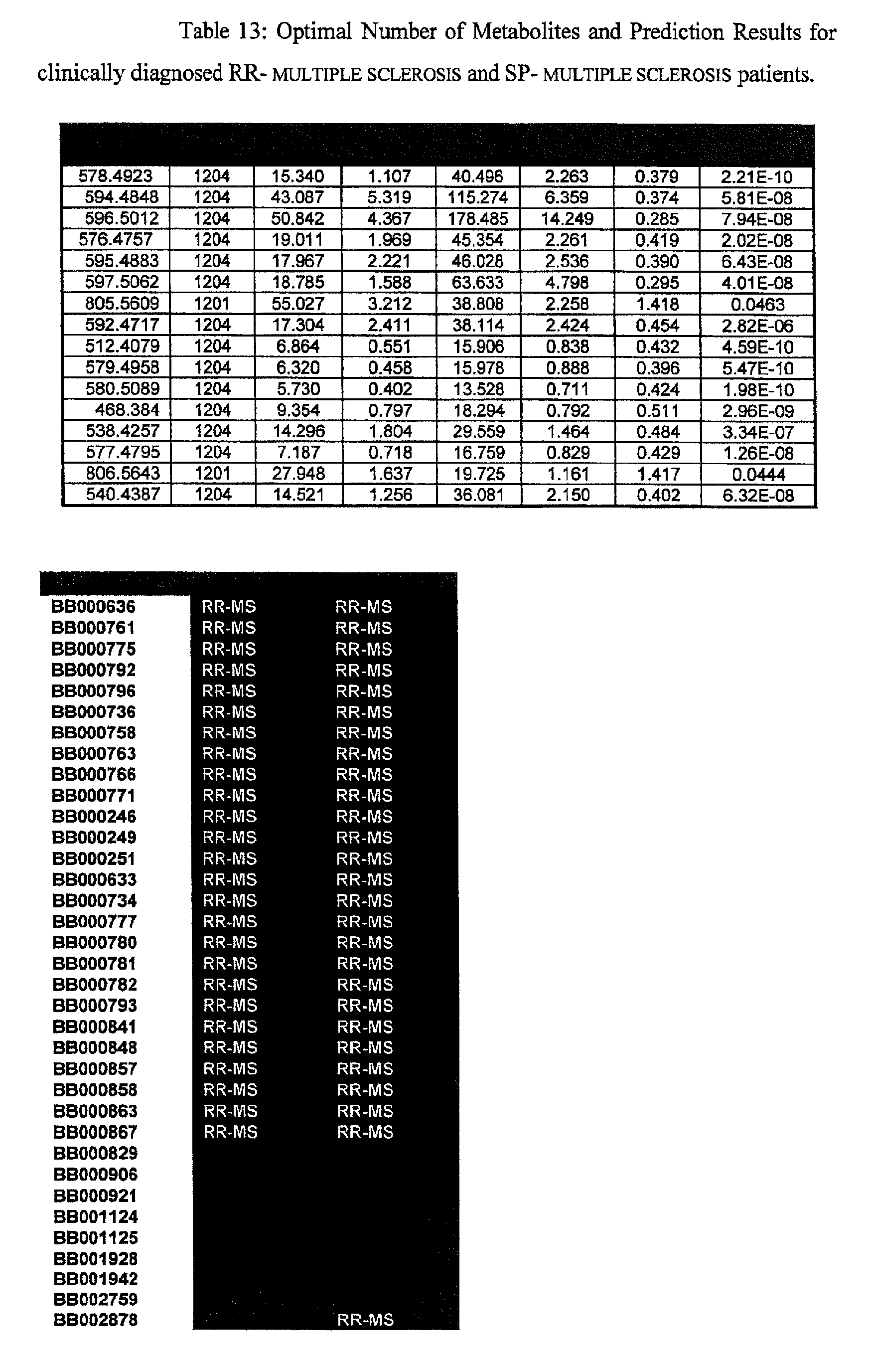

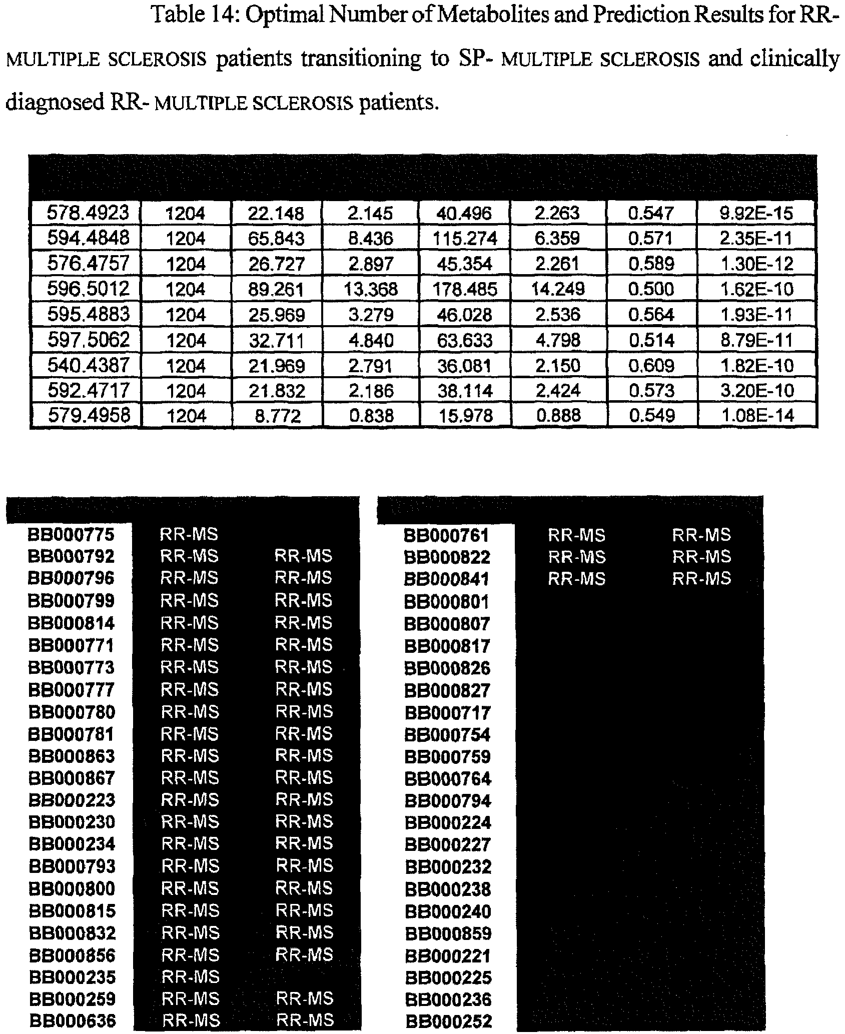

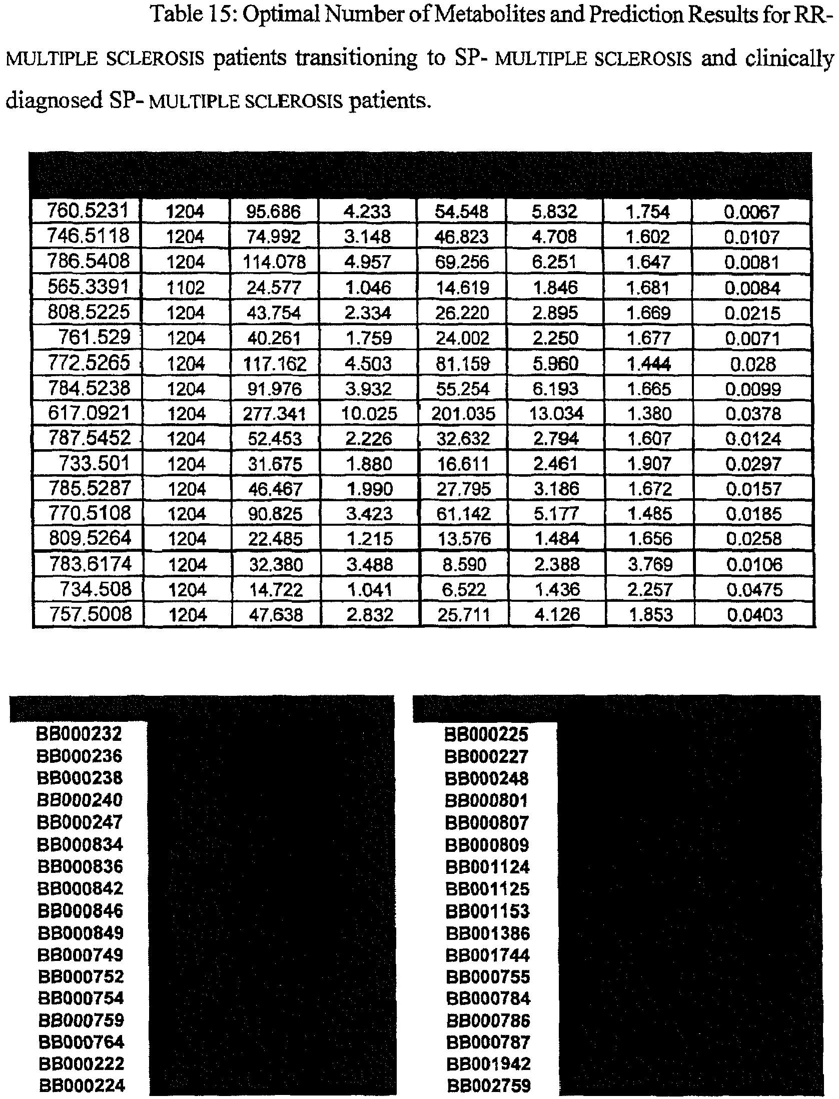

**Table 16: Accurate mass features differing between 10 clinically diagnosed RR- MULTIPLE SCLEROSIS patients and 10 controls (p<0.05).**

| | | | | | | | |
|---|---|---|---|---|---|---|---|
| 450.3729 | 1204 | 11.764 | 0.454 | 4.823 | 0.364 | 2.439 | 1.44E-10 |
| 512.3347 | 1201 | 3.928 | 0.689 | 2.788 | 0.548 | 1.409 | 1.44E-10 |
| 580.5089 | 1204 | 20.678 | 1.357 | 4.648 | 0.571 | 4.449 | 5.92E-10 |
| 513.4116 | 1204 | 7.981 | 0.438 | 2.392 | 0.342 | 3.337 | 1.89E-09 |
| 578.4923 | 1204 | 63.796 | 4.758 | 13.250 | 1.859 | 4.815 | 2.05E-09 |
| 579.4958 | 1204 | 24.980 | 1.842 | 5.626 | 0.692 | 4.440 | 3.44E-09 |
| 452.3868 | 1204 | 5.142 | 0.235 | 1.726 | 0.172 | 2.979 | 4.16E-09 |
| 581.5126 | 1204 | 8.401 | 0.625 | 1.791 | 0.257 | 4.691 | 5.10E-09 |
| 541.4422 | 1204 | 16.895 | 0.945 | 4.594 | 0.763 | 3.678 | 7.55E-09 |
| 596.5053 | 1202 | 22.164 | 1.738 | 4.557 | 0.776 | 4.864 | 1.19E-08 |
| 540.4387 | 1204 | 49.312 | 3.081 | 12.594 | 2.011 | 3.916 | 1.42E-08 |
| 448.3562 | 1204 | 12.704 | 0.509 | 6.139 | 0.508 | 2.069 | 1.69E-08 |
| 523.3637 | 1101 | 2.792 | 0.154 | 3.567 | 0.155 | 0.783 | 2.14E-08 |
| 494.3968 | 1204 | 19.629 | 1.092 | 6.605 | 0.867 | 2.972 | 2.25E-08 |
| 522.4313 | 1204 | 19.942 | 1.072 | 7.469 | 0.944 | 2.670 | 2.85E-08 |
| 594.4848 | 1204 | 165.993 | 10.796 | 45.401 | 7.979 | 3.656 | 4.81E-08 |
| 595.4883 | 1204 | 65.756 | 4.493 | 18.270 | 3.207 | 3.599 | 5.87E-08 |
| 597.5068 | 1202 | 8.430 | 0.707 | 2.001 | 0.308 | 4.213 | 6.69E-08 |
| 484.3788 | 1204 | 11.357 | 0.673 | 3.696 | 0.563 | 3.073 | 7.21E-08 |
| 568.4723 | 1204 | 19.140 | 1.666 | 4.476 | 0.627 | 4.276 | 7.36E-08 |
| 510.3937 | 1204 | 8.643 | 0.463 | 2.755 | 0.495 | 3.137 | 1.05E-07 |
| 610.482 | 1204 | 13.307 | 1.141 | 4.336 | 0.505 | 3.069 | 1.09E-07 |
| 552.3273 | 1201 | 6.531 | 1.036 | 4.365 | 0.492 | 1.496 | 1.11E-07 |
| 576.4757 | 1204 | 64.886 | 4.627 | 19.420 | 3.428 | 3.341 | 1.12E-07 |
| 495.4018 | 1204 | 6.142 | 0.341 | 2.325 | 0.300 | 2.642 | 1.21E-07 |
| 506.4338 | 1204 | 4.255 | 0.199 | 1.532 | 0.236 | 2.777 | 1.42E-07 |
| 478.4044 | 1204 | 4.567 | 0.254 | 2.095 | 0.208 | 2.180 | 1.54E-07 |
| 536.41 | 1204 | 13.137 | 0.707 | 5.601 | 0.743 | 2.345 | 1.66E-07 |
| 521.4188 | 1204 | 6.886 | 0.318 | 3.052 | 0.382 | 2.256 | 1.74E-07 |
| 468.384 | 1204 | 23.300 | 1.274 | 8.827 | 1.121 | 2.640 | 1.97E-07 |
| 569.4769 | 1204 | 13.342 | 0.782 | 16.192 | 0.834 | 0.824 | 2.06E-07 |
| 577.4795 | 1204 | 23.556 | 1.747 | 7.266 | 1.323 | 3.242 | 2.11E-07 |
| 508.3782 | 1204 | 6.701 | 0.397 | 2.628 | 0.357 | 2.550 | 2.43E-07 |
| 598.5107 | 1204 | 23.222 | 2.389 | 3.965 | 0.530 | 5.857 | 2.84E-07 |
| 550.4602 | 1204 | 14.919 | 1.141 | 4.800 | 0.796 | 3.108 | 2.93E-07 |
| 469.3863 | 1204 | 6.296 | 0.306 | 2.276 | 0.365 | 2.766 | 3.36E-07 |
| 466.3656 | 1204 | 17.573 | 0.871 | 7.234 | 0.974 | 2.429 | 3.53E-07 |
| 566.454 | 1204 | 11.369 | 0.761 | 4.079 | 0.698 | 2.787 | 5.16E-07 |
| 496.4157 | 1204 | 14.715 | 1.316 | 4.097 | 0.438 | 3.592 | 6.10E-07 |
| 597.5062 | 1204 | 105.816 | 11.948 | 17.041 | 2.549 | 6.209 | 6.44E-07 |
| 596.5012 | 1204 | 305.004 | 35.345 | 46.149 | 7.337 | 6.609 | 8.06E-07 |
| 548.4438 | 1204 | 9.290 | 0.523 | 3.896 | 0.575 | 2.384 | 8.46E-07 |
| 524.4448 | 1204 | 7.031 | 0.602 | 2.530 | 0.347 | 2.779 | 9.55E-07 |
| 467.3711 | 1204 | 5.622 | 0.304 | 2.318 | 0.352 | 2.425 | 1.13E-06 |
| 537.4142 | 1204 | 5.123 | 0.277 | 2.320 | 0.330 | 2.208 | 1.24E-06 |
| 590.4585 | 1204 | 15.750 | 1.532 | 5.639 | 0.766 | 2.793 | 1.25E-06 |
| 440.3526 | 1204 | 5.976 | 0.328 | 2.323 | 0.361 | 2.573 | 1.56E-06 |
| 520.4131 | 1204 | 19.275 | 0.868 | 8.963 | 1.213 | 2.151 | 1.63E-06 |
| 327.0307 | 1204 | 8.911 | 0.403 | 5.729 | 0.200 | 1.555 | 1.66E-06 |
| 562.4989 | 1204 | 15.568 | 0.969 | 6.545 | 0.819 | 2.379 | 1.91E-06 |
| 482.3604 | 1204 | 6.472 | 0.517 | 2.744 | 0.359 | 2.359 | 2.29E-06 |
| 538.4257 | 1204 | 35.521 | 2.265 | 14.248 | 2.317 | 2.493 | 2.63E-06 |
| 492.3832 | 1204 | 11.353 | 0.647 | 5.057 | 0.656 | 2.245 | 2.78E-06 |
| 570.4903 | 1204 | 7.316 | 0.836 | 1.596 | 0.209 | 4.584 | 2.84E-06 |
| 564.4396 | 1204 | 4.654 | 0.336 | 1.888 | 0.348 | 2.465 | 3.44E-06 |
| 551.4646 | 1204 | 5.429 | 0.463 | 1.857 | 0.313 | 2.924 | 3.83E-06 |
| 534.4645 | 1204 | 4.848 | 0.327 | 1.969 | 0.266 | 2.462 | 4.10E-06 |
| 534.3912 | 1204 | 5.803 | 0.287 | 2.348 | 0.490 | 2.471 | 5.38E-06 |
| 563.5013 | 1204 | 5.800 | 0.356 | 2.661 | 0.306 | 2.180 | 6.24E-06 |
| 564.513 | 1204 | 5.778 | 0.469 | 2.465 | 0.135 | 2.344 | 6.52E-06 |
| 594.4875 | 1202 | 12.561 | 1.194 | 4.445 | 0.981 | 2.826 | 6.62E-06 |
| 493.385 | 1204 | 3.804 | 0.230 | 1.686 | 0.272 | 2.256 | 8.96E-06 |
| 595.4928 | 1202 | 5.270 | 0.461 | 2.080 | 0.364 | 2.534 | 1.05E-05 |
| 576.4765 | 1202 | 5.732 | 0.787 | 1.685 | 0.289 | 3.402 | 1.18E-05 |
| 438.3354 | 1204 | 4.500 | 0.240 | 1.999 | 0.320 | 2.251 | 1.41E-05 |
| 518.3969 | 1204 | 14.324 | 0.833 | 7.105 | 1.015 | 2.016 | 1.55E-05 |
| 378.2921 | 1203 | 39.401 | 4.629 | 35.156 | 2.294 | 1.121 | 1.64E-05 |
| 464.3524 | 1204 | 10.992 | 0.512 | 5.327 | 0.783 | 2.063 | 1.83E-05 |
| 476.3869 | 1204 | 5.546 | 0.259 | 2.924 | 0.410 | 1.897 | 1.94E-05 |
| 519.3998 | 1204 | 5.121 | 0.373 | 2.423 | 0.396 | 2.113 | 2.59E-05 |
| 618.4834 | 1201 | 8.419 | 1.292 | 1.816 | 0.294 | 4.636 | 3.02E-05 |
| 480.3473 | 1204 | 4.616 | 0.238 | 2.369 | 0.377 | 1.949 | 4.26E-05 |
| 384.3399 | 1203 | 79.789 | 4.637 | 54.933 | 2.431 | 1.452 | 4.36E-05 |
| 593.4736 | 1204 | 21.988 | 2.537 | 7.306 | 1.312 | 3.010 | 4.64E-05 |
| 253.8165 | 1101 | 14.128 | 1.242 | 9.713 | 0.417 | 1.455 | 0.0001 |
| 264.9759 | 1204 | 7.668 | 0.276 | 5.890 | 0.250 | 1.302 | 0.0001 |
| 504.4188 | 1204 | 7.881 | 0.427 | 4.454 | 0.498 | 1.769 | 0.0001 |
| 591.4614 | 1204 | 5.417 | 0.569 | 2.303 | 0.323 | 2.352 | 0.0001 |
| 592.4717 | 1204 | 52.044 | 6.345 | 17.230 | 3.267 | 3.021 | 0.0001 |
| 612.4994 | 1204 | 8.902 | 0.755 | 3.979 | 0.613 | 2.237 | 0.0001 |
| 255.8135 | 1101 | 18.945 | 1.683 | 12.863 | 0.495 | 1.473 | 0.0002 |
| 385.3428 | 1203 | 25.610 | 1.669 | 17.850 | 0.822 | 1.435 | 0.0003 |
| 569.3687 | 1102 | 5.808 | 0.687 | 9.114 | 0.798 | 0.637 | 0.0003 |
| 616.4675 | 1201 | 5.743 | 0.847 | 2.166 | 0.403 | 2.651 | 0.0003 |
| 769.5638 | 1204 | 143.765 | 14.976 | 95.450 | 10.239 | 1.506 | 0.0003 |
| 770.569 | 1204 | 63.988 | 6.122 | 44.106 | 4.234 | 1.451 | 0.0003 |
| 474.3731 | 1204 | 5.273 | 0.308 | 2.975 | 0.401 | 1.772 | 0.0004 |
| 572.4455 | 1204 | 5.865 | 0.578 | 2.979 | 0.269 | 1.969 | 0.0004 |
| 446.341 | 1204 | 14.884 | 1.020 | 8.471 | 1.005 | 1.757 | 0.0005 |
| 447.3433 | 1204 | 4.850 | 0.350 | 2.489 | 0.366 | 1.949 | 0.0005 |
| 574.4594 | 1204 | 43.223 | 6.002 | 15.738 | 3.104 | 2.746 | 0.0005 |
| 462.3346 | 1204 | 4.094 | 0.171 | 2.281 | 0.372 | 1.795 | 0.0007 |
| 490.3676 | 1204 | 6.960 | 0.460 | 3.926 | 0.650 | 1.773 | 0.0007 |
| 502.4054 | 1204 | 7.152 | 0.530 | 4.183 | 0.460 | 1.710 | 0.0007 |
| 546.4298 | 1204 | 4.944 | 0.458 | 2.619 | 0.430 | 1.888 | 0.0008 |
| 575.4628 | 1204 | 16.464 | 2.289 | 6.352 | 1.189 | 2.592 | 0.0008 |
| 712.5074 | 1204 | 33.680 | 4.456 | 22.449 | 2.683 | 1.500 | 0.0011 |
| 1018.9399 | 1203 | 16.341 | 1.538 | 8.303 | 1.195 | 1.968 | 0.0011 |
| 558.3761 | 1204 | 7.224 | 0.825 | 4.791 | 0.249 | 1.508 | 0.0013 |
| 532.4503 | 1204 | 5.319 | 0.396 | 2.825 | 0.426 | 1.883 | 0.0015 |
| 716.4323 | 1204 | 17.596 | 2.776 | 9.512 | 0.571 | 1.850 | 0.0015 |
| 561.4863 | 1204 | 11.883 | 0.972 | 6.295 | 0.992 | 1.888 | 0.0016 |
| 713.5097 | 1204 | 14.360 | 1.899 | 9.557 | 1.160 | 1.503 | 0.0017 |
| 314.2461 | 1204 | 6.654 | 0.541 | 4.749 | 0.383 | 1.401 | 0.0018 |
| 160.1256 | 1203 | 7.861 | 0.351 | 6.088 | 0.622 | 1.291 | 0.0021 |
| 558.4649 | 1204 | 47.704 | 4.927 | 23.917 | 4.147 | 1.995 | 0.0021 |
| 781.6001 | 1204 | 11.335 | 1.338 | 7.350 | 0.701 | 1.542 | 0.0022 |
| 747.5761 | 1204 | 19.020 | 1.510 | 13.858 | 1.410 | 1.372 | 0.0024 |
| 539.4274 | 1204 | 10.318 | 1.781 | 3.468 | 0.856 | 2.975 | 0.0025 |
| 686.4879 | 1204 | 91.719 | 12.953 | 62.831 | 9.366 | 1.460 | 0.0025 |
| 546.3413 | 1204 | 4.730 | 0.643 | 2.304 | 0.359 | 2.053 | 0.003 |
| 688.5048 | 1204 | 31.508 | 3.980 | 22.044 | 3.110 | 1.429 | 0.0033 |
| 700.4371 | 1204 | 8.421 | 1.098 | 5.617 | 0.554 | 1.499 | 0.0033 |
| 1016.9279 | 1203 | 29.801 | 2.895 | 14.233 | 2.907 | 2.094 | 0.0037 |
| 560.478 | 1203 | 10.183 | 0.676 | 6.147 | 1.045 | 1.657 | 0.0039 |
| 559.4688 | 1204 | 17.743 | 1.887 | 9.237 | 1.570 | 1.921 | 0.004 |
| 367.3325 | 1203 | 12.234 | 0.329 | 10.201 | 0.434 | 1.199 | 0.0041 |
| 687.4916 | 1204 | 37.542 | 5.128 | 26.691 | 3.782 | 1.407 | 0.0044 |
| 523.3637 | 1101 | 2.792 | 0.154 | 3.567 | 0.155 | 0.783 | 0.0045 |
| 381.311 | 1203 | 65.856 | 6.771 | 55.622 | 3.160 | 1.184 | 0.005 |
| 574.4635 | 1202 | 4.109 | 0.785 | 1.733 | 0.354 | 2.371 | 0.005 |
| 376.2759 | 1203 | 18.196 | 1.641 | 16.364 | 0.695 | 1.112 | 0.0052 |
| 793.5663 | 1204 | 62.347 | 7.352 | 40.458 | 5.240 | 1.541 | 0.0056 |
| 746.5701 | 1204 | 59.736 | 5.308 | 43.321 | 5.482 | 1.379 | 0.0058 |
| 249.9677 | 1102 | 7.037 | 0.812 | 5.282 | 0.619 | 1.332 | 0.0059 |
| 544.3636 | 1204 | 4.822 | 0.603 | 3.228 | 0.162 | 1.494 | 0.0059 |
| 737.5045 | 1204 | 8.325 | 1.154 | 4.953 | 0.718 | 1.681 | 0.006 |
| 745.5643 | 1204 | 139.059 | 14.204 | 98.300 | 12.989 | 1.415 | 0.006 |
| 257.8106 | 1101 | 9.000 | 0.921 | 6.494 | 0.503 | 1.386 | 0.0063 |
| 794.5718 | 1204 | 32.136 | 3.430 | 21.759 | 2.588 | 1.477 | 0.0064 |
| 556.4497 | 1204 | 12.580 | 1.583 | 6.930 | 0.673 | 1.815 | 0.0067 |
| 689.5083 | 1204 | 13.040 | 1.560 | 9.335 | 1.306 | 1.397 | 0.0071 |
| 306.2568 | 1204 | 11.187 | 1.061 | 7.856 | 0.676 | 1.424 | 0.0072 |
| 370.351 | 1203 | 83.555 | 5.638 | 58.717 | 5.196 | 1.423 | 0.0073 |
| 205.8867 | 1101 | 8.244 | 0.345 | 7.034 | 0.240 | 1.172 | 0.0075 |
| 378,9906 | 1204 | 4.794 | 0.192 | 3.175 | 0.185 | 1.510 | 0.0075 |
| 557.4527 | 1204 | 5.130 | 0.622 | 2.610 | 0.443 | 1.966 | 0.008 |
| 369.3475 | 1203 | 641.745 | 44.780 | 441.804 | 42.240 | 1.453 | 0.0081 |
| 371.3542 | 1203 | 8.222 | 0.598 | 5.626 | 0.546 | 1.461 | 0.0088 |
| 702.4175 | 1204 | 12.357 | 2.087 | 7.404 | 0.542 | 1.669 | 0.0091 |
| 736.5031 | 1204 | 16.701 | 2.447 | 10.717 | 1.478 | 1.558 | 0.0092 |
| 743.5461 | 1204 | 452.234 | 52.550 | 321.639 | 51.754 | 1.406 | 0.0092 |
| 832.6022 | 1102 | 19.129 | 4.193 | 27.747 | 5.113 | 0.689 | 0.0092 |
| 744.55 | 1203 | 10.268 | 1.731 | 4.670 | 0.589 | 2.199 | 0.0095 |
| 722.5244 | 1204 | 11.878 | 1.668 | 7.942 | 0.684 | 1.496 | 0.0096 |
| 244.2189 | 1203 | 7.865 | 0.432 | 6.421 | 0.300 | 1.225 | 0.0103 |
| 263.8453 | 1101 | 8.105 | 0.644 | 6.599 | 0.299 | 1.228 | 0.0104 |
| 154.0035 | 1204 | 28.896 | 1.974 | 23.716 | 0.905 | 1.218 | 0.0105 |
| 530.3474 | 1204 | 54.770 | 6.770 | 41.447 | 1.894 | 1.321 | 0.0106 |
| 698.4885 | 1204 | 16.687 | 1.264 | 13.465 | 0.804 | 1.239 | 0.0106 |
| 776.556 | 1204 | 16.853 | 2.541 | 9.051 | 2.083 | 1.862 | 0.0107 |
| 779.5828 | 1204 | 29.410 | 4.417 | 18.711 | 2.200 | 1.572 | 0.0112 |
| 778.571 | 1204 | 13.161 | 2.066 | 7.907 | 1.215 | 1.664 | 0.0113 |
| 855.6009 | 1102 | 20.936 | 3.842 | 30.073 | 5.796 | 0.696 | 0.0113 |
| 743.5475 | 1203 | 22.097 | 3.492 | 10.450 | 1.319 | 2.115 | 0.0114 |
| 340.2407 | 1204 | 5.090 | 0.309 | 5.985 | 0.256 | 0.850 | 0.0115 |
| 831.5992 | 1102 | 44.121 | 10.631 | 65.931 | 13.152 | 0.669 | 0.0116 |
| 460.2681 | 1204 | 11.360 | 1.336 | 8.419 | 0.492 | 1.349 | 0.0117 |
| 624.5133 | 1203 | 24.990 | 1.800 | 16.782 | 1.739 | 1.489 | 0.0117 |
| 720.5081 | 1204 | 7.819 | 0.953 | 5.605 | 0.546 | 1.395 | 0.0117 |
| 730.4535 | 1204 | 31.096 | 4.976 | 20.165 | 0.999 | 1.542 | 0.012 |
| 432.2365 | 1204 | 3.845 | 0.328 | 2.860 | 0.316 | 1.344 | 0.0122 |
| 789.5658 | 1204 | 11.503 | 0.837 | 9.419 | 0.562 | 1.221 | 0.0127 |
| 446.2525 | 1204 | 6.209 | 0.643 | 4.533 | 0.209 | 1.370 | 0.0129 |
| 646.5702 | 1203 | 8.272 | 0.830 | 5.522 | 0.802 | 1.498 | 0.013 |
| 758.4785 | 1204 | 77.135 | 9.484 | 54.605 | 2.972 | 1.413 | 0.013 |
| 740.4966 | 1204 | 23.136 | 2.101 | 18.075 | 1.188 | 1.280 | 0.0131 |
| 744.5516 | 1204 | 183.026 | 19.581 | 136.722 | 20.173 | 1.339 | 0.0135 |
| 780.5872 | 1204 | 14.936 | 2.228 | 9.362 | 1.249 | 1.595 | 0.0135 |
| 907.7722 | 1203 | 26.436 | 2.735 | 17.366 | 2.098 | 1.522 | 0.0147 |
| 625.5161 | 1203 | 11.098 | 0.830 | 7.474 | 0.759 | 1.485 | 0.0148 |
| 623.5003 | 1203 | 8.971 | 1.018 | 5.816 | 0.536 | 1.542 | 0.0156 |
| 885.7866 | 1203 | 1.000 | 0.000 | 8.100 | 2.845 | 0.123 | 0.0158 |
| 906.7669 | 1203 | 45.385 | 5.161 | 29.567 | 3.715 | 1.535 | 0.0167 |
| 488.2996 | 1204 | 6.572 | 0.927 | 4.279 | 0.292 | 1.536 | 0.0168 |
| 558.4663 | 1202 | 5.415 | 1.033 | 3.113 | 0.430 | 1.739 | 0.0168 |
| 775.5514 | 1204 | 35.635 | 5.243 | 20.650 | 4.220 | 1.726 | 0.0168 |
| 239.939 | 1102 | 5.794 | 0.741 | 4.295 | 0.571 | 1.349 | 0.0171 |
| 462.3716 | 1204 | 3.522 | 0.290 | 2.681 | 0.165 | 1.314 | 0.0171 |
| 530.3474 | 1204 | 54.770 | 6.770 | 41.447 | 1.894 | 1.321 | 0.0181 |
| 856.6045 | 1102 | 10.732 | 1.955 | 15.064 | 2.836 | 0.712 | 0.0182 |
| 541.3415 | 1102 | 22.495 | 2.750 | 27.652 | 2.246 | 0.814 | 0.0189 |
| 648.5861 | 1203 | 28.975 | 1.870 | 20.476 | 3.455 | 1.415 | 0.019 |
| 211.8495 | 1102 | 6.647 | 0.650 | 4.835 | 0.395 | 1.375 | 0.0195 |
| 516.3324 | 1204 | 8.573 | 1.124 | 6.171 | 0.354 | 1.389 | 0.0201 |
| 729.5727 | 1204 | 11.687 | 1.327 | 8.167 | 1.211 | 1.431 | 0.0201 |
| 380.3079 | 1203 | 219.668 | 23.133 | 197.016 | 10.229 | 1.115 | 0.0202 |
| 232.2189 | 1203 | 9.603 | 0.999 | 6.990 | 0.603 | 1.374 | 0.0208 |
| 502.3165 | 1204 | 37.199 | 5.399 | 25.495 | 1.609 | 1.459 | 0.0213 |
| 570.3766 | 1201 | 2.370 | 0.366 | 1.292 | 0.159 | 1.834 | 0.0214 |
| 726.5438 | 1204 | 10.420 | 1.505 | 6.298 | 1.240 | 1.654 | 0.0219 |
| 146.11 | 1203 | 5.606 | 0.311 | 4.764 | 0.525 | 1.177 | 0.0221 |
| 503.3194 | 1204 | 10.490 | 1.478 | 6.992 | 0.517 | 1.500 | 0.0222 |
| 524.296 | 1201 | 5.049 | 0.769 | 3.162 | 0.424 | 1.597 | 0.0227 |
| 742.5366 | 1204 | 18.967 | 2.099 | 12.996 | 2.718 | 1.459 | 0.0231 |
| 777.5678 | 1204 | 26.326 | 4.120 | 16.489 | 2.472 | 1.597 | 0.0233 |
| 727.5554 | 1204 | 27.866 | 3.724 | 18.243 | 3.941 | 1.527 | 0.0234 |
| 286.2656 | 1203 | 10.402 | 1.248 | 7.166 | 0.711 | 1.452 | 0.0247 |
| 728.5605 | 1204 | 13.981 | 1.722 | 9.663 | 1.803 | 1.447 | 0.0254 |
| 260.2507 | 1204 | 21.769 | 3.306 | 14.449 | 1.741 | 1.507 | 0.026 |
| 265.8424 | 1101 | 7.482 | 0.580 | 6.008 | 0.368 | 1.245 | 0.026 |
| 753.5683 | 1204 | 26.430 | 4.780 | 16.198 | 2.044 | 1.632 | 0.0263 |
| 242.2032 | 1203 | 18.369 | 2.262 | 12.872 | 0.982 | 1.427 | 0.0272 |
| 545.3455 | 1101 | 2.905 | 0.196 | 3.734 | 0.258 | 0.778 | 0.0272 |
| 377.2801 | 1203 | 6.047 | 0.635 | 5.692 | 0.316 | 1.062 | 0.0275 |
| 649.5895 | 1203 | 13.892 | 1.020 | 9.866 | 1.723 | 1.408 | 0.0281 |
| 531.3504 | 1204 | 16.841 | 2.194 | 12.776 | 0.643 | 1.318 | 0.0285 |
| 763.5153 | 1204 | 29.196 | 5.814 | 13.222 | 5.672 | 2.208 | 0.0285 |
| 569.369 | 1202 | 13.342 | 0.782 | 16.192 | 0.834 | 0.824 | 0.0292 |
| 909.7867 | 1203 | 16.698 | 1.762 | 11.334 | 1.721 | 1.473 | 0.0301 |
| 311.7754 | 1101 | 5.876 | 0.894 | 3.902 | 0.528 | 1.506 | 0.0308 |
| 272.2501 | 1203 | 8.545 | 1.006 | 6.078 | 0.598 | 1.406 | 0.0309 |
| 622.4973 | 1203 | 19.859 | 2.358 | 13.139 | 1.241 | 1.511 | 0.0316 |
| 552.3273 | 1201 | 6.531 | 1.036 | 4.365 | 0.492 | 1.496 | 0.0319 |
| 672.586 | 1203 | 11.817 | 1.553 | 7.815 | 1.024 | 1.512 | 0.0324 |
| 340.2621 | 1204 | 5.577 | 0.638 | 4.305 | 0.400 | 1.295 | 0.0326 |
| 271.8051 | 1102 | 8.179 | 0.967 | 5.695 | 0.859 | 1.436 | 0.0328 |
| 855.6798 | 1204 | 5.556 | 1.040 | 2.729 | 0.579 | 2.036 | 0.0334 |
| 715.4864 | 1204 | 21.704 | 1.962 | 18.368 | 1.657 | 1.182 | 0.0338 |
| 899.5871 | 1102 | 10.315 | 2.129 | 13.414 | 2.488 | 0.769 | 0.0344 |
| 244.0559 | 1101 | 10.439 | 1.409 | 7.511 | 0.240 | 1.390 | 0.0346 |
| 512.4079 | 1204 | 24.363 | 1.277 | 6.085 | 0.936 | 4.004 | 0.0346 |
| 181.9806 | 1102 | 6.350 | 0.914 | 4.833 | 0.724 | 1.314 | 0.0357 |
| 754.5724 | 1204 | 11.650 | 1.883 | 7.945 | 0.856 | 1.466 | 0.0361 |
| 783.6174 | 1204 | 14.897 | 2.745 | 25.452 | 6.501 | 0.585 | 0.0368 |
| 379.2957 | 1203 | 10.169 | 1.195 | 9.312 | 0.567 | 1.092 | 0.0369 |
| 725.5376 | 1204 | 20.287 | 3.674 | 11.990 | 2.404 | 1.692 | 0.0369 |
| 764.5196 | 1204 | 13.325 | 3.070 | 5.424 | 2.939 | 2.457 | 0.037 |
| 345.8738 | 1101 | 5.195 | 0.436 | 6.130 | 0.367 | 0.847 | 0.0372 |
| 797.5973 | 1204 | 32.079 | 2.947 | 25.825 | 2.485 | 1.242 | 0.0381 |
| 330.2569 | 1204 | 3.288 | 0.416 | 2.317 | 0.210 | 1.419 | 0.0385 |
| 626.5271 | 1203 | 31.047 | 2.292 | 22.120 | 2.012 | 1.404 | 0.0385 |
| 202.0453 | 1101 | 27.142 | 4.189 | 18.281 | 0.929 | 1.485 | 0.0386 |
| 542.3447 | 1102 | 6.520 | 0.775 | 7.672 | 0.576 | 0.850 | 0.0395 |
| 738.5185 | 1204 | 27.693 | 3.648 | 20.578 | 2.843 | 1.346 | 0.04 |
| 144.0944 | 1203 | 6.264 | 0.355 | 5.320 | 0.523 | 1.177 | 0.0412 |
| 699.4908 | 1204 | 7.701 | 0.616 | 6.202 | 0.488 | 1.242 | 0.0422 |
| 584.2641 | 1202 | 5.916 | 1.460 | 3.159 | 0.549 | 1.873 | 0.0431 |
| 606.413 | 1204 | 4.879 | 1.664 | 2.072 | 0.870 | 2.355 | 0.0433 |
| 305.2439 | 1204 | 8.424 | 0.931 | 6.262 | 0.570 | 1.345 | 0.0435 |
| 207.8836 | 1101 | 6.837 | 0.380 | 5.851 | 0.405 | 1.169 | 0.044 |
| 780.5303 | 1204 | 10.924 | 0.738 | 8.993 | 0.851 | 1.215 | 0.044 |
| 773.5954 | 1204 | 32.726 | 3.281 | 27.668 | 2.679 | 1.183 | 0.0441 |
| 304.241 | 1204 | 39.983 | 4.615 | 29.496 | 2.692 | 1.356 | 0.0445 |
| 634.3951 | 1204 | 9.972 | 2.107 | 5.214 | 0.746 | 1.913 | 0.0446 |
| 792.555 | 1204 | 37.287 | 4.795 | 25.974 | 4.202 | 1.436 | 0.0446 |
| 688.4658 | 1204 | 12.687 | 1.417 | 9.572 | 0.751 | 1.325 | 0.0447 |
| 788.4794 | 1204 | 12.925 | 0.869 | 10.844 | 0.654 | 1.192 | 0.0447 |
| 627.5285 | 1203 | 13.706 | 1.127 | 9.720 | 0.884 | 1.410 | 0.0451 |
| 716.4987 | 1204 | 26.851 | 2.554 | 22.322 | 1.404 | 1.203 | 0.0452 |
| 765.5316 | 1204 | 37.075 | 5.186 | 23.443 | 5.659 | 1.581 | 0.0463 |
| 628.5393 | 1203 | 15.926 | 1.688 | 10.413 | 1.515 | 1.529 | 0.0466 |
| 791.5488 | 1204 | 72.427 | 10.198 | 49.500 | 8.854 | 1.463 | 0.0468 |
| 461.2707 | 1204 | 3.218 | 0.476 | 2.560 | 0.170 | 1.257 | 0.047 |
| 741.5302 | 1204 | 38.041 | 5.335 | 26.680 | 5.610 | 1.426 | 0.0472 |
| 781.5619 | 1204 | 12.501 | 1.566 | 7.960 | 1.653 | 1.570 | 0.0481 |
| 711.4947 | 1204 | 21.098 | 3.889 | 14.530 | 2.238 | 1.452 | 0.049 |

**Table 17: Accurate mass features differing between 10 clinically diagnosed PP- MULTIPLE SCLEROSIS patients and 10 controls (p<0.05).**

| | | | | | | | |
|---|---|---|---|---|---|---|---|
| 218.0371 | 1102 | 9.533 | 0.524 | 4.929 | 0.294 | 1.934 | 1.13E-08 |
| 244.0559 | 1101 | 13.840 | 1.110 | 6.736 | 0.509 | 2.055 | 3.93E-08 |
| 216.04 | 1102 | 28.201 | 1.565 | 14.157 | 0.586 | 1.992 | 7.45E-08 |
| 202.0453 | 1101 | 36.309 | 3.700 | 16.259 | 0.940 | 2.233 | 9.69E-07 |
| 226.0688 | 1102 | 14.690 | 1.479 | 8.398 | 0.768 | 1.749 | 2.99E-06 |
| 243.0719 | 1101 | 41.426 | 5.686 | 19.420 | 1.732 | 2.133 | 8.10E-06 |
| 273.9985 | 1102 | 5.556 | 0.606 | 1.951 | 0.368 | 2.848 | 2.31E-05 |
| 382.1084 | 1101 | 4.696 | 1.074 | 1.216 | 0.115 | 3.862 | 4.34E-05 |
| 253.8165 | 1101 | 12.798 | 0.768 | 8.954 | 0.705 | 1.429 | 0.0001 |
| 218.0192 | 1101 | 11.301 | 2.271 | 3.211 | 0.680 | 3.519 | 0.0002 |
| 188.0143 | 1102 | 6.273 | 1.597 | 1.293 | 0.196 | 4.852 | 0.0005 |
| 257.8106 | 1101 | 8.886 | 0.570 | 6.118 | 0.463 | 1.452 | 0.0005 |
| 260.004 | 1101 | 6.981 | 1.052 | 2.623 | 0.456 | 2.661 | 0.0005 |
| 333.9539 | 1102 | 5.156 | 0.719 | 1.864 | 0.382 | 2.766 | 0.0008 |
| 806.5643 | 1201 | 22.470 | 2.372 | 16.663 | 2.290 | 1.348 | 0.001 |
| 833.5931 | 1201 | 12.180 | 1.662 | 7.722 | 0.947 | 1.577 | 0.001 |
| 805.5609 | 1201 | 44.055 | 4.902 | 33.136 | 4.448 | 1.330 | 0.0013 |
| 263.8453 | 1101 | 8.384 | 0.323 | 6.535 | 0.524 | 1.283 | 0.0014 |
| 834.5963 | 1201 | 6.556 | 0.888 | 4.267 | 0.506 | 1.536 | 0.0014 |
| 506.2853 | 1201 | 5.751 | 0.973 | 2.233 | 0.235 | 2.575 | 0.0016 |
| 570.3766 | 1201 | 2.849 | 0.382 | 1.659 | 0.285 | 1.717 | 0.0017 |
| 311.7754 | 1101 | 5.404 | 0.343 | 3.232 | 0.556 | 1.672 | 0.0019 |
| 331.957 | 1102 | 5.296 | 0.724 | 2.066 | 0.378 | 2.563 | 0.0024 |
| 205.8867 | 1101 | 8.743 | 0.471 | 6.644 | 0.433 | 1.316 | 0.003 |
| 255.8135 | 1101 | 16.177 | 1.027 | 12.092 | 0.980 | 1.338 | 0.003 |
| 611.3724 | 1201 | 5.038 | 0.732 | 3.082 | 0.523 | 1.635 | 0.0031 |
| 271.8051 | 1102 | 8.408 | 1.095 | 4.389 | 0.612 | 1.916 | 0.0032 |
| 209.8525 | 1102 | 5.656 | 0.606 | 2.987 | 0.496 | 1.894 | 0.0038 |
| 275.8713 | 1101 | 5.952 | 0.369 | 4.788 | 0.389 | 1.243 | 0.0038 |
| 269.8081 | 1102 | 12.049 | 1.596 | 6.580 | 0.794 | 1.831 | 0.0042 |
| 610.3691 | 1201 | 14.008 | 2.142 | 8.756 | 1.600 | 1.600 | 0.0047 |
| 943.7452 | 1204 | 5.801 | 0.947 | 3.476 | 0.533 | 1.669 | 0.0055 |
| 882.7648 | 1203 | 131.949 | 15.074 | 87.892 | 8.198 | 1.501 | 0.0063 |
| 203.1157 | 1101 | 6.030 | 1.049 | 3.217 | 0.557 | 1.874 | 0.0064 |
| 428.295 | 1204 | 3.809 | 0.428 | 4.489 | 0.510 | 0.849 | 0.0066 |
| 828.5477 | 1201 | 6.444 | 0.675 | 4.716 | 0.655 | 1.366 | 0.0087 |
| 758.5655 | 1201 | 64.803 | 8.320 | 48.518 | 8.035 | 1.336 | 0.0089 |
| 267.811 | 1102 | 7.363 | 0.960 | 4.236 | 0.420 | 1.738 | 0.009 |
| 757.5622 | 1201 | 128.225 | 16.744 | 97.646 | 16.254 | 1.313 | 0.0098 |
| 884.7764 | 1203 | 65.646 | 8.766 | 39.419 | 4.888 | 1.665 | 0.0101 |
| 150.1413 | 1203 | 4.696 | 0.369 | 3.874 | 0.409 | 1.212 | 0.0102 |
| 766.5051 | 1201 | 3.970 | 0.401 | 2.771 | 0.518 | 1.433 | 0.0111 |
| 857.7516 | 1203 | 132.550 | 15.354 | 80.972 | 9.566 | 1.637 | 0.0111 |
| 452.244 | 1201 | 7.063 | 0.810 | 5.156 | 0.648 | 1.370 | 0.012 |
| 613.3404 | 1202 | 6.275 | 0.752 | 4.929 | 0.666 | 1.273 | 0.013 |
| 273.8743 | 1101 | 9.490 | 0.470 | 7.689 | 0.685 | 1.234 | 0.0131 |
| 265.8424 | 1101 | 7.220 | 0.470 | 6.038 | 0.317 | 1.196 | 0.0133 |
| 856.7475 | 1203 | 246.398 | 29.810 | 152.319 | 19.797 | 1.618 | 0.0133 |
| 337.2697 | 1203 | 5.612 | 0.449 | 4.461 | 0.237 | 1.258 | 0.0149 |
| 1253.124 | 1203 | 8.679 | 1.209 | 5.137 | 0.834 | 1.690 | 0.0161 |
| 813.5871 | 1202 | 4.817 | 0.467 | 6.593 | 0.671 | 0.731 | 0.0198 |
| 827.5445 | 1101 | 5.520 | 0.780 | 3.455 | 0.349 | 1.598 | 0.0201 |
| 861.5265 | 1102 | 6.955 | 0.882 | 7.014 | 1.062 | 0.992 | 0.0207 |
| 601.5163 | 1203 | 137.952 | 15.032 | 109.993 | 11.197 | 1.254 | 0.0215 |
| 1228.11 | 1203 | 14.619 | 3.088 | 7.593 | 1.801 | 1.925 | 0.0215 |
| 835.6094 | 1201 | 4.387 | 0.625 | 3.337 | 0.559 | 1.315 | 0.0223 |
| 858.7607 | 1203 | 104.669 | 15.454 | 57.677 | 8.571 | 1.815 | 0.0228 |
| 602.5287 | 1203 | 435.124 | 61.111 | 296.579 | 40.379 | 1.467 | 0.023 |
| 134.11 | 1203 | 11.334 | 1.148 | 10.055 | 1.025 | 1.127 | . 0.0235 |
| 785.5934 | 1201 | 66.177 | 9.947 | 48.757 | 9.132 | 1.357 | 0.0238 |
| 1254.131 | 1203 | 6.626 | 0.892 | 3.523 | 0.537 | 1.881 | 0.024 |
| 339.2851 | 1203 | 12.818 | 1.706 | 7.989 | 0.924 | 1.604 | 0.0249 |
| 603.532 | 1203 | 182.681 | 25.875 | 124.378 | 17.252 | 1.469 | 0.0259 |
| 827.5446 | 1201 | 12.164 | 1.506 | 9.243 | 1.482 | 1.316 | 0.0261 |
| 600.513 | 1203 | 329.443 | 38.573 | 257.344 | 27.668 | 1.280 | 0.0267 |
| 810.5967 | 1201 | 23.957 | 3.670 | 16.403 | 2.568 | 1.461 | 0.0268 |
| 136.1258 | 1203 | 5.285 | 0.522 | 4.442 | 0.479 | 1.190 | 0.0274 |
| 885.778 | 1203 | 29.109 | 5.738 | 17.223 | 3.290 | 1.690 | 0.0276 |
| 789.5163 | 1204 | 20.925 | 4.537 | 13.491 | 2.853 | 1.551 | 0.0278 |
| 285.1366 | 1201 | 3.390 | 0.933 | 1.221 | 0.221 | 2.776 | 0.028 |
| 859.7662 | 1203 | 48.036 | 6.665 | 26.489 | 3.984 | 1.813 | 0.0295 |
| 162.1412 | 1203 | 5.843 | 0.525 | 5.260 | 0.399 | 1.111 | 0.0296 |
| 211.8495 | 1102 | 6.585 | 0.809 | 4.119 | 0.402 | 1.599 | 0.0309 |
| 628.5393 | 1203 | 14.633 | 1.243 | 10.951 | 1.097 | 1.336 | 0.0309 |
| 828.5479 | 1101 | 3.221 | 0.413 | 2.144 | 0.126 | 1.502 | 0.031 |
| 336.266 | 1203 | 22.012 | 2.175 | 16.511 | 1.026 | 1.333 | 0.0313 |
| 258.2346 | 1203 | 9.388 | 0.794 | 12.892 | 1.074 | 0.728 | 0.0323 |
| 786.5967 | 1201 | 32.109 | 5.025 | 23.876 | 4.433 | 1.345 | 0.0328 |
| 881.7549 | 1203 | 71.086 | 7.371 | 55.715 | 3.669 | 1.276 | 0.0328 |
| 794.5419 | 1102 | 7.159 | 0.964 | 7.518 | 1.206 | 0.952 | 0.0336 |
| 338.2815 | 1203 | 63.145 | 7.870 | 40.778 | 4.091 | 1.549 | 0.034 |
| 781.497 | 1204 | 10.007 | 1.616 | 11.541 | 1.469 | 0.867 | 0.0347 |
| 184.1255 | 1203 | 5.899 | 0.492 | 5.451 | 0.380 | 1.082 | 0.0379 |
| 684.6037 | 1204 | 4.384 | 0.914 | 2.189 | 0.523 | 2.003 | 0.038 |
| 851.5686 | 1102 | 9.495 | 1.864 | 10.212 | 2.466 | 0.930 | 0.0392 |
| 880.7514 | 1203 | 127.222 | 15.132 | 97.678 | 7.882 | 1.302 | 0.0392 |
| 809.5934 | 1201 | 46.591 | 7.354 | 32.314 | 5.156 | 1.442 | 0.0408 |
| 148.1257 | 1203 | 7.366 | 0.769 | 6.753 | 0.637 | 1.091 | 0.0415 |
| 850.6899 | 1203 | 4.878 | 1.364 | 3.038 | 1.308 | 1.606 | 0.0417 |
| 161.1051 | 1101 | 4.609 | 0.742 | 3.270 | 0.598 | 1.409 | 0.0437 |
| 534.3166 | 1201 | 5.171 | 0.837 | 3.421 | 0.384 | 1.512 | 0.0444 |
| 852.5724 | 1102 | 5.230 | 0.908 | 5.565 | 1.313 | 0.940 | 0.0449 |
| 785.4799 | 1204 | 18.864 | 3.666 | 12.479 | 2.715 | 1.512 | 0.0455 |
| 207.8836 | 1101 | 6.333 | 0.187 | 5.541 | 0.328 | 1.143 | 0.0465 |
| 811.5718 | 1202 | 3.876 | 0.448 | 4.974 | 0.356 | 0.779 | 0.0466 |
| 793.5986 | 1201 | 6.760 | 0.935 | 5.649 | 0.925 | 1.197 | 0.0482 |
| 855.7361 | 1203 | 123.848 | 14.395 | 88.031 | 9.890 | 1.407 | 0.0482 |
| 720.4696 | 1204 | 6.438 | 0.985 | 4.636 | 0.451 | 1.389 | 0.0487 |
| 749.5762 | 1102 | 5.610 | 0.834 | 7.521 | 0.996 | 0.746 | 0.0489 |
| 283.903 | 1101 | 10.126 | 0.691 | 8.241 | 0.513 | 1.229 | 0.0491 |

**Table 18: Accurate mass features differing between 10 clinically diagnosed SP- MULTIPLE SCLEROSIS patients and 10 controls (p<0.05).**

| | | | | | | | |
|---|---|---|---|---|---|---|---|
| 550.4602 | 1204 | 3.472 | 0.470 | 12.653 | 1.135 | 0.274 | 6.38E-07 |
| 551.4646 | 1204 | 1.329 | 0.135 | 4.604 | 0.425 | 0.289 | 8.16E-07 |
| 578.4923 | 1204 | 11.751 | 0.881 | 46.817 | 5.096 | 0.251 | 2.37E-06 |
| 579.4958 | 1204 | 4.875 | 0.338 | 18.473 | 2.036 | 0.264 | 3.46E-06 |
| 580.5089 | 1204 | 4.485 | 0.410 | 14.745 | 1.530 | 0.304 | 4.29E-06 |
| 577.4795 | 1204 | 4.976 | 0.560 | 19.699 | 2.206 | 0.253 | 4.39E-06 |
| 576.4757 | 1204 | 12.835 | 1.389 | 53.107 | 6.179 | 0.242 | 5.45E-06 |
| 597.5068 | 1202 | 1.268 | 0.146 | 6.421 | 0.806 | 0.197 | 6.27E-06 |
| 597.5062 | 1204 | 12.819 | 1.444 | 77.858 | 10.610 | 0.165 | 9.69E-06 |
| 594.4848 | 1204 | 24.497 | 3.105 | 133.680 | 17.759 | 0.183 | 1.00E-05 |
| 598.5107 | 1204 | 3.097 | 0.299 | 17.223 | 2.358 | 0.180 | 1.27E-05 |
| 596.5012 | 1204 | 34.571 | 4.087 | 218.437 | 31.246 | 0.158 | 1.58E-05 |
| 595.4883 | 1204 | 10.287 | 1.282 | 53.490 | 7.294 | 0.192 | 1.59E-05 |
| 596.5053 | 1202 | 3.474 | 0.443 | 17.217 | 2.345 | 0.202 | 1.85E-05 |
| 616.4675 | 1201 | 1.137 | 0.137 | 5.287 | 0.713 | 0.215 | 2.01E-05 |
| 548.4438 | 1204 | 2.840 | 0.301 | 8.439 | 0.949 | 0.337 | 2.45E-05 |
| 563.5013 | 1204 | 2.423 | 0.366 | 5.674 | 0.449 | 0.427 | 2.55E-05 |
| 595.4928 | 1202 | 1.000 | 0.000 | 4.713 | 0.664 | 0.212 | 2.61E-05 |
| 581.5126 | 1204 | 2.311 | 0.187 | 5.718 | 0.588 | 0.404 | 3.07E-05 |
| 568.4723 | 1204 | 3.994 | 0.425 | 16.023 | 2.182 | 0.249 | 3.85E-05 |
| 558.4649 | 1204 | 14.333 | 1.960 | 47.412 | 5.855 | 0.302 | 4.31E-05 |
| 552.4784 | 1204 | 3.168 | 0.473 | 8.828 | 0.955 | 0.359 | 4.76E-05 |
| 493.385 | 1204 | 1.314 | 0.162 | 3.646 | 0.454 | 0.360 | 0.0001 |
| 508.3782 | 1204 | 2.236 | 0.298 | 5.212 | 0.525 | 0.429 | 0.0001 |
| 510.3937 | 1204 | 2.540 | 0.206 | 6.379 | 0.706 | 0.398 | 0.0001 |
| 522.4313 | 1204 | 6.350 | 0.553 | 17.263 | 2.060 | 0.368 | 0.0001 |
| 523.4337 | 1204 | 2.286 | 0.315 | 5.980 | 0.630 | 0.382 | 0.0001 |
| 534.4645 | 1204 | 1.957 | 0.270 | 3.828 | 0.264 | 0.511 | 0.0001 |
| 559.4688 | 1204 | 5.792 | 0.817 | 17.767 | 2.149 | 0.326 | 0.0001 |
| 562.4989 | 1204 | 6.687 | 0.835 | 14.101 | 1.150 | 0.474 | 0.0001 |
| 566.454 | 1204 | 2.751 | 0.317 | 9.505 | 1.346 | 0.289 | 0.0001 |
| 576.4765 | 1202 | 1.498 | 0.174 | 4.719 | 0.648 | 0.317 | 0.0001 |
| 594.4875 | 1202 | 2.493 | 0.219 | 10.648 | 1.606 | 0.234 | 0.0001 |
| 440.3526 | 1204 | 1.971 | 0.293 | 4.720 | 0.499 | 0.418 | 0.0002 |
| 446.341 | 1204 | 5.988 | 1.031 | 14.861 | 1.583 | 0.403 | 0.0002 |
| 448.3562 | 1204 | 5.280 | 0.797 | 11.199 | 0.980 | 0.471 | 0.0002 |
| 462.3346 | 1204 | 1.743 | 0.330 | 3.910 | 0.341 | 0.446 | 0.0002 |
| 469.3863 | 1204 | 2.014 | 0.324 | 5.088 | 0.570 | 0.396 | 0.0002 |
| 480.3473 | 1204 | 1.744 | 0.295 | 3.644 | 0.278 | 0.479 | 0.0002 |
| 492.3832 | 1204 | 3.984 | 0.530 | 10.155 | 1.249 | 0.392 | 0.0002 |
| 494.3968 | 1204 | 5.707 | 0.583 | 15.601 | 2.032 | 0.366 | 0.0002 |
| 502.4054 | 1204 | 2.918 | 0.427 | 7.569 | 0.875 | 0.386 | 0.0002 |
| 524.4448 | 1204 | 2.616 | 0.262 | 5.893 | 0.649 | 0.444 | 0.0002 |
| 532.4503 | 1204 | 2.510 | 0.310 | 5.587 | 0.587 | 0.449 | 0.0002 |
| 560.4821 | 1204 | 11.764 | 1.633 | 33.134 | 4.258 | 0.355 | 0.0002 |
| 561.4863 | 1204 | 4.978 | 0.592 | 13.298 | 1.719 | 0.374 | 0.0002 |
| 569.4769 | 1204 | 1.777 | 0.290 | 6.363 | 0.957 | 0.279 | 0.0002 |
| 610.482 | 1204 | 3.412 | 0.252 | 9.034 | 1.167 | 0.378 | 0.0002 |
| 466.3656 | 1204 | 6.322 | 0.835 | 14.385 | 1.618 | 0.439 | 0.0003 |
| 496.4157 | 1204 | 4.466 | 0.397 | 10.900 | 1.400 | 0.410 | 0.0003 |
| 513.4116 | 1204 | 2.094 | 0.261 | 6.272 | 0.900 | 0.334 | 0.0003 |
| 520.4131 | 1204 | 6.912 | 0.762 | 17.327 | 2.230 | 0.399 | 0.0003 |
| 540.4387 | 1204 | 11.594 | 1.267 | 37.542 | 5.765 | 0.309 | 0.0003 |
| 558.4663 | 1202 | 1.658 | 0.286 | 5.209 | 0.754 | 0.318 | 0.0003 |
| 570.4903 | 1204 | 1.413 | 0.178 | 5.019 | 0.800 | 0.282 | 0.0003 |
| 574.4594 | 1204 | 9.005 | 1.272 | 39.939 | 6.897 | 0.225 | 0.0003 |
| 618.4834 | 1201 | 1.781 | 0.291 | 7.306 | 1.195 | 0.244 | 0.0003 |
| 464.3524 | 1204 | 4.094 | 0.584 | 9.801 | 1.187 | 0.418 | 0.0004 |
| 484.3788 | 1204 | 3.708 | 0.358 | 8.598 | 1.058 | 0.431 | 0.0004 |
| 495.4018 | 1204 | 2.006 | 0.239 | 5.043 | 0.657 | 0.398 | 0.0004 |
| 538.4257 | 1204 | 10.128 | 1.453 | 29.656 | 4.218 | 0.342 | 0.0004 |
| 541.4422 | 1204 | 4.227 | 0.390 | 12.395 | 1.858 | 0.341 | 0.0004 |
| 482.3604 | 1204 | 2.285 | 0.295 | 5.003 | 0.565 | 0.457 | 0.0005 |
| 490.3676 | 1204 | 2.790 | 0.429 | 6.947 | 0.872 | 0.402 | 0.0005 |
| 504.4188 | 1204 | 3.750 | 0.382 | 8.301 | 0.997 | 0.452 | 0.0005 |
| 512.4079 | 1204 | 5.922 | 0.643 | 18.085 | 2.823 | 0.327 | 0.0005 |
| 590.4585 | 1204 | 3.711 | 0.299 | 11.905 | 1.918 | 0.312 | 0.0005 |
| 530.4379 | 1204 | 2.841 | 0.360 | 6.143 | 0.710 | 0.462 | 0.0006 |
| 572.4455 | 1204 | 1.967 | 0.311 | 5.226 | 0.721 | 0.376 | 0.0006 |
| 575.4628 | 1204 | 3.809 | 0.596 | 15.394 | 2.708 | 0.247 | 0.0006 |
| 468.384 | 1204 | 8.098 | 1.139 | 18.856 | 2.365 | 0.429 | 0.0007 |
| 592.4717 | 1204 | 9.648 | 1.214 | 44.121 | 8.330 | 0.219 | 0.0007 |
| 450.3729 | 1204 | 5.207 | 0.618 | 10.174 | 1.075 | 0.512 | 0.0008 |
| 557.4527 | 1204 | 2.309 | 0.458 | 5.268 | 0.578 | 0.438 | 0.0008 |
| 447.3433 | 1204 | 2.229 | 0.361 | 4.668 | 0.498 | 0.478 | 0.0009 |
| 474.3731 | 1204 | 2.438 | 0.304 | 5.290 | 0.652 | 0.461 | 0.0009 |
| 521.4188 | 1204 | 2.734 | 0.263 | 6.323 | 0.869 | 0.432 | 0.0009 |
| 556.4497 | 1204 | 5.393 | 1.130 | 12.738 | 1.469 | 0.423 | 0.0009 |
| 593.4736 | 1204 | 4.158 | 0.455 | 18.437 | 3.549 | 0.226 | 0.0009 |
| 478.4044 | 1204 | 2.134 | 0.241 | 4.281 | 0.489 | 0.498 | 0.001 |
| 564.4396 | 1204 | 1.437 | 0.188 | 3.702 | 0.547 | 0.388 | 0.001 |
| 662.4267 | 1204 | 4.620 | 0.544 | 7.882 | 0.625 | 0.586 | 0.001 |
| 438.3354 | 1204 | 1.835 | 0.244 | 3.452 | 0.337 | 0.532 | 0.0011 |
| 462.3716 | 1204 | 1.755 | 0.262 | 3.104 | 0.228 | 0.565 | 0.0011 |
| 467.3711 | 1204 | 2.039 | 0.329 | 4.570 | 0.573 | 0.446 | 0.0012 |
| 537.4142 | 1204 | 1.601 | 0.279 | 4.530 | 0.714 | 0.353 | 0.0013 |
| 539.4274 | 1204 | 2.969 | 0.646 | 9.921 | 1.707 | 0.299 | 0.0013 |
| 546.4298 | 1204 | 1.760 | 0.276 | 4.749 | 0.733 | 0.371 | 0.0013 |
| 634.3951 | 1204 | 4.190 | 0.858 | 9.781 | 1.192 | 0.428 | 0.0013 |
| 327.0307 | 1204 | 5.519 | 0.363 | 7.555 | 0.397 | 0.731 | 0.0014 |
| 518.3969 | 1204 | 4.953 | 0.798 | 13.176 | 2.065 | 0.376 | 0.0016 |
| 564.513 | 1204 | 2.491 | 0.383 | 5.132 | 0.600 | 0.485 | 0.0016 |
| 591.4614 | 1204 | 1.560 | 0.188 | 4.512 | 0.780 | 0.346 | 0.0017 |
| 780.5303 | 1204 | 7.051 | 0.543 | 10.487 | 0.764 | 0.672 | 0.0018 |
| 536.41 | 1204 | 4.849 | 0.569 | 11.044 | 1.618 | 0.439 | 0.002 |
| 476.3869 | 1204 | 2.592 | 0.310 | 5.647 | 0.807 | 0.459 | 0.0024 |
| 452.3868 | 1204 | 2.151 | 0.258 | 3.957 | 0.448 | 0.544 | 0.0026 |
| 684.6037 | 1204 | 4.873 | 0.976 | 1.387 | 0.258 | 3.513 | 0.0028 |
| 786.51 | 1204 | 28.214 | 4.540 | 47.124 | 3.077 | 0.599 | 0.0029 |
| 702.4175 | 1204 | 6.417 | 0.863 | 10.462 | 0.810 | 0.613 | 0.0031 |
| 1227.091 | 1203 | 20.780 | 2.685 | 7.389 | 2.870 | 2.812 | 0.0031 |
| 574.4635 | 1202 | 1.186 | 0.124 | 3.815 | 0.767 | 0.311 | 0.0033 |
| 590.4964 | 1204 | 2.971 | 0.514 | 5.910 | 0.704 | 0.503 | 0.0034 |
| 872.6715 | 1204 | 3.959 | 0.500 | 1.847 | 0.382 | 2.143 | 0.0035 |
| 534.3912 | 1204 | 2.256 | 0.293 | 4.670 | 0.675 | 0.483 | 0.0042 |
| 519.3998 | 1204 | 1.795 | 0.343 | 4.507 | 0.764 | 0.398 | 0.0045 |
| 566.3431 | 1102 | 4.903 | 0.724 | 8.188 | 0.735 | 0.599 | 0.0052 |
| 1253.124 | 1203 | 8.027 | 1.087 | 3.768 | 0.790 | 2.130 | 0.0053 |
| 1227.109 | 1203 | 1.000 | 0.000 | 6.463 | 1.747 | 0.155 | 0.0058 |
| 325.0805 | 1203 | 6.643 | 0.259 | 4.437 | 0.662 | 1.497 | 0.0061 |
| 565.3391 | 1102 | 16.189 | 2.611 | 27.761 | 2.683 | 0.583 | 0.0063 |
| 612.4994 | 1204 | 3.321 | 0.458 | 5.636 | 0.594 | 0.589 | 0.0064 |
| 428.3653 | 1201 | 8.555 | 1.414 | 4.218 | 0.485 | 2.028 | 0.0095 |
| 477.3218 | 1201 | 5.669 | 0.639 | 3.353 | 0.484 | 1.691 | 0.0098 |
| 786.5408 | 1204 | 69.825 | 12.284 | 114.325 | 9.307 | 0.611 | 0.0098 |
| 516.3324 | 1204 | 5.629 | 0.723 | 8.488 | 0.678 | 0.663 | 0.0099 |
| 787.5452 | 1204 | 32.885 | 5.441 | 52.366 | 4.008 | 0.628 | 0.0099 |
| 542.3447 | 1102 | 4.631 | 0.544 | 7.432 | 0.812 | 0.623 | 0.0103 |
| 716.4323 | 1204 | 9.262 | 1.168 | 13.477 | 0.896 | 0.667 | 0.0103 |
| 700.4371 | 1204 | 5.142 | 0.606 | 8.366 | 0.953 | 0.615 | 0.0105 |
| 780.4907 | 1204 | 8.547 | 1.272 | 13.226 | 1.042 | 0.646 | 0.0107 |
| 738.5448 | 1102 | 2.946 | 0.273 | 4.660 | 0.546 | 0.632 | 0.0116 |
| 758.4785 | 1204 | 48.142 | 6.547 | 70.822 | 4.748 | 0.680 | 0.0117 |
| 541.3415 | 1102 | 15.508 | 1.952 | 25.108 | 2.840 | 0.618 | 0.0122 |
| 832.5211 | 1204 | 4.586 | 0.642 | 6.726 | 0.423 | 0.682 | 0.0122 |
| 860.7729 | 1203 | 15.620 | 1.641 | 9.251 | 1.594 | 1.688 | 0.0123 |
| 772.5265 | 1204 | 80.234 | 11.146 | 116.817 | 7.132 | 0.687 | 0.0128 |
| 503.3194 | 1204 | 6.562 | 0.772 | 9.407 | 0.685 | 0.698 | 0.013 |
| 531.312 | 1102 | 4.205 | 0.597 | 6.308 | 0.485 | 0.667 | 0.0137 |
| 1226.078 | 1203 | 14.056 | 3.132 | 4.162 | 1.855 | 3.377 | 0.0141 |
| 1251.104 | 1203 | 7.607 | 1.295 | 3.433 | 0.835 | 2.216 | 0.0144 |
| 264.9759 | 1204 | 5.745 | 0.400 | 7.002 | 0.237 | 0.820 | 0.0145 |
| 569.3687 | 1102 | 4.709 | 0.805 | 7.253 | 0.491 | 0.649 | 0.0147 |
| 136.1258 | 1203 | 5.487 | 0.328 | 4.195 | 0.349 | 1.308 | 0.0148 |
| 468.3577 | 1201 | 4.938 | 0.656 | 2.794 | 0.451 | 1.767 | 0.0149 |
| 150.1413 | 1203 | 5.000 | 0.275 | 3.627 | 0.433 | 1.379 | 0.0154 |
| 610.5204 | 1204 | 6.706 | 0.995 | 16.481 | 3.554 | 0.407 | 0.0163 |
| 730.4535 | 1204 | 19.286 | 2.509 | 27.501 | 1.823 | 0.701 | 0.0163 |
| 1019.384 | 1102 | 4.944 | 0.506 | 6.898 | 0.538 | 0.717 | 0.0165 |
| 809.5264 | 1204 | 14.792 | 2.579 | 22.126 | 1.047 | 0.669 | 0.0168 |
| 812.6122 | 1201 | 6.364 | 0.891 | 3.185 | 0.813 | 1.998 | 0.0168 |
| 723.6395 | 1204 | 8.368 | 0.522 | 6.496 | 0.484 | 1.288 | 0.017 |
| 808.5225 | 1204 | 28.835 | 5.040 | 42.966 | 1.954 | 0.671 | 0.0176 |
| 748.5722 | 1102 | 9.684 | 0.818 | 15.985 | 2.287 | 0.606 | 0.0183 |
| 722.5244 | 1204 | 6.342 | 0.668 | 9.102 | 0.844 | 0.697 | 0.0196 |
| 368.1656 | 1102 | 1.185 | 0.185 | 3.565 | 0.913 | 0.332 | 0.0199 |
| 749.5762 | 1102 | 4.605 | 0.360 | 7.438 | 1.050 | 0.619 | 0.0201 |
| 828.5477 | 1201 | 7.432 | 0.648 | 5.374 | 0.484 | 1.383 | 0.0203 |
| 861.5265 | 1102 | 3.988 | 0.528 | 6.978 | 1.050 | 0.572 | 0.0203 |
| 170.11 | 1203 | 4.805 | 0.180 | 3.587 | 0.444 | 1.340 | 0.0204 |
| 506.4338 | 1204 | 2.346 | 0.274 | 3.523 | 0.376 | 0.666 | 0.021 |
| 728.5605 | 1204 | 6.704 | 0.883 | 10.409 | 1.177 | 0.644 | 0.0215 |
| 897.5729 | 1102 | 3.972 | 0.484 | 7.232 | 1.201 | 0.549 | 0.0215 |
| 859.7662 | 1203 | 43.601 | 5.294 | 27.020 | 3.953 | 1.614 | 0.0219 |
| 794.5126 | 1204 | 37.888 | 4.676 | 51.378 | 2.752 | 0.737 | 0.023 |
| 754.5724 | 1204 | 6.394 | 0.637 | 8.581 | 0.615 | 0.745 | 0.0238 |
| 858.7607 | 1203 | 96.197 | 12.594 | 58.519 | 8.619 | 1.644 | 0.0238 |
| 602.5287 | 1203 | 442.732 | 42.991 | 298.697 | 39.820 | 1.482 | 0.0243 |
| 793.5381 | 1102 | 9.315 | 1.086 | 16.255 | 2.606 | 0.573 | 0.0243 |
| 997.3968 | 1102 | 4.647 | 0.582 | 6.752 | 0.635 | 0.688 | 0.0251 |
| 886.5582 | 1102 | 5.308 | 0.511 | 8.358 | 1.146 | 0.635 | 0.0258 |
| 759.5145 | 1204 | 82.727 | 18.578 | 135.933 | 11.699 | 0.609 | 0.0261 |
| 603.532 | 1203 | 185.229 | 18.521 | 124.877 | 16.890 | 1.483 | 0.027 |
| 899.5871 | 1102 | 5.197 | 0.456 | 9.083 | 1.551 | 0.572 | 0.0272 |
| 502.3165 | 1204 | 23.972 | 3.305 | 34.085 | 2.678 | 0.703 | 0.0287 |
| 567.3547 | 1102 | 8.637 | 1.277 | 12.512 | 1.016 | 0.690 | 0.0289 |
| 194.0803 | 1203 | 4.092 | 0.903 | 10.360 | 2.484 | 0.395 | 0.0291 |
| 590.5287 | 1203 | 14.508 | 1.311 | 9.764 | 1.535 | 1.486 | 0.0304 |
| 784.5238 | 1204 | 61.252 | 12.412 | 93.059 | 5.519 | 0.658 | 0.0309 |
| 770.5108 | 1204 | 66.669 | 9.999 | 91.610 | 3.726 | 0.728 | 0.0312 |
| 134.11 | 1203 | 12.200 | 0.544 | 9.502 | 1.019 | 1.284 | 0.0313 |
| 833.5931 | 1201 | 13.307 | 1.528 | 8.766 | 1.203 | 1.518 | 0.0313 |
| 148.1257 | 1203 | 8.105 | 0.361 | 6.336 | 0.670 | 1.279 | 0.0321 |
| 781.497 | 1204 | 9.800 | 2.175 | 15.526 | 1.165 | 0.631 | 0.0322 |
| 835.6094 | 1201 | 5.359 | 0.712 | 3.476 | 0.392 | 1.542 | 0.0326 |
| 555.3102 | 1102 | 4.391 | 0.655 | 6.728 | 0.771 | 0.653 | 0.0329 |
| 729.5727 | 1204 | 6.021 | 0.638 | 8.508 | 0.871 | 0.708 | 0.0334 |
| 617.0921 | 1204 | 207.196 | 23.604 | 268.008 | 11.876 | 0.773 | 0.0335 |
| 576.51 | 1203 | 705.744 | 95.161 | 426.474 | 76.051 | 1.655 | 0.0341 |
| 788.5549 | 1204 | 13.572 | 1.893 | 19.042 | 1.460 | 0.713 | 0.0344 |
| 162.1412 | 1203 | 6.114 | 0.257 | 5.035 | 0.396 | 1.214 | 0.0346 |
| 758.5089 | 1204 | 123.531 | 26.082 | 193.464 | 16.177 | 0.639 | 0.0351 |
| 766.4759 | 1204 | 8.570 | 0.885 | 11.487 | 0.925 | 0.746 | 0.0351 |
| 779.5828 | 1204 | 15.783 | 1.495 | 21.637 | 2.090 | 0.729 | 0.0351 |
| 821.5714 | 1102 | 5.465 | 0.646 | 9.535 | 1.667 | 0.573 | 0.0352 |
| 888.5121 | 1204 | 7.012 | 1.000 | 11.029 | 1.455 | 0.636 | 0.0354 |
| 872.5557 | 1102 | 4.145 | 0.546 | 7.038 | 1.156 | 0.589 | 0.0362 |
| 827.5446 | 1201 | 14.730 | 1.373 | 10.914 | 0.983 | 1.350 | 0.0364 |
| 742.4745 | 1204 | 7.998 | 1.054 | 10.693 | 0.572 | 0.748 | 0.0375 |
| 378.9906 | 1204 | 3.444 | 0.209 | 4.104 | 0.207 | 0.839 | 0.0378 |
| 541.3141 | 1101 | 4.561 | 0.406 | 7.761 | 1.369 | 0.588 | 0.0379 |
| 785.5287 | 1204 | 31.042 | 6.421 | 46.658 | 2.719 | 0.665 | 0.038 |
| 830.7332 | 1203 | 24.238 | 3.418 | 14.491 | 2.708 | 1.673 | 0.0383 |
| 1226.099 | 1203 | 2.984 | 1.394 | 8.082 | 1.804 | 0.369 | 0.0383 |
| 184.1255 | 1203 | 6.461 | 0.228 | 5.272 | 0.481 | 1.226 | 0.0384 |
| 830.5881 | 1102 | 7.945 | 0.851 | 13.643 | 2.417 | 0.582 | 0.0392 |
| 727.5554 | 1204 | 13.082 | 1.418 | 20.151 | 2.857 | 0.649 | 0.0398 |
| 858.6843 | 1102 | 1.895 | 0.602 | 5.452 | 1.489 | 0.348 | 0.0399 |
| 474.2846 | 1204 | 6.995 | 0.821 | 9.629 | 0.862 | 0.726 | 0.04 |
| 488.2996 | 1204 | 4.260 | 0.569 | 5.842 | 0.432 | 0.729 | 0.04 |
| 829.5851 | 1102 | 17.810 | 1.960 | 31.309 | 5.797 | 0.569 | 0.0406 |
| 780.5872 | 1204 | 8.415 | 0.637 | 11.092 | 1.041 | 0.759 | 0.0416 |
| 519.3322 | 1101 | 4.242 | 0.386 | 6.534 | 0.971 | 0.649 | 0.0417 |
| 832.6022 | 1102 | 10.376 | 0.935 | 17.404 | 3.081 | 0.596 | 0.0425 |
| 172.1255 | 1203 | 6.904 | 0.268 | 5.742 | 0.462 | 1.202 | 0.043 |
| 699.5206 | 1204 | 4.894 | 0.732 | 8.253 | 1.358 | 0.593 | 0.043 |
| 577.5134 | 1203 | 257.861 | 34.886 | 159.577 | 28.694 | 1.616 | 0.0431 |
| 720.5081 | 1204 | 3.975 | 0.701 | 6.114 | 0.689 | 0.650 | 0.0431 |
| 281.2447 | 1204 | 20.974 | 3.610 | 29.938 | 1.991 | 0.701 | 0.0433 |
| 760.5231 | 1204 | 58.934 | 11.826 | 88.317 | 6.590 | 0.667 | 0.0436 |
| 744.4942 | 1204 | 127.238 | 21.145 | 181.030 | 13.054 | 0.703 | 0.0441 |
| 379.2536 | 1204 | 1.941 | 0.435 | 3.035 | 0.260 | 0.640 | 0.0448 |
| 633.3232 | 1102 | 3.047 | 0.515 | 4.505 | 0.439 | 0.676 | . 0.0451 |
| 804.5715 | 1102 | 19.957 | 2.117 | 36.054 | 7.169 | 0.554 | 0.0451 |
| 591.5321 | 1203 | 5.846 | 0.612 | 3.933 | 0.649 | 1.486 | 0.0458 |
| 832.7499 | 1203 | 13.911 | 2.292 | 7.895 | 1.626 | 1.762 | 0.0462 |
| 461.2707 | 1204 | 2.330 | 0.223 | 2.933 | 0.174 | 0.794 | 0.0467 |
| 302.2255 | 1204 | 3.093 | 0.424 | 4.225 | 0.320 | 0.732 | 0.0471 |
| 198.1411 | 1203 | 5.029 | 0.251 | 4.192 | 0.304 | 1.200 | 0.048 |
| 280.2413 | 1204 | 107.053 | 18.645 | 152.351 | 10.396 | 0.703 | 0.048 |
| 803.5683 | 1102 | 49.513 | 5.444 | 91.296 | 18.924 | 0.542 | 0.048 |
| 794.5419 | 1102 | 4.724 | 0.480 | 7.467 | 1.204 | 0.633 | 0.0486 |
| 558.3761 | 1204 | 4.723 | 0.502 | 6.382 | 0.605 | 0.740 | 0.049 |
| 777.5678 | 1204 | 13.748 | 1.328 | 18.871 | 2.037 | 0.729 | 0.0494 |
| 834.5963 | 1201 | 6.953 | 0.783 | 4.966 | 0.528 | 1.400 | 0.0497 |

**Table 19: Accurate mass features differing between 10 clinically diagnosed RR- MULTIPLE SCLEROSIS patients and SP- MULTIPLE SCLEROSIS controls (p<0.05).**

| | | | | | | | |
|---|---|---|---|---|---|---|---|
| 448.3562 | 1204 | 12.828 | 0.687 | 4.778 | 0.539 | 2.685 | 2.71E-08 |
| 467.3711 | 1204 | 5.915 | 0.391 | 1.851 | 0.225 | 3.196 | 4.32E-08 |
| 466.3656 | 1204 | 17.861 | 1.282 | 5.789 | 0.482 | 3.085 | 6.87E-08 |
| 484.3788 | 1204 | 10.785 | 0.702 | 3.694 | 0.316 | 2.920 | 7.22E-08 |
| 450.3729 | 1204 | 11.105 | 0.649 | 4.587 | 0.398 | 2.421 | 9.95E-08 |
| 580.5089 | 1204 | 18.694 | 1.434 | 4.911 | 0.628 | 3.807 | 1.10E-07 |
| 578.4923 | 1204 | 56.509 | 4.849 | 12.547 | 1.621 | 4.504 | 2.10E-07 |
| 579.4958 | 1204 | 22.270 | 1.913 | 5.359 | 0.654 | 4.156 | 3.04E-07 |
| 452.3868 | 1204 | 4.802 | 0.284 | 1.901 | 0.220 | 2.526 | 3.12E-07 |
| 469.3863 | 1204 | 6.668 | 0.510 | 2.064 | 0.276 | 3.231 | 3.69E-07 |
| 494.3968 | 1204 | 19.451 | 1.654 | 5.659 | 0.537 | 3.437 | 3.84E-07 |
| 468.384 | 1204 | 25.447 | 2.013 | 8.057 | 0.922 | 3.158 | 4.29E-07 |
| 581.5126 | 1204 | 7.479 | 0.640 | 2.018 | 0.219 | 3.706 | 4.72E-07 |
| 508.3782 | 1204 | 6.735 | 0.505 | 2.211 | 0.220 | 3.046 | 5.07E-07 |
| 618.4834 | 1201 | 10.132 | 1.055 | 2.065 | 0.335 | 4.907 | 6.11E-07 |
| 510.3937 | 1204 | 8.743 | 0.780 | 2.767 | 0.254 | 3.160 | 6.55E-07 |
| 495.4018 | 1204 | 6.117 | 0.522 | 1.938 | 0.194 | 3.156 | 6.94E-07 |
| 513.4116 | 1204 | 7.003 | 0.525 | 2.222 | 0.332 | 3.152 | 7.35E-07 |
| 596.5053 | 1202 | 23.532 | 2.544 | 4.292 | 0.670 | 5.483 | 7.38E-07 |
| 598.5107 | 1204 | 23.078 | 2.490 | 3.714 | 0.543 | 6.214 | 7.79E-07 |
| 597.5068 | 1202 | 8.982 | 0.941 | 1.674 | 0.317 | 5.366 | 7.99E-07 |
| 522.4313 | 1204 | 20.427 | 1.832 | 6.314 | 0.627 | 3.235 | 8.26E-07 |
| 568.4723 | 1204 | 19.787 | 2.282 | 4.028 | 0.450 | 4.912 | 1.01E-06 |
| 569.4769 | 1204 | 7.849 | 0.822 | 1.841 | 0.247 | 4.263 | 1.16E-06 |
| 597.5062 | 1204 | 106.868 | 12.859 | 15.045 | 2.165 | 7.103 | 1.20E-06 |
| 537.4142 | 1204 | 5.533 | 0.453 | 1.639 | 0.226 | 3.376 | 1.33E-06 |
| 610.482 | 1204 | 13.296 | 1.385 | 3.518 | 0.246 | 3.779 | 1.37E-06 |
| 551.4646 | 1204 | 5.117 | 0.491 | 1.286 | 0.215 | 3.979 | 1.43E-06 |
| 596.5012 | 1204 | 302.332 | 35.546 | 41.055 | 6.198 | 7.364 | 1.50E-06 |
| 512.4079 | 1204 | 20.539 | 1.811 | 6.306 | 0.840 | 3.257 | 1.90E-06 |
| 446.341 | 1204 | 15.895 | 1.428 | 5.248 | 0.644 | 3.029 | 2.20E-06 |
| 550.4602 | 1204 | 13.875 | 1.423 | 3.330 | 0.563 | 4.167 | 2.31E-06 |
| 464.3524 | 1204 | 11.766 | 1.096 | 3.915 | 0.357 | 3.005 | 2.60E-06 |
| 492.3832 | 1204 | 12.904 | 1.371 | 3.723 | 0.342 | 3.466 | 2.75E-06 |
| 595.4883 | 1204 | 74.421 | 9.813 | 11.967 | 1.663 | 6.219 | 3.22E-06 |
| 590.4585 | 1204 | 14.400 | 1.343 | 4.037 | 0.398 | 3.567 | 3.23E-06 |
| 577.4795 | 1204 | 24.484 | 2.935 | 5.137 | 0.663 | 4.766 | 3.38E-06 |
| 536.41 | 1204 | 14.183 | 1.310 | 4.859 | 0.376 | 2.919 | 3.48E-06 |
| 594.4848 | 1204 | 187.278 | 25.540 | 29.026 | 4.176 | 6.452 | 3.49E-06 |
| 523.4337 | 1204 | 6.812 | 0.627 | 2.209 | 0.276 | 3.084 | 3.70E-06 |
| 576.4757 | 1204 | 66.906 | 8.279 | 13.283 | 1.720 | 5.037 | 3.77E-06 |
| 524.4448 | 1204 | 6.702 | 0.649 | 2.252 | 0.265 | 2.976 | 3.94E-06 |
| 440.3526 | 1204 | 5.499 | 0.452 | 2.035 | 0.245 | 2.702 | 4.58E-06 |
| 482.3604 | 1204 | 6.620 | 0.663 | 2.324 | 0.131 | 2.849 | 4.58E-06 |
| 616.4675 | 1201 | 7.494 | 0.826 | 1.454 | 0.257 | 5.154 | 4.99E-06 |
| 594.4875 | 1202 | 14.552 | 1.781 | 3.055 | 0.390 | 4.763 | 5.09E-06 |
| 476.3869 | 1204 | 5.204 | 0.291 | 2.461 | 0.311 | 2.115 | 5.20E-06 |
| 534.3912 | 1204 | 6.113 | 0.560 | 2.085 | 0.205 | 2.932 | 5.42E-06 |
| 520.4131 | 1204 | 21.750 | 2.595 | 6.355 | 0.508 | 3.423 | 5.98E-06 |
| 566.454 | 1204 | 12.984 | 1.754 | 2.754 | 0.294 | 4.715 | 6.04E-06 |
| 570.4903 | 1204 | 5.738 | 0.678 | 1.315 | 0.165 | 4.363 | 6.16E-06 |
| 541.4422 | 1204 | 21.125 | 2.994 | 4.380 | 0.457 | 4.823 | 6.30E-06 |
| 496.4157 | 1204 | 16.461 | 1.840 | 4.566 | 0.456 | 3.605 | 6.77E-06 |
| 540.4387 | 1204 | 62.261 | 8.879 | 12.111 | 1.401 | 5.141 | 7.87E-06 |
| 538.4257 | 1204 | 44.839 | 5.751 | 10.268 | 1.135 | 4.367 | 7.94E-06 |
| 518.3969 | 1204 | 15.673 | 1.778 | 4.418 | 0.460 | 3.548 | 8.09E-06 |
| 462.3346 | 1204 | 4.382 | 0.382 | 1.547 | 0.236 | 2.833 | 8.53E-06 |
| 595.4928 | 1202 | 6.217 | 0.788 | 1.316 | 0.215 | 4.724 | 1.07E-05 |
| 519.3998 | 1204 | 5.809 | 0.641 | 1.523 | 0.270 | 3.814 | 1.10E-05 |
| 438.3354 | 1204 | 4.529 | 0.392 | 1.772 | 0.224 | 2.556 | 1.15E-05 |
| 591.4614 | 1204 | 5.115 | 0.467 | 1.890 | 0.269 | 2.706 | 1.28E-05 |
| 521.4188 | 1204 | 7.695 | 0.847 | 2.676 | 0.199 | 2.876 | 1.47E-05 |
| 552.4784 | 1204 | 10.058 | 1.199 | 2.886 | 0.350 | 3.485 | 1.70E-05 |
| 474.3731 | 1204 | 5.983 | 0.690 | 1.993 | 0.275 | 3.002 | 1.71E-05 |
| 548.4438 | 1204 | 10.315 | 1.447 | 2.758 | 0.308 | 3.740 | 1.83E-05 |
| 564.4396 | 1204 | 4.615 | 0.499 | 1.374 | 0.202 | 3.359 | 1.95E-05 |
| 447.3433 | 1204 | 4.979 | 0.485 | 1.964 | 0.273 | 2.535 | 2.69E-05 |
| 592.4717 | 1204 | 49.484 | 6.153 | 11.401 | 1.794 | 4.340 | 2.92E-05 |
| 480.3473 | 1204 | 4.690 | 0.393 | 2.037 | 0.277 | 2.302 | 3.09E-05 |
| 493.385 | 1204 | 4.379 | 0.513 | 1.374 | 0.155 | 3.187 | 3.09E-05 |
| 593.4736 | 1204 | 20.853 | 2.576 | 5.040 | 0.818 | 4.138 | 3.10E-05 |
| 576.4765 | 1202 | 5.839 | 0.693 | 1.624 | 0.253 | 3.595 | 4.93E-05 |
| 502.4054 | 1204 | 7.483 | 0.761 | 2.843 | 0.469 | 2.632 | 0.0001 |
| 504.4188 | 1204 | 7.357 | 0.554 | 3.472 | 0.503 | 2.119 | 0.0001 |
| 532.4503 | 1204 | 5.403 | 0.420 | 2.505 | 0.389 | 2.157 | 0.0001 |
| 534.4645 | 1204 | 4.466 | 0.372 | 1.859 | 0.315 | 2.402 | 0.0001 |
| 539.4274 | 1204 | 14.474 | 2.478 | 3.226 | 0.548 | 4.487 | 0.0001 |
| 563.5013 | 1204 | 5.244 | 0.403 | 2.149 | 0.406 | 2.440 | 0.0001 |
| 572.4455 | 1204 | 5.719 | 0.661 | 1.843 | 0.336 | 3.103 | 0.0001 |
| 327.0307 | 1204 | 8.810 | 0.616 | 5.574 | 0.325 | 1.581 | 0.0002 |
| 490.3676 | 1204 | 7.893 | 1.158 | 2.550 | 0.235 | 3.095 | 0.0002 |
| 574.4594 | 1204 | 41.888 | 6.536 | 9.914 | 1.605 | 4.225 | 0.0002 |
| 558.4649 | 1204 | 49.295 | 7.166 | 14.427 | 2.763 | 3.417 | 0.0003 |
| 559.4688 | 1204 | 18.364 | 2.624 | 5.551 | 0.957 | 3.308 | 0.0003 |
| 562.4989 | 1204 | 14.237 | 1.259 | 6.489 | 1.040 | 2.194 | 0.0003 |
| 575.4628 | 1204 | 16.105 | 2.497 | 4.318 | 0.711 | 3.730 | 0.0003 |
| 560.478 | 1203 | 9.998 | 0.752 | 6.482 | 0.540 | 1.542 | 0.0004 |
| 478.4044 | 1204 | 4.058 | 0.374 | 2.096 | 0.311 | 1.936 | 0.0006 |
| 530.4379 | 1204 | 6.139 | 0.698 | 2.640 | 0.429 | 2.325 | 0.0006 |
| 546.4298 | 1204 | 5.422 | 0.846 | 1.797 | 0.298 | 3.017 | 0.0006 |
| 557.4527 | 1204 | 4.641 | 0.521 | 2.044 | 0.378 | 2.271 | 0.0007 |
| 558.4663 | 1202 | 5.148 | 0.667 | 1.907 | 0.395 | 2.700 | 0.0007 |
| 612.4994 | 1204 | 8.097 | 0.941 | 3.294 | 0.473 | 2.458 | 0.0007 |
| 506.4338 | 1204 | 4.086 | 0.311 | 2.302 | 0.323 | 1.775 | 0.001 |
| 556.4497 | 1204 | 11.083 | 1.357 | 4.554 | 0.976 | 2.434 | 0.001 |
| 574.4635 | 1202 | 3.793 | 0.533 | 1.474 | 0.208 | 2.573 | 0.001 |
| 560.4821 | 1204 | 27.410 | 2.617 | 12.279 | 2.740 | 2.232 | 0.0011 |
| 561.4863 | 1204 | 11.089 | 1.073 | 5.196 | 1.045 | 2.134 | 0.0013 |
| 462.3716 | 1204 | 3.329 | 0.305 | 1.759 | 0.265 | 1.893 | 0.0031 |
| 856.6045 | 1102 | 7.793 | 1.319 | 6.545 | 0.691 | 1.191 | 0.0036 |
| 854.5884 | 1102 | 4.407 | 0.729 | 3.471 | 0.311 | 1.270 | 0.0042 |
| 634.3951 | 1204 | 11.113 | 2.036 | 4.104 | 0.836 | 2.708 | 0.0046 |
| 855.6009 | 1102 | 15.163 | 2.457 | 12.786 | 1.331 | 1.186 | 0.0047 |
| 519.3322 | 1101 | 5.790 | 0.368 | 4.417 | 0.460 | 1.311 | 0.0048 |
| 564.513 | 1204 | 5.141 | 0.573 | 2.558 | 0.458 | 2.010 | 0.005 |
| 611.3724 | 1201 | 5.966 | 0.607 | 3.351 | 0.968 | 1.780 | 0.0066 |
| 895.5575 | 1102 | 6.457 | 1.242 | 5.002 | 0.523 | 1.291 | 0.0109 |
| 853.5848 | 1102 | 8.298 | 1.332 | 6.301 | 0.585 | 1.317 | 0.0113 |
| 541.3141 | 1101 | 6.800 | 0.568 | 4.815 | 0.562 | 1.412 | 0.0121 |
| 610.5204 | 1204 | 18.173 | 4.912 | 7.931 | 1.596 | 2.291 | 0.0141 |
| 662.4267 | 1204 | 7.459 | 1.139 | 4.182 | 0.445 | 1.784 | 0.016 |
| 570.3766 | 1201 | 2.962 | 0.303 | 1.895 | 0.477 | 1.563 | 0.0171 |
| 827.5695 | 1102 | 26.452 | 5.262 | 20.283 | 2.505 | 1.304 | 0.0172 |
| 886.7804 | 1203 | 10.774 | 2.558 | 6.148 | 2.114 | 1.752 | 0.0192 |
| 546.3413 | 1204 | 4.855 | 0.708 | 2.749 | 0.410 | 1.766 | 0.0195 |
| 610.3691 | 1201 | 16.117 | 1.748 | 10.304 | 3.000 | 1.564 | 0.022 |
| 378.9906 | 1204 | 4.292 | 0.313 | 3.160 | 0.309 | 1.358 | 0.0233 |
| 570.376 | 1203 | 3.876 | 0.750 | 1.590 | 0.318 | 2.438 | 0.0239 |
| 162.1412 | 1203 | 6.102 | 0.386 | 6.220 | 0.259 | 0.981 | 0.0244 |
| 810.5967 | 1201 | 17.404 | 2.577 | 29.950 | 3.444 | 0.581 | 0.0247 |
| 835.6094 | 1201 | 3.249 | 0.425 | 5.886 | 0.673 | 0.552 | 0.0251 |
| 785.4799 | 1204 | 10.252 | 2.193 | 21.576 | 4.246 | 0.475 | 0.026 |
| 606.4872 | 1204 | 7.177 | 1.330 | 4.058 | 0.558 | 1.769 | 0.0281 |
| 639.4037 | 1201 | 3.594 | 0.326 | 2.974 | 0.457 | 1.208 | 0.0281 |
| 797.5257 | 1204 | 47.197 | 3.685 | 69.761 | 11.106 | 0.677 | 0.0296 |
| 264.9759 | 1204 | 7.268 | 0.440 | 5.734 | 0.361 | 1.268 | 0.0311 |
| 809.5934 | 1201 | 34.411 | 5.094 | 58.757 | 6.906 | 0.586 | 0.0313 |
| 828.5732 | 1102 | 12.694 | 2.293 | 9.640 | 1.101 | 1.317 | 0.0337 |
| 744.55 | 1203 | 9.050 | 1.592 | 6.269 | 1.174 | 1.444 | 0.0347 |
| 831.5758 | 1101 | 6.461 | 0.619 | 7.739 | 0.919 | 0.835 | 0.035 |
| 590.4964 | 1204 | 5.436 | 1.167 | 3.151 | 0.719 | 1.725 | 0.0382 |
| 795.5083 | 1204 | 30.689 | 2.188 | 40.150 | 5.184 | 0.764 | 0.039 |
| 769.4929 | 1204 | 65.355 | 5.025 | 88.784 | 12.171 | 0.736 | 0.0397 |
| 743.5475 | 1203 | 19.547 | 3.139 | 14.226 | 2.394 | 1.374 | 0.041 |
| 181.9806 | 1102 | 5.873 | 0.891 | 2.752 | 0.581 | 2.134 | 0.0422 |
| 748.5722 | 1102 | 12.839 | 2.174 | 9.039 | 0.655 | 1.420 | 0.0437 |
| 200.1566 | 1203 | 8.329 | 0.174 | 7.692 | 0.276 | 1.083 | 0.044 |
| 729.5727 | 1204 | 9.886 | 1.145 | 5.989 | 0.859 | 1.651 | 0.044 |
| 638.4003 | 1201 | 9.386 | 1.059 | 7.767 | 1.259 | 1.208 | 0.0446 |
| 832.6027 | 1202 | 26.976 | 3.806 | 14.960 | 2.367 | 1.803 | 0.0484 |
| 160.1256 | 1203 | 7.941 | 0.470 | 8.253 | 0.387 | 0.962 | 0.0489 |
| 566.3433 | 1202 | 18.924 | 1.569 | 15.150 | 0.718 | 1.249 | 0.0497 |

**Table 20: Accurate mass features differing between 10 RR- MULTIPLE SCLEROSIS patients transitioning to SP- MULTIPLE SCLEROSIS and 10 clinically diagnosed RR- MULTIPLE SCLEROSIS patients (p<0.05).**

| | | | | | | | |
|---|---|---|---|---|---|---|---|
| 580.5089 | 1204 | 4.522 | 0.292 | 18.548 | 1.208 | 0.244 | 7.78E-09 |
| 450.3729 | 1204 | 4.558 | 0.182 | 11.434 | 0.666 | 0.399 | 1.23E-08 |
| 578.4923 | 1204 | 12.054 | 1.113 | 56.683 | 4.013 | 0.213 | 1.66E-08 |
| 579.4958 | 1204 | 4.683 | 0.442 | 22.165 | 1.599 | 0.211 | 2.08E-08 |
| 448.3562 | 1204 | 5.180 | 0.347 | 13.087 | 0.778 | 0.396 | 3.80E-08 |
| 495.4018 | 1204 | 1.616 | 0.212 | 6.267 | 0.490 | 0.258 | 1.48E-07 |
| 581.5126 | 1204 | 1.907 | 0.240 | 7.358 | 0.552 | 0.259 | 1.68E-07 |
| 577.4795 | 1204 | 5.058 | 0.413 | 25.693 | 2.730 | 0.197 | 4.26E-07 |
| 550.4602 | 1204 | 3.541 | 0.391 | 14.589 | 1.385 | 0.243 | 4.63E-07 |
| 576.4757 | 1204 | 12.734 | 1.088 | 70.329 | 7.812 | 0.181 | 5.18E-07 |
| 484.3788 | 1204 | 3.788 | 0.327 | 10.428 | 0.737 | 0.363 | 5.84E-07 |
| 466.3656 | 1204 | 5.919 | 0.636 | 17.958 | 1.455 | 0.330 | 8.21E-07 |
| 551.4646 | 1204 | 1.432 | 0.191 | 5.465 | 0.509 | 0.262 | 9.00E-07 |
| 523.4337 | 1204 | 1.899 | 0.208 | 7.018 | 0.643 | 0.271 | 9.27E-07 |
| 561.4863 | 1204 | 5.053 | 0.475 | 12.026 | 0.798 | 0.420 | 9.30E-07 |
| 560.4821 | 1204 | 12.218 | 1.146 | 29.622 | 1.988 | 0.412 | 9.53E-07 |
| 494.3968 | 1204 | 5.859 | 0.504 | 19.493 | 1.724 | 0.301 | 9.75E-07 |
| 569.4769 | 1204 | 1.544 | 0.195 | 7.995 | 0.867 | 0.193 | 9.80E-07 |
| 512.4079 | 1204 | 5.655 | 0.647 | 19.615 | 1.654 | 0.288 | 1.05E-06 |
| 522.4313 | 1204 | 6.534 | 0.460 | 21.358 | 1.990 | 0.306 | 1.37E-06 |
| 598.5107 | 1204 | 3.116 | 0.474 | 22.331 | 2.356 | 0.140 | 1.53E-06 |
| 568.4723 | 1204 | 3.894 | 0.304 | 19.878 | 2.356 | 0.196 | 1.71E-06 |
| 504.4188 | 1204 | 3.450 | 0.155 | 7.928 | 0.540 | 0.435 | 1.91E-06 |
| 562.4989 | 1204 | 7.498 | 0.721 | 15.447 | 0.968 | 0.485 | 2.12E-06 |
| 521.4188 | 1204 | 2.171 | 0.302 | 8.244 | 0.832 | 0.263 | 2.22E-06 |
| 510.3937 | 1204 | 2.563 | 0.308 | 8.533 | 0.806 | 0.300 | 2.37E-06 |
| 595.4883 | 1204 | 11.511 | 1.238 | 75.048 | 9.692 | 0.153 | 2.53E-06 |
| 468.384 | 1204 | 8.388 | 0.798 | 25.639 | 2.296 | 0.327 | 2.54E-06 |
| 594.4848 | 1204 | 28.935 | 3.071 | 189.742 | 25.019 | 0.152 | 2.70E-06 |
| 476.3869 | 1204 | 2.471 | 0.222 | 5.512 | 0.344 | 0.448 | 2.71E-06 |
| 467.3711 | 1204 | 2.178 | 0.201 | 5.899 | 0.500 | 0.369 | 3.30E-06 |
| 559.4688 | 1204 | 5.645 | 0.677 | 20.172 | 2.260 | 0.280 | 3.64E-06 |
| 596.5012 | 1204 | 36.743 | 5.025 | 292.674 | 33.424 | 0.126 | 3.78E-06 |
| 548.4438 | 1204 | 2.435 | 0.275 | 10.928 | 1.436 | 0.223 | 4.42E-06 |
| 597.5062 | 1204 | 14.057 | 1.806 | 102.408 | 12.272 | 0.137 | 4.58E-06 |
| 452.3868 | 1204 | 1.961 | 0.284 | 4.714 | 0.297 | 0.416 | 4.71E-06 |
| 513.4116 | 1204 | 2.091 | 0.224 | 6.369 | 0.543 | 0.328 | 4.96E-06 |
| 469.3863 | 1204 | 2.527 | 0.300 | 6.732 | 0.561 | 0.375 | 5.15E-06 |
| 536.41 | 1204 | 4.313 | 0.401 | 14.234 | 1.396 | 0.303 | 5.27E-06 |
| 520.4131 | 1204 | 6.574 | 0.399 | 22.631 | 2.629 | 0.290 | 5.54E-06 |
| 496.4157 | 1204 | 4.416 | 0.420 | 16.630 | 1.813 | 0.266 | 5.67E-06 |
| 524.4448 | 1204 | 2.358 | 0.254 | 6.952 | 0.680 | 0.339 | 5.81E-06 |
| 558.4649 | 1204 | 15.025 | 1.572 | 53.745 | 6.329 | 0.280 | 6.09E-06 |
| 532.4503 | 1204 | 2.369 | 0.411 | 5.793 | 0.358 | 0.409 | 6.62E-06 |
| 610.482 | 1204 | 3.393 | 0.359 | 13.517 | 1.487 | 0.251 | 7.29E-06 |
| 596.5053 | 1202 | 3.960 | 0.749 | 21.759 | 2.655 | 0.182 | 7.39E-06 |
| 502.4054 | 1204 | 3.334 | 0.240 | 8.085 | 0.715 | 0.412 | 8.26E-06 |
| 492.3832 | 1204 | 4.090 | 0.389 | 13.443 | 1.453 | 0.304 | 9.14E-06 |
| 519.3998 | 1204 | 1.708 | 0.258 | 6.099 | 0.655 | 0.280 | 9.59E-06 |
| 597.5068 | 1202 | 1.593 | 0.313 | 8.239 | 1.011 | 0.193 | 1.17E-05 |
| 508.3782 | 1204 | 2.261 | 0.381 | 6.852 | 0.600 | 0.330 | 1.24E-05 |
| 534.3912 | 1204 | 1.750 | 0.274 | 6.155 | 0.599 | 0.284 | 1.41E-05 |
| 440.3526 | 1204 | 2.084 | 0.264 | 5.422 | 0.458 | 0.384 | 1.42E-05 |
| 541.4422 | 1204 | 3.411 | 0.379 | 20.925 | 3.127 | 0.163 | 1.44E-05 |
| 572.4455 | 1204 | 1.914 | 0.316 | 6.467 | 0.678 | 0.296 | 1.47E-05 |
| 590.4585 | 1204 | 4.211 | 0.410 | 15.828 | 1.796 | 0.266 | 1.60E-05 |
| 518.3969 | 1204 | 5.017 | 0.475 | 16.609 | 1.870 | 0.302 | 1.61E-05 |
| 557.4527 | 1204 | 2.731 | 0.271 | 5.357 | 0.471 | 0.510 | 1.62E-05 |
| 566.454 | 1204 | 3.340 | 0.340 | 13.366 | 1.792 | 0.250 | 1.63E-05 |
| 552.4784 | 1204 | 3.072 | 0.232 | 10.252 | 1.204 | 0.300 | 1.65E-05 |
| 482.3604 | 1204 | 2.387 | 0.293 | 6.828 | 0.684 | 0.350 | 1.72E-05 |
| 540.4387 | 1204 | 11.181 | 0.938 | 62.043 | 9.171 | 0.180 | 1.76E-05 |
| 594.4875 | 1202 | 2.688 | 0.532 | 13.976 | 1.893 | 0.192 | 1.78E-05 |
| 438.3354 | 1204 | 1.732 | 0.203 | 4.427 | 0.393 | 0.391 | 1.83E-05 |
| 464.3524 | 1204 | 4.499 | 0.474 | 12.320 | 1.229 | 0.365 | 2.20E-05 |
| 480.3473 | 1204 | 2.129 | 0.160 | 5.005 | 0.415 | 0.425 | 2.20E-05 |
| 537.4142 | 1204 | 1.910 | 0.208 | 5.646 | 0.543 | 0.338 | 2.22E-05 |
| 447.3433 | 1204 | 2.234 | 0.247 | 5.264 | 0.490 | 0.424 | 2.81E-05 |
| 563.5013 | 1204 | 3.247 | 0.289 | 5.586 | 0.321 | 0.581 | 3.14E-05 |
| 570.4903 | 1204 | 1.448 | 0.184 | 5.518 | 0.681 | 0.262 | 3.18E-05 |
| 618.4834 | 1201 | 1.560 | 0.468 | 8.422 | 1.166 | 0.185 | 3.74E-05 |
| 595.4928 | 1202 | 1.284 | 0.199 | 6.004 | 0.829 | 0.214 | 4.03E-05 |
| 591.4614 | 1204 | 1.801 | 0.306 | 5.617 | 0.656 | 0.321 | 4.23E-05 |
| 474.3731 | 1204 | 2.347 | 0.232 | 6.267 | 0.722 | 0.375 | 4.26E-05 |
| 478.4044 | 1204 | 2.307 | 0.091 | 4.386 | 0.373 | 0.526 | 4.46E-05 |
| 538.4257 | 1204 | 10.245 | 0.846 | 43.969 | 6.163 | 0.233 | 4.48E-05 |
| 446.341 | 1204 | 7.325 | 0.584 | 17.044 | 1.670 | 0.430 | 4.74E-05 |
| 462.3346 | 1204 | 2.077 | 0.306 | 4.729 | 0.424 | 0.439 | 0.0001 |
| 493.385 | 1204 | 1.231 | 0.155 | 4.527 | 0.575 | 0.272 | 0.0001 |
| 506.4338 | 1204 | 1.982 | 0.248 | 4.196 | 0.336 | 0.472 | 0.0001 |
| 534.4645 | 1204 | 2.424 | 0.211 | 4.518 | 0.357 | 0.537 | 0.0001 |
| 556.4497 | 1204 | 6.643 | 0.820 | 12.691 | 1.212 | 0.523 | 0.0001 |
| 574.4594 | 1204 | 9.293 | 1.092 | 47.951 | 7.271 | 0.194 | 0.0001 |
| 575.4628 | 1204 | 3.830 | 0.446 | 18.688 | 2.716 | 0.205 | 0.0001 |
| 576.4765 | 1202 | 1.606 | 0.237 | 5.861 | 0.728 | 0.274 | 0.0001 |
| 592.4717 | 1204 | 11.135 | 1.295 | 53.655 | 7.672 | 0.208 | 0.0001 |
| 593.4736 | 1204 | 4.494 | 0.518 | 22.565 | 3.209 | 0.199 | 0.0001 |
| 546.4298 | 1204 | 1.747 | 0.279 | 6.038 | 0.876 | 0.289 | 0.0002 |
| 558.4663 | 1202 | 1.899 | 0.327 | 5.605 | 0.622 | 0.339 | 0.0002 |
| 564.4396 | 1204 | 1.712 | 0.210 | 4.715 | 0.573 | 0.363 | 0.0002 |
| 616.4675 | 1201 | 1.703 | 0.290 | 6.509 | 0.964 | 0.262 | 0.0003 |
| 490.3676 | 1204 | 2.714 | 0.463 | 8.438 | 1.224 | 0.322 | 0.0005 |
| 327.0307 | 1204 | 6.681 | 0.286 | 9.139 | 0.550 | 0.731 | 0.0008 |
| 530.4379 | 1204 | 3.253 | 0.519 | 6.685 | 0.667 | 0.487 | 0.0009 |
| 612.4994 | 1204 | 3.768 | 0.404 | 8.583 | 0.926 | 0.439 | 0.0009 |
| 574.4635 | 1202 | 1.366 | 0.201 | 4.237 | 0.626 | 0.322 | 0.001 |
| 590.4964 | 1204 | 3.310 | 0.570 | 5.836 | 1.064 | 0.567 | 0.0011 |
| 564.513 | 1204 | 2.805 | 0.511 | 5.374 | 0.449 | 0.522 | 0.0012 |
| 606.4872 | 1204 | 5.701 | 0.704 | 7.360 | 1.168 | 0.775 | 0.0014 |
| 610.5204 | 1204 | 10.685 | 2.101 | 18.659 | 4.768 | 0.573 | 0.0021 |
| 539.4274 | 1204 | 3.329 | 0.516 | 13.055 | 2.878 | 0.255 | 0.0027 |
| 804.5715 | 1102 | 23.445 | 5.126 | 46.944 | 8.322 | 0.499 | 0.0042 |
| 871.5526 | 1102 | 10.664 | 2.211 | 20.033 | 3.436 | 0.532 | 0.0042 |
| 803.5683 | . 1102 | 57.160 | 13.215 | 116.596 | 21.332 | 0.490 | 0.0068 |
| 733.6414 | 1204 | 22.549 | 2.616 | 15.527 | 1.847 | 1.452 | 0.0125 |
| 829.5851 | 1102 | 20.138 | 2.786 | 39.735 | 6.642 | 0.507 | 0.0131 |
| 872.5557 | 1102 | 5.121 | 1.092 | 9.357 | 1.681 | 0.547 | 0.0132 |
| 569.369 | 1202 | 17.157 | 1.105 | 13.563 | 0.716 | 1.265 | 0.0147 |
| 603.5305 | 1201 | 1.757 | 0.345 | 4.631 | 1.000 | 0.379 | 0.0154 |
| 899.5871 | 1102 | 6.673 | 1.249 | 11.918 | 1.901 | 0.560 | 0.0163 |
| 576.5115 | 1201 | 4.861 | 1.392 | 15.148 | 3.461 | 0.321 | 0.0165 |
| 604.5428 | 1201 | 1.324 | 0.133 | 2.470 | 0.401 | 0.536 | 0.0183 |
| 601.515 | 1201 | 1.735 | 0.300 | 3.771 | 0.676 | 0.460 | 0.0204 |
| 707.6248 | 1204 | 12.429 | 2.115 | 8.439 | 1.276 | 1.473 | 0.0207 |
| 859.7715 | 1201 | 2.313 | 0.462 | 6.009 | 1.199 | 0.385 | 0.0227 |
| 856.7527 | 1201 | 4.217 | 1.499 | 13.836 | 3.049 | 0.305 | 0.0229 |
| 602.5271 | 1201 | 3.644 | 0.789 | 9.846 | 2.283 | 0.370 | 0.0239 |
| 600.5115 | 1201 | 3.502 | 0.762 | 9.257 | 2.057 | 0.378 | 0.0245 |
| 577.5148 | 1201 | 2.335 | 0.557 | 6.227 | 1.401 | 0.375 | 0.0253 |
| 719.6222 | 1204 | 17.497 | 2.578 | 12.859 | 1.904 | 1.361 | 0.0258 |
| 734.6429 | 1204 | 12.854 | 1.562 | 9.336 | 1.071 | 1.377 | 0.0258 |
| 687.4916 | 1204 | 22.840 | 3.156 | 37.699 | 5.234 | 0.606 | 0.0267 |
| 757.5622 | 1101 | 39.148 | 3.393 | 48.113 | 5.667 | 0.814 | 0.0274 |
| 784.5809 | 1101 | 9.351 | 0.779 | 10.960 | 1.260 | 0.853 | 0.0284 |
| 296.2357 | 1204 | 9.614 | 0.724 | 11.283 | 0.721 | 0.852 | 0.0288 |
| 574.4958 | 1201 | 4.124 | 0.837 | 9.107 | 1.722 | 0.453 | 0.0288 |
| 634.3951 | 1204 | 6.136 | 1.289 | 10.597 | 1.635 | 0.579 | 0.0302 |
| 758.5656 | 1101 | 20.676 | 1.792 | 25.277 | 2.995 | 0.818 | 0.0306 |
| 830.5881 | 1102 | 9.353 | 1.250 | 17.793 | 2.882 | 0.526 | 0.0309 |
| 260.2137 | 1203 | 5.046 | 0.345 | 5.892 | 0.213 | 0.856 | 0.031 |
| 854.737 | 1201 | 3.193 | 0.952 | 9.115 | 1.923 | 0.350 | 0.031 |
| 462.3716 | 1204 | 2.409 | 0.361 | 3.400 | 0.244 | 0.709 | 0.0313 |
| 686.4879 | 1204 | 53.749 | 7.668 | 90.851 | 13.784 | 0.592 | 0.0338 |
| 239.939 | 1102 | 4.050 | 0.706 | 5.407 | 0.631 | 0.749 | 0.0347 |
| 611.3724 | 1201 | 3.100 | 0.337 | 4.945 | 0.611 | 0.627 | 0.0349 |
| 673.4765 | 1204 | 7.595 | 1.041 | 10.654 | 0.589 | 0.713 | 0.0355 |
| 721.6382 | 1204 | 21.966 | 3.380 | 15.879 | 2.227 | 1.383 | 0.0356 |
| 550.4958 | 1201 | 1.889 | 0.508 | 5.003 | 1.096 | 0.378 | 0.036 |
| 857.7557 | 1201 | 3.271 | 0.869 | 8.666 | 1.828 | 0.377 | 0.0362 |
| 897.5729 | 1102 | 5.114 | 0.690 | 9.533 | 1.628 | 0.536 | 0.0387 |
| 735.6554 | 1204 | 22.156 | 3.657 | 15.289 | 1.767 | 1.449 | 0.0388 |
| 712.5074 | 1204 | 21.951 | 1.994 | 34.704 | 4.558 | 0.633 | 0.042 |
| 438.2993 | 1204 | 1.840 | 0.322 | 1.128 | 0.128 | 1.631 | 0.043 |
| 830.5634 | 1201 | 6.463 | 0.700 | 4.807 | 0.558 | 1.344 | 0.044 |
| 834.5372 | 1204 | 16.169 | 1.535 | 13.200 | 1.532 | 1.225 | 0.044 |
| 705.6086 | 1204 | 7.811 | 1.337 | 5.498 | 0.726 | 1.421 | 0.0461 |
| 598.4959 | 1201 | 2.137 | 0.341 | 3.489 | 0.474 | 0.612 | 0.0492 |

**Table 21: Accurate mass features differing between 10 RR- MULTIPLE SCLEROSIS patients transitioning to SP- MULTIPLE SCLEROSIS and 10 clinically diagnosed SP- MULTIPLE SCLEROSIS patients (p<0.05).**

| | | | | | | | |
|---|---|---|---|---|---|---|---|
| 761.529 | 1204 | 44.870 | 1.690 | 17.080 | 1.359 | 2.627 | 5.04E-10 |
| 760.5231 | 1204 | 109.100 | 5.078 | 36.556 | 3.639 | 2.984 | 2.17E-09 |
| 690.4843 | 1204 | 10.221 | 0.348 | 2.876 | 0.621 | 3.554 | 1.80E-08 |
| 758.5089 | 1204 | 220.433 | 11.793 | 77.867 | 7.929 | 2.831 | 1.86E-08 |
| 759.5145 | 1204 | 157.006 | 9.162 | 49.129 | 5.813 | 3.196 | 2.08E-08 |
| 784.5238 | 1204 | 110.088 | 6.582 | 37.629 | 3.285 | 2.926 | 2.20E-08 |
| 732.4929 | 1204 | 64.274 | 3.909 | 19.285 | 2.366 | 3.333 | 2.35E-08 |
| 742.4745 | 1204 | 11.943 | 0.592 | 5.829 | 0.243 | 2.049 | 3.27E-08 |
| 785.5287 | 1204 | 55.424 | 3.489 | 19.112 | 1.576 | 2.900 | 3.71E-08 |
| 812.5559 | 1204 | 27.252 | 1.631 | 10.799 | 0.817 | 2.524 | 7.82E-08 |
| 786.5408 | 1204 | 131.847 | 8.335 | 48.312 | 4.378 | 2.729 | 1.00E-07 |
| 787.5452 | 1204 | 60.356 | 3.628 | 23.335 | 2.087 | 2.586 | 1.08E-07 |
| 744.4942 | 1204 | 201.989 | 11.571 | 84.491 | 6.859 | 2.391 | 1.29E-07 |
| 733.501 | 1204 | 37.219 | 2.804 | 10.249 | 1.378 | 3.631 | 1.44E-07 |
| 731.4898 | 1204 | 139.168 | 10.350 | 37.560 | 5.674 | 3.705 | 1.55E-07 |
| 809.5264 | 1204 | 26.756 | 1.700 | 9.447 | 1.067 | 2.832 | 1.58E-07 |
| 770.5108 | 1204 | 106.431 | 6.784 | 45.101 | 2.511 | 2.360 | 1.74E-07 |
| 808.5225 | 1204 | 51.852 | 3.250 | 18.231 | 2.201 | 2.844 | 1.78E-07 |
| 734.508 | 1204 | 18.236 | 1.160 | 3.835 | 1.208 | 4.755 | 2.00E-07 |
| 788.5549 | 1204 | 22.912 | 1.296 | 10.186 | 0.813 | 2.249 | 2.60E-07 |
| 452.2536 | 1204 | 6.868 | 0.446 | 2.179 | 0.383 | 3.152 | 5.17E-07 |
| 780.4907 | 1204 | 16.058 | 1.092 | 6.003 | 0.661 | 2.675 | 5.41E-07 |
| 772.5265 | 1204 | 133.872 | 8.862 | 58.392 | 4.253 | 2.293 | 7.09E-07 |
| 757.5008 | 1204 | 61.528 | 5.970 | 15.151 | 1.849 | 4.061 | 1.03E-06 |
| 746.5118 | 1204 | 82.872 | 4.102 | 31.127 | 5.409 | 2.662 | 1.11E-06 |
| 810.5394 | 1204 | 89.171 | 6.119 | 33.859 | 4.327 | 2.634 | 1.34E-06 |
| 811.5436 | 1204 | 43.601 | 3.103 | 16.663 | 1.976 | 2.617 | 1.44E-06 |
| 836.5534 | 1204 | 10.314 | 0.657 | 4.772 | 0.395 | 2.161 | 1.68E-06 |
| 688.4658 | 1204 | 11.938 | 0.707 | 4.376 | 0.766 | 2.728 | 1.98E-06 |
| 794.5126 | 1204 | 60.457 | 4.186 | 26.308 | 2.449 | 2.298 | 2.32E-06 |
| 756.491 | 1204 | 31.271 | 2.881 | 9.848 | 1.070 | 3.175 | 2.38E-06 |
| 814.498 | 1204 | 13.596 | 1.071 | 3.697 | 0.945 | 3.677 | 3.28E-06 |
| 813.5617 | 1204 | 12.536 | 0.749 | 5.919 | 0.599 | 2.118 | 3.34E-06 |
| 781.497 | 1204 | 19.276 | 1.841 | 5.709 | 0.839 | 3.376 | 4.04E-06 |
| 779.4829 | 1204 | 12.474 | 1.000 | 3.574 | 0.849 | 3.490 | 4.20E-06 |
| 766.4759 | 1204 | 13.713 | 0.797 | 6.185 | 0.774 | 2.217 | 4.49E-06 |
| 783.5127 | 1204 | 85.018 | 7.493 | 29.020 | 3.903 | 2.930 | 4.83E-06 |
| 782.5084 | 1204 | 137.563 | 11.554 | 50.082 | 6.507 | 2.747 | 5.22E-06 |
| 718.4736 | 1204 | 7.136 | 0.524 | 2.565 | 0.481 | 2.782 | 8.51E-06 |
| 617.0921 | 1204 | 309.018 | 21.686 | 158.144 | 10.806 | 1.954 | 1.03E-05 |
| 712.4676 | 1204 | 10.933 | 0.771 | 4.225 | 0.746 | 2.588 | 1.20E-05 |
| 807.5103 | 1204 | 26.009 | 1.764 | 11.300 | 1.589 | 2.302 | 1.30E-05 |
| 716.4987 | 1204 | 25.836 | 1.753 | 13.642 | 1.011 | 1.894 | 1.58E-05 |
| 806.5068 | 1204 | 47.988 | 3.146 | 21.094 | 3.089 | 2.275 | 1.62E-05 |
| 755.4854 | 1204 | 48.306 | 5.655 | 12.351 | 2.199 | 3.911 | 1.76E-05 |
| 796.5278 | 1204 | 99.834 | 7.696 | 46.021 | 4.705 | 2.169 | 1.80E-05 |
| 816.5159 | 1204 | 9.394 | 0.509 | 5.772 | 0.339 | 1.628 | 1.98E-05 |
| 717.5011 | 1204 | 11.886 | 0.757 | 6.288 | 0.571 | 1.890 | 2.16E-05 |
| 379.2536 | 1204 | 3.809 | 0.419 | 1.217 | 0.160 | 3.130 | 2.37E-05 |
| 768.4944 | 1204 | 140.648 | 11.913 | 61.674 | 6.965 | 2.281 | 2.87E-05 |
| 835.5417 | 1204 | 10.463 | 0.567 | 5.827 | 0.576 | 1.796 | 3.32E-05 |
| 154.0035 | 1204 | 29.040 | 2.027 | 16.527 | 1.444 | 1.757 | 0.0001 |
| 306.2568 | 1204 | 9.934 | 0.611 | 5.961 | 0.492 | 1.667 | 0.0001 |
| 420.2651 | 1204 | 4.402 | 0.296 | 2.352 | 0.283 | 1.872 | 0.0001 |
| 712.5074 | 1204 | 27.892 | 2.096 | 14.835 | 1.259 | 1.880 | 0.0001 |
| 721.6382 | 1204 | 21.940 | 1.994 | 9.600 | 1.382 | 2.285 | 0.0001 |
| 815.5045 | 1204 | 10.228 | 0.990 | 3.882 | 0.751 | 2.635 | 0.0001 |
| 832.5211 | 1204 | 7.326 | 0.502 | 3.616 | 0.495 | 2.026 | 0.0001 |
| 834.5372 | 1204 | 18.398 | 1.170 | 9.245 | 1.179 | 1.990 | 0.0001 |
| 713.5097 | 1204 | 12.216 | 0.831 | 6.999 | 0.727 | 1.745 | 0.0002 |
| 740.4966 | 1204 | 21.462 | 1.753 | 12.268 | 0.852 | 1.749 | 0.0002 |
| 765.4894 | 1204 | 16.458 | 1.357 | 8.044 | 1.028 | 2.046 | 0.0002 |
| 780.5303 | 1204 | 10.368 | 0.680 | 5.616 | 0.727 | 1.846 | 0.0002 |
| 788.4794 | 1204 | 12.719 | 0.884 | 5.698 | 1.146 | 2.232 | 0.0002 |
| 872.6715 | 1204 | 1.743 | 0.360 | 4.635 | 0.476 | 0.376 | 0.0002 |
| 313.2702 | 1101 | 2.194 | 0.770 | 7.148 | 0.719 | 0.307 | 0.0003 |
| 714.5221 | 1204 | 35.796 | 2.655 | 19.874 | 2.112 | 1.801 | 0.0003 |
| 569.3687 | 1102 | 7.896 | 0.726 | 3.826 | 0.545 | 2.064 | 0.0004 |
| 690.5475 | 1204 | 6.802 | 0.612 | 3.252 | 0.496 | 2.092 | 0.0004 |
| 737.5045 | 1204 | 6.986 | 0.614 | 3.496 | 0.476 | 1.998 | 0.0004 |
| 789.5658 | 1204 | 11.139 | 0.523 | 6.630 | 0.841 | 1.680 | 0.0004 |
| 792.4954 | 1204 | 54.222 | 4.145 | 29.312 | 3.671 | 1.850 | 0.0004 |
| 686.4879 | 1204 | 64.699 | 6.152 | 34.789 | 3.217 | 1.860 | 0.0005 |
| 738.5185 | 1204 | 25.027 | 2.301 | 13.047 | 1.625 | 1.918 | 0.0006 |
| 757.5637 | 1204 | 13.400 | 0.954 | 7.554 | 0.966 | 1.774 | 0.0006 |
| 707.6248 | 1204 | 12.254 | 1.130 | 5.983 | 0.952 | 2.048 | 0.0007 |
| 736.5031 | 1204 | 14.979 | 1.418 | 7.958 | 0.879 | 1.882 | 0.0007 |
| 742.5142 | 1204 | 25.071 | 2.044 | 15.355 | 1.078 | 1.633 | 0.0007 |
| 886.5582 | 1102 | 9.158 | 1.042 | 4.660 | 0.365 | 1.965 | 0.0008 |
| 784.6228 | 1204 | 25.713 | 4.239 | 6.186 | 2.336 | 4.157 | 0.0009 |
| 820.5294 | 1204 | 23.887 | 1.921 | 13.544 | 1.608 | 1.764 | 0.0009 |
| 313.7724 | 1101 | 6.709 | 1.105 | 1.625 | 0.625 | 4.129 | 0.001 |
| 687.4916 | 1204 | 26.877 | 2.482 | 15.555 | 1.326 | 1.728 | 0.001 |
| 997.3968 | 1102 | 7.284 | 0.537 | 4.347 | 0.490 | 1.676 | 0.001 |
| 747.5121 | 1204 | 54.593 | 3.233 | 37.326 | 2.827 | 1.463 | 0.0011 |
| 735.6554 | 1204 | 20.623 | 2.274 | 10.165 | 1.429 | 2.029 | 0.0013 |
| 745.4938 | 1204 | 130.623 | 9.940 | 84.200 | 6.457 | 1.551 | 0.0013 |
| 495.3322 | 1201 | 3.373 | 0.314 | 5.878 | 0.549 | 0.574 | 0.0014 |
| 783.6174 | 1204 | 41.517 | 7.569 | 8.434 | 4.065 | 4.923 | 0.0014 |
| 688.5048 | 1204 | 26.182 | 2.869 | 14.168 | 1.295 | 1.848 | 0.0015 |
| 689.5083 | 1204 | 10.722 | 0.992 | 6.291 | 0.591 | 1.704 | 0.0015 |
| 771.5075 | 1204 | 73.947 | 6.085 | 47.202 | 3.498 | 1.567 | 0.0015 |
| 633.3232 | 1102 | 4.844 | 0.484 | 2.605 | 0.335 | 1.860 | 0.0016 |
| 773.5257 | 1204 | 93.493 | 6.310 | 60.586 | 5.795 | 1.543 | 0.0016 |
| 748.5722 | 1102 | 16.264 | 1.896 | 8.476 | 0.842 | 1.919 | 0.0017 |
| 770.569 | 1204 | 53.299 | 4.207 | 34.160 | 2.822 | 1.560 | 0.0017 |
| 812.6122 | 1201 | 3.134 | 0.525 | 6.820 | 0.801 | 0.460 | 0.0018 |
| 302.2255 | 1204 | 4.500 | 0.351 | 2.451 | 0.424 | 1.836 | 0.0022 |
| 1019.384 | 1102 | 7.026 | 0.536 | 4.415 | 0.461 | 1.591 | 0.0022 |
| 565.3391 | 1102 | 25.394 | 2.373 | 13.580 | 2.172 | 1.870 | 0.0023 |
| 715.4864 | 1204 | 18.244 | 1.938 | 10.740 | 0.788 | 1.699 | 0.0024 |
| 566.3431 | 1102 | 7.641 | 0.753 | 4.138 | 0.607 | 1.847 | 0.0025 |
| 794.5718 | 1204 | 28.883 | 2.201 | 18.210 | 1.958 | 1.586 | 0.0025 |
| 567.3547 | 1102 | 13.308 | 1.127 | 7.473 | 1.149 | 1.781 | 0.0026 |
| 833.5929 | 1101 | 4.412 | 0.340 | 3.011 | 0.199 | 1.465 | 0.0027 |
| 738.5448 | 1102 | 4.708 | 0.497 | 2.639 | 0.309 | 1.784 | 0.0028 |
| 719.6222 | 1204 | 16.138 | 1.622 | 9.274 | 1.107 | 1.740 | 0.0031 |
| 341.2443 | 1204 | 1.816 | 0.203 | 1.073 | 0.073 | 1.692 | 0.0032 |
| 795.5083 | 1204 | 45.741 | 3.585 | 29.214 | 3.139 | 1.566 | 0.0035 |
| 766.5153 | 1204 | 16.517 | 1.630 | 9.478 | 1.247 | 1.743 | 0.0037 |
| 714.4837 | 1204 | 38.123 | 4.503 | 22.200 | 1.520 | 1.717 | 0.0039 |
| 854.589 | 1202 | 12.123 | 1.922 | 5.379 | 0.618 | 2.254 | 0.004 |
| 722.5244 | 1204 | 8.482 | 0.797 | 4.920 | 0.683 | 1.724 | 0.0041 |
| 872.5557 | 1102 | 7.287 | 1.023 | 3.637 | 0.395 | 2.003 | 0.0041 |
| 541.3415 | 1102 | 24.790 | 2.463 | 14.323 | 1.902 | 1.731 | 0.0042 |
| 694.4953 | 1204 | 4.820 | 0.383 | 3.093 | 0.338 | 1.558 | 0.0042 |
| 749.5762 | 1102 | 7.246 | 0.867 | 4.089 | 0.392 | 1.772 | 0.0043 |
| 747.5761 | 1204 | 15.794 | 1.109 | 10.817 | 0.989 | 1.460 | 0.0045 |
| 854.5884 | 1102 | 6.310 | 0.977 | 3.038 | 0.217 | 2.077 | 0.0045 |
| 887.797 | 1203 | 7.782 | 1.662 | 1.939 | 0.632 | 4.014 | 0.0045 |
| 711.4947 | 1204 | 16.498 | 1.821 | 9.295 | 1.190 | 1.775 | 0.0046 |
| 769.5638 | 1204 | 114.290 | 10.000 | 74.462 | 6.683 | 1.535 | 0.0046 |
| 858.6843 | 1102 | 6.363 | 1.403 | 1.499 | 0.499 | 4.245 | 0.0047 |
| 861.5265 | 1102 | 6.739 | 0.942 | 3.423 | 0.386 | 1.969 | 0.0048 |
| 304.241 | 1204 | 35.178 | 2.794 | 23.043 | 2.384 | 1.527 | 0.0049 |
| 181.9806 | 1102 | 5.211 | 0.559 | 2.924 | 0.419 | 1.782 | 0.0051 |
| 280.2413 | 1204 | 153.282 | 19.301 | 81.661 | 11.092 | 1.877 | 0.0055 |
| 772.5842 | 1204 | 65.761 | 4.877 | 44.401 | 4.424 | 1.481 | 0.0056 |
| 830.5881 | 1102 | 14.539 | 2.244 | 7.110 | 0.677 | 2.045 | 0.0057 |
| 542.3447 | 1102 | 6.998 | 0.664 | 4.179 | 0.572 | 1.675 | 0.0059 |
| 281.2447 | 1204 | 29.936 | 3.733 | 16.187 | 2.186 | 1.849 | 0.006 |
| 696.4733 | 1204 | 10.289 | 2.321 | 2.124 | 1.124 | 4.844 | 0.006 |
| 744.5516 | 1204 | 132.030 | 11.098 | 87.905 | 8.188 | 1.502 | 0.006 |
| 699.4908 | 1204 | 7.332 | 0.636 | 4.685 | 0.530 | 1.565 | 0.0061 |
| 788.6128 | 1201 | 2.019 | 0.397 | 4.095 | 0.504 | 0.493 | 0.0062 |
| 853.5852 | 1202 | 23.738 | 3.949 | 10.596 | 1.444 | 2.240 | 0.0063 |
| 734.488 | 1204 | 15.884 | 2.046 | 8.871 | 0.912 | 1.791 | 0.0064 |
| 243.0719 | 1101 | 27.999 | 1.874 | 20.630 | 1.397 | 1.357 | 0.0066 |
| 256.24 | 1202 | 2.203 | 0.657 | 5.225 | 0.686 | 0.422 | 0.0066 |
| 345.8738 | 1101 | 6.834 | 0.780 | 3.405 | 0.747 | 2.007 | 0.0066 |
| 715.5228 | 1204 | 19.602 | 1.668 | 12.192 | 1.629 | 1.608 | 0.0066 |
| 746.5701 | 1204 | 50.317 | 4.437 | 33.781 | 2.855 | 1.489 | 0.0067 |
| 787.5995 | 1201 | 3.802 | 1.068 | 8.895 | 1.191 | 0.427 | 0.0067 |
| 897.5729 | 1102 | 7.234 | 1.101 | 3.570 | 0.427 | 2.026 | 0.0067 |
| 477.3218 | 1201 | 3.453 | 0.463 | 5.932 | 0.628 | 0.582 | 0.007 |
| 710.4916 | 1204 | 35.981 | 4.256 | 20.330 | 2.695 | 1.770 | 0.007 |
| 794.5419 | 1102 | 6.943 | 0.889 | 4.079 | 0.280 | 1.702 | 0.007 |
| 765.5704 | 1204 | 7.199 | 1.796 | 1.505 | 0.505 | 4.783 | 0.0073 |
| 829.5852 | 1202 | 51.097 | 8.689 | 23.637 | 2.394 | 2.162 | 0.0073 |
| 277.8861 | 1101 | 14.484 | 1.330 | 8.729 | 1.282 | 1.659 | 0.0075 |
| 803.5681 | 1202 | 107.752 | 18.096 | 50.203 | 5.542 | 2.146 | 0.0075 |
| 743.5461 | 1204 | 311.426 | 28.757 | 202.118 | 20.807 | 1.541 | 0.0076 |
| 830.5885 | 1202 | 22.923 | 3.777 | 11.038 | 1.041 | 2.077 | 0.0076 |
| 829.5851 | 1102 | 31.727 | 5.202 | 15.489 | 1.333 | 2.048 | 0.0077 |
| 825.5532 | 1202 | 4.799 | 0.761 | 2.209 | 0.389 | 2.172 | 0.008 |
| 827.5694 | 1202 | 62.787 | 10.158 | 29.412 | 4.314 | 2.135 | 0.008 |
| 773.5954 | 1204 | 28.422 | 2.040 | 19.815 | 1.943 | 1.434 | 0.0085 |
| 793.5381 | 1102 | 14.713 | 2.099 | 8.058 | 0.764 | 1.826 | 0.0086 |
| 828.5734 | 1202 | 29.459 | 4.653 | 14.227 | 2.071 | 2.071 | 0.0086 |
| 555.3102 | 1102 | 6.492 | 0.756 | 3.572 | 0.600 | 1.818 | 0.0087 |
| 804.5714 | 1202 | 45.461 | 7.448 | 22.273 | 2.328 | 2.041 | 0.0087 |
| 144.0944 | 1203 | 5.913 | 0.351 | 7.175 | 0.232 | 0.824 | 0.009 |
| 160.1256 | 1203 | 6.763 | 0.421 | 8.308 | 0.301 | 0.814 | 0.0093 |
| 809.5932 | 1101 | 15.522 | 1.647 | 10.274 | 0.674 | 1.511 | 0.0093 |
| 847.5315 | 1201 | 6.021 | 0.711 | 3.555 | 0.427 | 1.694 | 0.0093 |
| 1127.741 | 1204 | 3.217 | 0.760 | 1.000 | 0.000 | 3.217 | 0.0094 |
| 871.5526 | 1102 | 14.999 | 2.355 | 7.613 | 0.893 | 1.970 | 0.0096 |
| 531.312 | 1102 | 6.157 | 0.560 | 3.797 | 0.556 | 1.621 | 0.0097 |
| 824.5477 | 1201 | 2.172 | 0.368 | 1.078 | 0.078 | 2.015 | 0.0097 |
| 767.5827 | 1201 | 3.881 | 0.389 | 7.191 | 1.015 | 0.540 | 0.0099 |
| 634.4267 | 1201 | 1.649 | 0.225 | 1.000 | 0.000 | 1.649 | 0.01 |
| 757.5622 | 1201 | 98.678 | 7.301 | 147.948 | 14.540 | 0.667 | 0.01 |
| 797.5257 | 1204 | 75.239 | 6.508 | 49.628 | 5.701 | 1.516 | 0.0101 |
| 698.4885 | 1204 | 15.802 | 1.637 | 10.577 | 0.753 | 1.494 | 0.0104 |
| 305.2439 | 1204 | 7.384 | 0.624 | 5.028 | 0.505 | 1.469 | 0.0105 |
| 793.5986 | 1201 | 5.506 | 0.560 | 10.176 | 1.451 | 0.541 | 0.0108 |
| 805.5832 | 1202 | 35.953 | 5.189 | 20.118 | 1.873 | 1.787 | 0.0109 |
| 771.5792 | 1204 | 136.073 | 11.270 | 92.313 | 9.966 | 1.474 | 0.0113 |
| 798.6019 | 1204 | 14.243 | 1.131 | 9.780 | 1.037 | 1.456 | 0.0113 |
| 801.5543 | 1202 | 5.002 | 0.852 | 2.386 | 0.338 | 2.096 | 0.0113 |
| 856.6698 | 1102 | 6.227 | 0.961 | 3.318 | 0.356 | 1.877 | 0.0117 |
| 360.1467 | 1201 | 3.600 | 0.662 | 6.748 | 0.851 | 0.533 | 0.0118 |
| 739.4827 | 1204 | 11.114 | 1.363 | 6.723 | 0.728 | 1.653 | 0.012 |
| 742.5366 | 1204 | 14.637 | 1.420 | 9.248 | 1.224 | 1.583 | 0.0121 |
| 745.5643 | 1204 | 111.991 | 10.785 | 75.580 | 6.918 | 1.482 | 0.0123 |
| 806.5865 | 1202 | 15.199 | 2.072 | 8.845 | 0.893 | 1.718 | 0.0124 |
| 638.5138 | 1204 | 16.487 | 2.611 | 8.215 | 1.368 | 2.007 | 0.0129 |
| 821.5714 | 1102 | 9.269 | 1.328 | 5.241 | 0.559 | 1.769 | 0.0129 |
| 729.5727 | 1204 | 7.812 | 0.649 | 5.126 | 0.683 | 1.524 | 0.0131 |
| 852.5726 | 1202 | 9.104 | 1.529 | 4.422 | 0.690 | 2.059 | 0.0131 |
| 260.2507 | 1204 | 16.191 | 1.538 | 10.701 | 1.205 | 1.513 | 0.0135 |
| 593.3416 | 1204 | 1.607 | 0.248 | 3.779 | 0.717 | 0.425 | 0.0144 |
| 793.5663 | 1204 | 51.823 | 4.721 | 34.525 | 4.078 | 1.501 | 0.0148 |
| 758.5655 | 1201 | 49.966 | 3.650 | 73.507 | 7.476 | 0.680 | 0.015 |
| 796.5864 | 1204 | 43.191 | 4.094 | 28.514 | 3.400 | 1.515 | 0.0152 |
| 1225.093 | 1203 | 11.951 | 2.621 | 3.439 | 1.681 | 3.475 | 0.0154 |
| 501.3217 | 1201 | 3.393 | 0.263 | 5.242 | 0.602 | 0.647 | 0.0156 |
| 428.295 | 1204 | 5.120 | 0.550 | 2.795 | 0.634 | 1.832 | 0.0157 |
| 766.5372 | 1204 | 14.283 | 1.111 | 9.541 | 1.307 | 1.497 | 0.0159 |
| 817.5374 | 1102 | 6.843 | 1.095 | 3.764 | 0.350 | 1.818 | 0.0161 |
| 448.3194 | 1204 | 3.018 | 0.318 | 4.428 | 0.400 | 0.682 | 0.0162 |
| 832.6022 | 1102 | 19.054 | 3.035 | 10.450 | 1.065 | 1.823 | 0.0163 |
| 666.5449 | 1204 | 30.717 | 5.437 | 14.525 | 2.657 | 2.115 | 0.0167 |
| 278.2254 | 1204 | 14.617 | 3.007 | 5.989 | 1.213 | 2.441 | 0.017 |
| 134.11 | 1203 | 10.616 | 0.728 | 12.993 | 0.503 | 0.817 | 0.0171 |
| 736.4951 | 1201 | 1.974 | 0.296 | 3.154 | 0.318 | 0.626 | 0.0173 |
| 759.5779 | 1201 | 36.709 | 2.675 | 55.828 | 6.376 | 0.658 | 0.0173 |
| 787.6095 | 1201 | 3.839 | 1.154 | 8.481 | 1.266 | 0.453 | 0.0176 |
| 279.2284 | 1204 | 3.218 | 0.622 | 1.424 | 0.270 | 2.260 | 0.0177 |
| 338.2461 | 1204 | 2.353 | 0.284 | 3.961 | 0.516 | 0.594 | 0.018 |
| 851.5686 | 1202 | 17.271 | 3.006 | 8.654 | 1.290 | 1.996 | 0.018 |
| 853.5848 | 1102 | 11.637 | 2.132 | 5.890 | 0.529 | 1.976 | 0.0181 |
| 589.3398 | 1102 | 9.802 | 1.051 | 5.292 | 1.297 | 1.852 | 0.0182 |
| 440.308 | 1201 | 2.501 | 0.752 | 6.005 | 1.056 | 0.417 | 0.0184 |
| 769.4929 | 1204 | 100.639 | 10.775 | 65.749 | 7.683 | 1.531 | 0.019 |
| 759.5779 | 1101 | 20.424 | 2.309 | 13.845 | 1.041 | 1.475 | 0.0195 |
| 454.2969 | 1201 | 2.600 | 0.647 | 6.918 | 1.462 | 0.376 | 0.0196 |
| 283.2602 | 1204 | 39.989 | 6.317 | 20.305 | 4.126 | 1.969 | 0.0199 |
| 786.5967 | 1201 | 23.862 | 1.962 | 35.935 | 4.045 | 0.664 | 0.02 |
| 282.2572 | 1204 | 208.013 | 33.546 | 104.163 | 21.783 | 1.997 | 0.0204 |
| 785.5934 | 1201 | 49.962 | 3.937 | 74.679 | 8.353 | 0.669 | 0.0204 |
| 194.0803 | 1203 | 9.310 | 2.283 | 3.242 | 0.734 | 2.872 | 0.0219 |
| 612.4994 | 1204 | 4.837 | 0.644 | 3.004 | 0.327 | 1.610 | 0.0223 |
| 810.5966 | 1101 | 7.551 | 0.886 | 5.184 | 0.311 | 1.457 | 0.0223 |
| 146.11 | 1203 | 5.303 | 0.322 | 6.310 | 0.229 | 0.840 | 0.0227 |
| 722.486 | 1204 | 9.551 | 0.914 | 6.518 | 0.757 | 1.465 | 0.0229 |
| 741.5302 | 1204 | 27.862 | 3.508 | 17.251 | 2.288 | 1.615 | 0.0231 |
| 678.4528 | 1201 | 2.508 | 0.327 | 3.923 | 0.439 | 0.639 | 0.0232 |
| 765.5316 | 1204 | 26.722 | 2.313 | 17.611 | 2.680 | 1.517 | 0.0232 |
| 279.9312 | 1102 | 5.532 | 0.590 | 3.566 | 0.499 | 1.551 | 0.0234 |
| 681.5631 | 1204 | 5.859 | 1.282 | 2.128 | 0.748 | 2.753 | 0.0237 |
| 158.1101 | 1203 | 6.179 | 0.353 | 7.354 | 0.299 | 0.840 | 0.0238 |
| 831.5992 | 1102 | 42.798 | 7.551 | 23.095 | 2.383 | 1.853 | 0.0238 |
| 899.5871 | 1102 | 9.096 | 1.561 | 5.053 | 0.463 | 1.800 | 0.024 |
| 760.5811 | 1201 | 17.113 | 1.223 | 25.455 | 2.973 | 0.672 | 0.0242 |
| 799.5401 | 1204 | 22.934 | 2.034 | 16.153 | 1.745 | 1.420 | 0.0242 |
| 804.5715 | 1102 | 33.353 | 5.961 | 18.084 | 1.583 | 1.844 | 0.0242 |
| 856.6048 | 1202 | 18.380 | 2.965 | 10.122 | 1.484 | 1.816 | 0.0245 |
| 150.1413 | 1203 | 4.270 | 0.313 | 5.372 | 0.303 | 0.795 | 0.0247 |
| 640.5285 | 1204 | 19.392 | 3.594 | 9.651 | 1.655 | 2.009 | 0.0257 |
| 806.5863 | 1102 | 12.671 | 2.471 | 6.480 | 0.601 | 1.955 | 0.0262 |
| 462.3716 | 1204 | 2.921 | 0.398 | 1.744 | 0.262 | 1.675 | 0.0264 |
| 678.5469 | 1204 | 12.126 | 3.131 | 3.709 | 1.422 | 3.269 | 0.0265 |
| 797.5973 | 1204 | 28.011 | 2.531 | 19.814 | 2.126 | 1.414 | 0.0267 |
| 760.581 | 1101 | 9.590 | 1.054 | 6.767 | 0.476 | 1.417 | 0.0268 |
| 304.1111 | 1202 | 3.207 | 0.871 | 5.725 | 0.541 | 0.560 | 0.0269 |
| 738.4806 | 1204 | 20.803 | 3.350 | 11.861 | 1.518 | 1.754 | 0.0274 |
| 664.5313 | 1204 | 27.632 | 5.581 | 12.747 | 2.590 | 2.168 | 0.0281 |
| 803.5683 | 1102 | 83.168 | 15.663 | 44.392 | 3.974 | 1.873 | 0.0282 |
| 855.6011 | 1202 | 36.927 | 6.113 | 20.484 | 2.974 | 1.803 | 0.0282 |
| 674.4902 | 1204 | 8.970 | 0.728 | 6.706 | 0.575 | 1.338 | 0.0286 |
| 255.2283 | 1204 | 4.079 | 0.561 | 2.202 | 0.524 | 1.852 | 0.0291 |
| 781.5619 | 1204 | 9.821 | 0.767 | 6.454 | 1.145 | 1.522 | 0.031 |
| 828.5732 | 1102 | 17.547 | 3.618 | 8.840 | 0.788 | 1.985 | 0.031 |
| 446.219 | 1201 | 2.849 | 0.439 | 4.644 | 0.593 | 0.613 | 0.0313 |
| 172.1255 | 1203 | 6.279 | 0.331 | 7.346 | 0.297 | 0.855 | 0.0314 |
| 330.2569 | 1204 | 3.124 | 0.365 | 2.136 | 0.203 | 1.463 | 0.0316 |
| 827.5695 | 1102 | 37.092 | 7.880 | 18.168 | 1.787 | 2.042 | 0.0316 |
| 794.602 | 1201 | 2.964 | 0.305 | 4.814 | 0.690 | 0.616 | 0.0318 |
| 700.5037 | 1204 | 17.186 | 1.533 | 12.592 | 1.177 | 1.365 | 0.0323 |
| 720.5081 | 1204 | 5.498 | 0.665 | 3.262 | 0.656 | 1.685 | 0.0324 |
| 832.6027 | 1202 | 26.701 | 4.628 | 15.120 | 1.758 | 1.766 | 0.0325 |
| 768.5525 | 1204 | 84.034 | 8.929 | 56.831 | 7.192 | 1.479 | 0.0328 |
| 246.1468 | 1201 | 3.199 | 0.597 | 6.521 | 1.233 | 0.491 | 0.0331 |
| 252.2096 | 1204 | 2.857 | 0.418 | 1.603 | 0.327 | 1.782 | 0.0332 |
| 832.5793 | 1101 | 4.657 | 0.579 | 3.138 | 0.294 | 1.484 | 0.0333 |
| 831.5995 | 1202 | 58.297 | 10.567 | 32.015 | 4.088 | 1.821 | 0.0338 |
| 664.4374 | 1201 | 2.661 | 0.255 | 3.734 | 0.370 | 0.713 | 0.0344 |
| 701.5064 | 1204 | 7.572 | 0.687 | 5.461 | 0.588 | 1.387 | 0.0355 |
| 752.4902 | 1201 | 2.178 | 0.299 | 3.211 | 0.322 | 0.678 | 0.0356 |
| 856.6045 | 1102 | 11.620 | 2.428 | 5.891 | 0.628 | 1.973 | 0.0356 |
| 609.324 | 1102 | 4.496 | 0.485 | 3.054 | 0.386 | 1.472 | 0.036 |
| 702.5676 | 1101 | 4.257 | 0.546 | 2.826 | 0.297 | 1.506 | 0.0361 |
| 340.2621 | 1204 | 3.898 | 0.427 | 5.495 | 0.529 | 0.709 | 0.0364 |
| 1227.091 | 1203 | 10.143 | 2.889 | 19.624 | 2.874 | 0.517 | 0.0369 |
| 782.565 | 1101 | 16.036 | 1.944 | 11.211 | 0.883 | 1.430 | 0.0385 |
| 452.244 | 1201 | 5.926 | 0.441 | 7.858 | 0.702 | 0.754 | 0.0388 |
| 805.5833 | 1102 | 28.734 | 6.015 | 15.001 | 1.230 | 1.916 | 0.0388 |
| 884.709 | 1204 | 6.673 | 1.627 | 2.508 | 0.862 | 2.661 | 0.0388 |
| 512.3347 | 1201 | 3.117 | 0.364 | 6.248 | 1.279 | 0.499 | 0.039 |
| 156.0943 | 1203 | 3.520 | 0.264 | 4.172 | 0.120 | 0.844 | 0.0396 |
| 662.5164 | 1204 | 15.920 | 3.832 | 6.974 | 1.209 | 2.283 | 0.0402 |
| 783.5778 | 1201 | 50.681 | 3.561 | 70.630 | 7.811 | 0.718 | 0.0404 |
| 795.5814 | 1204 | 81.007 | 8.721 | 55.971 | 6.822 | 1.447 | 0.0406 |
| 868.7141 | 1204 | 6.709 | 1.697 | 2.424 | 0.889 | 2.768 | 0.0408 |
| 734.6429 | 1204 | 12.043 | 1.235 | 8.376 | 1.054 | 1.438 | 0.0412 |
| 148.1257 | 1203 | 7.248 | 0.541 | 8.624 | 0.296 | 0.840 | 0.0414 |
| 303.1081 | 1102 | 4.733 | 0.404 | 2.761 | 0.755 | 1.714 | 0.0414 |
| 438.2924 | 1201 | 1.630 | 0.290 | 3.698 | 0.851 | 0.441 | 0.0429 |
| 786.5965 | 1101 | 13.950 | 1.980 | 9.293 | 0.754 | 1.501 | 0.0429 |
| 781.5617 | 1101 | 32.132 | 3.891 | 22.886 | 1.609 | 1.404 | 0.0433 |
| 833.5931 | 1201 | 8.927 | 1.272 | 14.084 | 1.894 | 0.634 | 0.0439 |
| 811.6093 | 1201 | 8.938 | 1.013 | 13.019 | 1.497 | 0.687 | 0.0442 |
| 807.5754 | 1101 | 23.270 | 3.292 | 15.705 | 1.123 | 1.482 | 0.0447 |
| 257.1709 | 1201 | 4.487 | 0.776 | 9.661 | 2.145 | 0.464 | 0.0453 |
| 228.1362 | 1201 | 23.118 | 2.605 | 44.117 | 8.870 | 0.524 | 0.0455 |
| 767.5473 | 1204 | 179.498 | 20.657 | 121.949 | 16.310 | 1.472 | 0.0469 |
| 823.5427 | 1204 | 10.161 | 0.841 | 7.863 | 0.633 | 1.292 | 0.0469 |
| 821.4768 | 1204 | 7.624 | 0.798 | 11.838 | 1.704 | 0.644 | 0.0471 |
| 702.5676 | 1201 | 2.900 | 0.357 | 3.945 | 0.316 | 0.735 | 0.0473 |
| 855.6009 | 1102 | 23.110 | 5.234 | 11.619 | 1.220 | 1.989 | 0.0473 |
| 718.5348 | 1204 | 6.442 | 0.665 | 4.178 | 0.783 | 1.542 | 0.0475 |
| 162.1412 | 1203 | 5.557 | 0.362 | 6.454 | 0.204 | 0.861 | 0.0476 |
| 606.4872 | 1204 | 7.849 | 1.273 | 4.484 | 0.885 | 1.750 | 0.0477 |
| 784.5811 | 1201 | 24.262 | 1.796 | 33.594 | 3.775 | 0.722 | 0.0477 |
| 895.5575 | 1102 | 8.641 | 1.842 | 4.628 | 0.422 | 1.867 | 0.0487 |
| 705.6086 | 1204 | 7.166 | 0.897 | 4.887 | 0.562 | 1.466 | 0.0489 |
| 242.1519 | 1201 | 4.098 | 0.605 | 6.579 | 0.951 | 0.623 | 0.0494 |
| 264.9759 | 1204 | 6.810 | 0.414 | 5.630 | 0.354 | 1.210 | 0.0494 |

**Table 22: Accurate masses, mode of ionization, putative molecular formulae and proposed structures for multiple sclerosis biomarkers detected in aqueous and organic extracts of human serum.**

| | **Detected Mass** | **Exact Mass** | **Mode** | **Formula** | **Proposed Structure** |
|---|---|---|---|---|---|
| **1** | 452.3868 | 452.3866 | 1204 | C₂₈H₅₂O₄ | 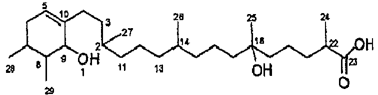 |
| **2** | 496.4157 | 496.4128 | 1204 | C₃₀H₅₆O₅ | 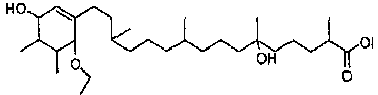 |
| **3** | 524.4448 | 524.4441 | 1204 | C₃₂H₆₀O₅ | 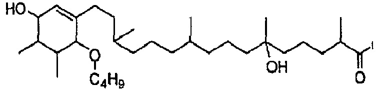 |
| **4** | 540.4387 | 540.4390 | 1204 | C₃₂H₆₀O₆ | 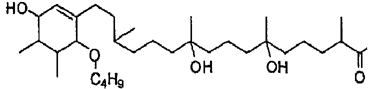 |
| **5** | 576.4757 | 576.4754 | 1204 | C₃₆H₆₄O₅ | 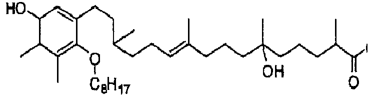 |
| **6** | 578.4923 | 578.4910 | 1204 | C₃₆H₆₆O₅ | 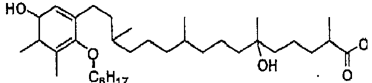 |
| **7** | 580.5089 | 580.5067 | 1204 | C₃₆H₆₈O₅ | 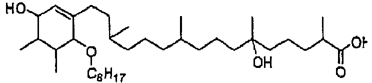 |
| **8** | 594.4848 | 594.4859 | 1204 | C₃₆H₆₆O₆ | 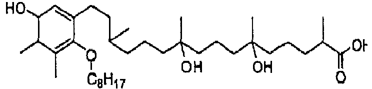 |
| **9** | 596.5012 | 596.5016 | 1204 | C₃₆H₆₈O₆ | 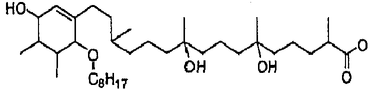 |
| **10** | 786.5408 | 786.5411 | 1204 | C₄₃H₇₉O₁₀P | 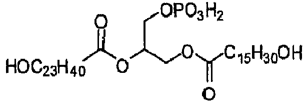 |
| **11** | 216.04 | 216.0399 | 1102 | C₅H₁₃O₇P | 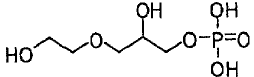 |
| **12** | 541.3415 | 541.3379 | 1102 | C₂₅H₅₂NO₉P | 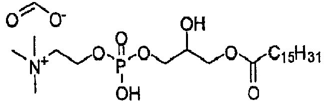 |
| **13** | 565.3391 | 565.3380 | 1102 | C₂₇H₅₂NO₉P | 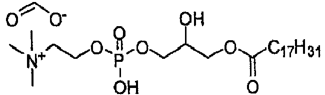 |
| **14** | 202.0453 | 202.0453 | 1101 | C₆H₁₁O₆Na | 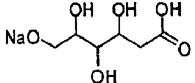 |
| **15** | 244.0559 | 244.0559 | 1101 | C₈H₁₃O₇Na | 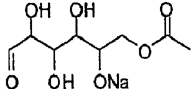 |
| **16** | 428.3653 | 428.3654 | 1201 | C₂₉H₄₈O₂ | 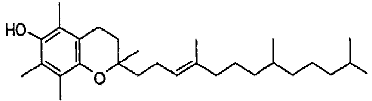 |
| **17** | 805.5609 | 805.5621 | 1201 | C₄₆H₈₀NO₈P | 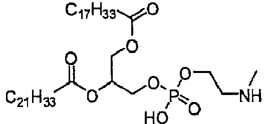 |
| **18** | 194.0803 | 194.0790 | 1203 | C₇H₁₄O₆ | 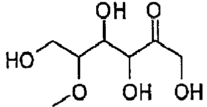 |
| **19** | 857.7516 | 857.7472 | 1203 | C₅₄H₉₉NO₆ | 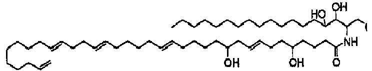 |

**Table 23: MS/MS fragmentation of multiple sclerosis biomarker 1, 452.3868 (C₂₈H₅₂O₄)**

| **m/z** | **Formula** | **Molecular fragment** | **Fragment loss** |
|---|---|---|---|
| 451 | C₂₈H₅₁O₄ | 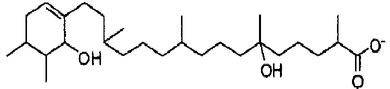 | -H⁺ |
| 433 | C₂₈H₄₃O₃ | 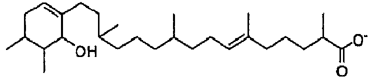 | -H₂O |
| 407 | C₂₇H₅₁O₂ | 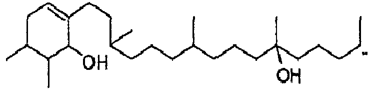 | -CO₂ |
| 389 | C₂₇H₄₉O | 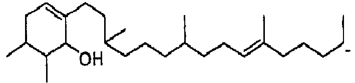 | 433 - CO₂ |
| 281 | C₁₈H₃₃O₂ | 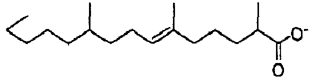 | 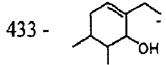 |
| 279 | C₁₈H₃₁O₂ | 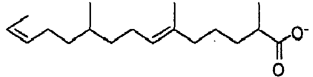 | 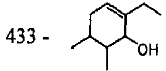 |
| 183 | C₁₁H₁₉O₂ | 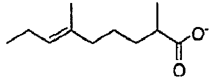 | 279-C₇H₁₂ |
| 169 | C₁₀H₁₇O₂ | 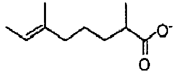 | 279-C₈H₁₄ |
| 153 | C₁₀H₁₇O | 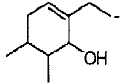 | -phytol chain |
| 139 | C₉H₁₅O | 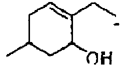 | 153-CH₄ |
| 125 | C₈H₁₃O | 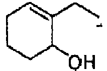 | 139-CH₄ |
| 111 | C₇H₁₁O | 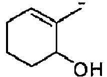 | 125-CH₄ |
| 97 | C₆H₉O | 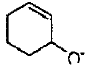 | 111-CH₄ |

**Table 24: MS/MS fragmentation of multiple sclerosis biomarker 2, 496.4157 (C₃₀H₅₆O₅)**

| **m/z** | **Formula** | **Molecular fragment** | **Fragment loss** |
|---|---|---|---|
| 495 | C₃₀H₅₅O₅ | 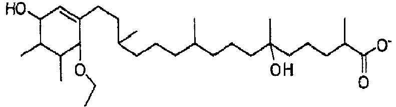 | -H⁺ |
| 477 | C₃₀H₅₃O₃ | 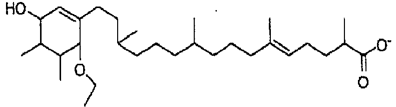 | -H₂O |
| 451 | C₂₉H₅₅O₃ | 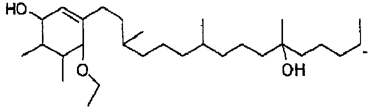 | -CO₂ |
| 433 | C₂₉H₅₃O₂ | 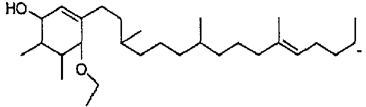 | -(CO₂ + H₂O) |
| 415 | C₂₉H₅₁O | 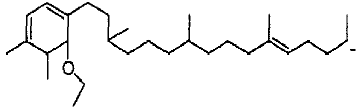 | -(CO₂ + 2H₂O) |
| 307 | C₂₀H₃₅O₂ | 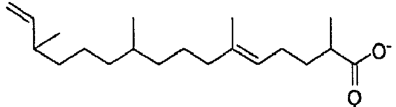 | 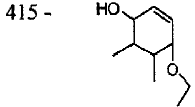 |
| 297 | C₁₈H₃₃O₃ | 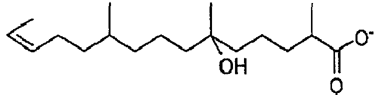 | 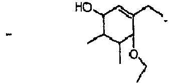 |
| 279 | C₁₈H₃₁O₂ | 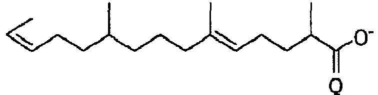 | 297-H₂O |
| 235 | C₁₆H₂₇O | 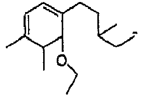 | 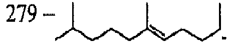 |
| 223 | C₁₄H₂₃O₂ | 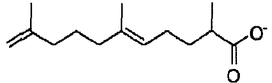 | 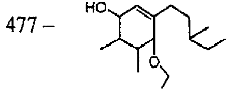 |
| 215 | C₁₂H₂₃O₂ | 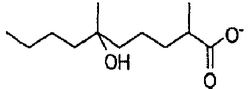 | Fragmentation at C13-C14 and loss of CH₃ |
| 197 | C₁₂H₂₁O₂ | 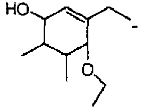 | -phytol chain |
| 179 | C₁₂H₁₉O | 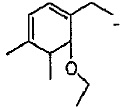 | 197-H₂O |
| 181 | C₁₃H₂₅ | 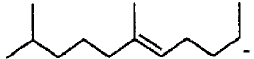 | 415-235 |
| 169 | C₁₀H₁₇O₂ | 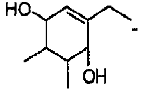 | 179-C₂H₄ |
| 157 | C₈H₁₃O₃ | 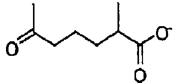 | 215-C₄H₁₀ |
| 155 | C₉H₁₅O₂ | 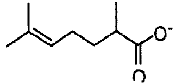 | 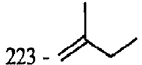 |
| 153 | C₁₀H₁₇O | 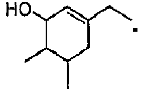 | 197-C₂H₄O |
| 141 | C₉H₁₇O | 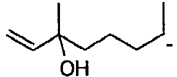 | 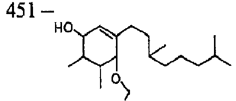 |
| 139 | C₉H₁₅O | 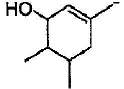 | 153-CH₄ |
| 127 | C₈H₁₅O | 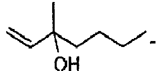 | 141-CH₂ |
| 125 | C₈H₁₃O | 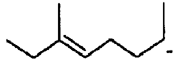 | 184-C₄H₈ |
| 113 | C₆H₉O₂ | 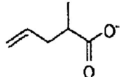 | 157-C₂H₄O |

**Table 25: MS/MS fragmentation of multiple sclerosis biomarker 3, 524.4448 (C₃₂H₆₀O₅)**

| **m/z** | **Formula** | **Molecular fragment** | **Fragment loss** |
|---|---|---|---|
| 523 | C₃₂H₅₉O₅ | 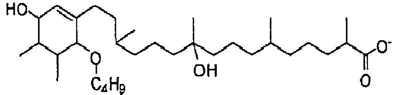 | - H⁺ |
| 505 | C₃₂H₅₇O₄ | 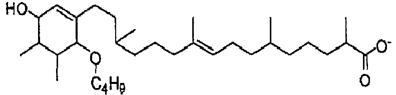 | - H₂O |
| 487 | C₃₂H₅₅O₃ | 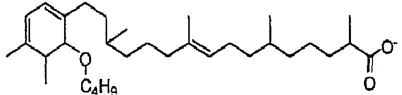 | - 2xH₂O |
| 479 | C₃₁H₅₉O₃ | 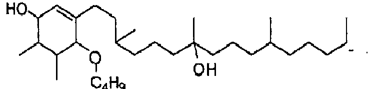 | -CO₂ |
| 463 | C₃₀H₅₅O₃ | 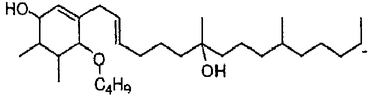 | 479-CH₄ |
| 461 | C₃₁H₅₇O₂ | 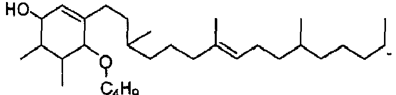 | -(CO₂ + H₂O) |
| 443 | C₃₁H₅₅O | 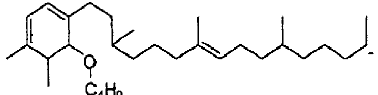 O C4H9 | -(CO₂ + 2H₂O) |
| 365 | C₂₃H₄₁O₃ | 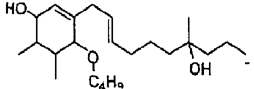 | 463 - C₇H₁₃ |
| 337 | C₂₁H₃₇O₃ | 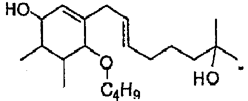 | 365 - C₂H₄ |
| 299 | C₁₈H₃₅O₃ | 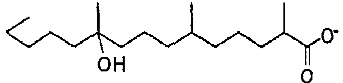 | 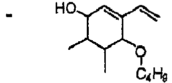 |
| 297 | C₁₈H₃₃O₃ | 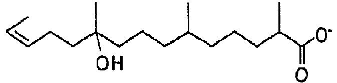 | 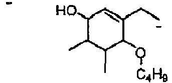 |
| 281 | C₁₈H₃₃O₂ | 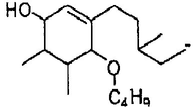 | 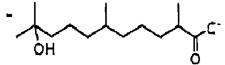 |
| 279 | C₁₈H₃₁O₂ | 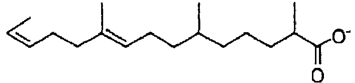 | 297 - H₂O |
| 271 | C₁₆H₃₁O₃ | 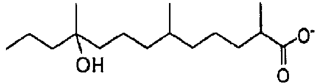 | 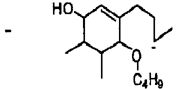 |
| 269 | C₁₆H₂₉O₃ | 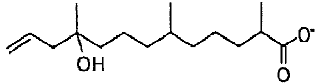 | 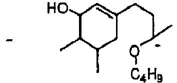 |
| 253 | C₁₆H₂₉O₂ | 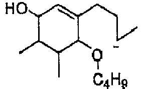 | -271 |
| 251 | C₁₆H₂₇O₂ | 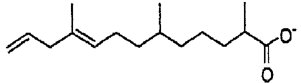 | 269 - H₂O |
| 243 | C₁₄H₂₇O₃ | 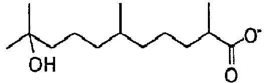 | -281 |
| 225 | C₁₄H₂₅O₂ | 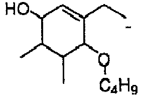 | - phytol chain |
| 197 | C₁₂H₂₁O₂ | 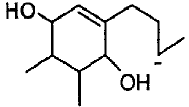 | 253- C₄H₈ |
| 171 | C₁₀H₁₉O₂ | 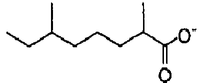 | 251- C₆H₈ |
| 169 | C₁₀H₁₇O₂ | 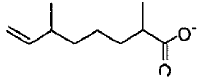 | 251 - C₆H₁₀ |
| 157 | C₉H₁₇O2 | 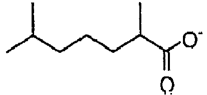 | 271 - CH₂ |
| 155 | C₉H₁₅O₂ | 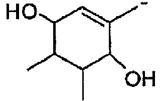 | 197 - C₃H₆ |
| 143 | C₈H₁₅O₂ | 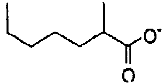 | 157 - CH₂ |
| 141 | C₉H₁₇O | 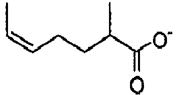 | 157 - CH₄ |
| 139 | C₈H₁₁O₂ | 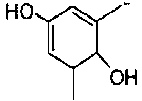 | 155 - CH₄ |
| 127 | C₇H₁₁O₂ | 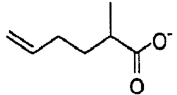 | 143 - CH₄ |
| 125 | C₈H₁₃O₂ | 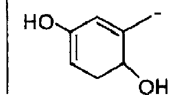 | 139 - CH₃ |
| 123 | C₇H₇O₂ | 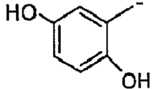 | 139 - CH₄ |
| 115 | C₆H₁₁O₂ | 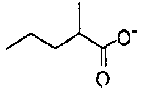 | 141 -C₃H₆ |
| 113 | C₆H₉O₂ | 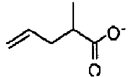 | 141- C₃H₄ |
| 111 | C₆H₇O₂ | 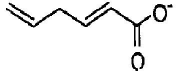 | 127 - CH₄ |
| 83 | C₄H₃O₂ | 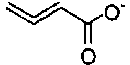 | 111 - C₂H₄ |

**Table 26: MS/MS fragmentation of multiple sclerosis biomarker 4, 540.4390 (C₃₂H₆₀O₆)**

| **m/z** | **Formula** | **Molecular fragment** | **Fragment loss** |
|---|---|---|---|
| 539 | C₃₂H₅₉O₆ | 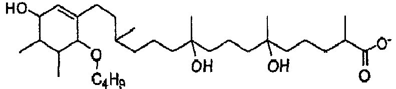 | - H⁺ |
| 521 | C₃₂H₅₇O₅ | 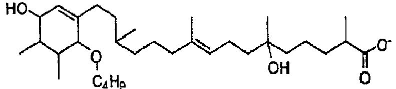 | - H₂O |
| 503 | C₃₂H₅₅O₄ | 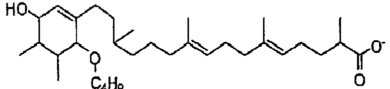 | -2xH₂O |
| 495 | C₃₁H₅₉O₄ | 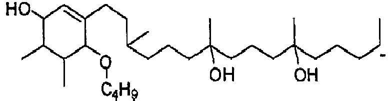 | - CO₂ |
| 477 | C₃₁H₅₇O₃ | 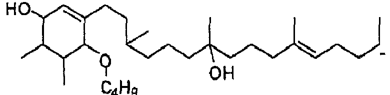 | - (CO₂ + H₂O) |
| 461 | C₃₀H₅₃O₃ | 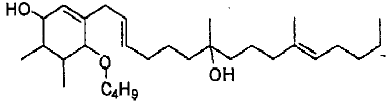 | 477 - CH₄ |
| 459 | C₃₁H₅₅O₂ | 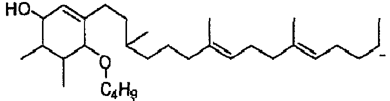 | - (CO₂ + 2xH₂O) |
| 419 | C₂₇H₄₇O₃ | 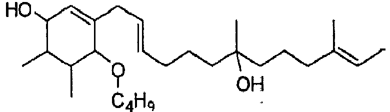 | 461 - C₃H₆ |
| 335 | C₂₂H₃₉O₂ | 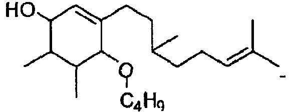 | 459 - C₉H₁₆ |
| 315 | C₁₈H₃₅O₄ | 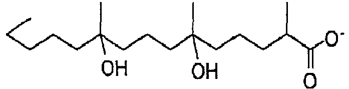 | 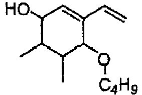 |
| 313 | C₁₈H₃₃O₄ | 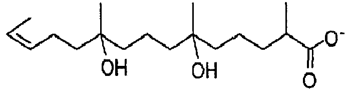 | 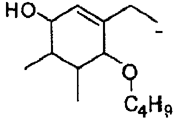 |
| 297 | C₁₈H₃₃O₃ | 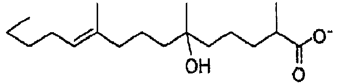 | 315-H₂O |
| 279 | C₁₈H₃₁O₂ | 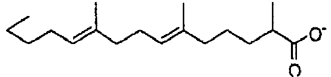 | 297 - H₂O |
| | | | 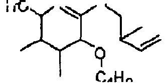 |
| 259 | C₁₄H₂₇O₄ | 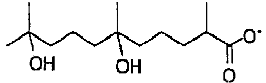 | - |
| 255 | C₁₅H₂₇O₃ | 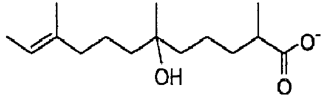 | 297 - C₃H₆ |
| 253 | C₁₆H₂₉O₂ | 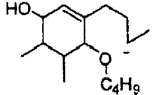 | 503 - phytol chain |
| 243 | C₁₄H₂₇O₃ | 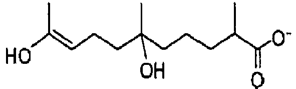 | 259 - CH₄ |
| 241 | C₁₅H₂₉O₂ | 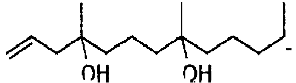 | 495 - 253 |
| 225 | C₁₄H₂₅O₂ | 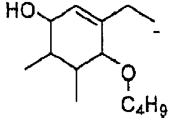 | - phytol chain |
| 223 | C₁₄H₂₃O₂ | 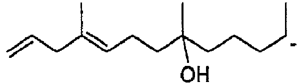 | 241 - H₂O |
| 213 | C₁₃H₂₅O₂ | 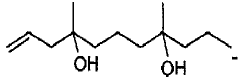 | 241- C₂H₄ |
| 179 | C₁₂H₁₉O | 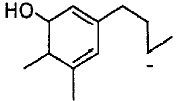 | 253-C₄H₉OH |
| 171 | C₁₀H₁₉O₂ | 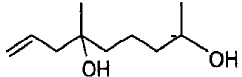 | 213 - C₃H₆ |
| 155 | C₉H₁₅O₂ | 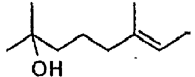 | 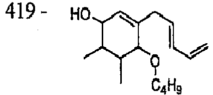 |
| 141 | C₈H₁₃O₂ | 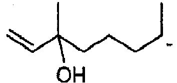 | 223 - C₆H₁₀ |
| 127 | C₈H₁₅O | 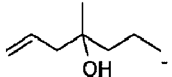 | 171 - C₂H₄O |

**Table 27: MS/MS fragmentation of multiple sclerosis biomarker 5, 576.4757 (C₃₆H₆₄O₅)**

| **m/z** | **Formula** | **Molecular fragment** | **Fragment loss** |
|---|---|---|---|
| 575 | C₃₆H₆₃O₅ | 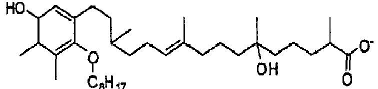 | -H⁺ |
| 557 | C₃₆H₆₁O₄ | 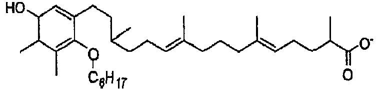 | -H₂O |
| 539 | C₃₆H₅₉O3 | 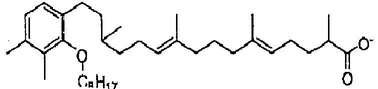 | -2xH₂O |
| 531 | C₃₅H₆₃O₃ | 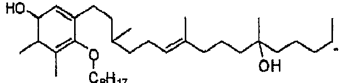 | - CO₂ |
| 513 | C₃₅H₆₁O₂ | 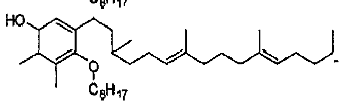 | 557 - CO₂ |
| 495 | C₃₅H₅₉O | 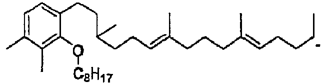 | 531 - CO₂ |
| 417 | C₂₈H₄₉O₂ | 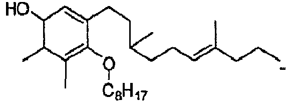 | 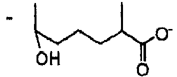 |
| 403 | C₂₈H₄₇O₂ | 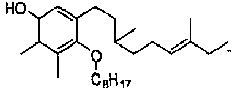 | 417 - CH₂ |
| 371 | C₂₆H₄₃O | 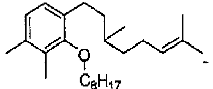 | 387 - CH₂ |
| 297 | C₁₈H₃₃O₃ | 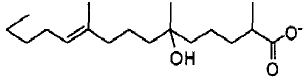 | 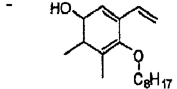 |
| 279 | C₁₈H₃₃O₂ | 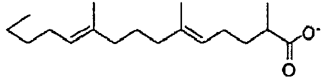 | 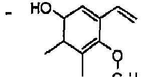 |
| 279 | C₁₈H₃₁O₂ | 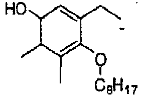 | - phytol chain |
| 251 | C₁₆H₂₇O₂ | 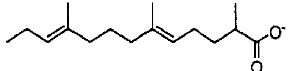 | 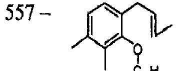 |
| 183 | C₁₁H₁₉O₂ | 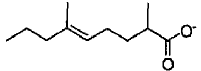 | 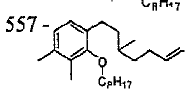 |

**Table 28: MS/MS fragmentation of multiple sclerosis biomarker 6, 578.4848 (C₃₆H₆₆O₅)**

| **m/z** | **Formula** | **Molecular fragment** | **Fragment loss** |
|---|---|---|---|
| 577 | C₃₆H₆₅O₅ | 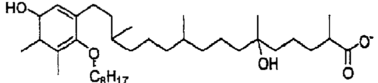 HO O C8H17 OH O- O | -H⁺ |
| 559 | C₃₆H₆₃O₄ | 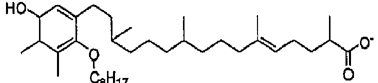 HO O C8H17 O- O | -H₂O |
| 541 | C₃₆H₆₁O₃ | 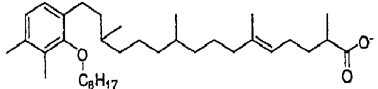 O⁻ O C8H17 O | -2xH₂O |
| 533 | C₃₅H₆₅O₃ | 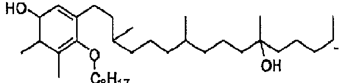 | - CO₂ |
| 515 | C₃₅H₆₃O₂ | 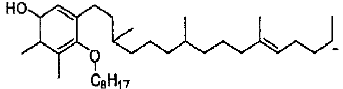 | 559 - CO₂ |
| 497 | C₃₅H₆₁O | 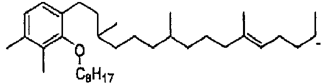 | 533 - CO₂ |
| 419 | C₂₈H₅₁O₂ | 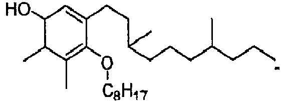 | 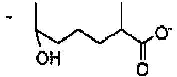 |
| 405 | C₂₈H₄₉O₂ | 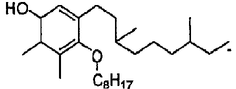 | 419 - CH₂ |
| 387 | C₂₇H₄₇O | 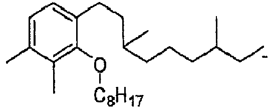 | 405 - H₂O |
| 373 | C₂₆H₄₅O | 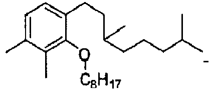 | 387 - CH₂ |
| 297 | C₁₈H₃₃O₃ | 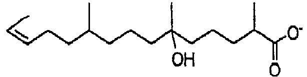 | 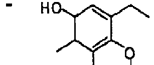 |
| 281 | C₁₈H₃₃O₂ | 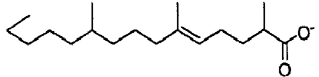 | |
| 279 | C₁₈H₃₁O₂ | 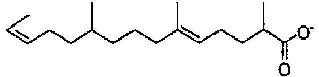 | 297 - H₂O |
| 279 | C₁₈H₃₁O₂ | 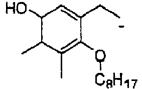 | - phytol chain |

**Table 29: MS/MS fragmentation of multiple sclerosis biomarker 7, 580.5089 (C₃₆H₆₈O₅)**

| **m/z** | **Formula** | **Molecular fragment** | **Fragment loss** |
|---|---|---|---|
| 579 | C₃₆H₆₇O₅ | 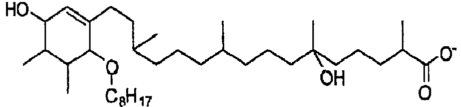 | -H⁺ |
| 561 | C₃₆H₆₅O₄ | 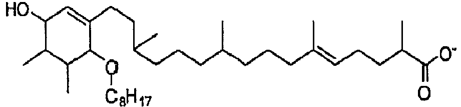 | -H₂O |
| 543 | C₃₅H₆₅O₃ | 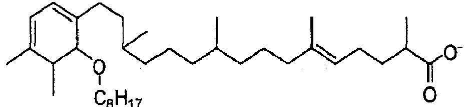 | -2xH₂O |
| 535 | C₃₅H₆₇O3 | 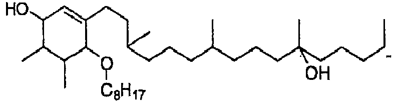 | -CO₂ |
| 517 | C₃₅H₆₅O₂ | 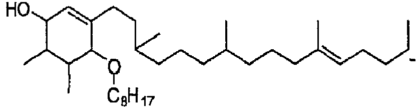 | 561 - CO₂ |
| 499 | C₃₅H₆₃O | 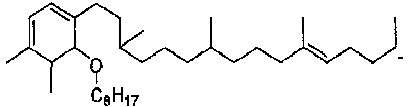 | 535 - CO₂ |
| 421 | C₂₈H₅₃O₂ | 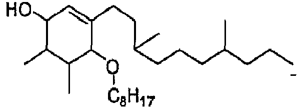 | 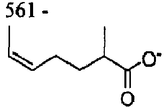 |
| 407 | C₂₇H₅₁O₂ | 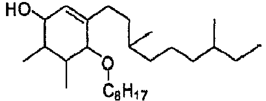 | 421 - CH₂ |
| 389 | C₂₇H₄₉O | 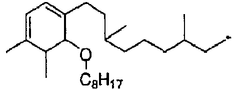 | 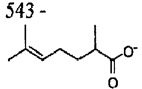 |
| 375 | C₂₆H₄₇O | 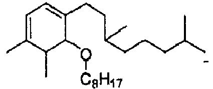 | 389 - CH₂ |
| 299 | C₁₈H₃₅O₃ | 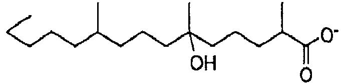 | 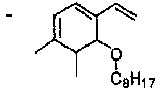 |
| 297 | C₁₈H₃₃O₃ | 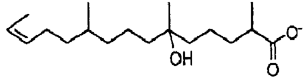 | 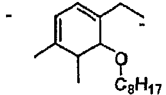 |
| 281 | C₁₈H₃₃O₂ | 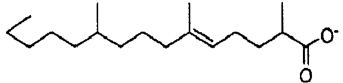 | 299 - H₂O |
| 281 | C₁₈H₃₃O₂ | 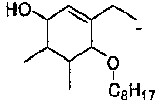 | - phytol chain |
| 279 | C₁₈H₃₁O₂ | 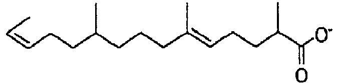 | 297 - H₂O |
| 263 | C₁₈H₃₁O | 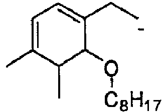 | 543 - phytol chain |
| 253 | C₁₇H₃₃O | 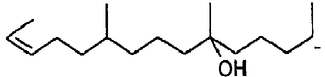 | 535 - 263 |
| 185 | C₁₀H₁₇O₃ | 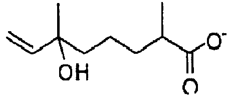 | 299 - C₈H₁₈ |
| 171 | C₉H₁₅O₃ | 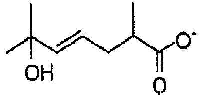 | |

**Table 30: MS/MS fragmentation of multiple sclerosis biomarker 8, 594.4848 (C₃₆H₆₆O₆)**

| **m/z** | **Formula** | **Molecular fragment** | **I Fragment loss** |
|---|---|---|---|
| 593 | C₃₆H₆₅O₆ | 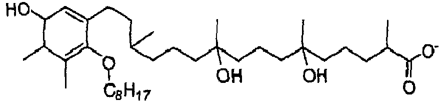 | -H⁺ |
| 575 | C₃₆H₆₅O₅ | 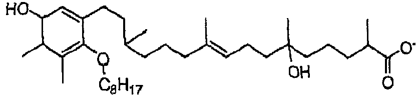 | -H₂O |
| 557 | C₃₆H₆₃O₄ | 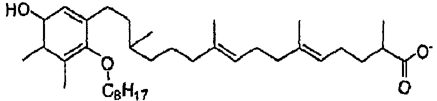 | -2xH₂O |
| 549 | C₃₅H₆₅O₄ | 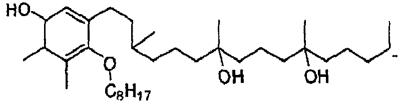 | - CO₂ |
| 531 | C₃₅H₆₃O₃ | 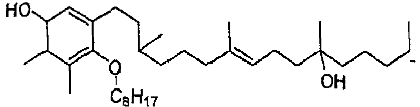 | 575 - CO₂ |
| 513 | C₃₅H₆₃O₂ | 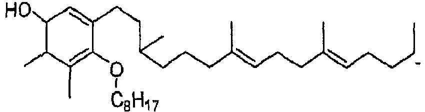 | 549 - CO₂ |
| 495 | C₃₅H₆₁O | 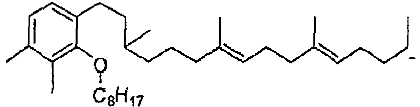 | 495 - H₂O |
| 421 | C₂₇H₄₉O₃ | 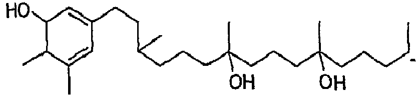 | 531 - C₈H₁₆O |
| 371 | C₂₆H₄₃O | 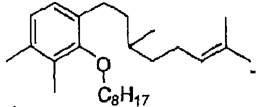 | 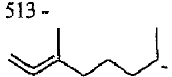 |
| 315 | C₁₈H₃₅O₄ | 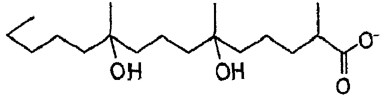 | 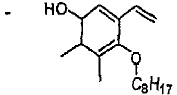 |
| 297 | C₁₈H₃₃O₃ | 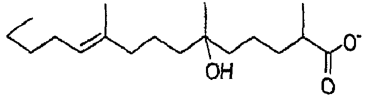 | 495 - H₂O |
| 279 | C₁₈H₃₁O₂ | 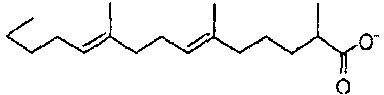 | 421 - H₂O |
| 279 | C₁₈H₃₁O₂ | 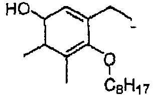 | - phytol chain |
| 201 | C₁₂H₂₅O₂ | 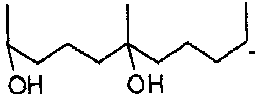 | 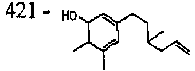 |
| 171 | C₉H₁₅O₃ | 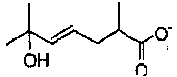 | |
| 141 | C₈H₁₃O₂ | 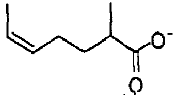 | |
| 127 | C₈H₁₅O | 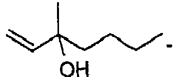 | |

**Table 31: MS/MS fragmentation of multiple sclerosis biomarker 9, 596.5012 (C₃₆H₆₈O₆)**

| **m/z** | **Formula** | **Molecular fragment** | **Fragment loss** |
|---|---|---|---|
| 595 | C₃₆H₆₇O₆ | 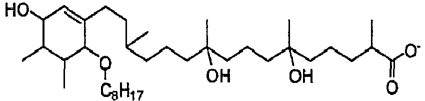 | -H⁺ |
| 577 | C₃₆H₆₅O₅ | 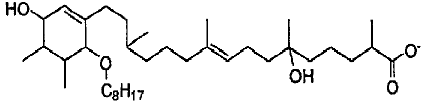 | -H₂O |
| 559 | C₃₆H₆₃O₄ | 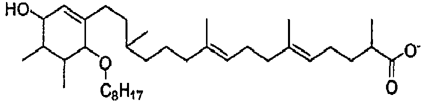 | -2xH₂O |
| 551 | C₃₅H₆₇O₂ | 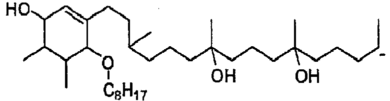 | - CO₂ |
| 515 | C₃₅H₆₃O₂ | 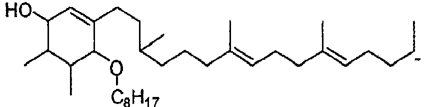 | 559 - CO₂ |
| 497 | C₃₅H₆₁O | 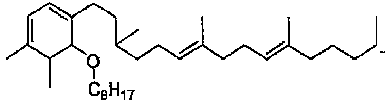 | 515 - H₂O |
| 423 | C₂₇H₅₁O₃ | 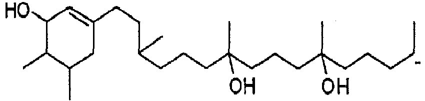 | 515 - C₈H₁₆O |
| 373 | C₂₆H₄₅O | 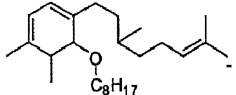 | 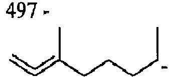 |
| 315 | C₁₈H₃₅O₄ | 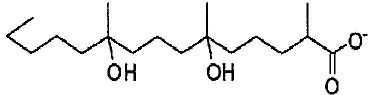 | 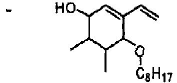 |
| 297 | C₁₈H₃₃O₃ | 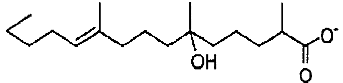 | 315 - H₂O |
| 281 | C₁₈H₃₂O₂ | 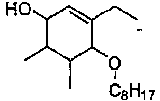 | - phytol chain |
| 279 | C₁₈H₃₁O₂ | 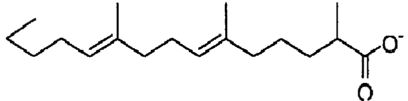 | 297 - H₂O |
| 269 | C₁₆H₂₉O₃ | 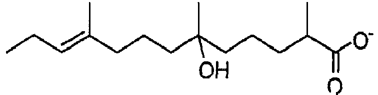 | 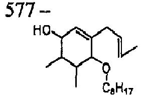 |
| 251 | C₁₆H₂₇O₂ | 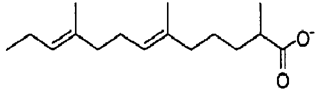 | 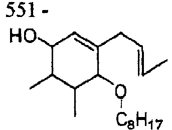 |
| 171 | C₉H₁₅O₃ | 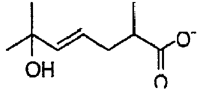 | |
| 155 | C₉H₁₅O₂ | 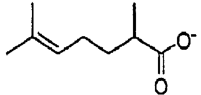 | |
| 153 | C₁₀H₁₇O | 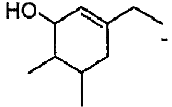 | |
| 141 | C₉H₁₇O | 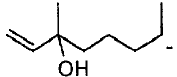 | |
| 139 | CgH₁₅0 | 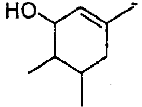 | |
| 127 | C₈H₁₅O | 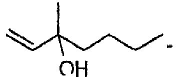 | |

**Table 32: MS/MS fragmentation of multiple sclerosis biomarker 10, 786.5408 (C₄₃H₇₉O₁₀P)**

| **m/z** | **Formula** | **Molecular fragment** | **Fragment loss** |
|---|---|---|---|
| 785 | C₄₃H₇₉O₁₀P | 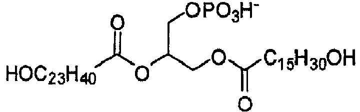 | -H⁺ |
| 529 | C₂₇H₄₆O₈P | 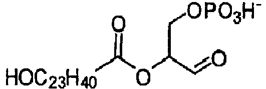 | 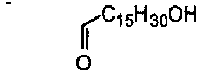 |
| 425 | C₁₉H₃₈O₈P | 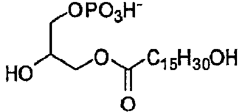 | 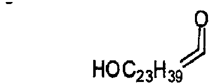 |
| 169 | C₃H₆O₆P | 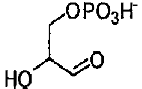 | 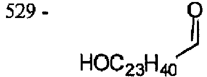 |
| 97 | H₂PO₄ | 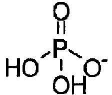 | |

**Table 33: MS/MS fragmentation of multiple sclerosis biomarker 11, 216.04 (C₅H₁₃O₇P)**

| **m/z** | **Formula** | **Molecular fragment** | **Fragment loss** |
|---|---|---|---|
| 215 | C₅H₁₂O₇P | 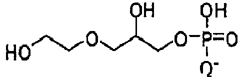 | - H⁺ |
| 197 | C₅H₁₀O₆P | 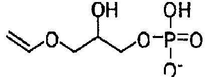 | - H₂O |
| 171 | C₃H₈O₆P | 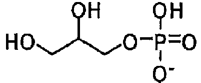 | 197 - C₂H₂ |
| 153 | C₃H₆O₅P | 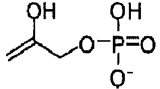 | 171 - H₂O |
| 135 | C₅H₁₁O₄ | 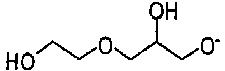 | 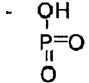 |

**Table 34: MS/MS fragmentation of multiple sclerosis biomarker 12, 541.3415 (C₂₅H₅₂NO₉P)**

| **m/z** | **Formula** | **Molecular fragment** | **Fragment loss** |
|---|---|---|---|
| 540 | C₂₅H₅₁NO₉P | 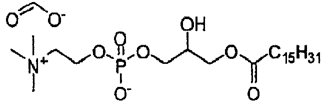 | -H⁺ |
| 480 | C₂₃H₄₇NO₇P | 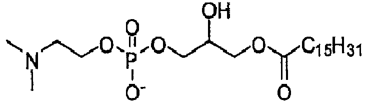 | 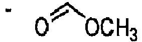 |
| 255 | C₁₆H₃₁O₂ | 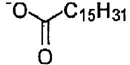 | 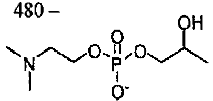 |
| 242 | C₇H₁₇NO₆P | 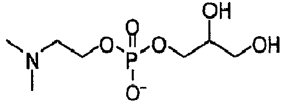 | 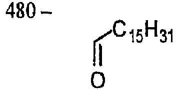 |
| 224 | C₇H₁₅NO₅P | 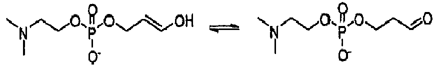 | 242 - H₂O |
| 168 | C₄H₁₁NO₄P | 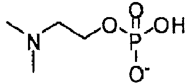 | 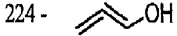 |
| 153 | C₃H₆O₅P | 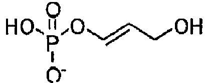 | 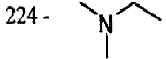 |
| 79 | PO₃ | 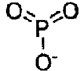 | |

**Table 35: MS/MS fragmentation of multiple sclerosis biomarker 13, 565.3391 (C₄₇H₈₃NO₁₃P)**

| **m/z** | **Formula** | **Molecular fragment** | **Fragment loss** |
|---|---|---|---|
| 564 | C₂₇H₅₁NO₉P | 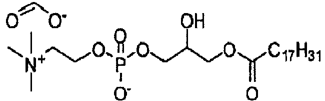 | -H+ |
| 504 | C₂₅H₄₅NO₈P | 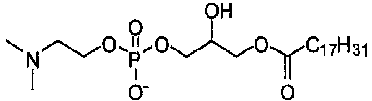 | 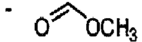 |
| 279 | C₁₈H₃₁O₂ | 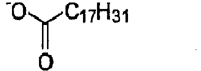 | 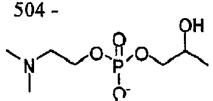 |
| 242 | C₇H₁₇NO₆P | 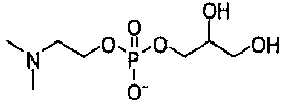 | 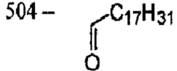 |
| 224 | C₇H₁₅NO₅P | 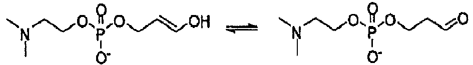 | 242 - H₂O |
| 168 | C₄H₁₁NO₄P | 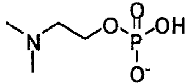 | 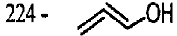 |
| 153 | C₃H₆O₅P | 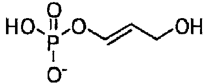 | 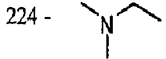 |
| 79 | PO₃ | 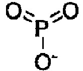 | |

**Table 36: MS/MS fragmentation of multiple sclerosis biomarker 14, 202.0453 (C₆H₁₁O₆Na)**

| **m/z** | **Formula** | **Molecular fragment** | **Fragment loss** |
|---|---|---|---|
| 203 | C₆H₁₂O₆Na | 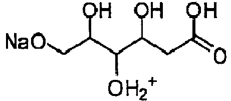 | + H⁺ |
| 159 | C₅H₁₂O₄Na | 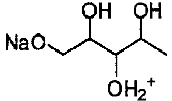 | - CO₂ |
| 115 | C₃H₈O₃Na | 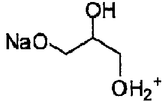 | 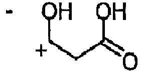 |
| 89 | C₃H₅O₃ | 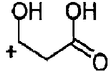 | 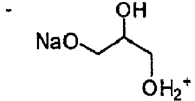 |
| 97 | C₃H₆O₂Na | 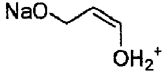 | 115 - H₂O |

**Table 37: MS/MS fragmentation of multiple sclerosis biomarker 15, 244.0559 (C₈H₁₃O₇Na)**

| **m/z** | **Formula** | **Molecular fragment** | **Fragment loss** |
|---|---|---|---|
| 245 | C₈H₁₄O₇Na | 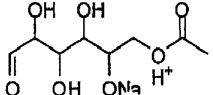 | + H⁺ |
| 227 | C₈H₁₂O₆Na | 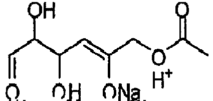 | - H₂O |
| 209 | C₈H₁₀O₅Na | 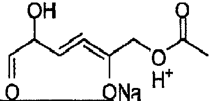 | 227 - H20 |
| 191 | C₈H₈O₄Na | 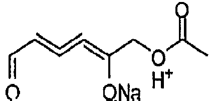 | 209 - H20 |
| 155 | C₅H₈O₄Na | 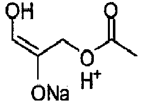 | 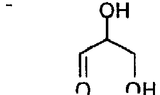 |
| 125 | C₄H₆O₃Na | 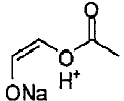 | 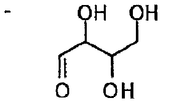 |
| 83 | C₂H₄O₂Na | 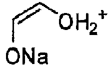 | 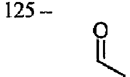 |

**Table 38: MS/MS fragmentation of multiple sclerosis biomarker 16, 428.3653 (C₂₉H₄₈O₂)**

| **m/z** | **Formula** | **Molecular fragment** | **Fragment loss** |
|---|---|---|---|
| 429 | C₂₉H₄₉O₂ | 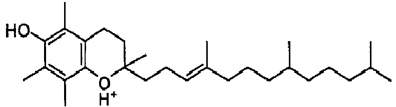 | + H⁺ |
| 205 | C₁₃H₁₇O₂ | 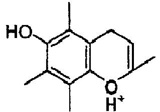 | 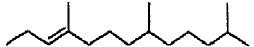 |
| 165 | C₁₀H₁₃O₂ | 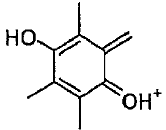 | |

**Table 39: MS/MS fragmentation of multiple sclerosis biomarker 17, 805.5609 (C₄₆H₈₀NO₈P)**

| **m/z** | **Formula** | **Molecular fragment** | **Fragment loss** |
|---|---|---|---|
| 806 | C₄₆H₈₁NO₈P | 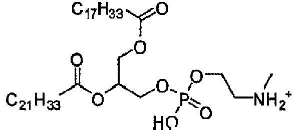 | +H⁺ |
| 478 | C₂₄H₄₉NO₆P | 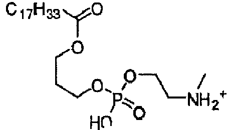 | 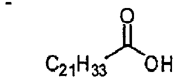 |
| 237 | C₁₇H₃₃ | 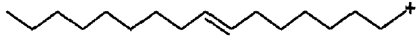 | |
| 184 | C₅H₁₅NO₄P | 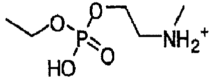 | |

**Table 40: MS/MS fragmentation of multiple sclerosis biomarker 18, 194.0803 (C₇H₁₄O₆)**

| **m/z** | **Formula** | **Molecular fragment** | **Fragment loss** |
|---|---|---|---|
| 195 | C₇H₁₅O₆ | 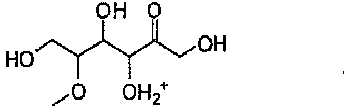 | +H⁺ |
| 177 | C₇H₁₃O₅ | 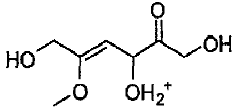 | -H₂O |
| 165 | C₆H₁₃O₅ | 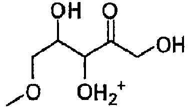 | -CH₂O |
| 163 | C₆H₁₁O₅ | 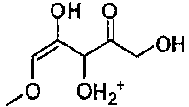 | - CH₃OH |
| 137 Observed 138 | C₅H₁₃O₄ | 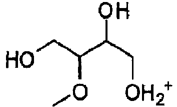 | 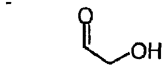 |
| 123 | C₄H₁₁O₄ | 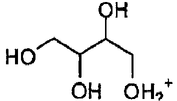 | 137-CH₂ |

**Table 41: MS/MS fragmentation of multiple sclerosis biomarker 19, 857.7516 (C₅₄H₉₉NO₆)**

| **m/z** | **Formula** | **Molecular fragment** | **Fragment loss** |
|---|---|---|---|
| 858 | C₅₄H₁₀₀NO₆ | 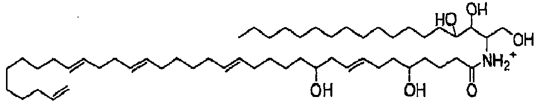 | + H⁺ |
| 602 | C₃₈H₆₈NO₄ | 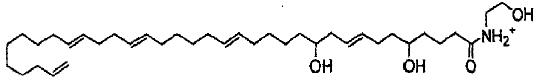 | - C₁₆H₃₄O₂ |
| 576 | C₃₆H₆₆NO₄ | 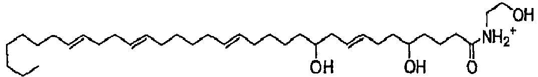 | 602 - C₂H₂ |
| 314 | C₁₇H₃₂NO₄ | 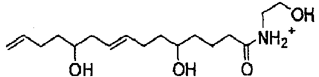 | 576-C₁₉H₃₄ |
| 165 | C₁₂H₂₁ | 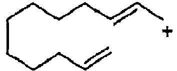 | |
| 151 | C₁₁H₁₉ | 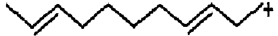 | |
| 95 | C₇H₁₁ | 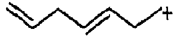 | |

### REFERENCES

1. MacDonald, B.K., et al. The incidence and lifetime prevalence of neurological disorders in a prospective community-based study in the UK. Brain, 2000. 123: p. 665-76.

2. Martyn, C. Epidemiology. McAlpine's Multiple Sclerosis. New York: Churchill Livingston, 1992.

3. Sayetta, R.B. Theories of the etiology of multiple sclerosis: a critical review. J Clin Lab Immunol, 1986. 21: p. 55-70.

4. Matthews, W.B. Clinical aspects. McAlpine's Multiple Sclerosis. New York: Churchill Livingston, 1992.

5. Poser, C.M. Trauma and multiple sclerosis: an hypothesis. J Neuro, 1987. 234: p. 155-9.

6. Chelmicka-Schorr, E., and Arnason, B.G. Nervous stem-immune system interactions and their role in multiple sclerosis. Ann Neurol, 1994. 36: p.29-s32.

7. Noseworthy, J.H., et al. Multiple sclerosis. N Engl J Med, 2000. 343: p.93 8-52.

8. ffrench-Constant, C. Pathogenesis of multiple sclerosis. Lancet, 1994. 343: p.271-5.

9. Prineas. J.W., and McDonald, W.I. Demyelinating diseases. Arnold: London, 1997.

10. Kidd, D., et al. Spinal cord MRI using multi-array coils and fast spin echo. II. Findings in multiple sclerosis. Neurology, 1993. 43: p. 2632-7.

11. Lucchinetti, C.W., et al. Heterogeneity of multiple sclerosis lesions: implication for the pathogenesis of demyelination. Ann Neurol, 2000. 47: p. 707-17.

12. Keegan. B.M., and Noseworthy, J.H. Multiple Sclerosis. Annu Rev Med, 2002. 52: 285-302.

13. McDonald, W.I, et al. Recommended diagnostic criteria for multiple sclerosis: guidelines from the international panel on the diagnosis of multiple sclerosis. Ann Neurol, 2001. 50: 121-7.

14. Reo, N.V., NMR-based metabolomics. Drug Chem Toxicol, 2002. 25(4): p. 375-82.

15. Fiehn, O., et al. Metabolite profiling for plant functional genomics. Nat Biotechnol, 2000. 18(11): p. 1157-61.

16. Hirai, M.Y., et al., Integration of transcriptomics and metabolomics for understanding of global responses to nutritional stresses in Arabidopsis thaliana. Proc Natl Acad Sci U S A, 2004.101(27): p. 10205-10.

17. Roessner, U., et al., Metabolic profiling allows comprehensive phenotyping of genetically or environmentally modified plant systems. Plant Cell, 2001.13(1): p. 11-29.

18. Castrillo, J.I., et al., An optimized protocol for metabolome analysis in yeast using direct infusion electrospray mass spectrometry. Phytochemistry, 2003. 62(6): p. 929-37.

19. Fiehn, O., Metabolomics--the link between genotypes and phenotypes. Plant Mol Biol, 2002. 48(1-2): p. 155-71.

20. Aharoni, A., et al., Nontargeted metabolome analysis by use of Fourier Transform Ion Cyclotron Mass Spectrometry. Omics, 2002. 6(3): p. 217-34.

21. Hirai, M.Y., et al., Elucidation ofgene-to-gene and metabolite-to-gene networks in arabidopsis by integration of metabolomics and transcriptomics. J Biol Chem, 2005. 280(27): p. 25590-5.

22. Murch, S.J., et al., A metabolomic analysis of medicinal diversity in Huang-qin (Scutellaria baicalensis Georgi) genotypes: discovery of novel compounds. Plant Cell Rep, 2004. 23(6): p. 419-25.

23. Tohge, T., et al., Functional genomics by integrated analysis of metabolome and transcriptome of Arabidopsis plants over-expressing an MYB transcription factor. Plant J, 2005. 42(2): p. 218-35.

24. Tibshirani, R., et al., Diagnosis of multiple cancer types by shrunken centroids ofgene expression. Proc Natl Acad Sci U S A, 2002. 99(10): p. 6567-72.

25. 15. Wu, B., et al., Comparison of statistical methods for classification of ovarian cancer using mass spectrometry data. Bioinformatics, 2003. 19(13): p. 1636-43.

26. Hager, J.W., et al., High-performance liquid chromatography-tandem mass spectrometry with a new quadrupole/linear ion trap instrument. J Chromatogr A, 2003. 1020(1): p. 3-9.

27. Hopfgartner, G., et al., Triple quadrupole linear ion trap mass spectrometer for the analysis of small molecules and macromolecules. J Mass Spectrom, 2004. 39(8): p. 845-55.

28. Xia, Y.Q., et al., Use of a quadrupole linear ion trap mass spectrometer in metabolite identification and bioanalysis. Rapid Commun Mass Spectrom, 2003.17(11): p. 1137-45.

29. Zhang, M.Y., et al., Hybrid triple quadrupole-linear ion trap mass spectrometry in fragmentation mechanism studies: application to structure elucidation of buspirone and one of its metabolites. J Mass Spectrom, 2005. 40(8): p. 1017-1029.
The present disclosure will now be further defined in the following numbered paragraphs:
1. A method of identifying one or more than one metabolite marker for diagnosing multiple sclerosis or another neurological disorder, comprising the steps of:
   a) introducing one or more than one sample from one or more than one patient with multiple sclerosis or another neurological disorder, said sample containing a plurality of metabolites into a high resolution mass spectrometer;
   b) obtaining quantifying data for the metabolites;
   c) creating a database of said quantifying data;
   d) comparing the quantifying data from the sample with corresponding data from a sample from one or more than one reference sample; and
   e) identifying one or more than one metabolite marker that differs between said sample and said one or more than one reference sample,
      wherein the one or more than one metabolite marker is selected from the metabolites listed in Table 1, 2, 3, 4, 5, 6 or any combination thereof.
2. The method of 1, further comprising selecting a minimal number of metabolite markers needed for optimal diagnosis.
3. The method of 1, wherein the high resolution mass spectrometer is a Fourier Transform Ion Cyclotron Resonance Mass Spectrometer (FTMS).
4. The method of 1, wherein the multiple sclerosis metabolite markers are selected from the group consisting of: relapsing-remitting as compared to a normal reference sample and the metabolites are listed in Table 1, primary-progressive as compared to a normal reference sample and the metabolites are listed in Table 2, secondary-progressive as compared to a normal reference sample and the metabolites are listed in Table 3, relapsing-remitting as compared to secondary-progressive and the metabolites are listed in Table 4, relapsing-remitting transiting to secondary-progressive as compared to relapsing-remitting and the metabolites are listed in Table 5, and relapsing-remitting transiting to secondary-progressive as compared to secondary-progressive and the metabolites are listed in Table 6.
5. A method for diagnosing multiple sclerosis or another neurological disorder or the risk of multiple sclerosis or another neurological disorder in a patient, the method comprising the steps of:
   a) obtaining a sample from said patient;
   b) analyzing said sample to obtain quantifying data for one or more than one metabolite marker;
   c) comparing the quantifying data for said one or more than one metabolite marker to corresponding data obtained from one or more than one reference sample; and
   d) using said comparison to diagnose multiple sclerosis or another neurological disorder or the risk of multiple sclerosis or another neurological disorder,
      wherein the one or more than one metabolite marker is selected from the metabolites listed in Table 1, 2, 3, 4, 5, 6 or any combination thereof.
6. The method of 5, wherein step b) comprises analyzing the sample by liquid chromatography mass spectrometry (LC-MS).
7. The method of 5, wherein the method is a high throughput method and step b) comprises analyzing the sample by direct injection or liquid chromatography and linear ion trap tandem mass spectrometry.
8. The method of 5, wherein said one or more than one reference sample is a plurality of samples obtained from control individuals; one or more than one baseline sample obtained from the patient at an earlier date; or a combination thereof.
9. The method of 5, wherein the multiple sclerosis metabolite markers are selected from the group consisting of: relapsing-remitting as compared to a normal reference sample and the metabolites are listed in Table 1, primary-progressive as compared to a normal reference sample and the metabolites are listed in Table 2, secondary-progressive as compared to a normal reference sample and the metabolites are listed in Table 3, relapsing-remitting as compared to secondary-progressive and the metabolites are listed in Table 4, relapsing-remitting transiting to secondary-progressive as compared to relapsing-remitting and the metabolites are listed in Table 5, and relapsing-remitting transiting to secondary-progressive as compared to secondary-progressive and the metabolites are listed in Table 6.
10. The method of 9, wherein the multiple sclerosis metabolite markers are relapsing-remitting as compared to a normal reference sample and the metabolite markers comprise metabolites with accurate masses in Daltons of, or substantially equivalent to, a) 496.4157, b) 524.4448. c) 540.4387, d) 580.5089, e) 594.4848, f) 596.5012 or g) 578.4923.
11. The method of 10 wherein the wherein the one or more than one metabolite is further characterized by molecular formula a) C₃₀H₅₆0₅, b) C₃₂H₆₀O₅, c) C₃₂H₆₀O₆, d) C₃₆H₆₈O₅, e) C₃₆H₆₆O₆, f) C₃₆H₆₈O₆, g) C₃₆H₆₆O₅.
12. The method of 11 wherein the one ore more than one metabolites is further characterized by the structure 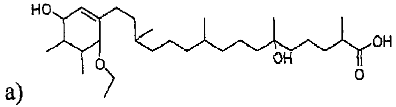 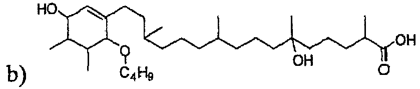 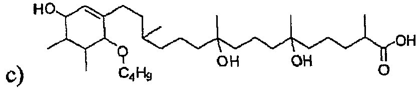 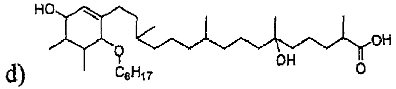 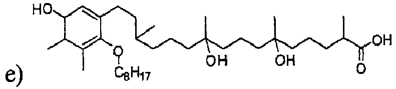 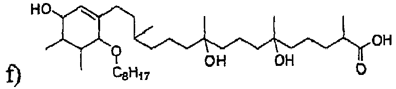 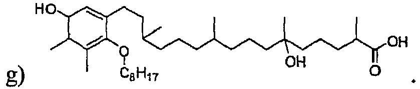
13. The method of 9, wherein the multiple sclerosis metabolite markers are primary-progressive as compared to a normal reference sample and the metabolite markers comprise metabolites with accurate masses in Daltons of, or substantially equivalent to a) 216.04, b) 202.0453, c) 244.0559, d) 857.7516.
14. The method of 13 wherein the wherein the one or more than one metabolite is further characterized by molecular formula a) C₅H₁₃O₇P, b) C₆H₁₁O₆Na, c) C₈H₁₃O₇Na, d) C₅₄H₉₉NO₆.
15. The method of 14 wherein the one ore more than one metabolites is further characterized by the structure 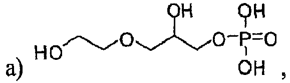 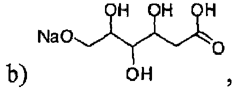 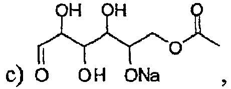 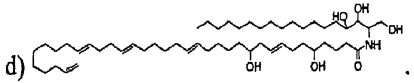
16. The method of 9, wherein the multiple sclerosis metabolite markers are secondary-progressive as compared to a normal reference sample and the metabolite markers comprise metabolites with accurate masses in Daltons of, or substantially equivalent to a) 541.3415, b) 565.3391, c) 428.3653, d) 805.5609, e) 194.0803, f) 578.423.
17. The method of 16 wherein the wherein the one or more than one metabolite is further characterized by molecular formula a) C₂₅H₅₂NO₉P, b) C₂₇H₅₂NO₉P, c) C₂₉H₄₈O₂, d) C₄₈H₈₀NO₈P, e) C₇H₁₄O₆, f) C₃₆H₆₆O₅.
18. The method of 17 wherein the one ore more than one metabolites is further characterized by the structure 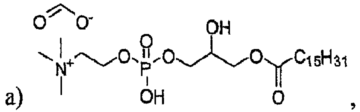 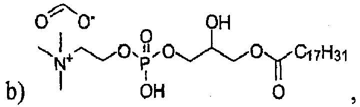 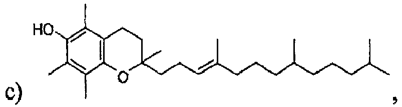 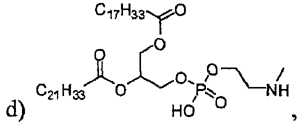 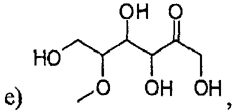 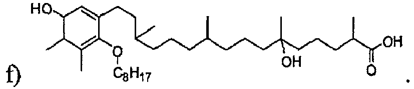
19. The method of . 9, wherein the multiple sclerosis metabolite markers are relapsing-remitting as compared to a secondary-progressive reference sample and the metabolite markers comprise metabolites with accurate masses in Daltons of, or substantially equivalent to a) 540.4387, b) 576.4757.
20. The method of 19 wherein the wherein the one or more than one metabolite is further characterized by molecular formula a) C₃₂H₆₀O₆, b) C₃₆H₆₄O₅.
21. The method of 20 wherein the one ore more than one metabolites is further characterized by the structure 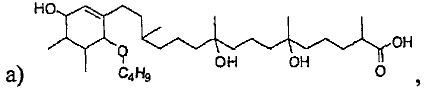 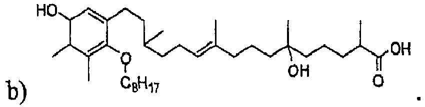
22. The method of 9, wherein the multiple sclerosis metabolite markers are relapsing-remitting transitioning to secondary-progressive as compared to a secondary-progressive reference sample and the metabolite markers comprise metabolites with accurate masses in Daltons of, or substantially equivalent to a) 786.5408.
23. The method of 22 wherein the wherein the one or more than one metabolite is further characterized by molecular formula a) C₄₃H₇₉O₁₀P.
24. The method of 23 wherein the one ore more than one metabolites is further characterized by the structure 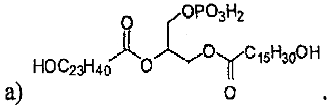
25. The method of 9, wherein the multiple sclerosis metabolite markers are relapsing-remitting transitioning to secondary-progressive as compared to a relapsing-remitting reference sample and the metabolite markers comprise metabolites with accurate masses in Daltons of, or substantially equivalent to a) 576.4757, b) 578.4923.
26. The method of 25 wherein the wherein the one or more than one metabolite is further characterized by molecular formula a) C₃₆H₆₄O₅, b) C₃₆H₆₆O₅.
27. The method of 26 wherein the one ore more than one metabolites is further characterized by the structure 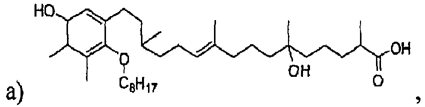 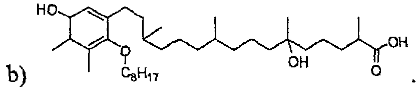
28. A compound selected from the group consisting of the metabolites with accurate masses measured in Daltons of, or substantially equivalent to, a) 452.3868, b) 496.4157, c) 524.4448, d) 540.4387, e) 576.4757, f) 578.4923, g) 580.5089, h) 594.4848, i) 596.5012, j) 786.5408 k) 216.04,1) 541.3415, m) 565.3391, n) 202.0453, o) 244.0559 p) 428.3653, q) 805.5609, r) 194.0803, s) 857.7516.
29. The compound of 28, further characterized by molecular formula a) C₂₈H₅₂O₄, b) C₃₀H₅₆O₅, c) C₃₂H₆₀O₅, d) C₃₂H₆₀O₆, e) C₃₆H₆₄O₅, f) C₃₆H₆₆O₅, g) C₃₆H₆₈O₅, h) C₃₆H₆₆O₆, i) C₃₆H₆₈O₆, j) C₄₃H₇₉O₁₀P, k) C₅H₁₃O₇P, 1) C₂₅H₅₂NO₉P, m) C₂₇H₅₂NO₉P n) C₆H₁₁O₆Na, o) C₈H₁₃O₇Na, p) C₂₉H₄₈O₂, q) C₄₆H₈₀NO₈P, r) C₇H₁₄O₆, s) C₅₄H₉₉NO₆ respectively.
30. The compound of 29, further characterized by the structure 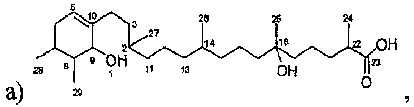 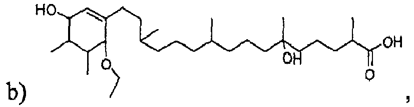 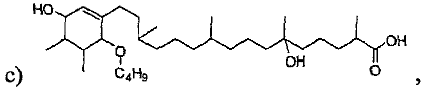 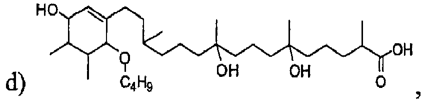 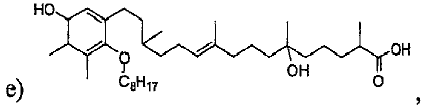 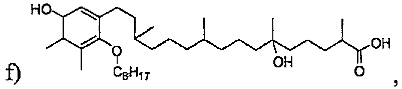 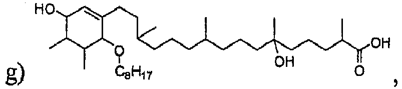 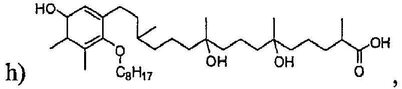 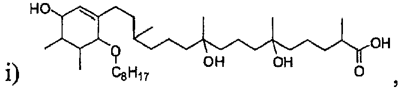 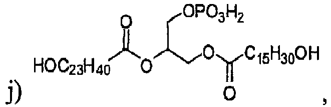 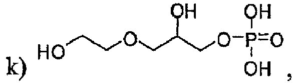 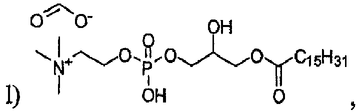 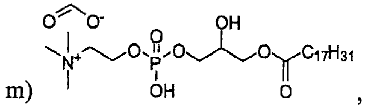 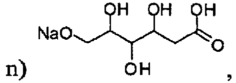 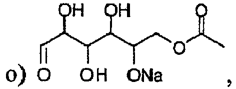 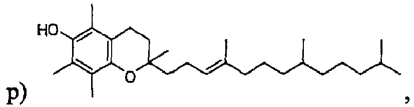 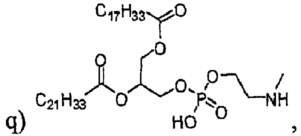 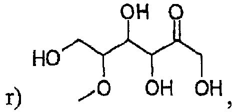 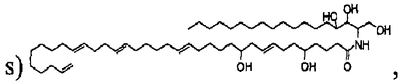 respectively.
31. A use of one or more than one compound of any one of 28-30 for the diagnosis of multiple sclerosis or another neurological disorder.

## Claims

1. A method for diagnosing a patient's multiple sclerosis health state, or change in multiple sclerosis health state, or for diagnosing another demyelinating disorder, or for diagnosing the risk of multiple sclerosis or another demyelinating disorder in a patient, the method comprising the steps of:
a) analyzing a sample obtained from said patient to obtain quantifying data for one or more than one metabolite marker;
b) comparing the quantifying data for said one or more than one metabolite marker to corresponding data obtained from one or more than one reference sample to identify an increase or decrease in the level of said one or more than one metabolite marker in said sample; and
c) using said increase or decrease in the level of said one or more than one metabolite marker in said sample to diagnose said patient's multiple sclerosis health state, or change in multiple sclerosis health state , or for diagnosing another demyelinating disorder, or for diagnosing the risk of multiple sclerosis or another demyelinating disorder,
wherein said one or more than one metabolite marker comprises one or more molecule selected from the molecular formulae consisting of C₂₈H₅₂O₄, C₃₀H₅₆O₅, C₃₂H₆₀O₅, C₃₂H₆₀O₆, C₃₆H₆₄O₅, C₃₆H₆₆O₅, C₃₆H₆₈O₅, C₃₆H₆₆O₆, C₃₆H₆₈O₆, C₄₃H₇₉O₁₀P, C₅H₁₃O₇P, C₂₅H₅₂NO₉P, C₂₇H₅₂NO₉P, C₆H₁₁O₆Na, C₈H₁₃O₇Na, C₂₉H₄₈O₂, C₄₆H₈₀NO₈P, C₇H₁₄O₆ and C₅₄H₉₉NO₆,
the metabolite having the molecular formula of C₂₈H₅₂O₄ being **characterized by** a collision induced dissociation (CID) MS/MS fragmentation pattern under negative ionization comprising the following daughter ions: 451, 433, 407, 389, 281, 279, 183, 169, 153, 139, 125, 111 and 97,
the metabolite having the molecular formula of C₃₀H₅₆O₅ being **characterized by** a CID MS/MS fragmentation pattern under negative ionization comprising the following daughter ions: 495, 477, 451, 433, 415, 307, 297, 279, 235, 223, 215, 197, 179, 181, 169, 157, 155, 153, 141, 139, 127, 125 and 113,
the metabolite having the molecular formula of C₃₂H₆₀O₅ being **characterized by** a CID MS/MS fragmentation pattern under negative ionization comprising the following daughter ions: 523, 505, 487, 479, 463, 461, 443, 365, 337, 299, 297, 281, 279, 271, 269, 253, 251, 243, 225, 197, 171, 169, 157, 155, 143, 141, 139, 127, 125, 123, 115, 113, 111 and 83,
the metabolite having the molecular formula of C₃₂H₆₀O₆ being **characterized by** a CID MS/MS fragmentation pattern under negative ionization comprising the following daughter ions: 539, 521, 503, 495, 477, 461, 459, 419, 335, 315, 313, 297, 279, 259, 255, 253, 243, 241, 225, 223, 213, 179, 171, 155, 141 and 127,
the metabolite having the molecular formula of C₃₆H₆₄O₅ being **characterized by** a CID MS/MS fragmentation pattern under negative ionization comprising the following daughter ions: 575, 557, 539, 531, 513, 495, 417, 403, 371, 297, 279, 279, 251 and 183,
the metabolite having the molecular formula of C₃₆H₆₆O₅ being **characterized by** a CID MS/MS fragmentation pattern under negative ionization comprising the following daughter ions: 577, 559, 541, 533, 515, 497, 419, 405, 387, 373, 297, 281, 279 and 279,
the metabolite having the molecular formula of C₃₆H₆₈O₅ being **characterized by** a CID MS/MS fragmentation pattern under negative ionization comprising the following daughter ions: 579, 561, 543, 535, 517, 499, 421, 407, 389, 375, 299, 297, 281, 281, 279, 263, 253, 185 and 171,
the metabolite having the molecular formula of C₃₆H₆₆O₆ being **characterized by** a CID MS/MS fragmentation pattern under negative ionization comprising the following daughter ions: 593, 575, 557, 549, 531, 513, 495, 421, 371, 315, 297, 279, 279, 201, 171, 141 and 127,
the metabolite having the molecular formula of C₃₆H₆₈O₆ being **characterized by** a CID MS/MS fragmentation pattern under negative ionization comprising the following daughter ions: 595, 577, 559, 551, 515, 497, 423, 373, 315, 297, 281, 279, 269, 251, 171, 155, 153, 141, 139 and 127,
the metabolite having the molecular formula of C₄₃H₇₉O₁₀P being **characterized by** a CID MS/MS fragmentation pattern under negative ionization comprising the following daughter ions: 785, 529, 425, 169 and 97,
the metabolite having the molecular formula of C₅H₁₃O₇P being **characterized by** a CID MS/MS fragmentation pattern under negative ionization comprising the following daughter ions: 215, 197, 171, 153 and 135,
the metabolite having the molecular formula of C₂₅H₅₂NO₉P being **characterized by** a CID MS/MS fragmentation pattern under negative ionization comprising the following daughter ions: 540, 480, 255, 242, 224, 168, 153 and 79,
the metabolite having the molecular formula of C₂₇H₅₂NO₉P being **characterized by** a CID MS/MS fragmentation pattern under negative ionization comprising the following daughter ions: 564, 504, 279, 242, 224, 168, 153 and 79,
the metabolite having the molecular formula of C₆H₁₁O₆Na being **characterized by** a CID MS/MS fragmentation pattern under positive ionization comprising the following daughter ions: 203, 159, 115, 89 and 97,
the metabolite having the molecular formula of C₈H₁₃O₇Na being **characterized by** a CID MS/MS fragmentation pattern under positive ionization comprising the following daughter ions: 245, 227, 209, 191, 155, 125 and 83,
the metabolite having the molecular formula of C₂₉H₄₈O₂ being **characterized by** a CID MS/MS fragmentation pattern under positive ionization comprising the following daughter ions: 429, 205 and 165,
the metabolite having the molecular formula of C₄₆H₈₀NO₈P being **characterized by** a CID MS/MS fragmentation pattern under positive ionization comprising the following daughter ions: 806, 478, 237 and 184,
the metabolite having the molecular formula of C₇H₁₄O₆ being **characterized by** a CID MS/MS fragmentation pattern under positive ionization comprising the following daughter ions: 195, 177, 165, 163, 138 and 123, and
the metabolite having the molecular formula of C₅₄H₉₉NO₆ being **characterized by** a CID MS/MS fragmentation pattern under positive ionization comprising the following daughter ions: 858, 602, 576, 314, 165, 151 and 95.

2. The method of claim 1, wherein the multiple sclerosis metabolite markers are relapsing-remitting as compared to a normal reference sample and are selected from the molecular formulae consisting of a) C₃₀H₅₆0₅, b) C₃₂H₆₀O₅, c) C₃₂H₆₀O₆, d) C₃₆H₆₈O₅, e) C₃₆H₆₆O₆, f) C₃₆H₆₈O₆, and g) C₃₆H₆₆O₅, including combinations thereof.

3. The method of claim 2, wherein the samples are analyzed by MS/MS analysis and one or more daughter ions are selected for:
a) C₃₀H₅₆O₅ selected from 495, 477, 451, 433, 415, 307, 297, 279, 235, 223, 215, 197, 179, 181, 169, 157, 155, 153, 141, 139, 127, 125 and 113;
b) C₃₂H₆₀O₅ selected from 523, 505, 487, 479, 463, 461, 443, 365, 337, 299, 297, 281, 279, 271, 269, 253, 251, 243, 225, 197, 171, 169, 157, 155, 143, 141, 139, 127, 125, 123, 115, 113, 111 and 83;
c) C₃₂H₆₀O₆ selected from 539, 521, 503, 495, 477, 461, 459, 419, 335, 315, 313, 297, 279, 259, 255, 253, 243, 241, 225, 223, 213, 179, 171, 155, 141 and 127;
d) C₃₆H₆₈O₅ selected from 579, 561, 543, 535, 517, 499, 421, 407, 389, 375, 299, 297, 281, 281, 279, 263, 253, 185 and 171;
e) C₃₆H₆₆O₆ selected from 593, 575, 557, 549, 531, 513, 495, 421, 371, 315, 297, 279, 279, 201, 171, 141 and 127;
f) C₃₆H₆₈O₆ selected from 595, 577, 559, 551, 515, 497, 423, 373, 315, 297, 281, 279, 269, 251, 171, 155, 153, 141, 139 and 127; or
g) C₃₆H₆₆O₅ selected from 577, 559, 541, 533, 515, 497, 419, 405, 387, 373, 297, 281, 279 and 279.

4. The method of claim 1, wherein the multiple sclerosis metabolite markers are primary-progressive as compared to a normal reference sample and are selected from the molecular formulae consisting of a) C₅H₁₃O₇P, b) C₆H₁₁O₆Na, c) C₈H₁₃O₇Na, and d) C₅₄H₉₉NO₆, including combinations thereof.

5. The method of claim 4, wherein the samples are analyzed by MS/MS analysis and one or more daughter ions are selected for:
a) C₅H₁₃O₇P selected from 215, 197, 171, 153 and 135;
b) C₆H₁₁O₆Na selected from 203, 159, 115, 89 and 97;
c) C₈H₁₃O₇Na selected from 245, 227, 209, 191, 155, 125 and 83; or
d) C₅₄H₉₉NO₆ selected from 858, 602, 576, 314, 165, 151 and 95.

6. The method of claim 4 or 5, wherein the one ore more than one metabolite is further **characterized by** the structure 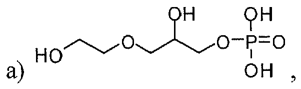 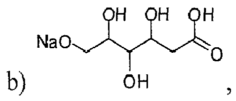 or

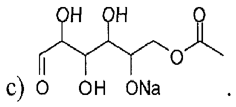

7. The method of claim 1, wherein the multiple sclerosis metabolite markers are secondary-progressive as compared to a normal reference sample and are selected from the molecular formulae consisting of a) C₂₅H₅₂NO₉P, b) C₂₇H₅₂NO₉P, c) C₂₉H₄₈O₂, d) C₄₈H₈₀NO₈P, e) C₇H₁₄O₆, f) C₃₆H₆₆O₅.

8. The method of claim 7, wherein the samples are analyzed by MS/MS analysis and one or more daughter ions are selected for:
a) C₂₅H₅₂NO₉P selected from 540, 480, 255, 242, 224, 168, 153 and 79;
b) C₂₇H₅₂NO₉P selected from 564, 504, 279, 242, 224, 168, 153 and 79;
c) C₂₉H₄₈O₂ selected from 429, 205 and 165;
d) C₄₆H₈₀NO₈P selected from 806, 478, 237 and 184;
e) C₇H₁₄O₆ selected from 195, 177, 165, 163, 138 and 123; or
f) C₃₆H₆₆O₅ selected from 577, 559, 541, 533, 515, 497, 419, 405, 387, 373, 297, 281, 279 and 279.

9. The method of claim 7 or 8, wherein the one ore more than one metabolite is further **characterized by** the structure: 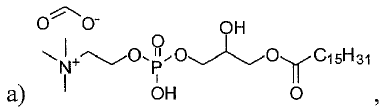 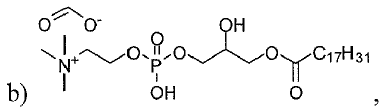 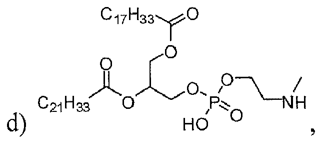 or

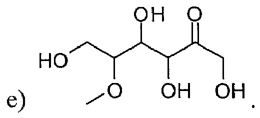

10. The method of claim 1, wherein the multiple sclerosis metabolite markers are relapsing-remitting as compared to a secondary-progressive reference sample and are selected from the molecular formulae consisting of a) C₃₂H₆₀O₆, and b) C₃₆H₆₄O₅, including combinations thereof.

11. The method of claim 2, wherein the samples are analyzed by MS/MS analysis and one or more daughter ions are selected for:
a) C₃₂H₆₀O₆ selected from 539, 521, 503, 495, 477, 461, 459, 419, 335, 315, 313, 297, 279, 259, 255, 253, 243, 241, 225, 223, 213, 179, 171, 155, 141 and 127; or
b) C₃₆H₆₄O₅ selected from 575, 557, 539, 531, 513, 495, 417, 403, 371, 297, 279, 279, 251 and 183.

12. The method of claim 1, wherein the multiple sclerosis metabolite markers are relapsing-remitting transitioning to secondary-progressive as compared to a secondary-progressive reference sample and comprise the molecular formula of a) C₄₃H₇₉O₁₀P.

13. The method of claim 12, wherein the samples are analyzed by MS/MS analysis and one or more daughter ions are selected for the metabolite having the molecular formula of a) C₄₃H₇₉O₁₀P selected from 785, 529, 425, 169 and 97.

14. The method of claim 12 or 13, wherein the metabolite is further **characterized by** the structure

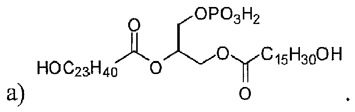

15. The method of claim 1, wherein the multiple sclerosis metabolite markers are relapsing-remitting transitioning to secondary-progressive as compared to a relapsing-remitting reference sample and are selected from the molecular formulae consisting of a) C₃₆H₆₄O₅, and b) C₃₆H₆₆O₅, including combinations thereof.

16. The method of claim 15, wherein the samples are analyzed by MS/MS analysis and one or more daughter ions are selected for:
a) C₃₆H₆₄O₅ selected from 575, 557, 539, 531, 513, 495, 417, 403, 371, 297, 279, 279, 251 and 183; or
b) C₃₆H₆₆O₅, selected from 577, 559, 541, 533, 515, 497, 419, 405, 387, 373, 297, 281, 279 and 279.

17. The method of claim 1, wherein the increase or decrease in the level of said one or more than one metabolite marker in said sample is identified in the comparing step (b) using a colorimetric chemical assay, an antibody-based enzyme-linked immunosorbant assay (ELISA), a dipstick chemical assay, or a mass spectrometry-based system.

18. The method of any one of claims 1 to 17, wherein the sample is whole blood, plasma, serum, or a subfraction of whole blood.

19. The method of any one of claims 1 to 18, wherein said one or more than one reference sample is a plurality of samples obtained from control individuals; one or more than one baseline sample obtained from the patient at an earlier date; or a combination thereof.

20. A compound selected from the following metabolites with accurate masses measured in Daltons at or ± 5 ppm of a) 452.3868, b) 496.4157, c) 524.4448, d) 540.4387, e) 576.4757, f) 578.4923, g) 580.5089, h) 594.4848, i) 596.5012, j) 786.5408 k) 216.04,1) 541.3415, m) 565.3391, n) 202.0453, o) 244.0559 p) 428.3653, and s) 857.7516, wherein said compounds are further characterized respectively by the molecular formulae of a) C₂₈H₅₂O₄, b) C₃₀H₅₆O₅, c) C₃₂H₆₀O₅, d) C₃₂H₆₀O₆, e) C₃₆H₆₄O₅, f) C₃₆H₆₆O₅, g) C₃₆H₆₈O₅, h) C₃₆H₆₆O₆, i) C₃₆H₆₈O₆, j) C₄₃H₇₉O₁₀P, k) C₅H₁₃O₇P, l) C₂₅H₅₂NO₉P, m) C₂₇H₅₂NO₉P n) C₆H₁₁O₆Na, o) C₈H₁₃O₇Na, p) C₂₉H₄₈O₂, and s) C₅₄H₉₉NO₆, and wherein
the metabolite having the molecular formula of C₂₈H₅₂O₄ is **characterized by** a collision induced dissociation (CID) MS/MS fragmentation pattern under negative ionization comprising the following daughter ions: 451, 433, 407, 389, 281, 279, 183, 169, 153, 139, 125, 111 and 97,
the metabolite having the molecular formula of C₃₀H₅₆O₅ is **characterized by** a CID MS/MS fragmentation pattern under negative ionization comprising the following daughter ions: 495, 477, 451, 433, 415, 307, 297, 279, 235, 223, 215, 197, 179, 181, 169, 157, 155, 153, 141, 139, 127, 125 and 113,
the metabolite having the molecular formula of C₃₂H₆₀O₅ is **characterized by** a CID MS/MS fragmentation pattern under negative ionization comprising the following daughter ions: 523, 505, 487, 479, 463, 461, 443, 365, 337, 299, 297, 281, 279, 271, 269, 253, 251, 243, 225, 197, 171, 169, 157, 155, 143, 141, 139, 127, 125, 123, 115, 113, 111 and 83,
the metabolite having the molecular formula of C₃₂H₆₀O₆ is **characterized by** a CID MS/MS fragmentation pattern under negative ionization comprising the following daughter ions: 539, 521, 503, 495, 477, 461, 459, 419, 335, 315, 313, 297, 279, 259, 255, 253, 243, 241, 225, 223, 213, 179, 171, 155, 141 and 127,
the metabolite having the molecular formula of C₃₆H₆₄O₅ is **characterized by** a CID MS/MS fragmentation pattern under negative ionization comprising the following daughter ions: 575, 557, 539, 531, 513, 495, 417, 403, 371, 297, 279, 279, 251 and 183,
the metabolite having the molecular formula of C₃₆H₆₆O₅ is **characterized by** a CID MS/MS fragmentation pattern under negative ionization comprising the following daughter ions: 577, 559, 541, 533, 515, 497, 419, 405, 387, 373, 297, 281, 279 and 279,
the metabolite having the molecular formula of C₃₆H₆₈O₅ is **characterized by** a CID MS/MS fragmentation pattern under negative ionization comprising the following daughter ions: 579, 561, 543, 535, 517, 499, 421, 407, 389, 375, 299, 297, 281, 281, 279, 263, 253, 185 and 171,
the metabolite having the molecular formula of C₃₆H₆₆O₆ is **characterized by** a CID MS/MS fragmentation pattern under negative ionization comprising the following daughter ions: 593, 575, 557, 549, 531, 513, 495, 421, 371, 315, 297, 279, 279, 201, 171, 141 and 127,
the metabolite having the molecular formula of C₃₆H₆₈O₆ is **characterized by** a CID MS/MS fragmentation pattern under negative ionization comprising the following daughter ions: 595, 577, 559, 551, 515, 497, 423, 373, 315, 297, 281, 279, 269, 251, 171, 155, 153, 141, 139 and 127,
the metabolite having the molecular formula of C₄₃H₇₉O₁₀P is **characterized by** a CID MS/MS fragmentation pattern under negative ionization comprising the following daughter ions: 785, 529, 425, 169 and 97,
the metabolite having the molecular formula of C₅H₁₃O₇P is **characterized by** a CID MS/MS fragmentation pattern under negative ionization comprising the following daughter ions: 215, 197, 171, 153 and 135,
the metabolite having the molecular formula of C₂₅H₅₂NO₉P is **characterized by** a CID MS/MS fragmentation pattern under negative ionization comprising the following daughter ions: 540, 480, 255, 242, 224, 168, 153 and 79,
the metabolite having the molecular formula of C₂₇H₅₂NO₉P is **characterized by** a CID MS/MS fragmentation pattern under negative ionization comprising the following daughter ions: 564, 504, 279, 242, 224, 168, 153 and 79,
the metabolite having the molecular formula of C₆H₁₁O₆Na is **characterized by** a CID MS/MS fragmentation pattern under positive ionization comprising the following daughter ions: 203, 159, 115, 89 and 97,
the metabolite having the molecular formula of C₈H₁₃O₇Na is **characterized by** a CID MS/MS fragmentation pattern under positive ionization comprising the following daughter ions: 245, 227, 209, 191, 155, 125 and 83,
the metabolite having the molecular formula of C₂₉H₄₈O₂ is **characterized by** a CID MS/MS fragmentation pattern under positive ionization comprising the following daughter ions: 429, 205 and 165, and
the metabolite having the molecular formula of C₅₄H₉₉NO₆ is **characterized by** a CID MS/MS fragmentation pattern under positive ionization comprising the following daughter ions: 858, 602, 576, 314, 165, 151 and 95.

21. The compound of claim 20, further characterized respectively by the structures:

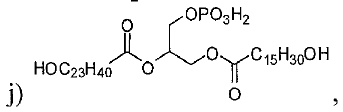

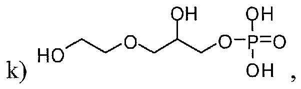

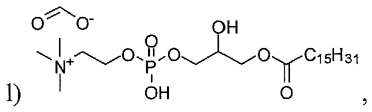

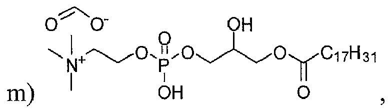

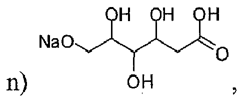

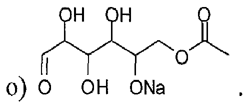

22. A use of one or more than one compound of claim 20 or 21 for the diagnosis of multiple sclerosis or another neurological disorder.
